# EUROPEAN PATENT SPECIFICATION

(11) **EP 2 970 316 B1**
(45) Date of publication and mention of the grant of the patent: **08.11.2017**
(21) Application number: 14726452.7
(22) Date of filing: 14.03.2014
(51) Int. Cl.: C07D 473/08, C07D 487/14, C07D 487/04

(54) **SUBSTITUTED XANTHINES AND METHODS OF USE THEREOF**
SUBSTITUIERTE XANTHINE UND VERFAHREN ZUR VERWENDUNG DAVON
XANTHINES SUBSTITUÉES ET LEURS PROCÉDÉS D'UTILISATION

(30) Priority: 14.03.2013 US 201361784448 P
(43) Date of publication of application: 20.01.2016
(73) Proprietor: Hydra Biosciences, Inc., Cambridge, MA 2138 (US)
(72) Inventor: CHENARD, Bertrand, Waterford, CT 06385 (US); KIMBALL, Spencer, David, East Windsor, NJ 08520 (US); GALLASCHUN, Randall, Lebanon, CT 06249 (US)
(74) Representative: Hoffmann Eitle
(86) International application number: PCT/US2014/027166
(87) International publication number: WO 2014/152287

(56) References cited:
- SEGURA-CABRERA A ET AL: "Structure-based prediction of Mycobacterium tuberculosis shikimate kinase inhibitors by high-throughput virtual screening", BIOORGANIC & MEDICINAL CHEMISTRY LETTERS, PERGAMON, AMSTERDAM, NL, vol. 18, no. 11, 1 June 2008 (2008-06-01), pages 3152-3157, XP022711187, ISSN: 0960-894X, DOI: 10.1016/J.BMCL.2008.05.003 [retrieved on 2008-05-04]
- EVSEYEVA. L. V. ET AL: "Synthesis and study of antioxidant activity of 8-R-thio-7-p-chlorobenzyltheophyllines", VISNIK FARMATSII, vol. 3, 2009, pages 3-6, XP009179920,
- KIM ET AL: "Molecular determinant of sensing extracellular pH in classical transient receptor potential channel 5", BIOCHEMICAL AND BIOPHYSICAL RESEARCH COMMUNICATIONS, ACADEMIC PRESS INC. ORLANDO, FL, US, vol. 365, no. 2, 5 November 2007 (2007-11-05), pages 239-245, XP022356980, ISSN: 0006-291X, DOI: 10.1016/J.BBRC.2007.10.154 cited in the application
- YOSHIDA J ET AL: "Capacitative Ca<2+> entries and mRNA expression for TRPC1 and TRPC5 channels in human epidermoid carcinoma A431 cells", EUROPEAN JOURNAL OF PHARMACOLOGY, ELSEVIER SCIENCE, NL, vol. 510, no. 3, 14 March 2005 (2005-03-14), pages 217-222, XP027695255, ISSN: 0014-2999 [retrieved on 2005-03-14] cited in the application

## Description

### CLAIM OF PRIORITY

This application claims priority to U.S.S.N. 61/784,448, filed March 14, 2013.

### BACKGROUND

A variety of ion channel proteins exist to mediate ion flux across cellular membranes. The proper expression and function of ion channel proteins is essential for the maintenance of cell function, intracellular communication, and the like. Numerous diseases are the result of misregulation of membrane potential or aberrant calcium handling. Given the central importance of ion channels in modulating membrane potential and ion flux in cells, identification of agents that can promote or inhibit particular ion channels are of great interest as research tools and as possible therapeutic agents.

### SUMMARY OF THE INVENTION

The present invention provides compounds of Formula I: 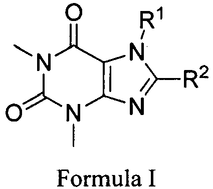 or a pharmaceutically acceptable salt thereof, wherein constituent members are provided herein.

The present invention further provides compounds of Formula II: 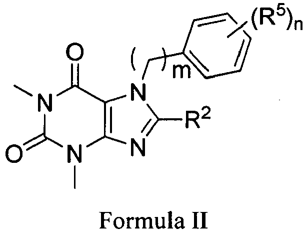 or a pharmaceutically acceptable salt thereof, wherein constituent members are provided herein.

The present invention further provides compositions comprising a compound of Formula I or II, and a pharmaceutically acceptable carrier.

The present invention further provides a compound of Formula I or Formula II, or a pharmaceutically acceptable salt thereof for use in methods of treating a TRPC5 mediated disorder in a subject, e.g. a human subject.

The present invention provides compositions for use in methods for treating conditions such as a neuropsychiatric disorder, a neurodegenerative disorder, nephropathy, and seizure disorder by modulating the activity of the transient receptor potential cation channel subfamily C, member 5 (TRPC5), which can exist in homomultimeric form as well as heteromultimeric form with other ion channels such as TRPC1 or TRPC3 (i.e., TRPC5-TRPC1 and TRPC1-TRPC3-TRPC5). The compound of Formula (I) modulate the function of TRPC5 by inhibiting a TRPC5-mediated ion flux or by inhibiting the inward current, the outward current, or both currents mediated by TRPC5. The inhibition of a particular current is the ability to inhibit or reduce such current (e.g., inward and/or outward) in an in vitro or an in vivo assay. The activation of a particular current is the ability to activate or increase such current (e.g., inward and/or outward) in an in vitro or an in vivo assay.

In one aspect, the invention relates to a TRPC5 antagonist, such as a compound of Formula I or Formula II, that inhibits TRPC5-mediated current and/or TRPC5-mediated ion flux for use in a method for treating a condition for which reduced TRPC5 activity can reduce the severity of the condition.

Structurally similar purine derivatives outside Formula I and Formula II having a different mechanism of action are described in Segura-Cabrera et al., Bioorganic & Medicinal Chemistry Letters 18 (11), 2008, pages 3152-3157, and Evseyeva et al., Visnik Farmatsii 3, 2009, pages 3-6.

Described in greater detail herein are compounds of Formula I and Formula II, which are TRPC5 antagonists that have a measured IC₅₀ for inhibition of TRPC5 of 10 nanomolar or less. In certain embodiments, the compounds of Formula I and Formula II, which are TRPC5 antagonists inhibit one or both of inward and outward TRPC5-mediated currents with an IC₅₀ 10 nanomolar or less. In certain embodiments, the compounds of Formula I and Formula II inhibit at least 95% of TRPC5-mediated current or TRPC5-mediated ion flux when administered at 1 micromolar or less.

In another aspect, compounds of Formula I or Formula II, which are TRPC5 antagonists can be used to inhibit a function of TRPC5, for example a TRPC5-mediated current and/or a TRPC5-mediated ion flux. In some embodiments, compounds of Formula I or Formula II can be used to inhibit a TRPC5 mediated current in vitro, for example in cells in culture. In other embodiments, compounds of Formula I or Formula II can be used to inhibit a TRPC5 mediated current in vivo. In certain embodiments, compounds of Formula I or Formula II inhibit both an inward and an outward TRPC5-mediated current.

Another aspect of the invention features a pharmaceutical preparation suitable for use in a human patient, or for veterinary use, comprising an effective amount of a compound of formula (I) (or a salt thereof, or a solvate, hydrate, oxidative metabolite or prodrug of the compound or its salt), and one or more pharmaceutically acceptable excipients. The invention further contemplates the use of compounds of Formula I or Formula II in methods to treat or reduce the symptoms of any of the diseases or conditions provided in the specification. The compounds of Formula I or Formula II for use in a method of treating a particular disease or condition can be formulated for administration via a route appropriate for the particular disease or condition.

Compounds of Formula I or Formula II can be administered alone or in combination with another therapeutic agent. For instance, the compounds of Formula I or Formula II can be administered conjointly with one or more of an anti-inflammatory agent, anti-acne agent, anti-wrinkle agent, anti-scarring agent, anti-psoriatic agent, anti-proliferative agent, anti-fungal agent, anti-viral agent, anti-septic agent, anti-migraine agent, keratolytic agent, or a hair growth inhibitor.

Compounds of Formula I or Formula II can be administered topically, orally, transdermally, rectally, vaginally, parentally, intranasally, intrapulmonary, intraocularly, intravenously, intramuscularly, intraarterially, intrathecally, intracapsularly, intraorbitally, intracardiacly, intradermally, intraperitoneally, transtracheally, subcutaneously, subcuticularly, intraarticularly, subcapsularly, subarachnoidly, intraspinally, intrasternally, sublingually, or by inhalation.

In some embodiments, compounds of Formula I or Formula II can be administered topically.

In some embodiments, compounds of Formula I or Formula II can be administered orally.

In some embodiments, compounds of Formula I or Formula II can be administered administered parentally.

### Detailed Description of the Invention

Cation channels such as TRPC5 modulate the flux of calcium and sodium ions across cellular membranes. Sodium and calcium influx leads to a depolarization of the cell. This increases the probability that voltage-gated ion channels will reach the threshold required for activation. As a result, activation of non-selective cation channels can increase electrical excitability and increase the frequency of voltage-dependent events. Voltage-dependent events include, but are not limited to, neuronal action potentials, cardiac action potentials, smooth muscle contraction, cardiac muscle contraction, and skeletal muscle contraction.

Calcium influx caused by the activation of non-selective cation channels such as TRPC5 also alters the intracellular free calcium concentration. Calcium is a ubiquitous second messenger molecule within the cell and the alterations in intracellular calcium levels have profound effects on signal transduction and gene expression. Thus, activation of non-selective cation channels such as TRPC5 can lead to changes in gene expression and cellular phenotype. Gene expression events include, but are not limited to, production of mRNAs encoding cell surface receptors, ion channels, and kinases. These changes in gene expression can lead to hyperexcitability in that cell.

Transient receptor potential (TRP) homomeric TRPC5 ion channels are signal transduction gated, Ca²⁺-permeable channels predominantly expressed in the neurons. TRPC5 forms homomultimeric structures such as tetramers (i.e., TRPC5 homomultimers) and heteromultimeric structures such as tetramers (i.e., TRPC5-TRPC1 heteromultimers). Unless expressly stated otherwise, when the term TRPC5 is used herein, for example, when identifying a modulator of TRPC5 such as a TRPC5 antagonist, the term TRPC5 is used generically so as to include either or both of a TRPC5 homomultimer or a heteromultimer (e.g., TRPC5 -TPRC1 or TRPC5-TRPC4 heteromultimer). Examples of TRPC5 in the literature include the following: Nature. 2008 Jan. 3; 451 (7174):69-72; Mol Pharmacol. 2008 January; 73 (1):42-9; J Biol Chem. 2007 Nov. 16; 282 (46):33868-78; Biochem Biophys Res Commun. 2008 Jan. 11; 365 (2):239-45; J Biol Chem. 2006 Nov. 3; 281 (44):33487-96; Eur J Pharmacol. 2005 Mar. 14; 510 (3):217-22; J Biol Chem. 2006 Feb. 24; 281 (8):4977-82; Biochem Soc Trans. 2007 February; 35 (Pt 1):101-4; Handb Exp Pharmacol. 2007; (179):109-23; J Biol Chem. 2005 Mar. 25; 280 (12):10997-1006; J Physiol. 2006 Jan. 15; 570 (Pt 2):219-35; and Nat Neurosci. (2003) 6: 837-45.

Modulating the function of TRPC5 proteins provides a means of modulating calcium homeostasis, sodium homeostasis, membrane polarization, and/or intracellular calcium levels, and compounds that can modulate TRPC5 function are useful in many aspects, including, but not limited to, maintaining calcium homeostasis, modulating intracellular calcium levels, modulating membrane polarization, and treating or preventing diseases, disorders, or conditions associated with calcium and/or sodium homeostasis or dyshomeostasis.

In one aspect, the present invention provides compounds of Formula I: 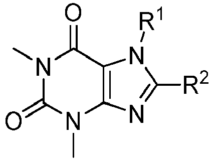 or a pharmaceutically acceptable salt thereof, wherein:
R¹ is 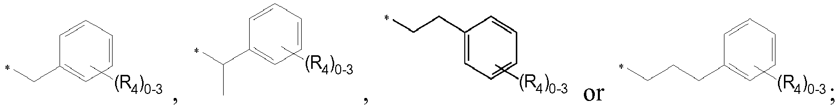
R² is C₁-C₆ alkyl, C₂-C₆ alkenyl, C₂-C₆ alkynyl, C₁-C₆ haloalkyl, hydroxyl, C₁-C₆ alkoxy, C₁-C₆ heteroalkyl, C₆-C₁₀ aryloxy, C₇-C₁₆ arylalkoxy, C₁-C₆ akylamino, C₂-C₁₂ dialkylamino, - C(O)-, -S(O)-, -O-, heterocycloalkyl, heteroaryl, sulfonamidyl, amido, urea, sulfonylurea, acyl, nitro, cyano, wherein each of C₁-C₆ alkyl, C₂-C₆ alkenyl, C₂-C₆ alkynyl, C₁-C₆ haloalkyl, C₁-C₆ alkoxy, C₁-C₆ heteroalkyl, C₆-C₁₀ aryloxy, C₇-C₁₆ arylalkoxy, C₁-C₆ akylamino, C₂-C₁₂ dialkylamino, -S(O)-, heterocycloalkyl, heteroaryl, sulfonamidyl, amido, urea, sulfonylurea, acyl, is optionally substituted with 1-3 R³;
each R³ is independently C₁-C₆ alkyl, C₁-C₆ heteroalkyl, halo, C₁-C₆ haloalkoxy, hydroxyl, C₁-C₆ alkoxy, amino, C₁-C₆ alkylamino, C₂-C₁₂ dialkylamino, cyano, nitro, amido, -S-, -S(O)₂-, -C(O)-, -C(O)O-C₁-C₆ alkyl, -C(O)-C₁-C₆ alkyl, C₃-C₇ cycloalkyl, heterocycloalkyl, phenyl, or naphthyl, wherein each of C₁-C₆ alkyl, C₁-C₆ heteroalkyl, C₁-C₆ haloalkoxy, C₁-C₆ alkoxy, amino, C₁-C₆ alkylamino, C₂-C₁₂ dialkylamino, amido, -S-, -S(O)₂-, -C(O)-, -C(O)O-C₁-C₆ alkyl, -C(O)-C₁-C₆ alkyl, C₃-C₇ cycloalkyl, heterocycloalkyl, phenyl, or naphthyl is optionally substituted with 1-3 R⁴; and
each R⁴ is independently C₁-C₆ alkyl, C₁-C₆ heteroalkyl, C₂-C₆ alkenyl, C₂-C₆ alkynyl, C₃-C₇ cycloalkyl, C₁-C₆ haloalkoxy, C₁-C₆ haloalkyl, heterocycloalkyl, C₆-C₁₀ aryl, heteroaryl, C₄-C₁₀ cycloalkylalkyl, heterocycloalkylalkyl, C₇-C₁₆ arylalkyl, heteroaryl-C₁-C₆ alkyl, halo, hydroxyl, C₁-C₆ alkoxy, C₁-C₆ hydroxyalkyl, C₆-C₁₀ aryloxy, C₇-C₁₆ arylalkoxy, C₂-C₈ alkoxyalkoxyl, amino, C₁-C₆ akylamino, C₂-C₁₂ dialkylamino, -S-, -S-C₁-C₆ alkyl, -S(O)₂-C₁-C₆ alkyl, sulfonamidyl, amido, urea, sulfonylurea, acyl, -C(O)-C₆-C₁₀ aryl, -NHC(O)-C₆-C₁₀ aryl, - C(O)NH-C₆-C₁₀ aryl, -C(O)OH, -C(O)O-C₁-C₆ alkyl, -C(O)-C₁-C₆ alkyl, acyl, nitro, or cyano.

In some embodiments, R⁴ is halo.

In some embodiments, R⁴ is bromo or chloro.

In some embodiments, R⁴ is C₁-C₆ alkyl and chloro.

In some embodiments, R⁴ is chloro and fluoro.

In some embodiments, R³ is phenyl or naphthyl.

In some embodiments, R⁴ is bromo.

In some embodiments, R³ is bromophenyl.

In some embodiments, R³ is 3-bromophenyl.

In some embodiments, R³ is chlorophenyl.

In some embodiments, R³ is 4-chlorophenyl.

In some embodiments, R² is -S-C₁-C₆ alkyl.

In some embodiments, R² is C₆-C₁₀ aryloxy.

In some embodiments, R² is C₇-C₁₆ arylalkoxy.

In some embodiments, the compounds have Formula II: 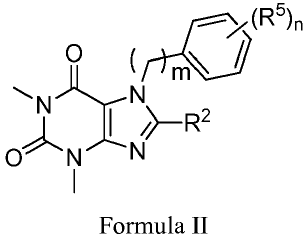 or a pharmaceutically acceptable salt thereof, wherein:
R² is C₁-C₆ alkoxy, C₁-C₆ heteroalkyl, C₆-C₁₀ aryloxy, C₇-C₁₆ arylalkoxy, or -C(O)-C₁-C₆ alkyl, each independently substituted with 1-3 R³;
each R³ is independently halo, C₁-C₆ alkyl, C₁-C₆ haloalkyl, C₁-C₆ haloalkoxy, C₁-C₆ heteroalkyl, hydroxyl, -C(O)O-C₁-C₆ alkyl, -C(O)-C₁-C₆ alkyl, phenyl, or heteroaryl, wherein each of C₁-C₆ alkyl, C₁-C₆ haloalkyl, C₁-C₆ haloalkoxy, C₁-C₆ heteroalkyl, -C(O)O-C₁-C₆ alkyl, -C(O)-C₁-C₆ alkyl, phenyl, or heteroaryl is optionally substituted with 1-3 R⁴;
R⁴ and R⁵ are each independently hydroxyl, C₁-C₆ alkyl, C₁-C₆ alkoxy, C₁-C₆ heteroalkyl, C₁-C₆ haloalkyl, C₁-C₆ haloalkoxy or halo;
n is 1 or 2; and
m is 1, 2, or 3.

In some embodiments, R² is C₁-C₆ alkoxy.

In some embodiments, R² is C₆-C₁₀ aryloxy substituted by 1-3 R³, wherein each R³ is independently selected from the group consisting of: C₁-C₆ haloalkoxy, halo, or -C(O)O-C₁-C₆ alkyl.

In some embodiments, R² is -S-C₁-C₆ alkyl substituted with -C(O)-C₁-C₆ alkyl, hydroxyl, or heteroaryl.

In certain embodiments, exemplary compounds of Formula I and Formula II include the compounds described in Table 1 and in the Examples. A compound described herein may be tested for its ability to block both inward and outward currents through the TRPC5 channel, e.g., by an assay as described in Example 1. For example, IC₅₀ of a compound of Formula I or Formula II was estimated by testing the compound at 5 µM and 500 nM. When 5 µM of a compound showed no block, IC₅₀ was estimated as >10 µM. When 5 µM of a compound showed 50% or less block, a rough estimate of IC₅₀ in the range of 5-10 µM could be made. IC₅₀ for a compound of Formula I or Formula II between 500 nM and 5 µM was similarly estimated. Exemplary compounds are shown in Table 1 below. As shown, "A" refers to an IC₅₀ < 100 nM. "B" refers to an IC₅₀ between 100 nM and 500 nM. "C" refers to an IC₅₀ between 500 nM and 1000 nM. "D" refers to an IC₅₀ between 1 µM and 2 µM. "E" refers to an IC₅₀ between 2 µM and 10 µM. "F" refers to agonist compounds. "ND" refers to compounds wherein the IC₅₀ was not determined.

**Table 1**

| **Compound Number** | **Structure** | **hTRPC5 Patch La3+ Inwd (nM) (All Batches)** | **hTRPC4 Patch Inwd (nM) (All Batches)** |
|---|---|---|---|
| 1 | 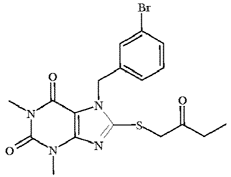 | C | ND |
| 2 | 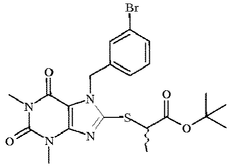 | F | ND |
| 3 | 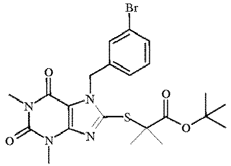 | B | ND |
| 4 | 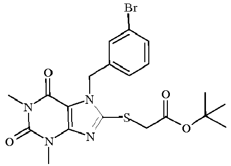 | F | ND |
| 5 | 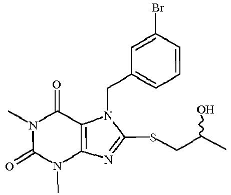 | E | ND |
| 6 | 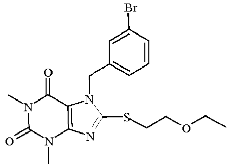 | B | ND |
| 7 | 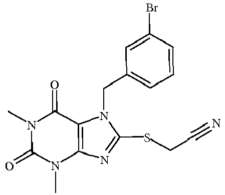 | E | ND |
| 8 | 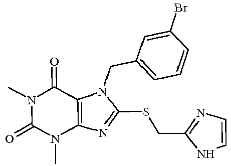 | E | ND |
| 9 | 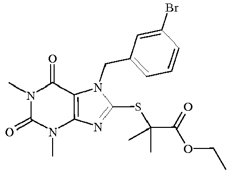 | A | ND |
| 10 | 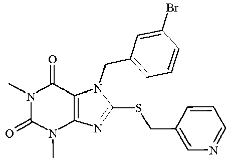 | E | ND |
| 11 | 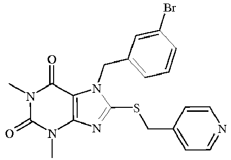 | E | ND |
| 12 | 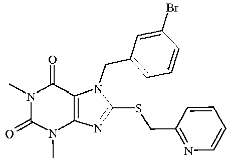 | C | ND |
| 13 | 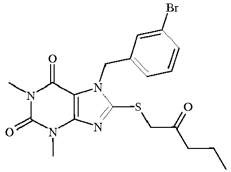 | A | ND |
| 14 | 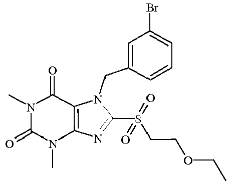 | D | ND |
| 15 | 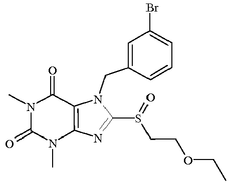 | E | ND |
| 16 | 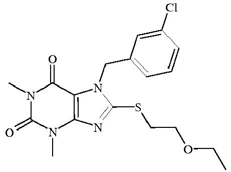 | E | ND |
| 17 | 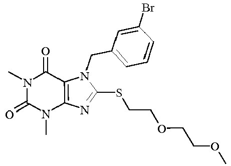 | B | ND |
| 18 | 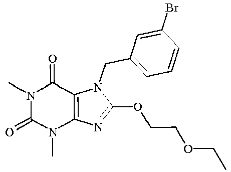 | D | ND |
| 19 | 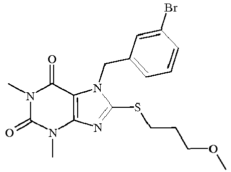 | B | ND |
| 20 | 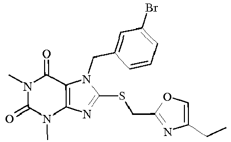 | B | ND |
| 21 | 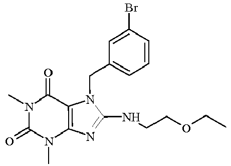 | E | ND |
| 22 | 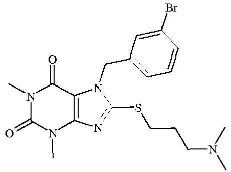 | E | ND |
| 23 | 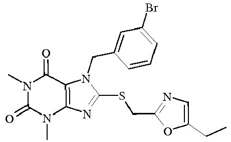 | B | ND |
| 24 | 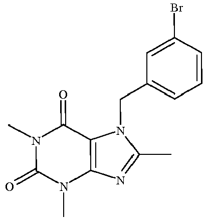 | E | ND |
| 25 | 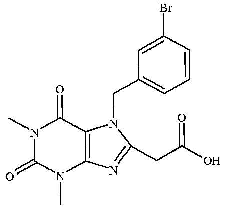 | E | ND |
| 26 | 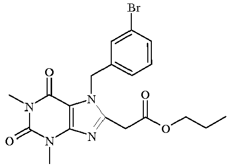 | E | ND |
| 27 | 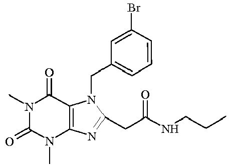 | E | ND |
| 28 | 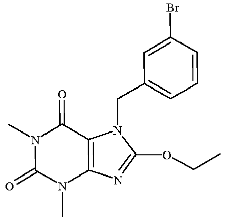 | E | ND |
| 29 | 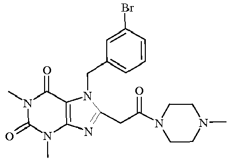 | E | ND |
| 30 | 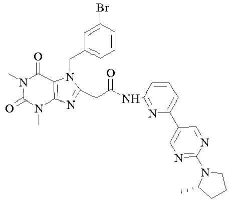 | E | ND |
| 31 | 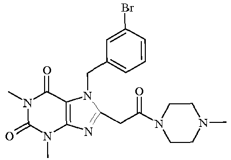 | E | ND |
| 32 | 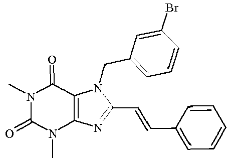 | E | ND |
| 33 | 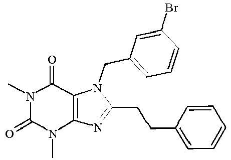 | C | ND |
| 34 | 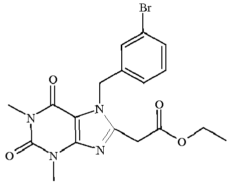 | E | ND |
| 35 | 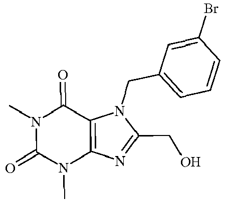 | E | ND |
| 36 | 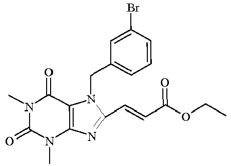 | E | ND |
| 37 | 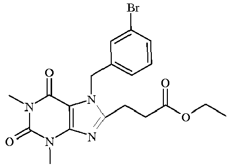 | E | ND |
| 38 | 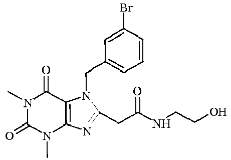 | E | ND |
| 39 | 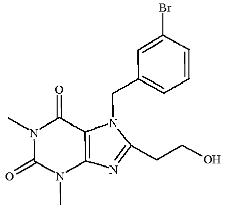 | E | ND |
| 40 | 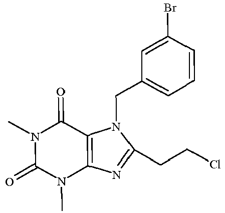 | E | ND |
| 41 | 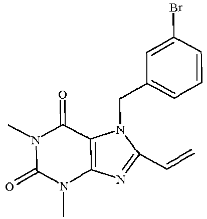 | E | ND |
| 42 | 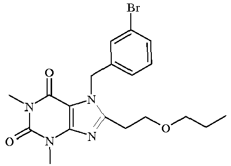 | D | ND |
| 43 | 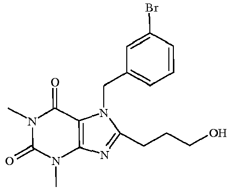 | E | ND |
| 44 | 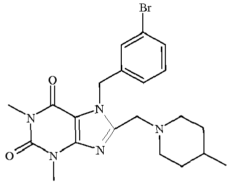 | E | ND |
| 45 | 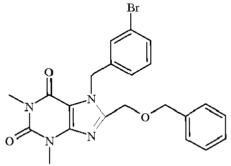 | E | ND |
| 46 | 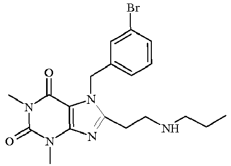 | E | ND |
| 47 | 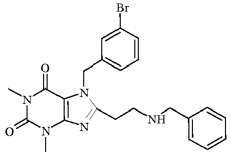 | E | ND |
| 48 | 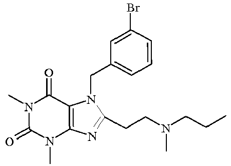 | E | ND |
| 49 | 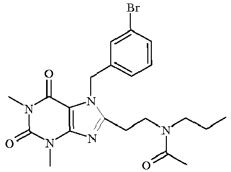 | E | ND |
| 50 | 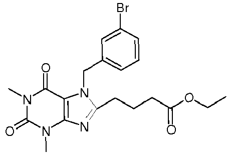 | D | ND |
| 51 | 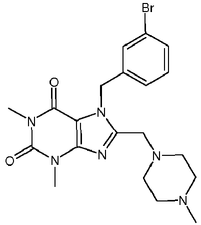 | E | ND |
| 52 | 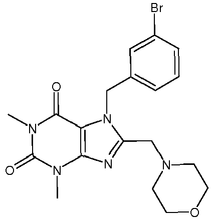 | E | ND |
| 53 | 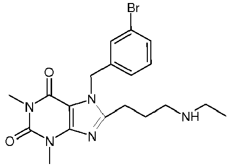 | E | ND |
| 54 | 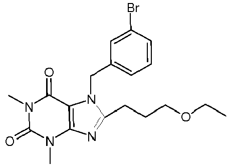 | E | ND |
| 55 | 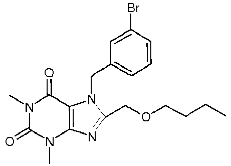 | C | ND |
| 56 | 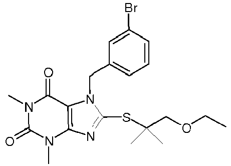 | D | ND |
| 57 | 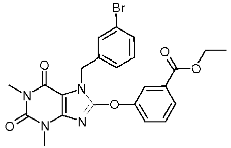 | A | ND |
| 58 | 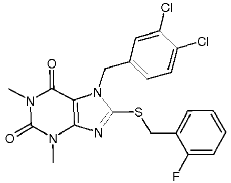 | B | ND |
| 59 | 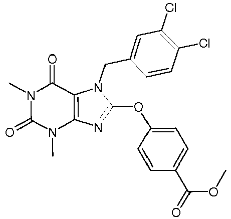 | ND | ND |
| 60 | 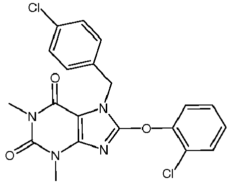 | ND | ND |
| 61 | 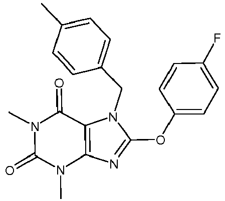 | ND | ND |
| 62 | 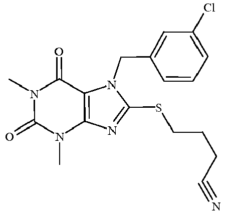 | D | ND |
| 63 | 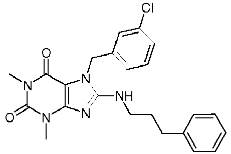 | ND | ND |
| 64 | 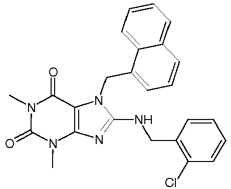 | ND | ND |
| 65 | 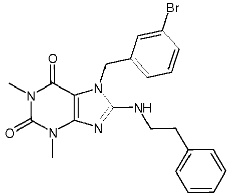 | ND | ND |
| 66 | 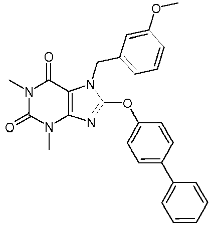 | ND | ND |
| 67 | 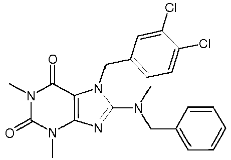 | E | ND |
| 68 | 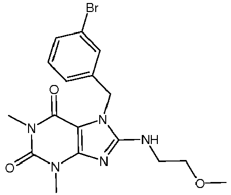 | ND | ND |
| 69 | 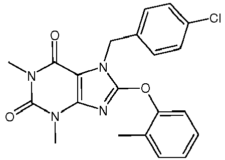 | ND | ND |
| 70 | 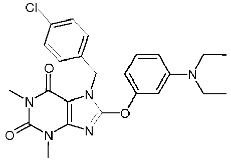 | F | ND |
| 71 | 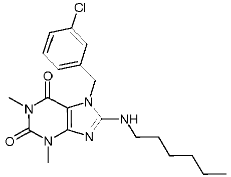 | ND | ND |
| 72 | 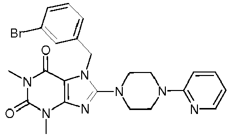 | ND | ND |
| 73 | 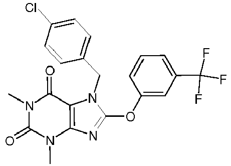 | B | ND |
| 74 | 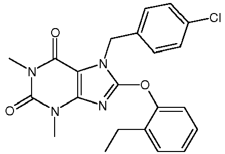 | B | ND |
| 75 | 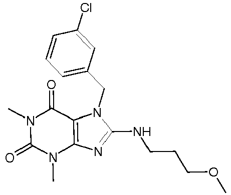 | ND | ND |
| 76 | 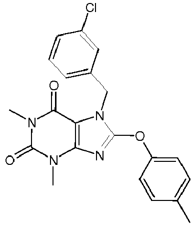 | E | ND |
| 77 | 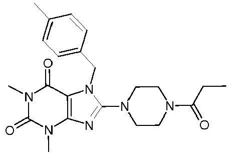 | ND | ND |
| 78 | 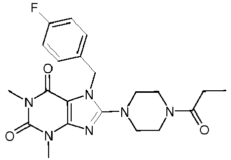 | ND | ND |
| 79 | 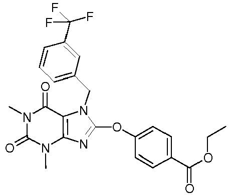 | ND | ND |
| 80 | 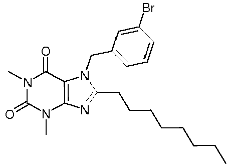 | ND | ND |
| 81 | 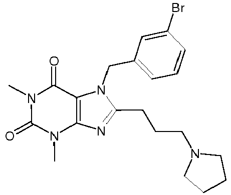 | E | ND |
| 82 | 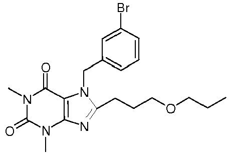 | D | ND |
| 83 | 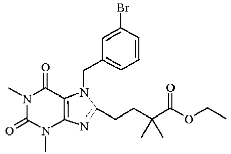 | E | ND |
| 84 | 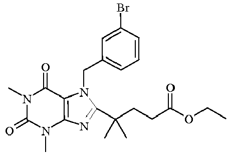 | D | ND |
| 85 | 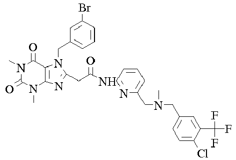 | E | ND |
| 86 | 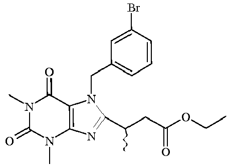 | B | ND |
| 87 | 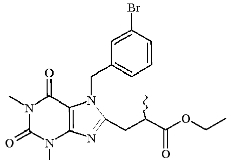 | C | ND |
| 88 | 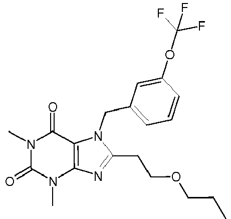 | C | ND |
| 89 | 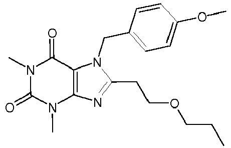 | E | ND |
| 90 | 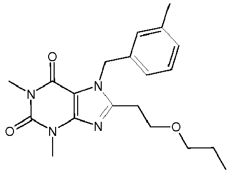 | D | ND |
| 91 | 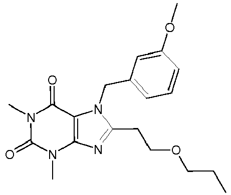 | E | ND |
| 92 | 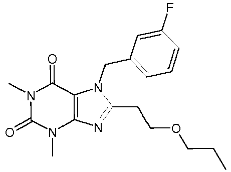 | E | ND |
| 93 | 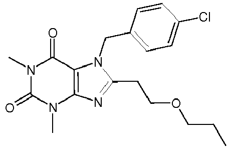 | C | ND |
| 94 | 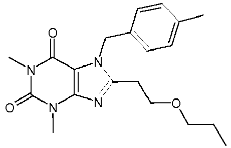 | E | ND |
| 95 | 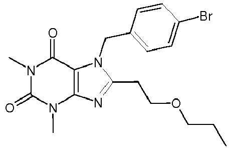 | D | ND |
| 96 | 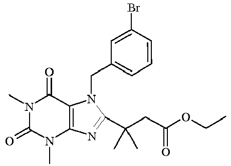 | C | ND |
| 97 | 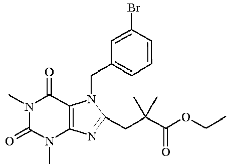 | B | ND |
| 98 | 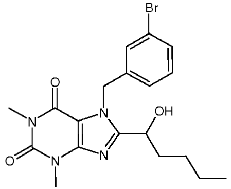 | E | ND |
| 99 | 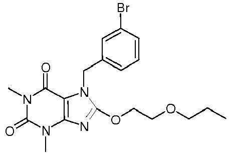 | B | ND |
| 100 | 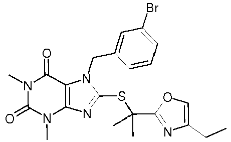 | B | ND |
| 101 | 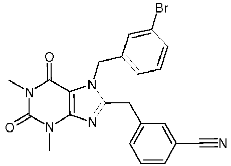 | E | ND |
| 102 | 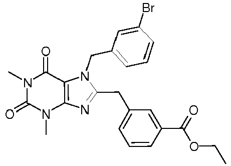 | D | ND |
| 103 | 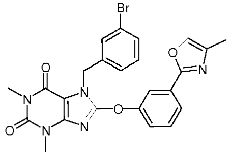 | E | ND |
| 104 | 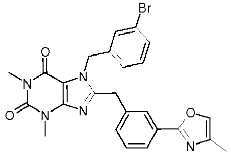 | E | ND |
| 105 | 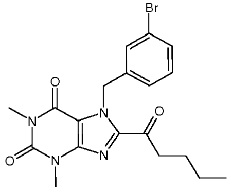 | A | ND |
| 106 | 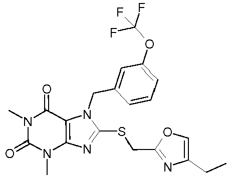 | B | ND |
| 107 | 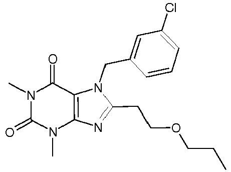 | C | ND |
| 108 | 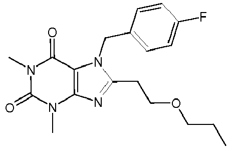 | E | ND |
| 109 | 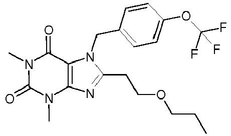 | D | ND |
| 110 | 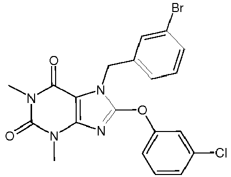 | B | ND |
| 111 | 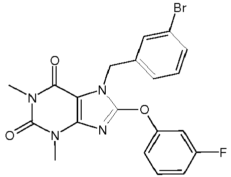 | D | ND |
| 112 | 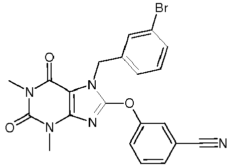 | E | ND |
| 113 | 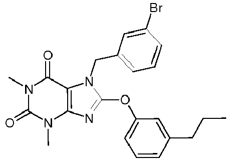 | A | ND |
| 114 | 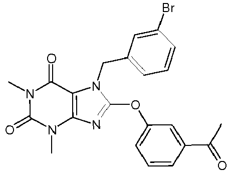 | D | ND |
| 115 | 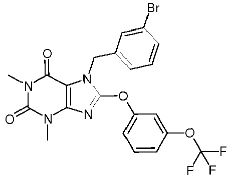 | A | ND |
| 116 | 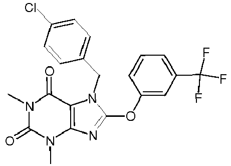 | B | ND |
| 117 | 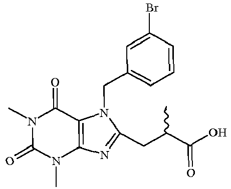 | E | ND |
| 118 | 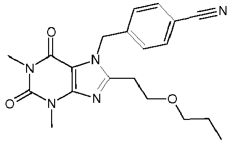 | E | ND |
| 119 | 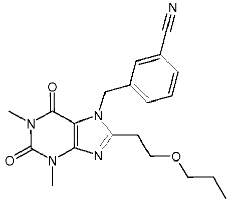 | E | ND |
| 120 | 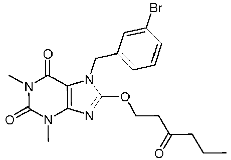 | E | ND |
| 121 | 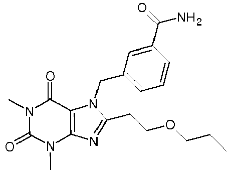 | E | ND |
| 122 | 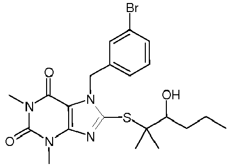 | A | ND |
| 123 | 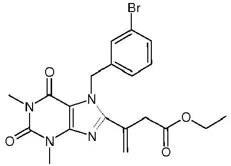 | D | ND |
| 124 | 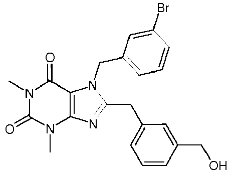 | E | ND |
| 125 | 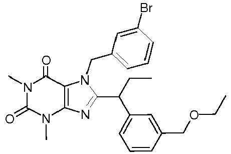 | C | ND |
| 126 | 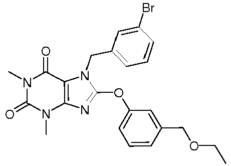 | B | ND |
| 127 | 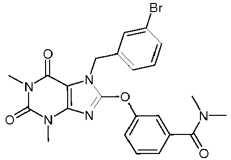 | E | ND |
| 128 | 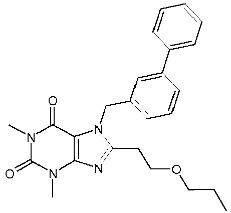 | B | ND |
| 129 | 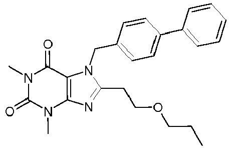 | C | ND |
| 130 | 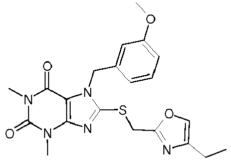 | C | ND |
| 131 | 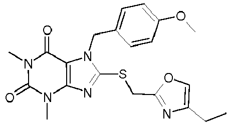 | C | ND |
| 132 | 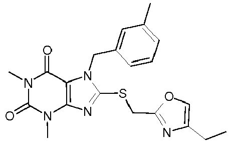 | B | ND |
| 133 | 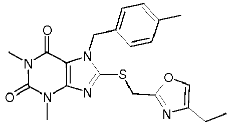 | B | ND |
| 134 | 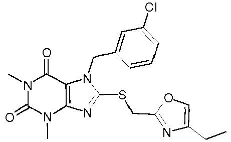 | B | ND |
| 135 | 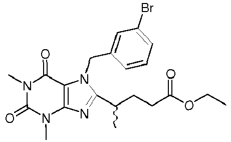 | C | ND |
| 136 | 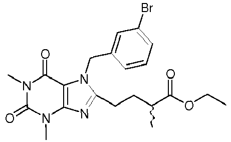 | D | ND |
| 137 | 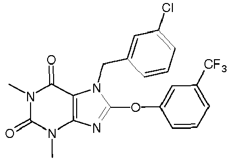 | B | ND |
| 138 | 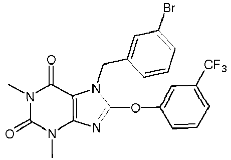 | B | ND |
| 139 | 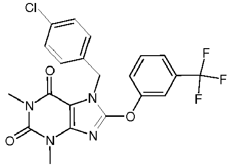 | B | ND |
| 140 | 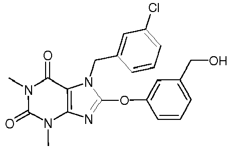 | E | ND |
| 141 | 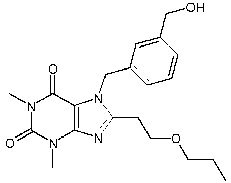 | E | ND |
| 142 | 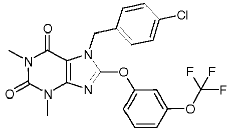 | A | A |
| 143 | 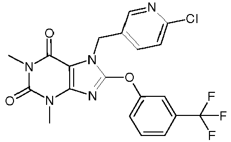 | E | ND |
| 144 | 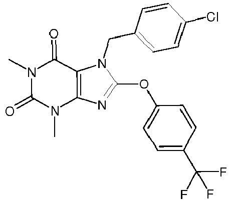 | C | ND |
| 145 | 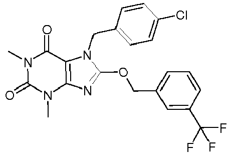 | A | ND |
| 146 | 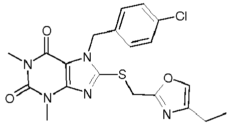 | A | ND |
| 147 | 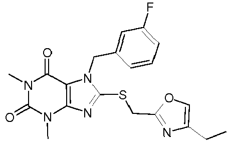 | B | ND |
| 148 | 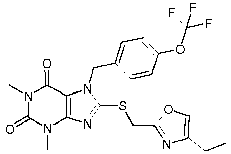 | B | ND |
| 149 | 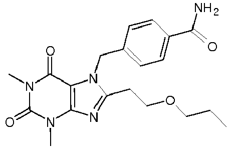 | E | ND |
| 150 | 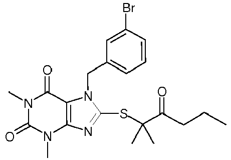 | A | ND |
| 151 | 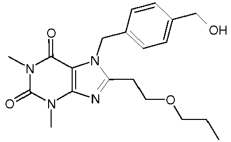 | E | ND |
| 152 | 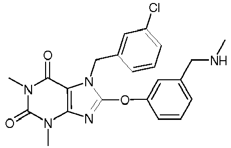 | E | ND |
| 153 | 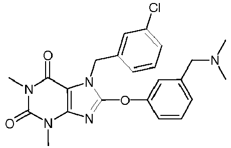 | E | ND |
| 154 | 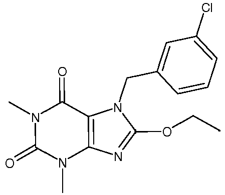 | E | ND |
| 155 | 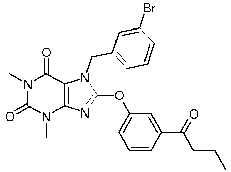 | ND | ND |
| 156 | 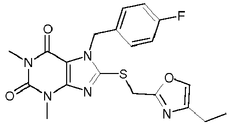 | B | ND |
| 157 | 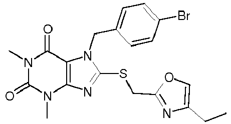 | A | ND |
| 158 | 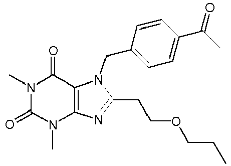 | E | ND |
| 159 | 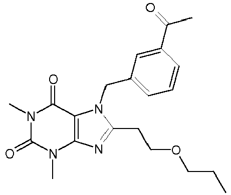 | E | ND |
| 160 | 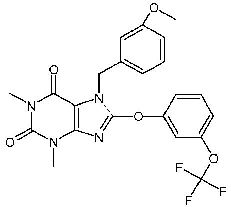 | B | ND |
| 161 | 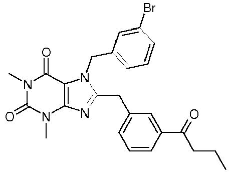 | E | ND |
| 162 | 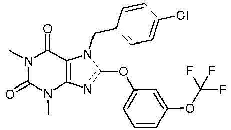 | A | A |
| 163 | 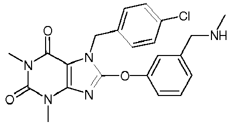 | E | ND |
| 164 | 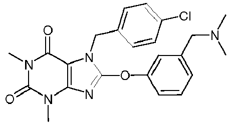 | E | ND |
| 165 | 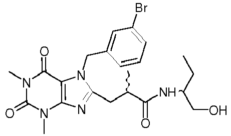 | E | ND |
| 166 | 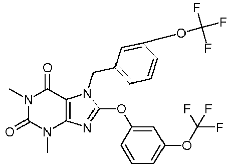 | B | ND |
| 167 | 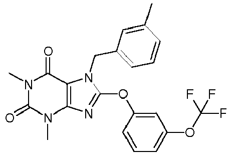 | A | ND |
| 168 | 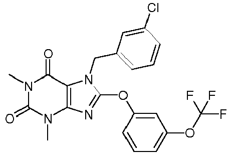 | B | ND |
| 169 | 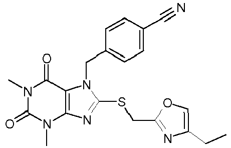 | D | ND |
| 170 | 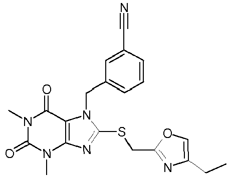 | E | ND |
| 171 | 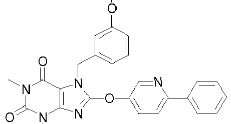 | E | ND |
| 172 | 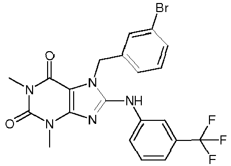 | E | ND |
| 173 | 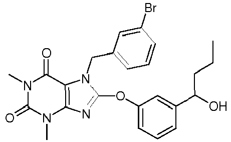 | E | ND |
| 174 | 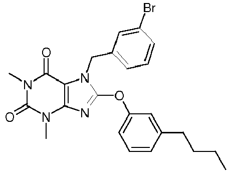 | E | ND |
| 175 | 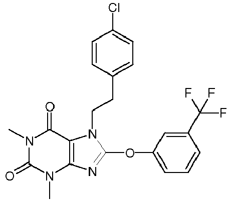 | F | ND |
| 176 | 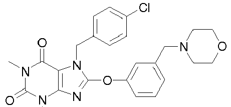 | F | ND |
| 177 | 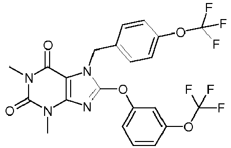 | ND | ND |
| 178 | 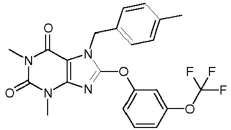 | B | ND |
| 179 | 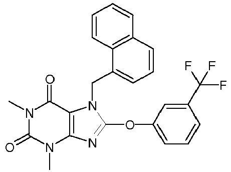 | B | ND |
| 180 | 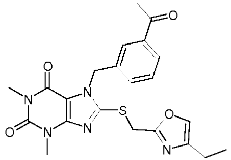 | E | ND |
| 181 | 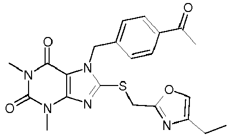 | E | ND |
| 182 | 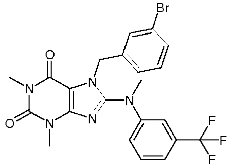 | F | ND |
| 183 | 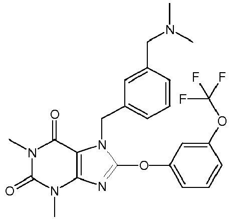 | E | ND |
| 184 | 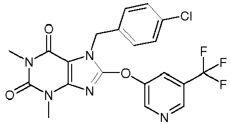 | D | ND |
| 185 | 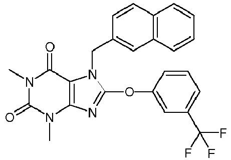 | E | ND |
| 186 | 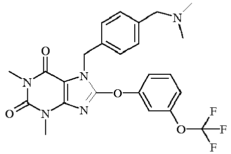 | E | ND |
| 187 | 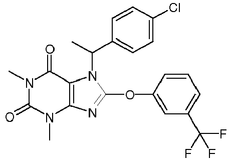 | B | ND |
| 188 | 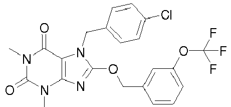 | ND | ND |
| 189 | 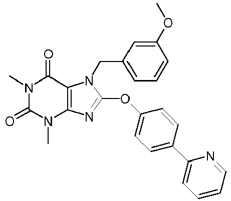 | E | ND |
| 190 | 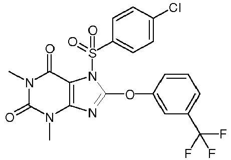 | E | ND |
| 191 | 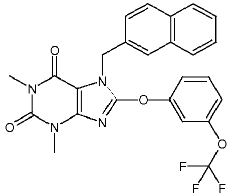 | A | ND |
| 192 | 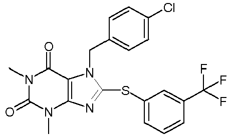 | B | ND |
| 193 | 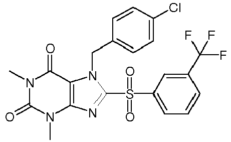 | ND | ND |
| 194 | 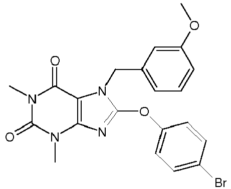 | D | ND |
| 195 | 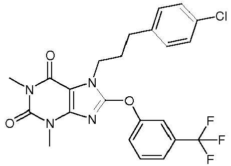 | E | ND |
| 196 | 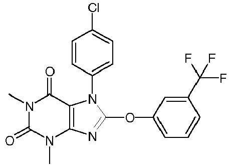 | E | ND |
| 197 | 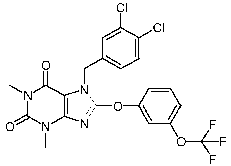 | A | ND |
| 198 | 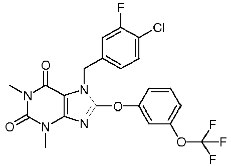 | A | ND |
| 199 | 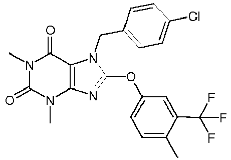 | E | ND |
| 200 | 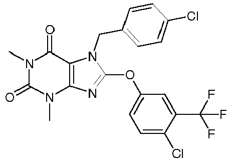 | F | ND |
| 201 | 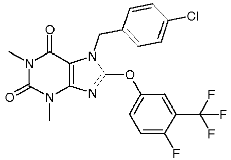 | ND | ND |
| 202 | 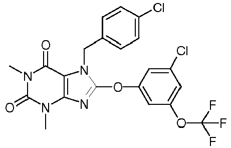 | A | ND |
| 203 | 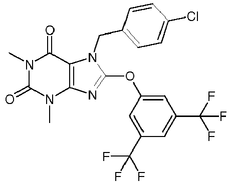 | F | ND |
| 204 | 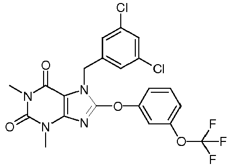 | A | ND |
| 205 | 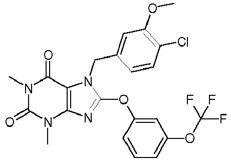 | B | ND |
| 206 | 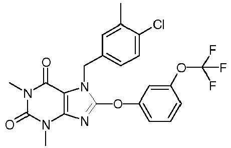 | A | ND |
| 207 | 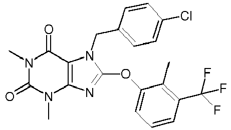 | E | ND |
| 208 | 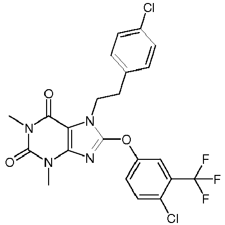 | F | ND |
| 209 | 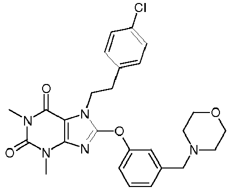 | ND | ND |
| 210 | 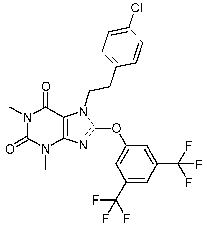 | ND | ND |
| 211 | 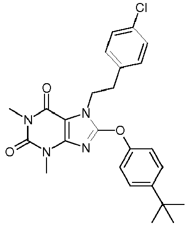 | ND | ND |
| 212 | 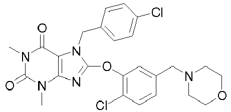 | ND | ND |
| 213 | 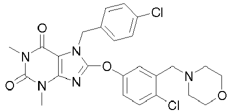 | F | ND |
| 214 | 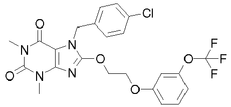 | ND | ND |
| 215 | 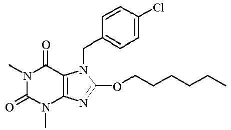 | A | ND |
| 216 | 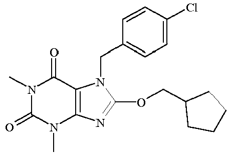 | B | ND |
| 217 | 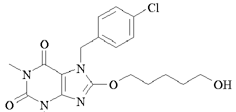 | E | ND |

### Definitions

At various places in the present specification, substituents of compounds of the invention are disclosed in groups or in ranges. It is specifically intended that the invention include each and every individual subcombination of the members of such groups and ranges. For example, the term "C₁₋₆ alkyl" is specifically intended to individually disclose methyl, ethyl, C₃ alkyl, C₄ alkyl, C₅ alkyl, and C₆ alkyl.

For compounds of the invention in which a variable appears more than once, each variable can be a different moiety selected from the Markush group defining the variable. For example, where a structure is described having two R groups that are simultaneously present on the same compound; the two R groups can represent different moieties selected from the Markush group defined for R.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

As used herein, "acyl" refers to the group (C₁-C₆ alkyl)-C(O)-.

As used herein, "alkyl," by itself or as part of another substituent, means, unless otherwise stated, a straight or branched chain, and can have a number of carbon atoms optionally designated (*i.e.,* C₁-C₆ means one to six carbons). Examples of saturated hydrocarbon groups include, but are not limited to, groups such as methyl, ethyl, n-propyl, isopropyl, n-butyl, t-butyl, isobutyl, sec-butyl, n-pentyl, isopentyl, homologs and isomers of, for example, n-pentyl, n-hexyl, and the like.

As used herein, "alkenyl" can be a straight or branched hydrocarbon chain, containing at least one double bond, and having from two to six carbon atoms (i.e. C₂-C₆ alkenyl). Examples of alkenyl groups, include, but are not limited to, groups such as ethenyl (*i.e.,* vinyl), prop-1-enyl (*i.e.,* allyl), but-1-enyl, pent-1-enyl, penta-1,4-dienyl, and the like.

As used herein, "alkoxy" can be a straight chain or branched alkoxy group having from one to six carbon atoms (i.e., C₁-C₆ alkoxy). Examples of alkoxy groups, include, but are not limited to, groups such as methoxy, ethoxy, propyloxy, isopropyloxy, butyloxy, isobutyloxy, tert-butyloxy, pentyloxy, or hexyloxy, and the like.

As used herein, "alkynyl" can be a straight or branched hydrocarbon chain, containing at least one triple bond, having from two to six carbon atoms (i.e. C₂-C₆ alkynyl). Examples of alkynyl groups, include, but are not limited to, groups such as ethynyl, propynyl, butynyl, pentynyl, hexynyl, and the like.

As used herein, "amide" or "amido" refers to a chemical moiety with the formula - C(O)NR^{a}- or -NR^{a}C(O)- wherein R^{a} is H or C₁-C₆ alkyl.

As used herein, "amino" or "amine" refers to a -NH₂ radical group.

As used herein, "alkylamino" refers to a group of formula -NH(alkyl), wherein the alkyl group each has 1 to 6 carbons.

As used herein, the term "dialkylamino" refers to a group of formula -N(alkyl)₂, wherein the two alkyl groups each independently have, 1 to 6 carbons.

As used herein, "aryl" refers to a polyunsaturated, aromatic, hydrocarbon moiety which can be a single ring or multiple rings (e.g., 1 to 2 rings) which are fused together or linked covalently, having from six to twelve carbon atoms (i.e. C₆-C₁₂ aryl). Non-limiting examples of aryl groups include phenyl, 1-naphthyl, 2-naphthyl, and 4-biphenyl.

As used herein, "arylalkyl" refers to an (aryl)alkyl- radical wherein aryl and alkyl moieties are as disclosed herein.

As used herein, "aryloxy" refers to -O-(aryl), wherein the heteroaryl moiety is as defined herein.

As used herein, "arylalkoxy" refers to -O-(arylalkyl), wherein the heteroaryl moiety is as defined herein.

As used herein, "carboxyl" refers to a -(C=O)OH radical.

As used herein, "cyano" refers to a -CN radical.

As used herein, "cycloalkyl" refers to a monocyclic or polycyclic radical that contains only carbon and hydrogen, and may be saturated, or partially unsaturated. Cycloalkyl groups include groups having from 3 to 10 ring atoms (i.e. C₃-C₁₀ cycloalkyl). Examples of cycloalkyl groups include, but are not limited to, groups such as cyclopropyl, cyclobutyl, cyclopentyl, cyclopentenyl, cyclohexyl, cyclohexenyl, cycloseptyl, cyclooctyl, cyclononyl, cyclodecyl, norbornyl, and the like.

As used herein, "C₃-C₇ cycloalkyloxy" refers to -O-(C₃-C₇ cycloalkyl), wherein the C₃-C₇cycloalkyl moiety is as defined herein.

As used herein, "halo" or "halogen," independently or as part of another substituent, mean, unless otherwise stated, a fluorine, chlorine, bromine, or iodine atom. The term "halide" by itself or as part of another substituent, refers to a fluoride, chloride, bromide, or iodide atom.

As used herein, "haloalkyl" and "haloalkoxy" can include alkyl and alkoxy structures that are substituted with one or more halo groups or with combinations thereof. For example, the terms "fluoroalkyl" and "fluoroalkoxy" include haloalkyl and haloalkoxy groups, respectively, in which the halo is fluorine.

As used herein, "heteroalkyl" can include an optionally substituted alkyl, which has one or more skeletal chain atoms selected from an atom other than carbon, e.g., oxygen, nitrogen, sulfur, phosphorus or combinations thereof. A numerical range may be given, e.g. C₁-C₆ heteroalkyl which refers to the number of carbons in the chain, which in this example includes 1 to 6 carbon atoms. For example, a -CH₂OCH₂CH₃ radical is referred to as a "C₃" heteroalkyl. Connection to the rest of the molecule may be through either a heteroatom or a carbon in the heteroalkyl chain.

As used herein, "heteroaryl" refers to a 5- to 14-membered aromatic radical (e.g., C₂-C₁₃ heteroaryl) that includes one or more ring heteroatoms selected from nitrogen, oxygen and sulfur, and which may be a monocyclic or bicyclic ring system. Bivalent radicals derived from univalent heteroaryl radicals whose names end in "-yl" by removal of one hydrogen atom from the atom with the free valence are named by adding "-idene" to the name of the corresponding univalent radical, e.g., a pyridyl group with two points of attachment is a pyridylidene. An N-containing "heteroaromatic" or "heteroaryl" moiety refers to an aromatic group in which at least one of the skeletal atoms of the ring is a nitrogen atom. The polycyclic heteroaryl group may be fused or non-fused. The heteroatom(s) in the heteroaryl radical is optionally oxidized. One or more nitrogen atoms, if present, are optionally quaternized. The heteroaryl is attached to the rest of the molecule through any atom of the ring(s). Examples of heteroaryl groups include without limitation, pyridyl, pyrimidinyl, pyrazinyl, pyridazinyl, triazinyl, furyl (furanyl), quinolyl, isoquinolyl, thienyl, imidazolyl, thiazolyl, indolyl, pyrryl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, indolinyl, and the like.

As used herein, "heteraryloxy" refers to -O-(heteroaryl), wherein the heteroaryl moiety is as defined herein.

As used herein, "heterocycloalkyl" can be a stable 3- to 18-membered non-aromatic ring radical that comprises two to twelve carbon atoms and from one to six heteroatoms selected from nitrogen, oxygen and sulfur. Examples of heterocycloalkyl groups include, but are not limited to, groups such as dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, 1,1-dioxo-thiomorpholinyl, and the like.

As used herein, "hydroxy" or "hydroxyl" refers to -OH.

As used herein, "hydroxyalkyl" refers to an alkyl group having 1 to 6 carbon atoms, which is substituted with a hydroxyl group, e.g., hydroxypropyl.

As used herein, "nitro" refers to -NO₂.

As used herein, "urea" refers to -NR^{a}-C(O)-NR^{a}₂ or -NR^{a}-C(O)NR^{a}-, wherein R^{a} is H or C₁-C₆ alkyl.

As used herein, "sulfonylurea" refers to -S(O)₂-NR^{a}-C(O)-NR^{a}- or -NR^{a}-C(O)-NR^{a}-SO₂-, wherein R^{a} is H or C₁-C₆ alkyl.

As used herein, "sulfonamidyl" refers to -S(O)₂-NR^{a}- or -NR^{a}-S(O)₂-, wherein R^{a} is H or C₁-C₆ alkyl.

The terms "antagonist" and "inhibitor" are used interchangeably to refer to an agent that decreases or suppresses a biological activity, such as to repress an activity of an ion channel, such as TRPC5. TRPC5 ion channels as described herein include homomultimeric and heteromultimeric structures (e.g., homomultimeric TRPC5 and heteromeric TRPC5-TRPC1 or TRPC5-TRPC4). TRPC5 antagonists include inhibitors having any combination of the structural and/or functional properties disclosed herein.

An "effective amount" of, e.g., a TRPC5 antagonist, with respect to the subject methods of inhibition or treatment, refers to an amount of the antagonist in a preparation which, when applied as part of a desired dosage regimen brings about a desired clinical or functional result. Without being bound by theory, an effective amount of a TRPC5 antagonist for use in the methods of the present invention includes an amount of a TRPC5 antagonist effective to decrease one or more in vitro or in vivo function of a TRPC5 channel. Exemplary functions include, but are not limited to, membrane polarization (e.g., an antagonist may promote hyperpolarization of a cell), ion flux, ion concentration in a cell, outward current, and inward current. Compounds that antagonize TRPC5 function include compounds that antagonize an in vitro or in vivo functional activity of TRPC5. When a particular functional activity is only readily observable in an in vitro assay, the ability of a compound to inhibit TRPC5 function in that in vitro assay serves as a reasonable proxy for the activity of that compound. In certain embodiments, an effective amount is an amount sufficient to inhibit a TRPC5-mediated current and/or the amount sufficient to inhibit TRPC5 mediated ion flux.

The TRPC5 anagonists for use in the methods of the present invention may be characterized according to their activity, or lack of activity, against one or more other ion channels. When other ion channels are referred to, inhibition of a function of such other ion channels is defined similarly. For example, inhibition of an ion channel or an activity of an ion channel means the antagonist inhibits one or more functional activities of the other ion channel. Such functions include the current mediated by the particular ion channel, ion flux, or membrane polarization.

The term "preventing" is art-recognized, and when used in relation to a condition, such as a local recurrence, a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount. Prevention of an infection includes, for example, reducing the number of diagnoses of the infection in a treated population versus an untreated control population, and/or delaying the onset of symptoms of the infection in a treated population versus an untreated control population. Prevention of pain includes, for example, reducing the magnitude of, or alternatively delaying, pain sensations experienced by subjects in a treated population versus an untreated control population.

The term "prodrug" is intended to encompass compounds that, under physiological conditions, are converted into the therapeutically active agents of the present invention. A common method for making a prodrug is to include selected moieties that are hydrolyzed under physiological conditions to reveal the desired molecule. In other embodiments, the prodrug is converted by an enzymatic activity of the host animal.

The term "small molecule" refers to a compound having a molecular weight less than about 2500 amu, preferably less than about 2000 amu, even more preferably less than about 1500 amu, still more preferably less than about 1000 amu, or most preferably less than about 750 amu.

The terms "TRPC5", "TRPC5 protein", and "TRPC5 channel" are used interchangeably throughout the application. Unless expressly stated, the term TRPC5 includes homomultimeric structures (e.g., homomultimeric TRPC5) and heteromultimeric structures (e.g., heteromultimeric TRPC5-TRPC1).

The term "oxidative metabolite" is intended to encompass compounds that are produced by metabolism of the parent compound under normal physiological conditions. Specifically, an oxidative metabolite is formed by oxidation of the parent compound during metabolism. For example, a thioether group may be oxidized to the corresponding sulfoxide or sulfone.

The term "solvate" as used herein, refers to a compound formed by solvation (e.g., a compound formed by the combination of solvent molecules with molecules or ions of the solute).

The term "hydrate" as used herein, refers to a compound formed by the union of water with the parent compound.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The terms "compound" and "agent" are used interchangeably to refer to the inhibitors/antagonists of the invention. In certain embodiments, the compounds are small organic or inorganic molecules, e.g., with molecular weights less than 7500 amu, preferably less than 5000 amu, and even more preferably less than 2000, 1500, 1000, or 600 amu. Such compounds can bind to and inhibit a function of TRPC5. In certain other embodiments, the compounds are nucleic acids, for example, TRPC5 antisense oligonucleotides or TRPC5 RNAi constructs. Such compounds can inhibit the expression of TRPC5, thereby inhibiting the activity of TRPC5. Other exemplary compounds that may act as inhibitors include ribozymes and peptide fragments.

In general, the compound of the present invention may be prepared by the methods illustrated in the general reaction schemes as, for example, described below, or by modifications thereof, using readily available starting materials, reagents and conventional synthesis procedures. In these reactions, it is also possible to make use of variants which are in themselves known, but are not mentioned here.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

The compounds described herein can be asymmetric (e.g., having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms.

Resolution of racemic mixtures of compounds can be carried out by any of numerous methods known in the art. An example method includes fractional recrystallizaion using a "chiral resolving acid" which is an optically active, salt-forming organic acid. Suitable resolving agents for fractional recrystallization methods are, for example, optically active acids, such as the D and L forms of tartaric acid, diacetyltartaric acid, dibenzoyltartaric acid, mandelic acid, malic acid, lactic acid or the various optically active camphorsulfonic acids such as β-camphorsulfonic acid. Other resolving agents suitable for fractional crystallization methods include stereoisomerically pure forms of α-methylbenzylamine (e.g., S and R forms, or diastereomerically pure forms), 2-phenylglycinol, norephedrine, ephedrine, N-methylephedrine, cyclohexylethylamine, 1,2-diaminocyclohexane, and the like.

Resolution of racemic mixtures can also be carried out by elution on a column packed with an optically active resolving agent (e.g., dinitrobenzoylphenylglycine). Suitable elution solvent composition can be determined by one skilled in the art. Compounds of the invention also include tautomeric forms, such as keto-enol tautomers.

Compounds of the invention can also include all isotopes of atoms occurring in the intermediates or final compounds. For example, the compound of formula (I) may be radiolabeled with radioactive isotopes, such as for example tritium (³H) or carbon-14 (¹⁴C). All isotopic variations, whether radioactive or not, are intended to be encompassed within the scope of the present invention.

Compounds of the invention can exist in unsolvated forms as well as solvated forms, including hydrated forms. A compound of formula (I) may exist in multiple crystalline or amorphous forms.

The term "pharmaceutically acceptable salts" includes salts of a compound of formula (I) which are prepared with relatively nontoxic acids or bases. Base addition salts can be obtained by contacting the neutral form of a compound of formula (I) with a sufficient amount of the desired base, either neat or in a suitable inert solvent. Examples of pharmaceutically acceptable base addition salts include sodium, potassium, calcium, ammonium, organic amino, or magnesium salt, or a similar salt. Acid addition salts can be obtained by contacting the neutral form of a compound of formula (I) with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, trifluoroacetic, propionic, isobutyric, maleic, malonic, benzoic, succinic, suberic, fumaric, lactic, mandelic, phthalic, benzensulfonic, p-tolylsulfonic, citric, tartaric, methanesulfonic, and the like. Also included are the salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19).

The neutral forms of a compound of formula (I) is preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

The term "low enough pyrogen activity", with reference to a pharmaceutical preparation, refers to a preparation that does not contain a pyrogen in an amount that would lead to an adverse effect (e.g., irritation, fever, inflammation, diarrhea, respiratory distress, endotoxic shock, etc.) in a subject to which the preparation has been administered. For example, the term is meant to encompass preparations that are free of, or substantially free of, an endotoxin such as, for example, a lipopolysaccharide (LPS).

### Diseases, Disorders, or Conditions Related to TR-PC5 Function

In certain embodiments, the invention provides methods and compositions for antagonizing a function of a TRPC5 channel in vitro or in vivo. Exemplary functions include, but are not limited to, TRPC5-mediated current. In certain embodiments, the invention provides a compound of the invention for use in methods for treating a disease or disorder or condition. In other embodiments, the compound as described herein selectively inhibits the expression level and/or activity of a TRPC5 protein. In other words, in certain embodiment, the compound as described herein inhibits the activity of a TRPC5 protein preferentially in comparison to the activity of one or more other ion channels.

### Treatment of Anxiety and Fear-Related Disorders

In certain embodiments, the compounds of the invention can be used in methods for preventing or treating anxiety and fear-related disorders (see, e.g., Riccio et al. (2009) Cell 137:761-72). Examples of such disorders include post-traumatic stress disorder, panic disorder, agoraphobia, social phobias, generalized anxiety disorder, panic disorder, social anxiety disorder, obsessive-compulsive disorder, and separation anxiety.

### Memory, Motion and Mood Disorders

A compound of Formula I or Formula II is also useful in methods for the treatment of Parkinson's disease, epilepsy, memory disorders, stroke, seizure, and mood disorders. Mood disorders include depression (e.g., major depression, psychiatric depression, dysthymia, and postpartum depression) and bipolar disorder (e.g., bipolar I, bipolar II, and cyclothymia). Memory disorders are conditions associated with any memory loss and may result from Alzheimer's disease, amnesia, aphasia, atherosclerosis, brain injury or disorder, brain tumor, chronic fatigue syndrome, Creutzfedt-Jacob disease, dissociative amnesia, depression, fuge amnesia, Huntington's disease, learning disorders, sleeping disorders, multiple personality disorder, pain, post-traumatic stress disorder, schizophrenia, sports injuries, stroke, and Wernicke-Korsakoff syndrome.

### Treatment of Pain, Sensitivity to Pain and Touch, or Pain-Related Diseases or Disorders

In certain embodiments, a compound of Formula I or Formula II is used in methods to treat or ameliorate pain. Exemplary classes of pain that can be treated using a a compound of formula (I)include, but are not limited to nociceptive pain, inflammatory pain, and neuropathic pain. The pain can be chronic or acute.

A compound of Formula I or Formula II may be particularly useful in methods for the treatment of pain associated with cancer, osteoarthritis, rheumatoid arthritis, post-herpetic neuralgia, bums, and other indications detailed above. To further illustrate, additional exemplary indications for which a compound of Formula I or Formula II can be used include oral pain, pelvic pain, Fabry's disease, complex regional pain syndrome, pancreatitis, and fibromyalgia syndrome.

A compound of Formula I or Formula II may also be used in methods in connection with prevention or treatment of sensitivity to pain and touch. Pain or sensitivity to pain and touch may be indicated in a variety of diseases, disorders or conditions, including, but not limited to, diabetic neuropathy, breast pain, psoriasis, eczema, dermatitis, burn, post-herpetic neuralgia (shingles), nociceptive pain, peripheral neuropathic and central neuropathic pain, chronic pain, cancer and tumor pain, spinal cord injury, crush injury and trauma induced pain, migraine, cerebrovascular and vascular pain, sickle cell disease pain, rheumatoid arthritis pain, musculoskeletal pain including treating signs and symptoms of osteoarthritis and rheumatoid arthritis, orofacial and facial pain, including dental, temperomandibular disorder, and cancer related, lower back or pelvic pain, surgical incision related pain, inflammatory and non-inflammatory pain, visceral pain, psychogenic pain and soft tissue inflammatory pain, fibromyalgia-related pain, and reflex sympathetic dystrophy, and pain resulting from kidney stones or urinary tract infection.

The foregoing are merely exemplary of diseases and conditions that cause or lead to inflammation, lesions, ulcers, or other sources of oral pain. In other embodiments, the oral pain is due to an injury to the mouth, jaw, lips, gums, or teeth. In other embodiments, the oral pain is due to oral surgery, for example, surgery for cancer, tooth extraction, or jaw remodeling. Other conditions that may lead to oral ulcers, and thus oral pain, include, but are not limited to chickpox, herpes zoster, infectious mononucleosis, syphilis, tuberculosis, acute necrotizing gingivitis, and burning mouth syndrome.

Fibromyalgia (FMS; fibromyalgia syndrome) is a widespread musculoskeletal pain and fatigue disorder. Fibromyalgia is characterized by pain in the muscles, ligaments, and tendons. The condition affects more women than men, and occurs in people of all ages. Overall, FMS is estimated to afflict 3-6% of the population. Patients have described the pain associated with fibromylagia as deep muscular aching, throbbing, shooting, and stabbing. The pain sometimes includes an intense burning sensation. The pain and stiffness are often worse in the morning or after repetitive use of a particular muscle group.

Additionally, varying levels of fatigue ranging from mild to incapacitating are often associated with fibromylagia. Other symptoms of fibromylagia include gastrointestinal symptoms. Irritable bowel syndrome and IBS-like symptoms such as constipation, diarrhea, frequent abdominal pain, abdominal gas, and nausea occur in roughly 40 to 70% of FMS patients. Acid reflux or gastroesophogeal reflux disease (GERD) occurs at a similar frequency.

Complex Regional Pain Syndrome (CRPS; also known as chronic regional pain syndrome) is a chronic pain condition. CRPS was formerly known as reflex sympathetic dystrophy (RSD). CRPS is a chronic, painful, and progressive neurological condition that affects skin, muscles, joints, and bones. The syndrome usually develops in an injured limb, such as a broken leg or following surgery. However, many cases involve only a minor injury, such as a sprain, and sometimes no precipitating injurious event can be identified. CRPS involves continuous, intense pain that is disproportionate to the severity of the injury. The pain worsens, rather than improves, over time.

Although CRPS can affect a variety of regions of the body, it most often affects the arms, legs, hands, or feet. Often the pain begins in one portion of a limb, but spreads over time to include the entire limb or even to include a different limb. Typical features include dramatic changes in the color and temperature of the skin over the affected limb or body part, accompanied by intense burning pain, skin sensitivity, sweating, and swelling.

The compounds disclosed herein can also be used in methods to treat endometriosis and the pain associated therewith.

### Neurological or Neurodegenerative Diseases and Disorders

Neurodegenerative diseases and disorders include but are not limited to Alzheimer's disease (AD), Parkinson's disease, Huntington's disease, amyotrophic lateral sclerosis (ALS), and other brain disorders caused by trauma or other insults including aging.

Mechanisms associated with calcium signaling may be altered in many neurodegenerative diseases and in disorders resulting from brain injury. For example, fibroblasts or T-lymphocytes from patients with AD have consistently displayed an increase in Ca²⁺ release from intracellular stores compared to controls (Ito et al. (1994) Proc. Natl. Acad. Sci. U.S.A. 91:534-538; Gibson et al. (1996) Biochem. Biophys. ACTA 1316:71-77; Etchenberrigaray et al. (1998) Neurobiology of Disease, 5:37-45). Consistent with these observations, mutations in presenilin genes (PS1 or PS2) associated with familial AD (FAD) have been shown to increase InsP3-mediated Ca²⁺ release from internal stores (Guo et al. (1996) Neuro Report, 8:379-383; Leissring et al. (1999) J. Neurochemistry, 72:1061-1068; Leissring et al. (1999) J. Biol. Chem. 274 (46):32535-32538; Leissring et al. (2000) J. Cell Biol. 149 (4):793-797; Leissring et al. (3):561-572).

Experimental traumatic brain injury has been shown to initiate massive disturbances in Ca²⁺ concentrations in the brain that may contribute to further neuronal damage. Intracellular Ca²⁺ may be elevated by many different ion channels. It has been further shown that channel blockers may be beneficial in the treatment of neurological motor dysfunction when administered in the acute posttraumatic period (Cheney et al. (2000) J. Neurotrauma, 17 (1):83-91).

### Seizure

Excitotoxicity of a variety of origins leads to seizures. Commonly excess neuronal firing can drive seizure activity. Compounds that reduce the hyperexcitability of relevant neuronal populations have significant potential in reducing seizure activity.

### Proteinuric Kidney Disease

TRPC5 is also expressed in the podocyte of the kidney. It has been proposed that there is an antagonistic regulation of actin dynamics and cell in the podocytes by TRPC5 and TRPC6 (Tian et al., (2010) Science Signaling). Thus, inhibiting TRPC5 may impact the reaction of the podocyte to injury.

### Combination Therapy

The present invention provides a compound of Formula I or Formula II for use in vitro and in vivo. The present invention also provides compositions and pharmaceutical compositions comprising a compound of formula (I) that inhibits TRPC5 activity. In certain embodiments, the compound of formula (I) is selective. In other words, in certain embodiments, the compound of formula (I) inhibits TRPC5 activity preferentially over the activity of other ion channels. In certain embodiments, the compound of formula (I) inhibits TRPC5 activity preferentially over TRPV1, TRPV2, TRPV3, TRPV4, TRPC3, TRPC6, TRPC7, TRPA1, and/or TRPM8 activity. For example, in certain embodiments, the compound of formula (I) inhibits the activity of TRPC5 and also inhibits the activity of one or more of TRPC4, TRPV1, TRPV2, TRPV3, TRPV4, TRPC3, TRPC6, TRPC7, TRPA1, and TRPM8.

A compound of Formula I or Formula II can be used alone or in combination with other pharmaceutically active agents. Examples of such other pharmaceutically active agents include, but are not limited to, anti-depressants, anti-anxiety agents, anti-epileptic agents, anti-inflammatory agents (e.g., NSAIDS, bradykinin receptor antagonists, hormones and autacoids such as corticosteroids), or anti-migraine agents. Certain active agents belong to more than one category.

In certain embodiments, a compound of formula (I) is conjointly administered with an analgesic. Suitable analgesics include, but are not limited to, opioids, glucocorticosteroids, non-steroidal anti-inflammatories, naphthylalkanones, oxicams, para-aminophenol derivatives, propionic acids, propionic acid derivatives, salicylates, fenamates, fenamate derivatives, pyrozoles, and pyrozole derivatives. Examples of such analgesic compounds include, but are not limited to, codeine, hydrocodone, hydromorphone, levorpharnol, morphine, oxycodone, oxymorphone, butorphanol, dezocine, nalbuphine, pentazocine, etodolac, indomethacin, sulindac, tolmetin, nabumetone, piroxicam, acetaminophen, fenoprofen, flurbiprofen, ibuprofen, ketoprofen, naproxen, diclofenac, oxaprozin, aspirin, diflunisal, meclofenamic acid, mefanamic acid, prednisolone, and dexamethasone. Preferred analgesics are non-steroidal anti-inflammatories and opioids (preferably morphine).

In some embodiments, a compound of formula (I) can be administered in conjunction with a therapeutic whose administration causes pain. For example, a compound of Formula (I) can be administered in conjunction with an anesthetic, to reduce the pain caused by the administration of the anaesthetic. A compound of Formula (I) can also be administered in conjunction with a chemotherapeutic agent, to reduce the pain caused by administration of the chemotherapeutic agent.

In certain embodiments, a compound of formula (I) is conjointly administered with a non-steroidal anti-inflammatory. Suitable non-steroidal anti-inflammatory compounds include, but are not limited to, piroxicam, diclofenac, etodolac, indomethacin, ketoralac, oxaprozin, tolmetin, naproxen, flubiprofen, fenoprofen, ketoprofen, ibuprofen, mefenamic acid, sulindac, apazone, phenylbutazone, aspirin, celecoxib and rofecoxib.

### Pharmaceutical Compositions

While it is possible for a compound of formula (I) to be administered alone, it is preferable to administer the compound as a pharmaceutical formulation, where the compound is combined with one or more pharmaceutically acceptable excipients or carriers. The compound of Formula I or Formula II may be formulated for administration in any convenient way for use in human or veterinary medicine. In certain embodiments, the compound of Formula I or Formula II may be a prodrug, e.g., capable of being converted to an active compound in a physiological setting.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Examples of pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; (21) cyclodextrins such as Captisol®; and (22) other non-toxic compatible substances employed in pharmaceutical formulations.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Solid dosage forms (e.g., capsules, tablets, pills, dragees, powders, granules and the like) can include one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents.

Liquid dosage forms can include pharmaceutically acceptable emulsions, microemulsions, solutions, suspensions, syrups and elixirs. In addition to the compound of formula (I), the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Suspensions, in addition to the compound of Formula I or Formula II, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Ointments, pastes, creams and gels may contain, in addition to the compound of formula (I), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compound of Formula I or Formula II, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The formulations may conveniently be presented in unit dosage form and may be prepared by any methods well known in the art of pharmacy. The amount of a compound of Formula I or Formula II which can be combined with a carrier material to produce a single dosage form will vary depending upon the host being treated, the particular mode of administration. The amount of a compound of Formula I or Formula II that can be combined with a carrier material to produce a single dosage form will generally be that amount of the compound which produces a therapeutic effect. Generally, out of one hundred percent, this amount will range from about 1 percent to about ninety-nine percent of a compound of Formula I or Formula II, preferably from about 5 percent to about 70 percent, most preferably from about 10 percent to about 30 percent.

The tablets, and other solid dosage forms of the pharmaceutical compositions disclosed herein, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain pacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Dosage forms for the topical or transdermal administration of a compound of Formula I or Formula II include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The formulations disclosed herein can be delivered via a device. Exemplary devices include, but are not limited to, a catheter, wire, stent, or other intraluminal device. Further exemplary delivery devices also include a patch, bandage, mouthguard, or dental apparatus. Transdermal patches have the added advantage of providing controlled delivery of a compound of formula (I) to the body. Such dosage forms can be made by dissolving or dispersing the compound of formula (I) in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, drops, solutions and the like, are also contemplated as being within the scope of this invention.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

When a compound of Formula I or Formula II is administered as a pharmaceutical, to humans and animals, it can be given per se or as a pharmaceutical composition containing, for example, 0.1 to 99.5% (more preferably, 0.5 to 90%) of the compound of Formula I or Formula II in combination with a pharmaceutically acceptable carrier.

The formulations can be administered topically, orally, transdermally, rectally, vaginally, parentally, intranasally, intrapulmonary, intraocularly, intravenously, intramuscularly, intraarterially, intrathecally, intracapsularly, intraorbitally, intracardiacly, intradermally, intraperitoneally, transtracheally, subcutaneously, subcuticularly, intraarticularly, subcapsularly, subarachnoidly, intraspinally, intrasternally, sublingually, or by inhalation.

### Dosages

Actual dosage levels of the the compound of Formula I or Formula II in the pharmaceutical compositions of this invention may be varied so as to obtain an amount of the active ingredient that is effective to achieve the desired therapeutic response for a particular patient, composition, and mode of administration, without being toxic to the patient.

The selected dosage level will depend upon a variety of factors including the activity of the compound of Formula I or Formula II disclosed herein employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of Formula I or Formula II in the pharmaceutical composition at levels lower than that required in order to achieve the desired therapeutic effect and gradually increase the dosage until the desired effect is achieved.

In general, a suitable daily dose of a compound of Formula I or Formula II will be that amount of the compound that is the lowest dose effective to produce a therapeutic effect. Such an effective dose will generally depend upon the factors described above. Generally, intravenous, intracerebroventricular and subcutaneous doses of the compounds of Formula I or Formula II for a patient will range from about 0.0001 to about 100 mg per kilogram of body weight per day. For example, the dose can be 0.1-50, 0.1-25, 0.5-10, 1-10, or 5-10 mg/kg.

If desired, the effective daily dose of the compound of Formula I or Formula II may be administered as two, three, four, five, six or more sub-doses administered separately at appropriate intervals throughout the day, optionally, in unit dosage forms.

### Disease and Injury Models

A compound of Formula I or Formula II which antagonizes TRPC5 function may be useful in the prophylaxis and treatment of any of the foregoing injuries, diseases, disorders, or conditions. In addition to in vitro assays of the activity of the compound of Formula I or Formula II, its efficacy can be readily tested in one or more animal models. By way of example, numerous well known animal models exist. One or more suitable animal models (e.g., suitable in light of the particular indication) can be selected.

Fear-related behaviors can be measured as described, e.g., in Riccio et al. Pain behaviors can be studied using various agents or procedures to simulate pain resulting from injuries, diseases, or other conditions. Blackburn-Munro (2004) Trends in Pharmacological Sciences 25: 299-305. Behavioral characteristics of challenged animals can then be observed. Compounds or procedures that may reduce pain in the animals can be readily tested by observing behavioral characteristics of challenged animals in the presence versus the absence of the test compound(s) or procedure.

Exemplary behavioral tests used to study chronic pain include tests of spontaneous pain, allodynia, and hyperalgesia. Id. To assess spontaneous pain, posture, gait, nocifensive signs (e.g., paw licking, excessive grooming, excessive exploratory behavior, guarding of the injured body part, and self-mutilation) can be observed. To measure evoked pain, behavioral responses can be examined following exposure to heat (e.g., thermal injury model).

Exemplary animal models of pain include, but are not limited to, the Chung model, the carageenan induced hyperalgesia model, the Freund's complete adjuvant induced hyperalgesia model, the thermal injury model, the formalin model and the Bennett Model. The Chung model of neuropathic pain (without inflammation) involves ligating one or more spinal nerves. Chung et al. (2004) Methods Mol Med 99: 35-45; Kim and Chung (1992) Pain 50: 355-363. Ligation of the spinal nerves results in a variety of behavioral changes in the animals including heat hyperalgesia, cold allodynia, and ongoing pain. Compounds that antagonize TRPC5 can be administered to ligated animals to assess whether they diminish these ligation-induced behavioral changes in comparison to that observed in the absence of compound.

Useful anxiety and depression models include the maternal separation model, the elevated plus-maze model, the forced swim test, the tail suspension test, the light/dark preference model, the light-enhanced startle model, and the ultrasonic vocalization model.

Useful seizure models include but are not limited to maximal electric shock (MES), acoustic startle in susceptible animals (eg DBA mice), and chemical induced seizure (with proconvulsant compounds such as pilocarpine, pentalene tetrazole, kainic acid, N-methyl-D-aspartic acid).

Useful models of kidney function include the LPS-induced proteinuria (waiting for a reference for others).

### Examples

### Example 1: Patch Clamp Experiments

Patch clamp experiments permit the detection of currents through the TRPC5 channel in the cell line described above. In normal whole-cell patch clamp recordings, a glass electrode is brought into contact with a single cell and a high-resistance (gigaohm) seal is established with the cell membrane. The membrane is then ruptured to achieve the whole-cell configuration, permitting control of the voltage of the cell membrane and measurement of currents flowing across the membrane using the amplifier attached to the electrode and resulting in the replacement of cytoplasm with the pipette solution. A perfusion system permits control of the extracellular solution, including the addition of blockers and activators of the current. The current can be activated by including 1.4 µM free Ca²⁺ in the pipette (intracellular) solution, and 80 µM LaCl₃ in the extracellular solution.

TRPC5 cells were induced 20-48 hours, removed from growth plates, and replated at low density (to attain good single-cell physical separation) on glass coverslips for measurement. In some cases, cells were grown in low density overnight on glass coverslips. Patch clamp recordings were made in the whole-cell mode with a holding potential of -40 mV. Every 5 seconds, a voltage ramp was applied from -120 to +100 mV, 400 ms in duration. Currents elicited were quantified at -80 mV and +80 mV. The internal solution consisted of 140 mM cesium aspartate, 10 mM HEDTA, 2 mM CaCl₂, 2.27 mM MgCl₂ and 10 mM HEPES, pH 7.2, with 1,400 nM calculated free Ca²⁺. The external solution consisted of 150 mM NaCl, 4.5 mM KCl, 1 mM MgCl₂, 2 mM CaCl₂,10 mM HEPES, 10 mM glucose, 1 mM EGTA, pH 7.4. Upon addition of LaCl₃, TRPC5 current was induced only in TRPC5-expressing cells and not in parental HEK293 TREx cells. Removal of the LaCl₃ stimulus causes most of the current to go away. Potential blockers were tested for ability to block both inward and outward currents in the continued presence of LaCl₃.

IC₅₀ of a compound of Formula I or Formula II was estimated by testing the compound at 5 µM and 500 nM. When 5 µM of a compound showed no block, IC₅₀ was estimated as >10 µM. When 5 µM of a compound showed 50% or less block, a rough estimate of IC₅₀ in the range of 5-10 µM could be made. IC₅₀ for a compound of Formula I or Formula II between 500 nM and 5 µM was similarly estimated.

### Example 2:

### General experimental procedures

### General Procedures

All reagents were purchased from commercial suppliers and used without further purification unless otherwise stated. THF was continuously refluxed and freshly distilled from sodium and benzophenone under nitrogen, DCM was continuously refluxed and freshly distilled from CaH₂ under nitrogen.

Reactions were monitored via TLC on silica gel plates (either 60 HSGF254 percolated plates (0.15-0.2 mm SiO₂) or Baker-flex IB2-F TLC plates), and visualized using UV light (254 nm or 365 nm) and/or staining with a solution of DNP (12 g, 2,4-dinitrofenylhydrazin, 60 mL concentrated H₂SO₄, 80 ml H₂O, 200 mL ethanol) and subsequent heating or monitored via LCMS.

Microwave reactions were carried out with a Biotage Smith Synthesizer.

LCMS were performed on a SHIMADZU LCMS-2010EV instrument using one of two sets of conditions. LCMS conditions one: (Chromolith SpeedROD, RP-18e column, 50x4.6 mm, mobile phase: Solvent A: CH₃CN/H₂O /HCOOH=10/90/0.05, Solvent B: CH₃CN/H₂O /HCOOH=90/10/0.05, 0.8min@ 10% B, 2.7min gradient (10-95% B), then 0.8min@95%B, Flow rate: 3mL/min, temperature: 40°C). LCMS conditions two: (Zorbax , 3.5 micron, 2.1 x 50 mm C18 column. Mobile phase: Solvent A: 0.1% formic acid /acetonitrile Solvent B: 0.1% formic acid / water. Gradient: 5% to 95% B using a 5 min or 8 min runtime).

Preparative HPLC were performed either on a SHIMADZU LC-8A instrument. (Column: YMC Pack ODS-A (150*30mm, 10µm)) or LC-6AD (Column: Shim=Pack PREP-ODS-H (250*20mm, 10µm)) with UV detection which were controlled by LC solution Chemstation software. H₂O (0.1% HCOOH) and methanol (MeCN) as mobile phase at the indicated flow rate.

Analytical HPLC were performed on a SHIMADZU LC-2010A instrument. (Chromolith SpeedROD, RP-18e, 50x4.6 mm, mobile phase: Solvent A: CH₃CN/H₂O /HCOOH=10/90/0.05, Solvent B: CH₃CN/H₂O /HCOOH=90/10/0.05, 0.8min@ 10% B, 2.7min gradient (10-95% B), then 0.8min@95%B, Flow rate: 3mL/min, temperature: 40°C).
¹*H*-NMR spectra were recorded on either a Bruker Avance II 400MHz or a Varian Unity Inova 400 MHz instrument. Chemical shifts (δ) are reported in ppm relative to tetramethylsilane (δ = 0.000 ppm) and the spectra were calibrated to the residual solvent signal of chloroform (δ = 7.26), Dimethyl sulfoxide (δ = 2.50), methanol (δ = 3.30). Data for ¹*H*-NMR spectra are reported as follows: chemical shift (multiplicity, number of hydrogens). Abbreviations are as follows: s (singlet), d (doublet), t (triplet), q (quartet), quint (quintet), m (multiplet), br (broad).

**List of abbreviations and terms**

| | |
|---|---|
| BPO | benzoyl peroxide |
| Chromatography | compound purification using silica gel |
| Concentrated [or concentrated at reduced pressure] solvent removal with the aid of a rotary evaporation device | |
| DCM | dichloromethane |
| DBU | 1,8-diazabicyclo[5.4.0]undec-7-ene |
| Dess Martin [or Dess Martin periodinane] | 1,1,1-triacetoxy-1,1-dihydro-1,2-benziodoxol-3(1H)-one |
| Dilute HCl | 1N hydrochloric acid |
| DMF | N,N-dimethylformamide |
| DMAP | 4-dimethylaminopyridine |
| Dried, referring to removal of residual water from organic solutions implies the use of an inorganic drying agent such as sodium sulfate | |
| Dried in vacuo [or dried under vacuum] | residual solvent removal with the aid of a vacuum pump |
| DMSO | dimethyl sulfoxide |
| Eaton's reagent | 7.7 wt% phosphorus pentoxide in methanesulfonic acid |
| EDCI | 1-ethyl-3-(3-dimethylaminopropyl)carbodiimide) |
| Evaporated | solvent removal with the aid of a rotary evaporation device |
| h | hour |
| HMDS | hexamethyldisilazane |
| LAH | lithium aluminum hydride |
| MCPBA | 3-chloroperoxybenzoic acid |
| min | minutes |
| n-BuLi | n-Butyllithium |
| NBS | N-bromosuccinimide |
| NCS | N-chlorosuccinimide |
| NMP | N-methylpyrolidinone |
| Oxone | potassium peroxomonosulfate |
| Pd/C | palladium on activated carbon |
| Pd-dppf | 1,1'-Bis(diphenylphosphino)ferrocene-palladium(II)dichloride dichloromethane complex |
| PPTS | pyridinium p-toluenesulfonate |
| Preparative TLC | preparative thin layer chromatography |
| SEM | (trimethylsilyl)ethoxy)methyl |
| TBAI | tetrabutylammonium iodide |
| TBAF | tetrabutylammonium fluoride |
| TBAH | tetrabutylammonium hydroxide |
| TEA | triethylamine |
| TFA | trifluoroacetic acid |
| THF | tetrahydrafuran |
| TLC | thin layer chromatography |
| X-phos | 2-(dicyclohexylphosphino)-2',4',6'-triisopropylbiphenyl |

### Purine numbering

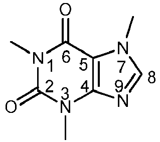

### Preparation of intermediates

### Intermediate 1 7-(3-bromobenzyl)-8-mercapto-1,3-dimethyl-1H-purine-2,6(3H,7H)-dione

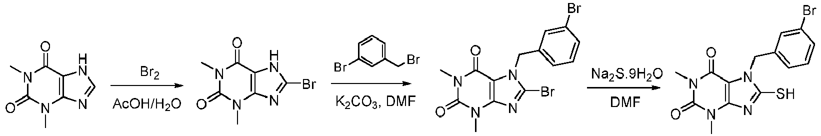

### Step 1 8-bromo-1,3-dimethyl-1H-purine-2,6(3H,7H)-dione

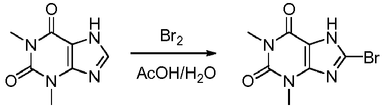

To a solution of theophylline (10 g, 55.5 mmol) in acetic acid /water (90 mL/ 60 mL) was added bromine (9.76 g, 61 mmol) dropwise at 50 °C. The resulting mixture was stirred at this temperature for 4 h. The mixture was cooled to room temperature and the product was precipitated. The mixture was filtered and the filter cake was washed with water twice, dried in vacuo to give 8-bromo-1,3-dimethyl-1H-purine-2,6(3H,7H)-dione (13.6 g, 94.5%) as white solid. LCMS MH⁺ 259.

### Step 2 8-bromo-7-(3-bromobenzyl)-1,3-dimethyl-1H-purine-2,6(3H,7H)-dione

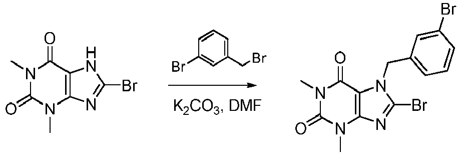

To a solution of 8-bromo-1,3-dimethyl-1H-purine-2,6(3H,7H)-dione (5 g, 19.3 mmol) in DMF (40 mL) was added 1-bromo-3-(bromomethyl)benzene (5.3 g, 21.2 mmol) followed by potassium carbonate (4 g, 28.95 mmol). The resulting mixture was stirred at room temperature for 3 h. The mixture was diluted with water (100 mL) and filtered. The filter cake was washed with water twice, dried in vacuo to give 8-bromo-7-(3-bromobenzyl)-1,3-dimethyl-1H-purine-2,6(3H,7H)-dione (7.6 g, 91.9%) as white solid. LCMS MH⁺ 429.

### Step 3 7-(3-bromobenzyl)-8-mercapto-1,3-dimethyl-1H-purine-2,6(3H,7H)-dione

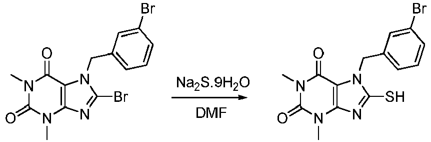

To a solution of 8-bromo-7-(3-bromobenzyl)-1,3-dimethyl-1H-purine-2,6(3H,7H)-dione (5 g, 11.7 mmol) in DMF (50 mL) was added sodium sulfide nonahydrate (3.36 g, 14.02 mmol). The mixture was stirred at 80 °C for 2 h. The mixture was poured into ice-water and the solution was acidified by adding acetic acid. The mixture was filtered and the filter cake was washed successively with water, ethanol, then dried in vacuo to give 7-(3-bromobenzyl)-8-mercapto-1,3-dimethyl-1H-purine-2,6(3H,7H)-dione(4.1 g, 92.1) as white solid. LCMS MH⁺ 383.

### Intermediate 2 8-((4-ethyloxazol-2-yl)methylthio)-1,3-dimethyl-1H-purine-2,6(3H,7H)-dione

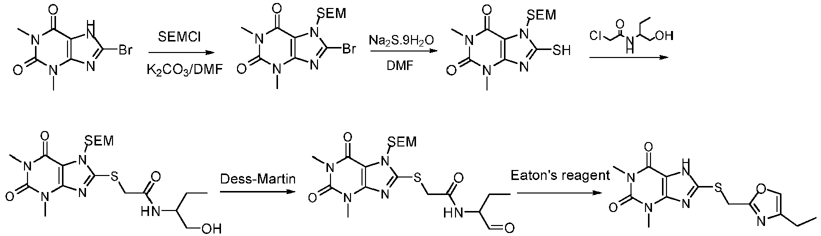

### Step 1 8-bromo-1,3-dimethyl-7-((2-(trimethylsilyl)ethoxy)methyl)-1H-purine-2,6(3H,7H)-dione

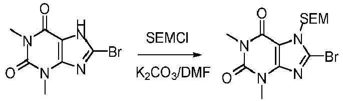

To a solution of the8-bromo-1,3-dimethyl-1H-purine-2,6(3H,7H)-dione (8 g, 31 mmol, Intermediate 1 Step 1 product) in DMF(20 mL) was added potassium carbonate (8.6g, 62.32 mmol), followed by 2-(trimethylsilyl)ethoxymethyl chloride (6.2g, 37.4 mmol) at room temperature. Then the resulting mixture was stirred at 80 °C for 2 h. The mixture was diluted with water (100 mL) and filtered. The filter cake was washed with water twice, dried in vacuo to give 8-bromo-1,3-dimethyl-7-((2-(trimethylsilyl)ethoxy)methyl)-1H-purine-2,6(3H,7H)-dione (11 g, 94.5%) as white solid. LCMS retention time 1.686 min, LCMS MH⁺-28 363.

### Step 2 8-mercapto-1,3-dimethyl-7-((2-(trimethylsilyl)ethoxy)methyl)-1H-purine-2,6(3H,7H)-dione

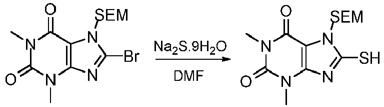

To a solution of 8-bromo-1,3-dimethyl-7-((2-(trimethylsilyl)ethoxy)methyl)-1H-purine-2,6(3H,7H)-dione (11 g, 28.4 mmol) in DMF (20 mL) was added sodium sulfide nonahydrate (8.6 g, 35.8mmol). The mixture was stirred at 80 °C for 2 h. The mixture was poured into ice-water and extracted with ethyl acetate twice, the layers were separated. The organic layer was dried over sodium sulfate, filtered and concentrated to give crude product 8-mercapto-1,3-dimethyl-7-((2-(trimethylsilyl)ethoxy)methyl)-1H-purine-2,6(3H,7H)-dione (8.9 g, 92.8%) as yellow oil. LCMS retention time 1.368 min, LCMS MH⁺-28 315.

### Step 3 2-(1,3-dimethyl-2,6-dioxo-7-((2-(trimethylsilyl)ethoxy)methyl)-2,3,6,7-tetrahydro-1H-purin-8-ylthio)-N-(1-hydroxybutan-2-yl)acetamide

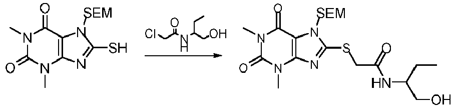

To a solution of 8-mercapto-1,3-dimethyl-7-((2-(trimethylsilyl)ethoxy)methyl)-1H-purine-2,6(3H,7H)-dione (3.5 g, 11.2mmol) in DMF (15 mL) was added 2-chloro-N-(1-hydroxybutan-2-yl)acetamide (2.6 g, 15.7mmol), sodium iodide(20mg) and potassium carbonate (2.8g, 20.3mmol). The mixture was stirred at 80 °C for 16 h. The mixture was diluted with ethyl acetate and water, and the layers were separated. The organic layer was dried over sodium sulfate, filtered and concentrated to give crude product, then purified by silica gel chromatography eluting with DCM/ methanol = 50 : 1 to 40 :1 to give 2-(1,3-dimethyl-2,6-dioxo-7-((2-(trimethylsilyl)ethoxy)methyl)-2,3,6,7-tetrahydro-1H-purin-8-ylthio)-N-(1-hydroxybutan-2-yl)acetamide(2.3 g, 47.7%) as yellow solid. LCMS retention time 1.241, LCMS MH⁺ 472.

### Step 4 2-(1,3-dimethyl-2,6-dioxo-7-((2-(trimethylsilyl)ethoxy)methyl)-2,3,6,7-tetrahydro-1H-purin-8-ylthio)-N-(1-oxobutan-2-yl)acetamide

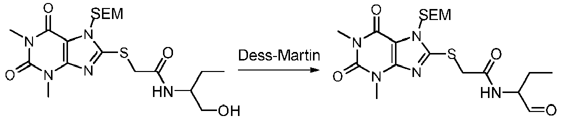

To a solution of 2-(1,3-dimethyl-2,6-dioxo-7-((2-(trimethylsilyl)ethoxy)methyl)-2,3,6,7-tetrahydro-1H-purin-8-ylthio)-N-(1-hydroxybutan-2-yl)acetamide (2.3g, 4.9mmol) in DCM (30 mL) was added Dess-Martin periodinane (3.0g, 7.1mmol) at 0 °C under N₂ atmosphere. The mixture was stirred at room temperature for 16 h. The mixture was diluted with DCM and water, washed with saturated aqueous sodium bicarbonate and the organic layers were separated. The organic layer was dried over sodium sulfate, filtered and concentrated to give crude product, then purified by chromatography eluting with DCM/ methanol (50 : 1 to 40 :1) to give 2-(1,3-dimethyl-2,6-dioxo-7-((2-(trimethylsilyl)ethoxy)methyl)-2,3,6,7-tetrahydro-1H-purin-8-ylthio)-N-(1-oxobutan-2-yl)acetamide (2.2 g, 96.1%) as yellow solid. LCMS retention time 1.138 min, LCMS MH⁺ 470.

### Step 5 8-((4-ethyloxazol-2-yl)methylthio)-1,3-dimethyl-1H-purine-2,6(3H,7H)-dione

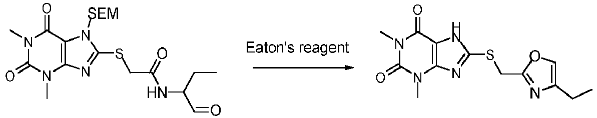

2-(1,3-dimethyl-2,6-dioxo-7-((2-(trimethylsilyl)ethoxy)methyl)-2,3,6,7-tetrahydro-1H-purin-8-ylthio)-N-(1-oxobutan-2-yl)acetamide (1g, 2.1mmol) and Eaton's reagent mixed together , then heated to 80°C with stirring for 16 h. The mixture was diluted with ethyl acetate and water and the phases were separated. The organic phase was washed with saturated aqueous sodium bicarbonate and the organic layers were separated. The organic layer was dried over sodium sulfate, filtered and concentrated to give crude product, then purified by silica gel chromatography eluting with DCM/ methanol = 50 : 1 to 40 :1 to give 8-((4-ethyloxazol-2-yl)methylthio)-1,3-dimethyl-1H-purine-2,6(3H,7H)-dione (400 mg, 58.7%) as yellow solid. LCMS retention time 0.389 min, LCMS MH⁺ 322.

### Intermediate 3 1,3-dimethyl-8-(propoxymethyl)-1H-purine-2,6(3H,7H)-dione

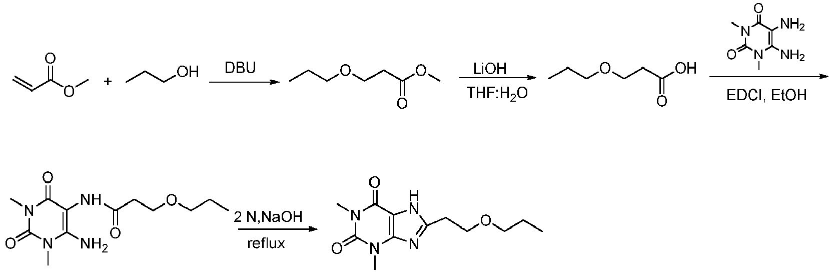

### Step 1 methyl 3-propoxypropanoate (GG-000317-008)

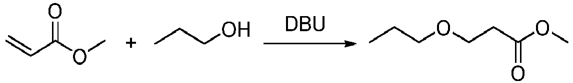

To a solution of methyl acrylate (4.75 g, 55 mmol) in propan-1-ol (15 mL,200mmol) was added DBU (0.418 g, 2.75 mmol) dropwise at room temperature. The resulting mixture was stirred overnight. The solution was diluted with ethyl acetate, washed with aq. NH₄Cl. The organic layers were combined, dried over sodium sulfate, and concentrated to afford methyl 3-propoxypropanoate (6.0 g, 75%)as a light yellow oil.

### Step 2 3-propoxypropanoic acid

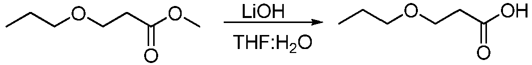

To a solution of methyl 3-propoxypropanoate (5.4 g, 37 mmol, product of intermediate 3 step 1) in THF/water (10mL/10 mL) was added lithium hydroxide (2.33 g, 55 mmol) portionwise at room temperature. The resulting mixture was stirred overnight. THF was evaporated, the residue was treated with conc. HCl to adjust the pH to 1-2.Then it was partitioned between ethyl acetate and water, the organic layers were combined, dried over sodium sulfate concentrated to afford 3-propoxypropanoic acid (3.5 g, 71%)as a colorless oil.

### Step 3 N-(6-amino-1,3-dimethyl-2,4-dioxo-1,2,3,4-tetrahydropyrimidin-5-yl)-3-propoxypropanamide

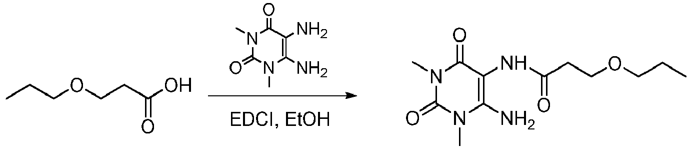

To a solution of 5,6-diamino-1,3-dimethylpyrimidine-2,4(1H,3H)-dione (5 g, 29.4 mmol,) and 3-propoxypropanoic acid (3.9 g, 29.4 mmol, product of intermediate 3 step 2)in ethanol (20 mL) was added EDCI (5.7 g, 29.4 mmol) The mixture was stirred at room temperature for 2 h. Then ethanol was evaporated , the residue was partitioned between chloroform: propan-2-ol (3:1) and water, the aqueous phase was washed with chloroform: propan-2-ol (3:1) for several times, the organic layers were combined, dried over sodium sulfate, and concentrated to give N-(6-amino-1,3-dimethyl-2,4-dioxo-1,2,3,4-tetrahydropyrimidin-5-yl)-3-propoxypropanamide (7.0 g, 83%) as a light yellow solid. LCMS retention time 0.345 min, LCMS MH⁺ 285.

### Step 4 1,3-dimethyl-8-(propoxymethyl)-1H-purine-2,6(3H,7H)-dione

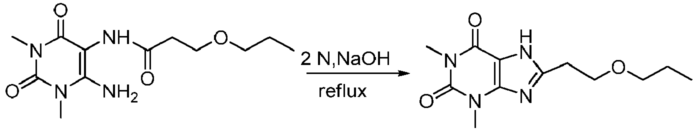

To a suspension of N-(6-amino-1,3-dimethyl-2,4-dioxo-1,2,3,4-tetrahydropyrimidin-5-yl)-3-propoxypropanamide (7.0 g, 24.7 mmol, product of intermediate 3 step 3) in water (10 mL) was added sodium hydroxide (3.0 g, 73.9 mmol).The mixture was refluxed at 100 °C for 2 h. Then it was cooled to 0°C, conc.HCl was added to adjust the pH to 4-5, some solid formed. The mixture was filtered, the residue was washed with water, dried in vacuo to afford 1,3-dimethyl-8-(propoxymethyl)-1H-purine-2,6(3H,7H)-dione (4.5 g, 68%) as a light yellow solid. LCMS retention time 0.379 min, LCMS MH⁺ 267.

### Intermediate 4 1,3-dimethyl-8-(3-(trifluoromethyl)phenoxy)-1H-purine-2,6(3H,7H)-dione

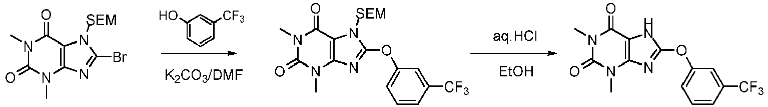

### Step 1 1,3-dimethyl-8-(3-(trifluoromethyl)phenoxy)-7-((2-(trimethylsilyl)ethoxy)methyl)-1H-purine-2,6(3H,7H)-dione

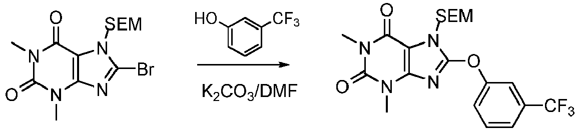

To a solution of the 8-bromo-1,3-dimethyl-7-((2-(trimethylsilyl)ethoxy)methyl)-1H-purine-2,6(3H,7H)-dione (5 g, 12.9 mmol, Intermediate 2 Step 1 product) in DMF(20 mL) was added potassium carbonate (2.7 g, 19.6 mmol). After stirring for 10 min at room temperature 3-(trifluoromethyl)phenol (2.5 g, 15.4 mmol) was added at room temperature. Then the resulting mixture was stirred at 70 °C for 2 h. The mixture was concentrated and filtered. The filter cake was washed with ethanol twice, dried in vacuo to give 1,3-dimethyl-8-(3-(trifluoromethyl)phenoxy)-7-((2-(trimethylsilyl)ethoxy)methyl)-1H-purine-2,6(3H,7H)-dione (4.5 g, 74.4 %) as white solid. LCMS retention time 2.117 min, LCMS MH⁺ 471.

### Step 2 1,3-dimethyl-8-(3-(trifluoromethyl)phenoxy)-1H-purine-2,6(3H,7H)-dione

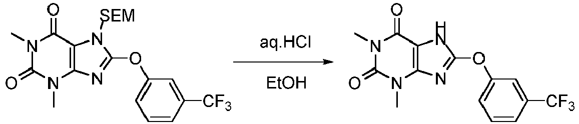

To a solution of 1,3-dimethyl-8-(3-(trifluoromethyl)phenoxy)-7-((2-(trimethylsilyl)ethoxy)methyl)-1H-purine-2,6(3H,7H)-dione (4.5 g, 9.6 mmol) in ethanol (40 mL) was added HCl (16 ml, 36% , w/w%). The mixture was stirred at 80 °C for 16 h. The mixture was concentrated and filtered. The filter cake was washed with water twice, dried in vacuo to give product 1,3-dimethyl-8-(3-(trifluoromethyl)phenoxy)-1H-purine-2,6(3H,7H)-dione(3.16 g, 96 %) as white solid. LCMS retention time 1.434 min, LCMS MH⁺ 341.

### Intermediate 5 1,3-dimethyl-8-(3-(trifluoromethoxy)phenoxy)-1H-purine-2,6(3H,7H)-dione

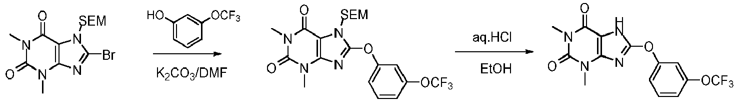

### Step 1 1,3-dimethyl-8-(3-(trifluoromethoxy)phenoxy)-7-((2-(trimethylsilyl)ethoxy)methyl)-1H-purine-2,6(3H,7H)-dione

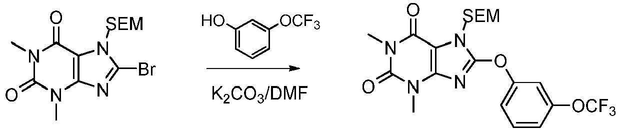

The title compound was prepared using the method of intermediate 4 step 1 to give 1,3-dimethyl-8-(3-(trifluoromethoxy)phenoxy)-7-((2-(trimethylsilyl)ethoxy)methyl)-1H-purine-2,6(3H,7H)-dione (0.62 g, 50.5 %) as white solid. LCMS retention time 2.133 min, LCMS MH⁺ 487.

### Step 2 1,3-dimethyl-8-(3-(trifluoromethoxy)phenoxy)-1H-purine-2,6(3H,7H)-dione

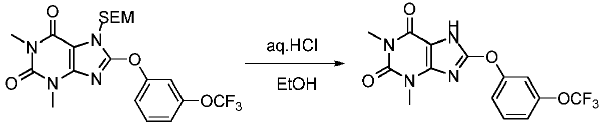

The title compound was prepared using the method of intermediate 4 step 2 to give 1,3-dimethyl-8-(3-(trifluoromethoxy)phenoxy)-1H-purine-2,6(3H,7H)-dione (0.22 g, 47.7%) as white solid. LCMS retention time 1.457 min, LCMS MH⁺ 357.

### Intermediate 6 8-bromo-7-(4-chlorobenzyl)-1,3-dimethyl-1H-purine-2,6(3H,7H)-dione

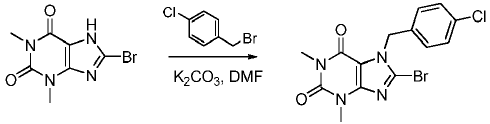

The title compound was prepared using the method of example 1 step 2 to give 8-bromo-7-(4-chlorobenzyl)-1,3-dimethyl-1H-purine-2,6(3H,7H)-dione (7.6 g, 89.1% yield) as white solid. LCMS retention time 1.791 min, LCMS MH⁺ 383.

### Intermediate 7 8-bromo-7-(4-chlorophenethyl)-1,3-dimethyl-1H-purine-2,6(3H,7H)-dione

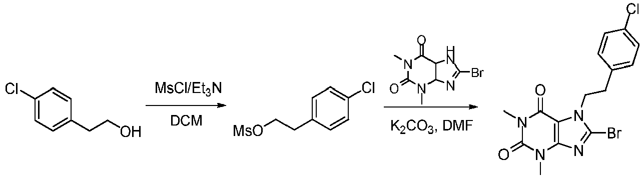

### Step 1 4-chlorophenethyl methanesulfonate

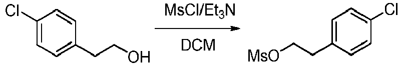

To a solution of 2-(4-chlorophenyl)ethanol (500 mg, 3.21 mmol) and TEA (650 mg, 6.43 mmol) in DCM (5 mL) was added methanesulfonyl chloride (548 mg, 4.81 mmol) at 0 °C. The resulting mixture was stirred at room temperature overnight. The mixture was diluted with DCM and water, and the phases were separated. The organic phase was washed with brine dried over sodium sulfate, filtered and concentrated to give product 4-chlorophenethyl methanesulfonate (570 mg, 75.6%) as white solid. ¹*H*-NMR (CDCl₃) δ 7.31-7.33 (d, 2H), 7.18-7.20 (d, 2H), 4.40-4.43 (t, 2H), 3.03-3.06(t, 2H), 2.91 (s, 3H).

### Step 2 8-bromo-7-(4-chlorophenethyl)-1,3-dimethyl-1H-purine-2,6(3H,7H)-dione

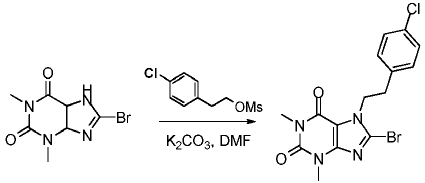

The title compound was prepared using the method of example 1 step 2 to give 8-bromo-7-(4-chlorophenethyl)-1,3-dimethyl-1H-purine-2,6(3H,7H)-dione (1.63 g, 90.9% yield) as white solid. LCMS retention time 1.519 min; LCMS MH⁺ 397.

### Intermediate 8 2-bromo-2-methylpropanal

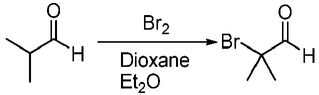

To a solution of isobutyraldehyde (9.5 mL, 0.1 mol) in ethoxyethane was added dioxane (0.38 mL); then bromine (0.11mL) was added. The mixture was cooled to 0°C, another bromine (5mL) was added. The resulting solution was stirred at this temperature for 20 min. The reaction was poured to ice-water (50 mL), then sodium bicarbonate (6.0 g) was added portionwise. The organic layer was separated, dried over sodium sulfate, filtered and concentrated to afford 2-bromo-2-methylpropanal (4.0 g, 27%) as yellow oil that was used without further purification.

### Intermediate 9 2-bromo-N-(1-hydroxybutan-2-yl)-2-methylpropanamide

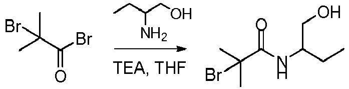

To a solution of 2-aminobutan-1-ol (1.28 g, 14.3 mmol) in THF (30 mL) was added TEA (2.6 g, 26.1 mmol). The mixture was cooled to -30 °C and 2-bromo-2-methylpropanoyl bromide (3 g, 13.05 mmol) dropwise. The resulting mixture was stirred at room temperature for 16 h. The mixture was quenched with brine (20 mL) and extracted with ethyl acetate. The organic layer was washed with saturated aqueous sodium bicarbonate, dried and concentrated to give 2-bromo-N-(1-hydroxybutan-2-yl)-2-methylpropanamide (2.2 g, 70.8%) as yellow solid. ¹*H*-NMR (DMSO-*d₆*) δ 6.83 (w, 1H), 3.82-3.85 (m, 1H), 3.61-3.73 (m, 2H), 1.99 (s, 6H), 1.64-1.71 (m, 1H), 1.51-1.57 (m, 1H), 0.96-0.99 (t, 3H).

### Intermediate 10 1,3-dimethyl-8-phenethyl-1H-purine-2,6(3H,7H)-dione

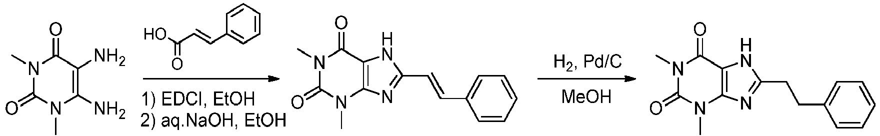

### Step 1 (E)-1,3-dimethyl-8-styryl-1H-purine-2,6(3H,7H)-dione

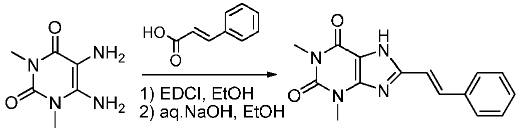

To a solution of 5,6-diamino-1,3-dimethylpyrimidine-2,4(1H,3H)-dione (300 mg, 1.76 mmol) in ethanol (5 mL) was added cinnamic acid (394 mg, 2.65 mmol) followed by EDCI (508 mg, 2.65 mmol). The resulting mixture was stirred at room temperature for 2 h. The mixture was concentrated to dryness and the residue was taken up in ethyl acetate This organic phase was washed with saturated aqueous ammonium chloride, and brine, dried and concentrated to give N-(6-amino-1,3-dimethyl-2,4-dioxo-1,2,3,4-tetrahydropyrimidin-5-yl)cinnamamide (370 mg) as yellow solid that was used without characterization. To a solution of N-(6-amino-1,3-dimethyl-2,4-dioxo-1,2,3,4-tetrahydropyrimidin-5-yl)cinnamamide (370 mg, 1.23 mmol) in ethanol (5 mL) was added 1N aqueous sodium hydroxide solutions (1.85 mL, 1.85 mmol). The resulting mixture was heated to 80 °C for 2 h. The mixture was concentrated and the residue was taken up in ethyl acetate This organic phase was washed with water, dried and concentrated to give crude product, which was collected by filtration and washed with ethanol to give (E)-1,3-dimethyl-8-styryl-1H-purine-2,6(3H,7H)-dione (230 mg, 46.3% yield) as white solid. LCMS MH⁺ 283.

### Step 2 1,3-dimethyl-8-phenethyl-1H-purine-2,6(3H,7H)-dione

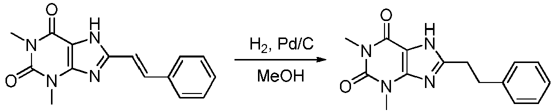

To a solution of (E)-1,3-dimethyl-8-styryl-1H-purine-2,6(3H,7H)-dione (230 mg, 0.815 mmol) in methanol (5 mL) was added 10% Pd/C (20 mg). The mixture was stirred under 50 psi of hydrogen at room temperature for 2 h. The mixture was filtered and the filtrate was concentrated to give 1, 3-dimethyl-8-phenethyl-1H-purine-2,6(3H,7H)-dione (230 mg, 100% yield) as yellow syrup. LCMS MH⁺ 285.

### Intermediate 11 ethyl 3-(1,3-dimethyl-2,6-dioxo-2,3,6,7-tetrahydro-1H-purin-8-yl)propanoate

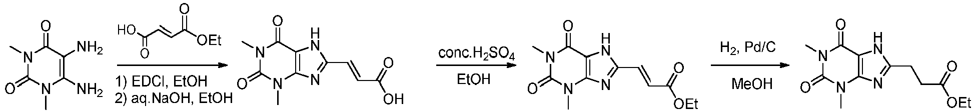

### Step 1 (E)-1,3-dimethyl-8-styryl-1H-purine-2,6(3H,7H)-dione

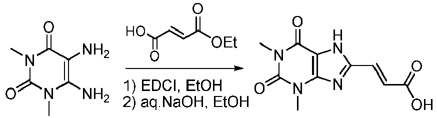

The title compound was prepared using the method of intermediate 10, step 1 to give (E)-1,3-dimethyl-8-styryl-1H-purine-2,6(3H,7H)-dione (210 mg, 25.67% yield) as brown solid. LCMS MH⁺ 251.

### Step 2 (E)-ethyl 3-(1,3-dimethyl-2,6-dioxo-2,3,6,7-tetrahydro-1H-purin-8-yl)acrylate

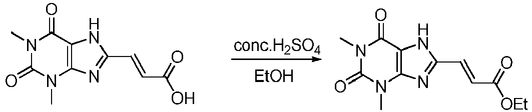

To a solution of (E)-3-(1,3-dimethyl-2,6-dioxo-2,3,6,7-tetrahydro-1H-purin-8-yl)acrylic acid (210 mg, 0.84 mmol) in ethanol (3 mL) was added concentrated sulfuric acid (0.2 mL). The resulting mixture was stirred at 80 °C 16 h. The mixture was cooled to room temperature and water 5 mL was added. The product precipitated from the mixture. The was collected and dried in vacuo to give (E)-ethyl 3-(1,3-dimethyl-2,6-dioxo-2,3,6,7-tetrahydro-1H-purin-8-yl)acrylate (190 mg, 81.4% yield) as yellow solid. LCMS MH⁺ 279.

### Step 3 ethyl 3-(1,3-dimethyl-2,6-dioxo-2,3,6,7-tetrahydro-1H-purin-8-yl)propanoate

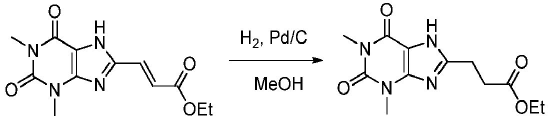

The title compound was prepared using the method of intermediate 10, step 2 to give ethyl 3-(1,3-dimethyl-2,6-dioxo-2,3,6,7-tetrahydro-1H-purin-8-yl)propanoate (190 mg, 99% yield) as yellow solid. LCMS MH⁺ 281.

### Intermediate 12 ethyl 3-(1,3-dimethyl-2,6-dioxo-2,3,6,7-tetrahydro-1H-purin-8-yl)but-3-enoate

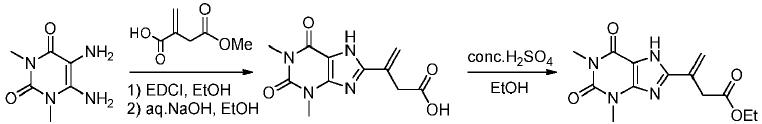

### Step 1 3-(1,3-dimethyl-2,6-dioxo-2,3,6,7-tetrahydro-1H-purin-8-yl)but-3-enoic acid

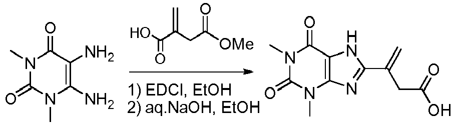

The title compound was prepared using the method of intermediate 10, step 1 to give 3-(1,3-dimethyl-2,6-dioxo-2,3,6,7-tetrahydro-1H-purin-8-yl)but-3-enoic acid (550 mg, 35.4% yield) as brown solid. LCMS MH⁺ 265.

### Step 2 ethyl 3-(1,3-dimethyl-2,6-dioxo-2,3,6,7-tetrahydro-1H-purin-8-yl)but-3-enoate

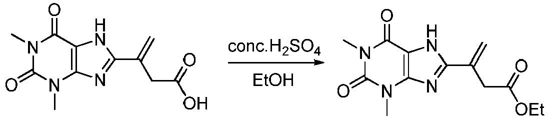

The title compound was prepared using the method of intermediate 11, step 2 to give ethyl 3-(1,3-dimethyl-2,6-dioxo-2,3,6,7-tetrahydro-1H-purin-8-yl)but-3-enoate (210 mg, 70.8%) as yellow solid. LCMS MH⁺ 293.

### Intermediate 13 ethyl 4-(1,3-dimethyl-2,6-dioxo-2,3,6,7-tetrahydro-1H-purin-8-yl)butanoate

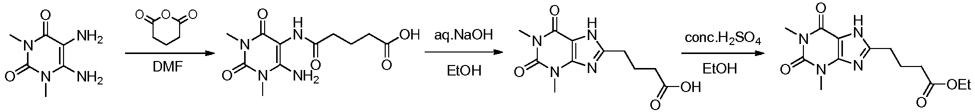

### Step 1 5-(6-amino-1,3-dimethyl-2,4-dioxo-1,2,3,4-tetrahydropyrimidin-5-ylamino)-5-oxopentanoic acid

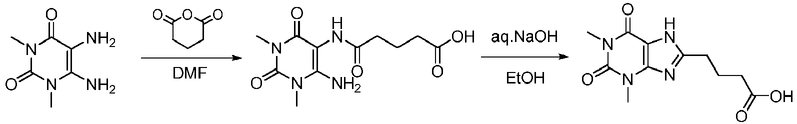

To a solution of 5,6-diamino-1,3-dimethylpyrimidine-2,4(1H,3H)-dione (0.6 g, 3.5 mmol) in DMF (5 mL) was added dihydro-2H-pyran-2,6(3H)-dione (0.4 g, 3.5 mmol). The mixture was stirred at room temperature for 16 h. The mixture was concentrated to give the crude uncyclized amide. To a solution of the amide intermediate in ethanol (6 mL) was added 1N aqueous sodium hydroxide (5.25 mL, 5.25 mmol) and the mixture was stirred at 80 °C for 12 h. The mixture was cooled and neutralized with 1N HCl. The product precipitated from the mixture and was filtered. The filter cake was washed with water and dried in vacuo to give 5-(6-amino-1,3-dimethyl-2,4-dioxo-1,2,3,4-tetrahydropyrimidin-5-ylamino)-5-oxopentanoic acid (0.68 g, 66% yield) as light yellow solid. LCMS MH⁺ 267.

### Step 2 ethyl 4-(1,3-dimethyl-2,6-dioxo-2,3,6,7-tetrahydro-1H-purin-8-yl)butanoate

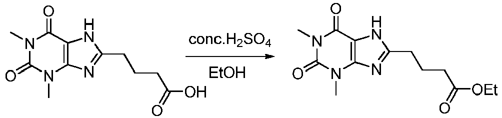

The title compound was prepared using the method of intermediate 11, step 2 to give 4-(1,3-dimethyl-2,6-dioxo-2,3,6,7-tetrahydro-1H-purin-8-yl)butanoate (0.69 g, 96%) as brown solid. LCMS MH⁺ 295.

### Intermediate 14 8-(hydroxymethyl)-1,3-dimethyl-1H-purine-2,6(3H,7H)-dione

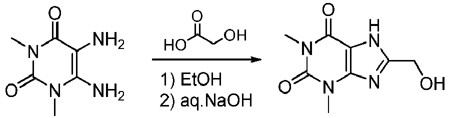

A stirred mixture of 5,6-diamino-1,3-dimethylpyrimidine-2,4(1H,3H)-dione (1.5 g, 8.8 mmol) and glycolic acid (1.5 g, 19.7 mmol) was heated to 100 °C. The mixture melted and then solidified. ethanol (10 mL) was added and the mixture was stirred at 100 °C for 2 h. The mixture was cooled and diluted with ethanol (10 mL). To the resulting mixture, aqueous sodium hydroxide (20 mL, 2N, 40 mmol) was added, and the mixture was heated to 80 °C for 3 h. The mixture was cooled to room temperature and neutralized with acetic acid. Then it was diluted with water and extracted with ethyl acetate. The organic layer was concentrated to dryness to give 8-(hydroxymethyl)-1,3-dimethyl-1H-purine-2,6(3H,7H)-dione (1.1 g, 59.5% yield) as brown solid. LCMS MH⁺ 211.

### Intermediate 15 8-(3-bromobenzyl)-1,3-dimethyl-1H-purine-2,6(3H,7H)-dione

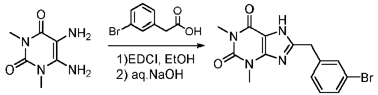

The title compound was prepared using the method of intermediate 10, step 1 to give 8-(3-bromobenzyl)-1,3-dimethyl-1H-purine-2,6(3H,7H)-dione (367 mg, 85.3% yield) as yellow solid. LCMS retention time 1.361 min; LCMS MH⁺ 349.

### Intermediate 16 2-(chloromethyl)-5-methylthiazole

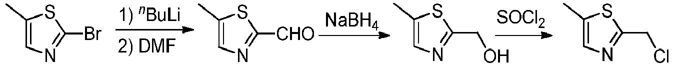

### Step 1 (5-methylthiazol-2-yl)methanol

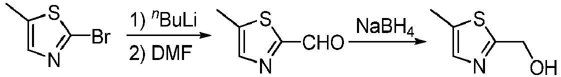

To a solution of n-BuLi (8.4 ml, 13.48 mmol) in THF (30 mL) was added 2-bromo-5-methylthiazole(2.0 g, 11.23 mmol) dropwise under a nitrogen atmosphere at -70 °C ; then it was stirred at this temperature for 1.5 h. DMF (1.3 ml, 16.85 mmol) was added dropwise under nitrogen atmosphere at -70 °C. The resulting mixture was stirred at this temperature for 1 h. Then the mixture was quenched with aqueous saturated ammonium chloride (5 mL), the mixture was partitioned between ethyl acetate and water. The organic layers were combined, dried over sodium sulfate, filtered and concentrated to give a yellow oil. The yellow oil was dissolved in methanol (15 ml), sodium borohydride (512 mg, 13.48 mmol) was added portionwise under a nitrogen atmosphere at -60 °C. The mixture was stirred at this temperature for 1 h. The reaction mixture was quenched with acetone and concentrated. The residue was partitioned between ethyl acetate and water. The organic layers were dried over sodium sulfate, filtered and concentrated, then purified by silica gel chromatography eluting with petroleum/ ethyl acetate= 3:1 to give thiazol-2-ylmethanol (1.3 g, 90.3%) as brown oil. LCMS MH⁺ 130.

### Step 2 2-(chloromethyl)-5-methylthiazole

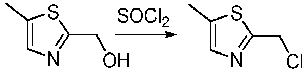

To a solution of (5-methylthiazol-2-yl)methanol (0.5 g, 3.87 mmol)in DCM (5 mL) was added thionyl chloride (0.19 ml, 2.6 mmol) at 0 °C, then the mixture was stirred at room temperature for 2 h. The solvent was concentrated to give 2-(chloromethyl)thiazole (570 mg) which was without purification.

### Intermediate 17 2-(3-(trifluoromethoxy)phenoxy)ethanol

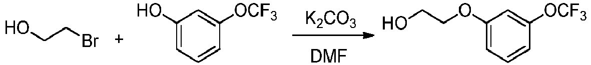

To a solution of 3-(trifluoromethoxy)phenol(3 g, 16.8 mmol) in DMF (30 mL) was added 2-bromoethanol (3.16 g, 25.3 mmol), potassium carbonate(4.65 g, 33.7 mmol). The mixture was heated at 80 °C overnight. The mixture was cooled, partitioned between ethyl acetate and water. The organic layers were combined, dried over sodium sulfate, filtered and concentrated to give a crude product which was purified by a column chromatography eluting with petroleum ether/methanol (60:1 to 30:1) to give 2-(3-(trifluoromethoxy)phenoxy)ethanol (3.5 g, 94.5 % yield) as a yellow oil. ¹*H*-NMR ¹*H*-NMR (DMSO-*d₆*) δ 7.28-7.30 (t, 1H), 6.81-6.86 (m, 2H), 6.78 (s, 1H), 4.07-4.09 (t, 2H), 3.95-3.99 (m, 2H), 2.58 (t, 1H).

### Intermediate 18 ethyl 3-((,3-dimethyl-2,6-dioxo-2,3,6,7-tetrahydro-1H-purin-8-yl)methyl)benzoate

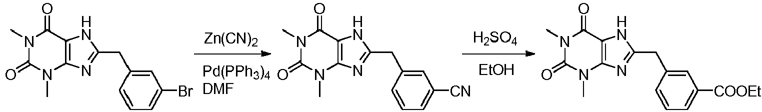

### Step 1 3-((1,3-dimethyl-2,6-dioxo-2,3,6,7-tetrahydro-1H-purin-8-yl)methyl)benzonitrile

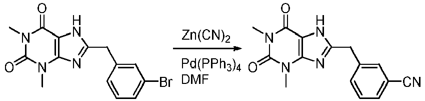

To a mixture of 8-(3-bromobenzyl)-1,3-dimethyl-1H-purine-2,6(3H,7H)-dione (250 mg, 0.72 mmol, intermediate 15) and zinc cyanide (101 mg, 0.86 mmol) in degassed DMF was added tetrakis(triphenylphosphine)palladium (44.5 mg, 0.035 mmol) under nitrogen atmosphere. The mixture was subjected to microwave irradiation at 160 °C for 45 min in a sealed tube. The mixture was diluted with ethyl acetate and washed with saturated aqueous ammonium chloride, Then the organic phase was dried and concentrated to give crude product, which was purified via silica gel chromatography eluting with DCM/methanol (40:1) to give 3-((1,3-dimethyl-2,6-dioxo-2,3,6,7-tetrahydro-1H-purin-8-yl)methyl)benzonitrile (130 mg, 61.5%) as yellow solid. LCMS MH⁺ 295.

### Step 2 ethyl 3-((1,3-dimethyl-2,6-dioxo-2,3,6,7-tetrahydro-1H-purin-8-yl)methyl)benzoate

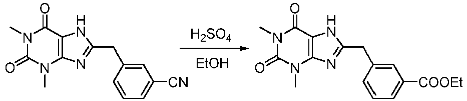

To a solution of 3-((1,3-dimethyl-2,6-dioxo-2,3,6,7-tetrahydro-1H-purin-8-yl)methyl)benzonitrile (130 mg, 0.44 mmol) in ethanol (3 mL) was added concentrated sulfuric acid (0.5 mL). The resulting mixture was stirred at 80 °C 16 h. The mixture was cooled to room temperature and water 5 mL was added. The product precipitated from the mixture. The mixture was filtered and the solid product was dried under vacuum to give ethyl 3-((1,3-dimethyl-2,6-dioxo-2,3,6,7-tetrahydro-1H-purin-8-yl)methyl)benzoate (110 mg, 73% yield) as light yellow solid. LCMS MH⁺ 343.

### Intermediate 19 1-(4-(bromomethyl)phenyl)ethanone

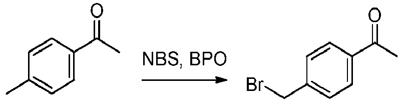

To a solution ofl-p-tolylethanone(600 mg, 4.47 mmol) in carbon tetrachloride 15 mL) was added N-bromosuccinimide (955 mg, 5.37 mmol) and BPO (31mg, 0.13 mmol). The mixture was refluxed for 3 h. Then it was cooled, and filtered. The filtrate was concentrated to give 1-(4-(bromomethyl)phenyl)ethanone (683 mg, 71%) as a brown oil. LCMS MH⁺ 213.

### Intermediate 20 2-(3-(4-methyloxazol-2-yl)phenyl)acetic acid

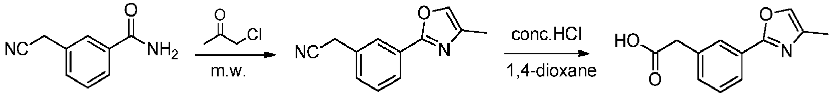

### Step 1 2-(3-(4-methyloxazol-2-yl)phenyl)acetonitrile

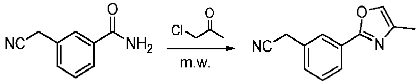

A solution of 3-(cyanomethyl)benzamide (200 mg, 1.25 mmol) in 1-chloropropan-2-one (2 mL) was microwave irradiated at 120 °C for 20 min in a sealed tube. The mixture was concentrated to give crude product (110 mg), which was directly used to the next reation without purification. LCMS MH⁺ 199.

### Step 2 2-(3-(4-methyloxazol-2-yl)phenyl)acetic acid

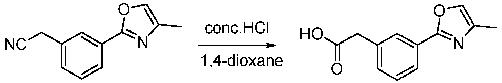

To a solution of 2-(3-(4-methyloxazol-2-yl)phenyl)acetonitrile (110 mg, 0.555 mmol) in 1,4-dioxane (3 mL) was added hydrochloride acid (1 mL, 6N). Then the mixture was stirred at 80 °C for 2 h. The mixture was concentrated and purified via silica gel chromatography eluted with DCM/ methanol (20 :1) to give 2-(3-(4-methyloxazol-2-yl)phenyl)acetic acid (85 mg, 70.5%) as yellow syrup. LCMS retention time 0.920min, LCMS MH⁺ 218.

### Intermediate 21 1-(4-chlorophenyl)ethyl methanesulfonate (LY-000266-172)

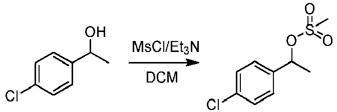

The title compound was prepared using the method of intermediate 7, step 1 to give 1-(4-chlorophenyl)ethyl methanesulfonate (258 mg, 23.6% yield) as yellow oil, which was directly used to the next reaction without purification.

### Intermediate 22 2-(bromomethyl)-5-methylthiazole

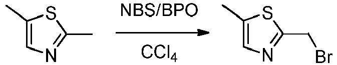

To a solution of 2,5-dimethylthiazole (200 mg, 1.77 mmol) in carbon tetrachloride (5 mL) was added NBS (377 mg, 2.12 mmol), followed by BPO (20 mg, 0.083 mmol). Then the mixture was heated to reflux for 4 h with stirring. The mixture was cooled to room temperature and filtered, the filtrate was concentrated to give crude product 2-(bromomethyl)-5-methylthiazole (260 mg, 76.9%) as yellow oil, which was used without purification.

### Intermediate 23 2-(chloromethyl)thiazole

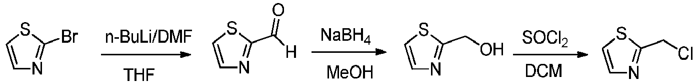

### Step 1 thiazol-2-ylmethanol

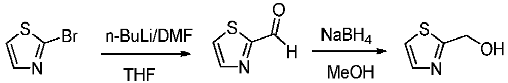

To a solution of n-BuLi (8.4 ml, 1.6 mol/l, 13.4 mmol) in THF (30 mL) was added 2-bromothiazole (377 mg, 2.12 mmol) dropwise under nitrogen atmosphere at -70 °C, and the mixture was stirred at the temperature for 1 h. Then DMF (1.4 ml, 18.3 mmol) was added into the solution dropwise under nitrogen atmosphere at -70 °C. The resulting mixture was stirred at the temperature for 1 h. Then the mixture was quenched with aqueous saturated ammonium chloride, diluted with ethyl acetate and water, and the phases were separated. The organic phase was washed with brine, dried over sodium sulfate, filtered and concentrated to give yellow oil. The yellow oil was dissolved in methanol (15 ml), cooled to -60 °C, and sodium borohydride (463 mg , 12.2 mmol) was added portionwise under nitrogen atmosphere. The mixture was stirred at the temperature for 1 h. The reaction was quenched with acetone and concentrated. The residue was diluted with ethyl acetate and water, and the phases were separated. The organic layer was dried over sodium sulfate, filtered and concentrated, then purified by silica gel chromatography eluting with petroleum/ ethyl acetate= 3:1 to give thiazol-2-ylmethanol (230 mg, 16.4 % yield) as brown oil. LCMS MH⁺ 116.

### Step 2 2-(chloromethyl)thiazole

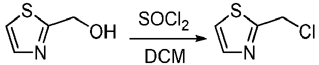

To a solution of thiazol-2-ylmethanol (230 mg, 2.0 mmol)in DCM (5 mL) was added thionyl chloride (0.19 ml, 2.6 mmol) at 0 °C and the mixture was stirred at room temperature for 1 h. The mixture was diluted with DCM and water, and the phases were separated. The organic layer was dried over sodium sulfate, filtered and concentrated to give 2-(chloromethyl)thiazole (240 mg, crude), which was used without purification.

### Intermediate 24 5-(chloromethyl)-2-methylpyrimidine

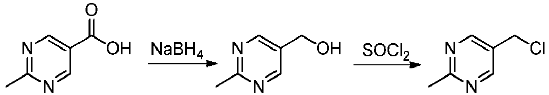

### Step 1 (2-methylpyrimidin-5-yl)methanol

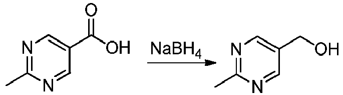

To a solution of 2-methylpyrimidine-5-carboxylic acid (150 mg, 1.23 mm01) in ethanol (5 mL) was added sodium borohydride (93 mg, 2.46 mmol). The mixture was stirred at room temperature for 3 h. It was quenched with aqueous HCl (2 N, 2 mL), extracted with DCM, dried over sodium sulfate, filtered and concentrated give the yellow oil product (2-methylpyrimidin-5-yl)methanol (95 mg, 62.6%). LCMS MH⁺ 125.

### Step 2 5-(chloromethyl)-2-methylpyrimidine

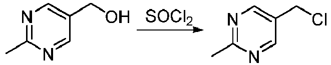

A solution of (2-methylpyrimidin-5-yl)methanol (95 mg, 0.77 mmol) in thionyl chloride(1 mL) was stirred at room temperature for 1 h. The mixture was concentrated to dryness and used without purification. LCMS MH⁺ 143.

### Intermediate 25 3-(4-methyloxazol-2-yl)phenol

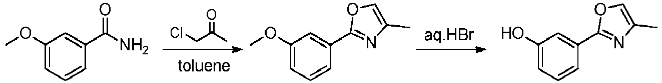

### Step 1 2-(3-methoxyphenyl)-4-methyloxazole

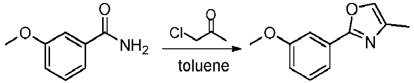

To a solution of 3-methoxybenzamide (1.5 g, 9.9 mmol) in toluene (15 mL) was added 1-chloropropan-2-one (1.37 g, 14.9 mmol), and the mixture was stirred at reflux for 16 h. The mixture was cooled and concentrated to give crude product, which was purified via silica gel chromatography eluted with ethyl acetate/petroleum ether (1: 5) to give 2-(3-methoxyphenyl)-4-methyloxazole (1.21 g, 64.6% yield) as yellow syrup. LCMS MH⁺ 190.

### Step 2 3-(4-methyloxazol-2-yl)phenol

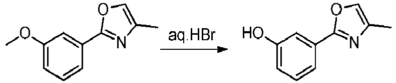

A solution of 2-(3-methoxyphenyl)-4-methyloxazole (1.1 g, 5.79 mmol) in aqueous hydrogen bromide (10 mL, 48% w/w) was heated to 100 °C for 16 h. The mixture was concentrated to give 3-(4-methyloxazol-2-yl)phenol (0.97 g, crude), which was directly used to the next reaction without purification. LCMS MH⁺ 176.

### Intermediate 26 8-bromo-7-(3-methoxybenzyl)-1,3-dimethyl-1H-purine-2,6(3H,7H)-dione

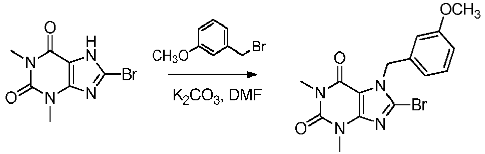

The title compound was prepared using the method of intermediate 1 step 2 to give 8-bromo-7-(3-methoxybenzyl)-1,3-dimethyl-1H-purine-2,6(3H,7H)-dione (165 mg, 78.9% yield) as off white solid. LCMS retention time 1.967min; LCMS MH⁺ 379.

### Intermediate 27 3-(morpholinomethyl)phenol

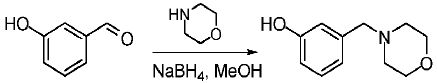

To a solution of 3-hydroxybenzaldehyde (1 g, 8.19 mmol) in methanol (15 mL) was added morpholine (1.42 g, 16.4 mmol). Then the mixture was stirred at room temperature for 1 h. The mixture was cooled to -5 °C, and sodium borohydride (403 mg, 10.6 mmol) was added in small portions. The resulting mixture was stirred at room temperature for 4 h. The mixture was quenched with diluted hydrochloride acid and concentrated. The aqueous solution was washed with ethyl acetate and then made basic by addition of ammonium hydroxide. The basic aqueous slurry was extracted with ethyl acetate and this organic layer was dried and concentrated to give 3-(morpholinomethyl)phenol (0.76 g, 48.2%) as white solid. LCMS retention time 0.871 min, LCMS MH⁺ 194.

### Intermediate 28a and 28b 2-chloro-5-hydroxybenzaldehyde (28a) and 4-chloro-3-hydroxybenzaldehyde (28b)

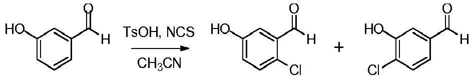

To a solution of 3-hydroxybenzaldehyde (1 g, 10 mmol) in acetonitrile (50 mL) was added p-toluenesulfonic acid (3.4 g, 20 mmol) portionwise. The mixture was stirred at room temperature for 5 min, and NCS (1.33 g, 10 mmol) was added and the resulting mixture was stirred at room temperature for 2 h. The mixture was quenched with aqueous sodium thiosulfate, and diluted with ethyl acetate and brine. The organic layer was separated, dried, and concentrated to give the crude products which were purified by silica gel chromatography eluting with petroleum ether /ethyl acetate (10:1 to 5:1) to give 2-chloro-5-hydroxybenzaldehyde (340 mg, 21.7% yield intermediate 28a) as yellow solid; ¹*H*-NMR ¹*H*-NMR (CDCl₃) δ 10.43 (s, 1H), 7.54-7.56 (d, 1H), 7.30-7.37 (m, 2H) and then 4-chloro-3-hydroxybenzaldehyde (310 mg, 19.7% yield, intermediate 28b) as yellow solid. ¹*H*-NMR ¹*H*-NMR (CDCl₃) δ 10.43 (s, 1H), 7.40 (d, 1H), 7.33 (d, 1H), 7.06-7.09 (dd, 1H).

### Intermediate 29 4-chloro-3-(morpholinomethyl)phenol

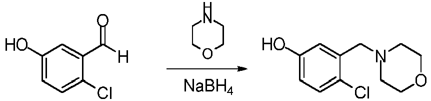

To a mixture of 2-chloro-5-hydroxybenzaldehyde (200 mg, 1.27 mmol, intermediate 28a) and morpholine (280 mg, 3.21 mmol) in methanol (10 mL) was added 2 drops of acetic acid and the mixture was stirred at room temperature for 2 h. To the mixture, sodium borohydride (97 mg, 2.56 mmol) was added and the resulting mixture was stirred at room temperature for 4 h. The mixture was quenched with dilute hydrochloride acid and concentrated to give crude product. This crude material was purified by silica gel chromatography eluting with petroleum ether/ethyl acetate (5:1 to 1:1) to give 4-chloro-3-(morpholinomethyl)phenol (150 mg, 51.8% yield) as white solid. LCMS retention time 0.378; LCMS MH⁺ 228.

### Intermediate 30 4-chloro-3-(morpholinomethyl)phenol

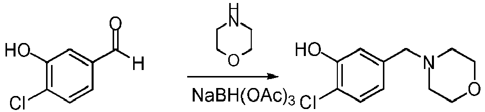

The title compound was prepared using the method of intermediate 29 starting with intermediate 28b but using sodium triacetoxyborohydride as the reducing agent to give 4-chloro-3-(morpholinomethyl)phenol (190 mg, 68.1% yield) as yellow solid. LCMS retention time 0.347; LCMS MH⁺ 228.

### Intermediate 31 1,3-dimethyl-8-(3-(trifluoromethyl)phenylamino)-1H-purine-2,6(3H,7H)-dione

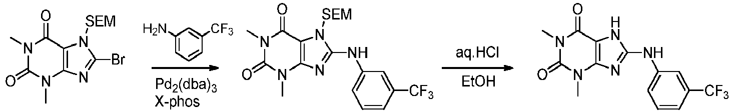

### Step 1 1,3-dimethyl-8-(3-(trifluoromethyl)phenylamino)-7-((2-trimethylsilyl)ethoxy)methyl)-1H-purine-2,6(3H,7H)-dione

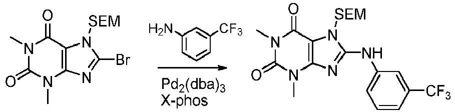

To a mixture of 8-bromo-1,3-dimethyl-7-((2-(trimethylsilyl)ethoxy)methyl)-1H-purine-2,6(3H,7H)-dione (300 mg, 0.77 mmol, intermediate 2 step 1 product) and 3-(trifluoromethyl)aniline (149 mg, 0.92 mmol) in degassed toluene was added potassium tert-butoxide (129.6 mg, 1.15 mmol) followed by tris(dibenzylideneacetone)dipalladium (35.2 mg, 0.038 mmol) and X-phos (18.3 mg, 0.038 mmol) under nitrogen. The resulting mixture was stirred at 120 °C for 16 h under nitrogen. The mixture was diluted with ethyl acetate and washed with brine. The organic phase was concentrated to give crude product, which was purified via silica gel chromatography eluting with DCM/methanol (80:1) to give 1,3-dimethyl-8-(3-(trifluoromethyl)phenylamino)-7-((2-(trimethylsilyl)ethoxy)methyl)-1H-purine-2,6(3H,7H)-dione (259 mg, 71.6%) as yellow solid. LCMS retention time 1.792 min, LCMS MH⁺ 470.

### Step 2 1,3-dimethyl-8-(3-(trifluoromethyl)phenylamino)-1H-purine-2,6(3H,7H)-dione

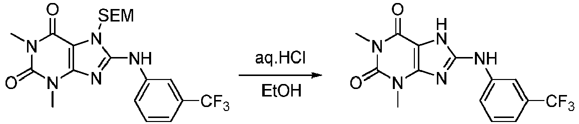

To a solution of 1,3-dimethyl-8-(3-(trifluoromethyl)phenylamino)-7-((2-(trimethylsilyl)ethoxy)methyl)-1H-purine-2,6(3H,7H)-dione (259 mg, 0.551 mmol) in ethanol (5 mL) was added hydrochloride acid (2 mL, 6N). The mixture was stirred at 80 °C for 16 h. The mixture was concentrated and the product precipitated from the mixture. The slurry was filtered and the filter cake was washed with water twice then dried under vacuum to give 1,3-dimethyl-8-(3-(trifluoromethyl)phenylamino)-1H-purine-2,6(3H,7H)-dione (149 mg, 79.7% yield) as white solid. LCMS retention time 1.271 min, LCMS MH⁺ 340.

### Intermediate 32 1-(3-(bromomethyl)phenyl)ethanone

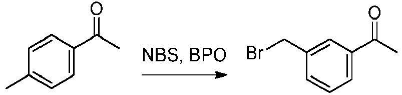

The title product was prepared using the method of intermediate 19. LCMS MH⁺ 213.

### Intermediate 33 Diethyl 2-(7-(3-bromobenzyl)-1,3-dimethyl-2,6-dioxo-2,3,6,7-tetrahydro-1H-purin-8-yl)malonate

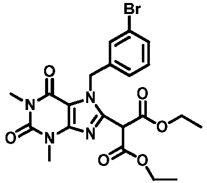

Sodium hydride (60% in oil, 3.36 g, 83.94 mmol) was slurried in DMF (110 mL) cooled in an ice bath and diethyl malonate (15.9 mL, 104.9 mmol) was carefully added slowly drop wise. The reaction was allowed to warm to room temperature, stirred for 20 min then 7-(3-bromobenzyl)-8-chloro-1,3-dimethyl-1*H*-purine-2,6(3*H*,7*H*)-dione (16.1 g, 41.97 mmol) was added. The reaction was heated at 100°C for 4 h yielding a clear light golden solution. The reaction was cooled to room temperature, diluted with water (1L) and extracted with ethyl acetate (2 x 200 mL). The aqueous layer was cooled in an ice bath and enough 2N aqueous HCl was added until PH = 1 and a white solid formed. Solid was filtered, washed with water (2 x 100 mL), diethyl ether (2 x 100 mL) air dried for 15 h to give diethyl 2-(7-(3-bromobenzyl)-1,3-dimethyl-2,6-dioxo-2,3,6,7-tetrahydro-1H-purin-8-yl)malonate (13.6 g, 64 % yield) as a white solid. LCMS retention time = 3.506 min and 96% pure, LCMS MH⁺ 507.

### Intermediate 34 Ethyl 3-(1,3-dimethyl-2,6-dioxo-2,3,6,7-tetrahydro-1H-purin-8-yl)2-methylpropanoate and Ethyl 3-(1,3-dimethyl-2,6-dioxo-2,3,6,7-tetrahydro-1H-purin-8-yl)butanoate

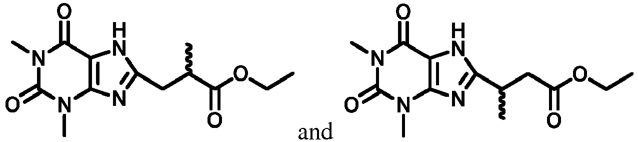

### Step 1 4-((6-Amino-1,3-dimethyl-2,4-dioxo-1,2,3,4-tetrahydropyrimidin-5-yl)amino)-3-methyl-4-oxobutanoic acid and 4-((6-Amino-1,3-dimethyl-2,4-dioxo-1,2,3,4-tetrahydropyrimidin-5-yl)amino)-2-methyl-4-oxobutanoic acid

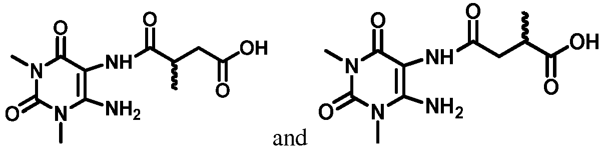

5,6-Diamino-1,3-dimethylpyrimidine-2,4(1H,3H)-dione (7.46 g, 43.82 mmol) and 3-methyldihydrofuran-2,5-dione (5.0 g , 43.82 mmol) were combined in DMF (15 mL). The reaction was stirred at room temperature for 30 min then ethyl acetate (150 mL) was added. The tan solid that precipitated was collected and washed with ethyl acetate (2 x 50 mL) and high vacuum dried for 6 h to give a mixture of 4-((6-amino-1,3-dimethyl-2,4-dioxo-1,2,3,4-tetrahydropyrimidin-5-yl)amino)-3-methyl-4-oxobutanoic acid and 4-((6-amino-1,3-dimethyl-2,4-dioxo-1,2,3,4-tetrahydropyrimidin-5-yl)amino)-2-methyl-4-oxobutanoic acid (10.1 g, 81% yield) as a tan solid that were used without purification.

### Step 2 3-(1,3-Dimethyl-2,6-dioxo-2,3,6,7-tetrahydro-1H-purin-8-yl)-2-methylpropanoic acid and 3-(1,3-Dimethyl-2,6-dioxo-2,3,6,7-tetrahydro-1H-purin-8-yl)butanoic acid

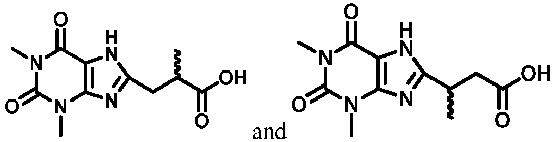

A mixture of 4-((6-Amino-1,3-dimethyl-2,4-dioxo-1,2,3,4-tetrahydropyrimidin-5-yl)amino)-3-methyl-4-oxobutanoic acid and 4-((6-amino-1,3-dimethyl-2,4-dioxo-1,2,3,4-tetrahydropyrimidin-5-yl)amino)-2-methyl-4-oxobutanoic acid (2.0 g, 7.04 mmol) were dissolved in 2N sodium hydroxide (18mL)) and heated at 85°C for 3 h. The reaction was cooled in an ice bath and 2N HCl was added until pH = 2. Then 2 N sodium hydroxide was added to bring the mixture to pH to 6. The reaction was concentrated under reduced pressure. The residues were diluted with ethanol (100 mL) and filtered. The filtrate containing a mixture of 3-(1,3-dimethyl-2,6-dioxo-2,3,6,7-tetrahydro-1*H*-purin-8-yl)-2-methylpropanoic acid and 3-(1,3-dimethyl-2,6-dioxo-2,3,6,7-tetrahydro-1*H*-purin-8-yl)butanoic acid was used directly in the next step.

### Step 3 Ethyl 3-(1,3-dimethyl-2,6-dioxo-2,3,6,7-tetrahydro-1H-purin-8-yl)2-methylpropanoate and Ethyl 3-(1,3-dimethyl-2,6-dioxo-2,3,6,7-tetrahydro-1H-purin-8-yl)butanoate

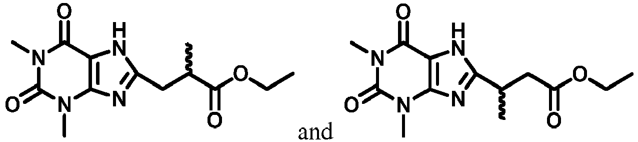

To a mixture of 3-(1,3-dimethyl-2,6-dioxo-2,3,6,7-tetrahydro-1H-purin-8-yl)-2-methylpropanoic acid and 3-(1,3-dimethyl-2,6-dioxo-2,3,6,7-tetrahydro-1H-purin-8-yl)butanoic acid (1.87 g, 7.02 in ethanol (100 mL) was added 2N HCl in diethyl ether (3 mL) was added and the reaction was refluxed for 15 h. The reaction was concentrated under reduced pressure to give a mixture of ethyl 3-(1,3-dimethyl-2,6-dioxo-2,3,6,7-tetrahydro-1*H*-purin-8-yl)2-methylpropanoate and ethyl 3-(1,3-dimethyl-2,6-dioxo-2,3,6,7-tetrahydro-1*H*-purin-8-yl)butanoate (2.15 g, >100% yield) as a golden semi-solid. LCMS retention time = 2.595 min and 95% purity, LCMS MH⁺ 295.

### Intermediate 35 8-Chloro-7-(4-chlorobenzyl)-1,3-dimethyl-1H-purine-2,6(3H,7H)-dione

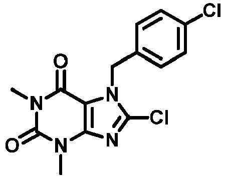

8-Chloro-1,3-dimethyl-1*H*-purine-2,6(3*H*,7*H*)-dione (10.0 g, 46.6 mmol), potassium carbonate (9.66 g, 69.9 mmol) and 1-(bromomethyl)-4-chlorobenzene (10.5 g, 51.3 mmol) were combined in DMF (150 mL) and heated at 70°C for 24 h. The reaction was cooled to room temperature, diluted with water (600 mL) and extracted with ethyl acetate (3 x 200 mL). The combined extracts were dried with magnesium sulfate, filtered and evaporated under reduced pressure to give 8-chloro-7-(4-chlorobenzyl)-1.3-dimethyl-1H-purine-2,6(3H,7H)-dione (15.8 g, 100% yield) as a white solid. LCMS retention time = 3.453 min and 96% purity, LCMS MH⁺ 339.

### Intermediate 36 7-(4-Chlorobenzyl)-8-(3-hydroxymethyl)phenoxy) 1,3-dimethyl-1H-purine-2,6(3H,7H)-dione

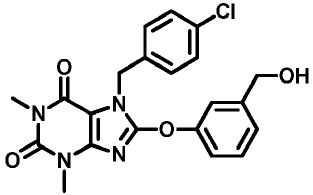

8-Chloro-7-(4-chlorobenzyl)-1,3-dimethyl-1*H*-purine-2,6(3*H*,7*H*)-dione (2.0 g, 5.90 mmol), potassium carbonate (2.45 g, 17.70 mmol) and 3-(hydroxymethyl)phenol (0.77 g, 6.20 mmol) were combined in DMF (20 mL) and heated at 70°C for 15 h. The reaction was cooled to room temperature, diluted with water (200 mL) and extracted with ethyl acetate (2 x 150 mL). The combined extracts were washed with 1N lithium chloride (2 x 100 mL) dried with magnesium sulfate, filtered and evaporated under reduced pressure to give 7-(4-chlorobenzyl)-8-(3-hydroxymethyl)phenoxy)1,3-dimethyl-1*H*-purine-2,6(3*H*,7*H*)-dione (2.5 g, 100% yield) as a white solid. LCMS retention time = 3.360 min and 88% purity, LCMS MH⁺ 427.

### Intermediate 37 7-(4-Chlorobenzyl)-8-(3-chloromethyl)phenoxy)-1,3-dimethyl-1H-purine-2,6(3H,7H)-dione

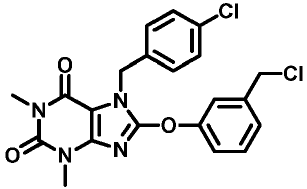

7-(4-Chlorobenzyl)-8-(3-hydroxymethyl)phenoxy)-1,3-dimethyl-1*H*-purine-2,6(3*H*,7*H*)-dione (2.5 g, 1.07 mmol, intermediate 36) was dissolved in thionyl chloride (20 mL) and heated at reflux for 15 h. The reaction was evaporated under reduced pressure, azeotroped with toluene (2 x 100 mL) to give 7-(4-chlorobenzyl)-1,3-dimethyl-8-(3-chloromethyl)phenoxy)1,3-dimethyl-1*H*-purine-2,6(3*H*,7*H*)-dione (2.53 g, 98% yield) as a yellow solid. LCMS retention time = 4.126 min and 90% purity, LCMS MH⁺ 445.

### Intermediate 38 7-(3-Chlorobenzyl)-8-(3-chloromethyl)phenoxy)-1,3-dimethyl-1H-purine-2,6(3H,7H)-dione

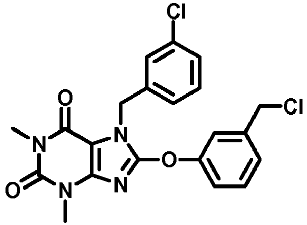

The title compound was prepared using the methods of intermediates 36 and 37. Golden solid. LCMS retention time = 4.126 min and 90% purity, LCMS MH⁺ 445.

### Intermediate 39 7-(3-Bromobenzyl)-8-chloro-1,3-dimethyl-1H-purine-2,6(3H,7H)-dione

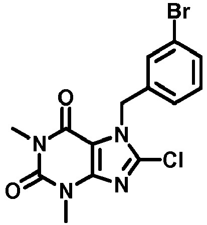

8-Chloro-1,3-dimethyl-1H-2,6(3H,7H)-dione (1.0 g, 4.66 mmol), 1-bromo-3-(bromomethyl)benzene (1.74 g, 6.99 mmol) and potassium carbonate (0.97 g, 6.99 mmol) were combined in DMF (20 mL); then heated at 70°C for 18 h. The reaction was cooled and poured into water (75 mL) cooled in an ice bath. After stirring for 15 minutes a white solid formed. The solid was collected and washed with water (10 ml), air dried 30 min, high vacuumed dried 15 h to give 7-(3-bromobenzyl)-8-chloro-1,3-dimethyl-1*H*-purine-2,6(3*H*,7*H*)-dione (1.69 g, 94 % yield) as a white solid. LCMS retention time = 3.355 min and 99% purity, LCMS MH⁺ 385. ¹*H* NMR (DMSO-d₆) δ 7.48-7.54 (m, 2H), 7.31 (t, 1H, J = 8 Hz), 7.23 (d, 1H, J = 8 Hz)), 5.52 (s, 2H), 3.38 (s, 3H), 3.21 (s, 3H).

### Intermediate 40 Synthesis of 2-(chloromethyl)-4-methylthiazole

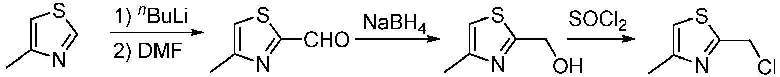

### Step 1 Synthesis of (4-methylthiazol-2-yl)methanol

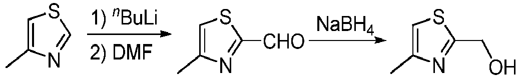

To a solution of 4-methylthiazole (1.0 g, 10.1 mmol) in tetrahydrofuran (30 mL) was added n-BuLi (7.56 mL, 13.48 mmol) dropwise under a nitrogen atmosphere at -60 °C. The reaction was stirred for 1 hour; then DMF (1.4 ml, 18.2 mmol) was added dropwise while maintaining -60 °C. The resulting mixture was stirred at this temperature for 30 min. The reaction was quenched with aqueous saturated ammonium chloride (5 mL). The mixture was partitioned between ethyl acetate and water. The combined organic layer was dried over sodium sulfate, filtered and concentrated to give a yellow oil. This yellow oil was dissolved in methanol (15 ml) and sodium borohydride (460 mg, 12.1 mmol) was added portionwise under nitrogen atmosphere at -60 °C. The mixture was stirred at this temperature for 1 hour. The reaction mixture was quenched with acetone, warmed to room temperature, and concentrated. The residue was partitioned between ethyl acetate and water. The combined organic layer was dried over sodium sulfate, filtered and concentrated. The residue was purified by silica gel chromatography eluting with petroleum ether/ ethyl acetate (3:1) to give thiazol-2-ylmethanol (1.3 g, 90.3%) as brown oil. LCMS retention time 0.375 min; LCMS MH⁺ 130.

### Step 2 Synthesis of 2-(chloromethyl)-4-methylthiazole

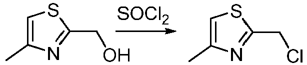

To a solution of (4-methylthiazol-2-yl)methanol (0.5 g, 3.87 mmol) in DCM (5 mL) was added thionyl chloride (0.19 ml, 2.6 mmol) at 0 °C. The mixture was stirred at room temperature for 2 hour. The reaction was concentrated to give 2-(chloromethyl)thiazole (570 mg, crude) as yellow oil, which was used without purification. LCMS retention time 0.895 min; LCMS MH⁺ 148

### Intermediate 41 2-(chloromethyl)-5-methylpyrazine

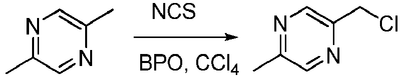

To a solution of 2,5-dimethylpyrazine (500 mg, 4.62 mmol) in carbon tetrachloride (7 mL) was added NCS (679 mg, 5.09 mmol) followed by BPO (20 mg) and the mixture was heated to 80 °C for 6 hours. The mixture was diluted with DCM and extracted with saturated aqueous sodium sulfite solution and brine. The organic layer was dried and concentrated to give a crude product which was purified via silica gel chromatography eluting with petroleum ether/ ethyl acetate (1:0 to 15:1) to give 2-(chloromethyl)-5-methylpyrazine (133 mg, 20.19% yield) as yellow oil. LCMS retention time 0.557 min; LCMS MH⁺ 143.

### Example 1 7-(3-bromobenzyl)-1,3-dimethyl-8-(2-oxobutylthio)-1H-purine-2,6(3H,7H)-dione (Compound 1)

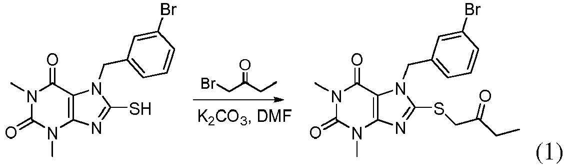

To a solution of 7-(3-bromobenzyl)-8-mercapto-1,3-dimethyl-1H-purine-2,6(3H,7H)-dione (150 mg, 0.393 mmol, Intermediate 1) in DMF (3 mL) was added 1-bromobutan-2-one(59.4 mg, 0.393 mmol) followed by potassium carbonate (65.2 mg, 0.472 mmol). The mixture was stirred at room temperature for 4 h. The mixture was diluted with ethyl acetate and water, and the layers were separated. The organic layer was dried over sodium sulfate, filtered and concentrated to give crude product, which was washed with ethanol and dried under vacuum to give 7-(3-bromobenzyl)-1,3-dimethyl-8-(2-oxobutylthio)-1H-purine-2,6(3H,7H)-dione (90 mg, 50.7%) as white solid. ¹*H*-NMR (DMSO-d₆) δ 7.53 (s, 1H), 7.52 (d, 1H), 7.34 (t, 1H), 7.26 (d, 1H), 5.46 (s, 2H), 4.32 (s, 2H), 3.38 (s, 3H), 3.21 (s, 3H), 2.63 (m, 2H), 0.97 (t, 3H). LCMS retention time 2.653 min; LCMS MH⁺ 453.

### Example 2a Tert-butyl 2-(7-(3-bromobenzyl)-1,3-dimethyl-2,6-dioxo-2,3,6,7-tetrahydro-1H-purin-8-ylthio)propanoate (Compound 2)

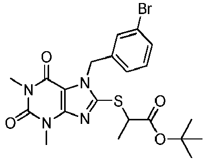

The title compound was prepared using the method of example 1 to give 110mg (54.9% yield) as white solid. ¹*H*-NMR (DMSO-d₆) δ 7.52 (d, 1H), 7.50 (s, 1H), 7.33 (t, 1H), 7.22 (d, 1H), 5.48 (d, 2H), 4.35 (m, 2H), 3.42 (s, 3H), 3.22 (s, 3H), 1.50 (d, 3H), 1.36 (s, 9H). LCMS retention time 3.195 min; LCMS MH⁺ 511.

### Example 2b Tert-butyl 2-(7-(3-bromobenzyl)-1,3-dimethyl-2,6-dioxo-2,3,6,7-tetrahydro-1H-purin-8-ylthio)-2-methylpropanoate (Compound 3)

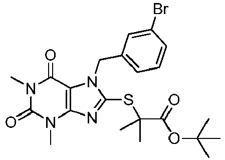

The title compound was prepared using the method of example 1 except the temperature was 80 °C to give 96 mg (46.7% yield) as white solid. ¹*H*-NMR (DMSO-*d₆*) δ 7.50 (d, 1H), 7.46 (s, 1H), 7.31 (t, 1H), 7.18 (d, 1H), 5.52 (s, 2H), 3.42 (s, 3H), 3.22 (s, 3H), 1.58 (s, 6H), 1.35 (s, 9H). LCMS retention time 3.322 min; LCMS MH⁺ 525.

### Example 2c Tert-butyl 2-(7-(3-bromobenzyl)-1,3-dimethyl-2,6-dioxo-2,3,6,7-tetrahydro-1H-purin-8-ylthio)acetate (Compound 4)

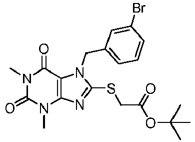

The title compound was prepared using the method of example 1 to give 56 mg (43.1% yield) as white solid. ¹*H*-NMR (DMSO-d₆) δ 7.54(d, 1H), 7.52 (s, 1H), 7.33 (t, 1H), 7.28 (d, 1H), 5.46 (s, 2H), 4.08 (s, 2H), 3.40 (s, 1H), 3.22 (s, 3H), 1.38 (s, 9H). LCMS retention time 3.937 min; LCMS MH⁺ 497.

### Example 2d Ethyl 2-(7-(3-bromobenzyl)-1,3-dimethyl-2,6-dioxo-2,3,6,7-tetrahydro-1H-purin-8-ylthio)-2-methylpropanoate (Compound 9)

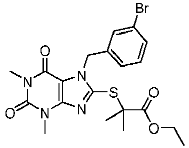

The title compound was prepared using the method of example 2b to give 90 mg (48.8% yield) as white solid. ¹*H*-NMR (DMSO-*d₆*) δ 7.49 (d, 1H), 7.47 (s, 1H), 7.29 (t, 1H), 7.14 (d, 1H), 5.49 (s, 2H),4.05-4.10 (m, 2H), 3.38 (s, 3H), 3.19 (s, 3H), 1.57 (s, 6H), 1.10 (t, 3H). LCMS retention time 3.036 min; LCMS MH⁺ 497.

### Example 2e 7-(3-bromobenzyl)-1,3-dimethyl-8-(pyridin-3-ylmethylthio)-1H-purine-2,6(3H,7H)-dione (Compound 10)

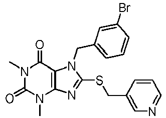

The title compound was prepared using the method of example 1 except the temperature was 60 °C to give 36 mg (19.4% yield) as white solid. ¹*H*-NMR (DMSO-d₆) δ 8.90 (s, 1H), 8.72 (d, 1H), 8.38 (d, 1H), 7.79-7.83(m, 1H), 7.50 (d, 1H), 7.42 (s, 1H), 7.26 (t, 1H), 7.11 (d, 1H), 5.40 (s, 2H), 4.64 (s, 2H), 3.47(s, 3H), 3.20 (s, 3H). LCMS retention time 2.374 min; LCMS MH⁺ 472.

### Example 2f 7-(3-bromobenzyl)-1,3-dimethyl-8-(pyridin-4-ylmethylthio)-1H-purine-2,6(3H,7H)-dione (Compound 11)

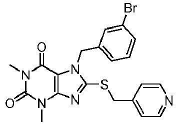

The title compound was prepared using the method of example 1 except the temperature was 60 °C to give 90 mg (48.5% yield) as white solid. ¹*H*-NMR (DMSO-*d₆*) δ 8.77 (d, 2H), 7.95 (d, 2H), 7.50 (d, 1H), 7.41 (s, 1H), 7.28 (t, 1H), 7.12 (d, 1H), 5.42 (s, 2H), 4.71 (s, 2H), 3.49 (s, 3H), 3.21 (s, 3H). LCMS retention time 2.103 min; LCMS MH⁺ 472.

### Example 2g 7-(3-bromobenzyl)-1,3-dimethyl-8-(pyridin-2-ylmethylthio)-1H-purine-2,6(3H,7H)-dione (Compound 12)

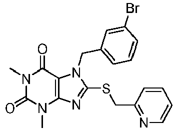

The title compound was prepared using the method of example 1 except the temperature was 60 °C to give 121 mg (65.2% yield) as white solid.¹*H*-NMR (DMSO-*d₆*) δ 8.65 (d, 1H), 8.04 (t, 1H), 7.70 (d, 1H), 7.49-7.55 (m, 2H), 7.46 (s, 1H), 7.29 (t, 1H), 7.16 (d, 1H), 5.43 (s, 2H), 4.73 (s, 2H), 3.43 (s, 3H), 3.21 (s, 3H). LCMS retention time 2.649 min; LCMS MH⁺ 472.

### Example 2h 7-(3-bromobenzyl)-1,3-dimethyl-8-(2-oxopentylthio)-1H-purine-2,6(3H,7H)-dione (Compound 13)

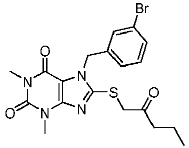

The title compound was prepared using the method of example 1 except the temperature was 60 °C to give 86 mg (47.0% yield) as white solid. ¹*H*-NMR (DMSO-d₆) δ 7.54 (s, 1H), 7.52 (d, 1H), 7.32 (t, 1H), 7.26 (d, 1H), 5.47 (s, 2H), 4.32 (s, 2H), 3.39 (s, 3H), 3.21 (s, 3H), 2.59 (s, 2H), 1.53 (q, 2H), 0.86 (t, 3H). LCMS retention time 2.837 min; LCMS MH⁺ 467.

### Example 3 7-(3-bromobenzyl)-8-(3-hydroxy-2-methylhexan-2-ylthio)-1,3-dimethyl-1H-purine-2,6(3H,7H)-dione (Compound 122)

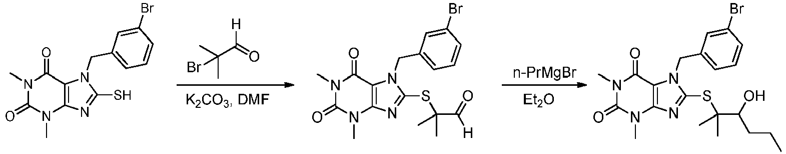

### Step 1 2-(7-(3-bromobenzyl)-1,3-dimethyl-2,6-dioxo-2,3,6,7-tetrahydro-1H-purin-8-ylthio)-2-methylpropanal

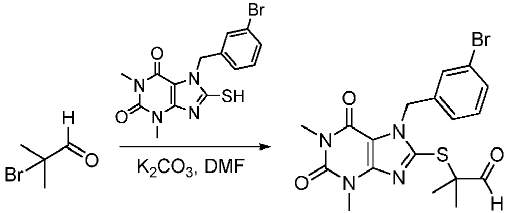

To a solution of 7-(3-bromobenzyl)-1,3-dimethyl-8-thioxo-8,9-dihydro-1H-purine-2,6(3H,7H)-dione (400 mg, 1.05 mmol, Intermediate 1) and 2-bromo-2-methylpropanal (238 mg, 1.57 mmol, intermediate 8) in DMF (5 mL) was added potassium carbonate (217 mg, 1.57 mmol).The mixture was heated at 50°C for 1.5 h. The mixture was cooled to room temperature, and partitioned between ethyl acetate and water. The organic layers were combined, dried over sodium sulfate, filtered and concentrated to afford a crude residue which was purified by flash chromatography eluting with petroleum ether/ethyl acetate (4:1) to give 2-(7-(3-bromobenzyl)-1,3-dimethyl-2,6-dioxo-2,3,6,7-tetrahydro-1H-purin-8-ylthio)-2-methylpropanal (293 mg,62%) as a yellow oil. LCMS retention time 1.626 min; LCMS MH⁺ 451.

### Step 2 7-(3-bromobenzyl)-8-(3-hydroxy-2-methylhexan-2-ylthio)-1,3-dimethyl-1H-purine-2,6(3H,7H)-dione

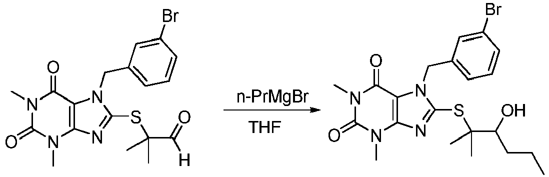

To a solution of 2-(7-(3-bromobenzyl)-1,3-dimethyl-2,6-dioxo-2,3,6,7-tetrahydro-1H-purin-8-ylthio)-2-methylpropanal (260 mg, 0.58 mmol) in THF(10 mL) was added isopropylmagnesium bromide (0.52 mL, 1.16 mmol) dropwise at -78°C.The mixture was stirred at this temperature for 1 h. The reaction was quenched with aqueous ammonium chloride (2 mL); then it was warmed to room temperature, and partitioned between ethyl acetate and water. The organic layers were combined, dried over sodium sulfate, filtered and concentrated to afford a crude residue which was purified by chromatography eluting with petroleum ether/ethyl acetate (4:1) to give 7-(3-bromobenzyl)-8-(3-hydroxy-2-methylhexan-2-ylthio)-1,3-dimethyl-1H-purine-2,6(3H,7H)-dione(150 mg, 53%) as a light yellow solid.¹*H*-NMR (DMSO-*d₆*) *δ* 7.50(d, 1 H), 7.45(s, 1 H), 7.31(t, 1H), 7.17(d, 1 H), 5.57(s, 2 H), 5.09(d, 1H), 3.59(t, 1 H),3.45(s, 3 H), 3.33(s, 3H), 1.55(q, 2 H),1.34(s, 3 H),1.26-1.31(m, 5 H), 0.89(t, 3H). LCMS retention time 2.741 min; LCMS MH⁺ 495

### Example 4 7-(3-bromobenzyl)-1,3-dimethyl-8-(2-methyl-3-oxohexan-2-ylthio)-1H-purine-2,6(3H,7H)-dione (Compound 150)

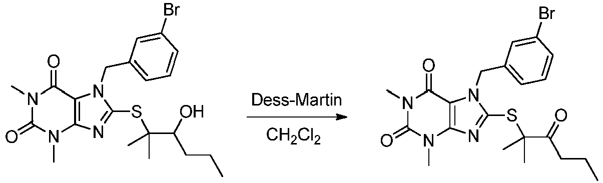

To a solution of 7-(3-bromobenzyl)-8-(3-hydroxy-2-methylhexan-2-ylthio)-1,3-dimethyl-1H-purine-2,6(3H,7H)-dione(50 mg, 0.1 mmol, Product of example 3) in DCM(3 mL) was added Dess-Martin periodinane (90 mg, 0.2 mmol). The mixture was stirred at room temperature for 1.5 h; then it was partitioned between DCM and water. The organic layers were combined, dried over sodium sulfate, and concentrated to afford a crude residue which was purified by preparative-HPLC to give 7-(3-bromobenzyl)-1,3-dimethyl-8-(2-methyl-3-oxohexan-2-ylthio)-1H-purine-2,6(3H,7H)-dione(30 mg, 60%) as a white solid. ¹*H*-NMR (DMSO-*d₆*) *δ* 7.50(d, 1 H), 7.46(s, 1 H), 7.32(t, 1H), 7.19(d, 1 H), 5.51(s, 2 H), 3.36(s, 3 H), 3.21(s, 3 H), 2.79(t, 2 H), 1.48-1.55(m, 8 H), 0.87(t, 3 H). LCMS retention time 2.815; LCMS MH⁺ 493.

### Example 5 7-(3-bromobenzyl)-8-(1-ethoxy-2-methylpropan-2-ylthio)-1,3-dimethyl-1H-purine-2,6(3H,7H)-dione (Compound 56)

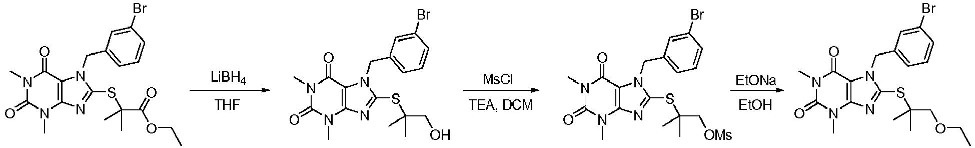

### Step 1 7-(3-bromobenzyl)-8-(1-hydroxy-2-methylpropan-2-ylthio)-1,3-dimethyl-1H-purine-2,6(3H,7H)-dione

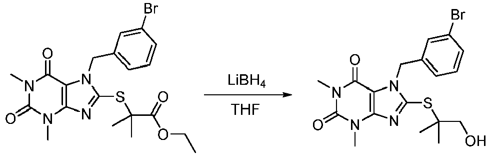

To a solution of ethyl 2-(7-(3-bromobenzyl)-1,3-dimethyl-2,6-dioxo-2,3,6,7-tetrahydro-1H-purin-8-ylthio)-2-methylpropanoate (300 mg, 0.628 mmol, example 2d product) in THF (6 mL) was added lithium borohydride (30 mg, 1.25 mmol) in portions. After addition, the mixture was stirred at room temperature for 3 h. The mixture was quenched with dilute HCl (5 mL, 1N), extracted with ethyl acetate, dried over sodium sulfate and concentrated to give 7-(3-bromobenzyl)-8-(1-hydroxy-2-methylpropan-2-ylthio)-1,3-dimethyl-1H-purine-2,6(3H,7H)-dione (230 mg, 80.8% yield) as yellow solid. LCMS MH⁺ 453

### Step 2 2-(7-(3-bromobenzyl)-1,3-dimethyl-2,6-dioxo-2,3,6,7-tetrahydro-1H-purin-8-ylthio)-2-methylpropyl methanesulfonate

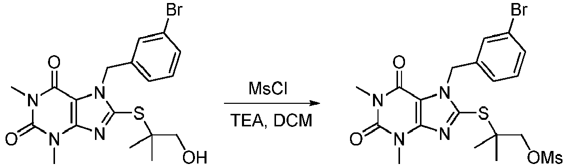

To a solution of 7-(3-bromobenzyl)-8-(1-hydroxy-2-methylpropan-2-ylthio)-1,3-dimethyl-1H-purine-2,6(3H,7H)-dione (100 mg, 0.221 mmol) in anhydrous DCM (3 mL) was added TEA (45 mg, 0.442 mmol) followed by methanesulfonyl chloride (50 mg, 0.442 mmol). The resulting mixture was stirred at room temperature for 2 h. The mixture was diluted with DCM (5 mL), washed with water, dried over sodium sulfate and concentrated to give 2-(7-(3-bromobenzyl)-1,3-dimethyl-2,6-dioxo-2,3,6,7-tetrahydro-1H-purin-8-ylthio)-2-methylpropyl methanesulfonate (79 mg, 67.3% yield) as yellow syrup. LCMS MH⁺ 533.

### Step 3 7-(3-bromobenzyl)-8-(1-ethoxy-2-methylpropan-2-ylthio)-1,3-dimethyl-1H-purine-2,6(3H,7H)-dione

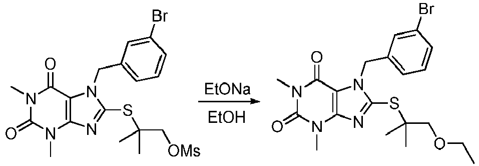

To a solution of 2-(7-(3-bromobenzyl)-1,3-dimethyl-2,6-dioxo-2,3,6,7-tetrahydro-1H-purin-8-ylthio)-2-methylpropyl methanesulfonate (79 mg, 0.149 mmol) in anhydrous ethanol (1 mL) was added sodium (13.7 mg, 0.596 mmol). Then the mixture was heated to 80 °C for 6 h. The mixture was concentrated and purified via preparative HPLC to give 7-(3-bromobenzyl)-8-(1-ethoxy-2-methylpropan-2-ylthio)-1,3-dimethyl-1H-purine-2,6(3H,7H)-dione (31 mg, 43.2%) as white solid. ¹*H*-NMR (DMSO-*d₆*) *δ* 7.52(d, 1 H), 7.43-7.45 (d, 1 H), 7.41-7.43 (d, 1H), 7.19-7.22 (t, 1 H), 5.47(s, 2 H), 3.58(s, 3 H), 3.55 (t, 2H), 3.43 (d, 2H), 3.41(s, 3 H), 1.32 (s, 6H), 1.13-1.16(t, 3H). LCMS retention time 2.815; LCMS MH⁺ 493.

### Example 6 7-(3-bromobenzyl)-8-(2-ethoxyethylthio)-1,3-dimethyl-1H-purine-2,6(3H,7H)-dione (Compound 6)

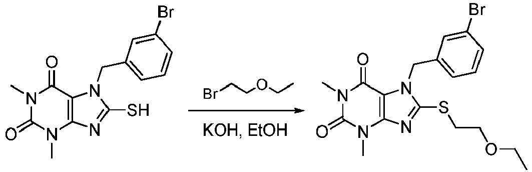

To a solution of 7-(3-bromobenzyl)-8-mercapto-1,3-dimethyl-1H-purine-2,6(3H,7H)-dione (100 mg, 0.262mmol, Intermediate 1) in ethanol (4 mL) was added aqueous potassium hydroxide solution (0.5 mL, 1N) and 1-bromo-2-ethoxyethane (0.05 mL, 0.394 mmol). Then the mixture was heated to reflux for 16 h. The mixture was concentrated to dryness and the residue was dissolved in DCM, washed with brine, dried over sodium sulfate and concentrated to give crude product, which was purified via preparative HPLC to give 7-(3-bromobenzyl)-8-(2-ethoxyethylthio)-1,3-dimethyl-1H-purine-2,6(3H,7H)-dione (65 mg, 54.7% yield) as white solid. ¹*H*-NMR (DMSO-*d₆*) δ 7.51 (d, 1H), 7.49 (s, 1H), 7.32 (t, 1H), 7.22 (d, 1H), 5.45 (s, 2H), 3.64 (t, 2H), 3.41-3.46 (m, 7H), 3.22 (s, 3H), 1.07 (t, 3H). LCMS MH⁺ 455.

### Example 7a 7-(3-bromobenzyl)-8-(2-(2-methoxyethoxy)ethylthio)-1,3-dimethyl-1H-purine-2,6(3H,7H)-dione (Compound 17)

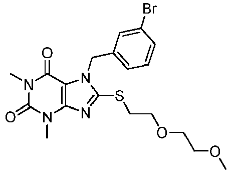

The title compound was prepared using the method of example 6 to give 79 mg (41.4% yield) as white solid. ¹*H*-NMR (DMSO-d₆) δ 7.51 (d, 1H), 7.49 (s, 1H), 7.32 (t, 1H), 7.22 (d, 1H), 5.45 (d, 2H), 3.67 (t, 2H), 3.52 (q, 2H), 3.40-3.44 (m, 7H), 3.22 (s, 3H), 3.21 (s, 3H). LCMS retention time 2.579 min; LCMS MH⁺ 485.

### Example 7b 7-(3-bromobenzyl)-8-(3-methoxypropylthio)-1,3-dimethyl-1H-purine-2,6(3H,7H)-dione (Compound 19)

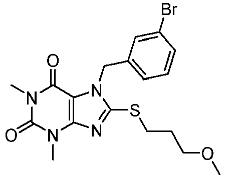

The title compound was prepared using the method of example 6 to give 105 mg (58.6% yield) as white solid. ¹*H*-NMR (DMSO-*d₆*) δ 7.50 (d, 1H), 7.48 (s, 1H), 7.32 (t, 1H), 7.21 (d, 1H), 5.44 (d, 2H), 3.43 (s, 3H), 3.35 (t, 2H), 3.28 (t, 2H), 3.22 (s, 3H), 3.21 (s, 3H).1.88-1.91 (m, 2H). LCMS retention time 2.765 min; LCMS MH⁺ 455.

### Example 8 7-(3-bromobenzyl)-8-(2-ethoxyethylsulfonyl)-1,3-dimethyl-1H-purine-2,6(3H,7H)-dione (Compound 14)

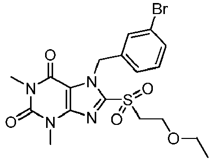

To a solution of 7-(3-bromobenzyl)-8-(2-ethoxyethylthio)-1,3-dimethyl-1H-purine-2,6(3H,7H)-dione (100 mg, 0.22 mmol, product of example 6) in THF/water (4 mL/ 1mL) was added oxone (406.8 mg, 0.66 mmol). The mixture was stirred at room temperature 16 h. The mixture was diluted with ethyl acetate and washed with brine, dried over sodium sulfate and concentrated to give crude product, which was purified via preparative HPLC to give 7-(3-bromobenzyl)-8-(2-ethoxyethylsulfonyl)-1,3-dimethyl-1H-purine-2,6(3H,7H)-dione (66 mg, 61.8%) as white solid. ¹*H*-NMR (DMSO-*d₆*) δ 7.49 (d, 1H), 7.47 (s, 1H), 7.27 (t, 1H), 7.19 (d, 1H), 5.84 (s, 2H), 3.83 (t, 2H),3.73 (t, 2H), 3.44 (s, 3H), 3.26 (q, 2H), 3.21 (s, 3H), 0.84 (t, 3H). LCMS retention time 2.636 min; LCMS MH⁺ 487.

### Example 9 7-(3-bromobenzyl)-8-(2-ethoxyethoxy)-1,3-dimethyl-1H-purine-2,6(3H,7H)-dione (Compound 18)

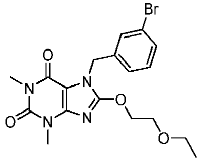

To a solution of 8-bromo-7-(3-bromobenzyl)-1,3-dimethyl-1H-purine-2,6(3H,7H)-dione (100 mg, 0.23 mmol, intermediate 1 Step 2) in 2-ethoxyethanol (2 mL) was added sodium (16.1 mg, 0.70mmol). The mixture was stirred at room temperature for 2 h . The mixture was diluted with ethyl acetate and washed with brine, dried over sodium sulfate and concentrated to give crude product, which was purified via preparative HPLC to give 7-(3-bromobenzyl)-8-(2-ethoxyethoxy)-1,3-dimethyl-1H-purine-2,6(3H,7H)-dione (51 mg, 49.9%) as white solid. ¹*H-*NMR (DMSO-d₆) δ 7.53 (s, 1H), 7.51 (d, 1H), 7.30-7.32 (m, 2H), 5.23 (s, 3H), 5.84 (s, 2H), 4.59 (t, 2H),3.46 (q, 2H), 3.35 (s, 3H), 3.21 (s, 3H), 1.09 (t, 3H). LCMS MH⁺ 437.

### Example 10a 7-(3-bromobenzyl)-8-ethoxy-1,3-dimethyl-1H-purine-2,6(3H,7H)-dione (Compound 28)

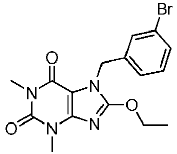

The title compound was prepared using the method of example 9 to give 36 mg (41.6% yield) as white solid. ¹*H*-NMR (DMSO-*d₆*) δ 7.49 (s, 1H), 7.47 (d, 1H), 7.28 (t, 1H), 7.25 (d, 1H), 5.21 (s, 2H), 4.50 (q, 2H), 3.36 (s, 3H), 3.19 (s, 3H), 1.33 (t, 3H). LCMS retention time 2.657 min; LCMS MH⁺ 393.

### Example 10b 7-(3-bromobenzyl)-1,3-dimethyl-8-(2-propoxyethoxy)-1H-purine-2,6(3H,7H)-dione (Compound 99)

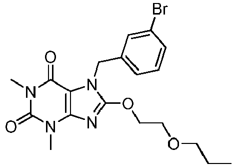

The title compound was prepared using the method of example 9 to give 39 mg (37.1% yield) as white solid. ¹*H*-NMR (DMSO-d₆) δ 7.52 (s, 1H), 7.50 (d, 1H), 7.29-7.31 (m, 2H), 5.22 (s, 2H), 4.56 (t, 2H), 3.72 (t, 2H), 3.37 (s, 3H), 3.20 (s, 3H),1.47 (q, 2H), 0.81 (t, 3H). LCMS retention time 3.055 min; LCMS MH⁺ 451.

### Example 11 8-((4-ethyloxazol-2-yl)methylthio)-1,3-dimethyl-7-(3-(trifluoromethoxy)benzyl)-1H-purine-2,6(3H,7H)-dione (Compound 106)

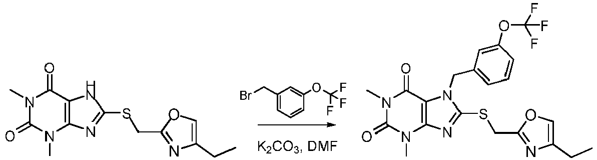

To a solution of 8-((4-ethyloxazol-2-yl)methylthio)-1,3-dimethyl-1H-purine-2,6(3H,7H)-dione (70 mg, 0.218 mmol, Intermediate 2) in DMF (3 mL) was added 1-(bromomethyl)-3-(trifluoromethoxy)-benzene (66 mg, 0.26 mmol) followed by potassium carbonate (60mg, 0.43 mmol). The mixture was stirred at 50°C for 2 h. The mixture was diluted with ethyl acetate and water, and the layers were separated. The organic layer was dried over sodium sulfate, filtered and concentrated to give crude product which was purified by preparative HPLC to give 8-((4-ethyloxazol-2-yl)methylthio)-1,3-dimethyl-7-(3-(trifluoromethoxy)benzyl)-1H-purine-2,6(3H,7H)-dione (16 mg, 10.4%) as white solid. ¹*H*-NMR (CDCl₃) δ 1.22-1.18(t,3H), 2.55-2.49(q,2H), 3.39(s,3H), 3.56(s,3H), 4.56(s,2H), 5.46(s,2H), 7.16-7.13(d,1H), 7.22(bs,1H), 7.36-7.28(m,3H). LCMS MH⁺ 496.

### Example 12a 8-((4-ethyloxazol-2-yl)methylthio)-7-(3-methoxybenzyl)-1,3-dimethyl-1H-purine-2,6(3H,7H)-dione (Compound 130)

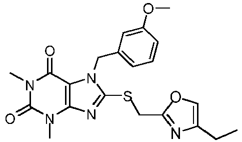

The title compound was prepared using the method of example 11 to give 34mg (16.5% yield) as white solid.¹*H*-NMR (CDCl₃) δ 1.24-1.21(t,3H), 2.55-2.53(q,2H), 3.42(s,3H), 3.58(s,3H), 3.79(s,3H), 4.56(s,2H), 5.45(s,2H), 6.85-6.82(m,1H), 6.93-6.90(m,2H), 7.27-7.23(m,1H), 7.31(s,1H). LCMS retention time 2.428 min; LCMS MH⁺ 442.

### Example 12b 8-((4-ethyloxazol-2-yl)methylthio)-7-(4-methoxybenzyl)-1,3-dimethyl-1H-purine-2,6(3H,7H)-dione (Compound 131)

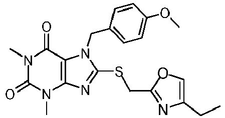

The title compound was prepared using the method of example 11 to give 33mg (16.5% yield) as white solid. ¹*H*-NMR (CDCl₃) δ 1.25-1.21(t,3H), 2.58-2.52(q,2H), 3.43(s,3H), 3.57(s,3H), 3.79(s,3H), 4.57(s,2H), 5.04(s,2H), 6.86-6.84(d,2H), 7.31(s,1H), 7.36-7.34(d,2H). LCMS retention time 2.424 min; LCMS MH⁺ 442.

### Example 12c 8-((4-ethyloxazol-2-yl)methylthio)-1,3-dimethyl-7-(3-methylbenzyl)-1H-purine-2,6(3H,7H)-dione (Compound 132)

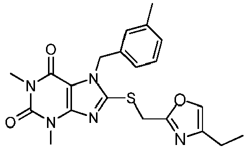

The title compound was prepared using the method of example 11 to give 30 mg (15.1% yield) as white solid. ¹*H*-NMR (DMSO-*d₆*) δ 1.12-1.08((t,3H), 2.26(s,3H), 2.44-2.39(q,2H), 3.22(s,3H), 3.42(s,3H), 4.60(s,2H), 5.40(s,2H), 6.99-6.97(d,1H), 7.06(s,1H), 7.11-7.09(d,1H), 7.23-7.19(m,1H), 7.75(s,1H). LCMS retention time 2.595 min; LCMS MH⁺ 426.

### Example 12d 8-((4-ethyloxazol-2-yl)methylthio)-1,3-dimethyl-7-(4-methylbenzyl)-1H-purine-2,6(3H,7H)-dione (Compound 133)

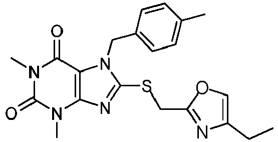

The title compound was prepared using the method of example 11 to give 30 mg (15.1% yield) as white solid. ¹*H*-NMR (DMSO-*d₆*) δ 1.13-1.09(t,3H), 2.27(s,3H), 2.44-2.39(q,2H), 3.22(s,3H), 3.41(s,3H), 4.06(s,2H), 5.39(s,2H), 7.13(s,4H), 7.75-7.74(m,1H). LCMS retention time 2.603 min; LCMS MH⁺ 426.

### Example 12e 7-(3-chlorobenzyl)-8-((4-ethyloxazol-2-yl)methylthio)-1,3-dimethyl-1H-purine-2,6(3H,7H)-dione (Compound 134)

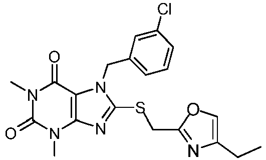

The title compound was prepared using the method of example 11 to give 30 mg (14.4% yield) as white solid. ¹*H*-NMR (DMSO-*d₆*) δ 1.12-1.08(t,3H), 2.44-2.38(q,2H), 3.22(s,3H), 3.42(s,3H), 4.62(s,2H), 5.45(s,2H), 7.16-7.13(m,1H), 7.38-7.33(m,3H), 7.73-7.72(t,1H). LCMS retention time 2.595 min; LCMS MH⁺ 446.

### Example 12f 7-(4-chlorobenzyl)-8-((4-ethyloxazol-2-yl)methylthio)-1,3-dimethyl-1H-purine-2,6(3H,7H)-dione (Compound 146)

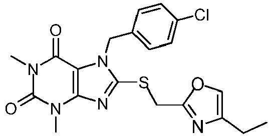

The title compound was prepared using the method of example 11 to give 15mg (7.2% yield) as white solid. ¹*H*-NMR (400 Hz, CDCl₃) δ 1.23-1.19(t,3H), 2.55-2.49(q,2H), 3.39(s,3H), 3.55(s,3H), 4.55(s,2H), 5.41(s,2H), 7.30(s,5H). LCMS retention time 2.636 min; LCMS MH⁺ 446.

### Example 12g 8-((4-ethyloxazol-2-yl)methylthio)-7-(3-fluorobenzyl)-1,3-dimethyl-1H-purine-2,6(3H,7H)-dione (Compound 147)

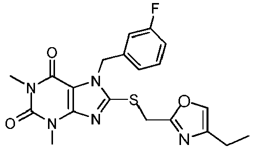

The title compound was prepared using the method of example 11 to give 18mg (8.9% yield) as white solid. ¹*H*-NMR (CDCl₃) δ 1.22-1.18(t,3H), 2.55-2.49(q,2H), 3.39(s,3H), 3.56(s,3H), 4.55(s,2H), 5.43(s,2H), 7.02-6.95(m,2H), 7.13-7.10(m,1H), 7.31-7.28(m,2H). LCMS retention time 2.292 min; LCMS MH⁺ 430.

### Example 12h 8-((4-ethyloxazol-2-yl)methylthio)-1,3-dimethyl-7-(4-(trifluoromethoxy)benzyl)-1H-purine-2,6(3H,7H)-dione (Compound 148)

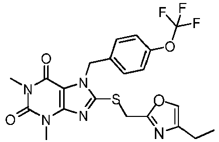

The title compound was prepared using the method of example 11 to give 18mg (7.8% yield) as white solid. ¹*H*-NMR (CDCl₃) δ 1.25-1.21(t,3H), 2.58-2.51(q,2H), 3.42(s,3H), 3.58(s,3H), 4.59(s,2H), 5.46(s,2H), 7.187.16(d,2H), 7.31-7.30(m,1H), 7.44-7.42(d,2H). LCMS retention time 2.601 min; LCMS MH⁺ 496.

### Example 12i 8-((4-ethyloxazol-2-yl)methylthio)-7-(4-fluorobenzyl)-1,3-dimethyl-1H-purine-2,6(3H,7H)-dione (Compound 156)

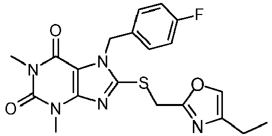

The title compound was prepared using the method of example 11 to give 20 mg (16.6% yield) as white solid. ¹*H*-NMR (CDCl₃) δ 1.22-1.18(t,3H), 2.55-2.49(m,2H), 3.39(s,3H), 3.55(s,3H), 4.55(s,2H), 5.41(s,2H), 7.01-6.97(t,2H), 7.28(s,1H), 7.38-7.28(m,2H). LCMS retention time 2.309 min; LCMS MH⁺ 430.

### Example 12j 7-(4-bromobenzyl)-8-((4-ethyloxazol-2-yl)methylthio)-1,3-dimethyl-1H-purine-2,6(3H,7H)-dione (Compound 157)

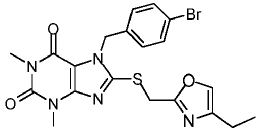

The title compound was prepared using the method of example 11 to give 20 mg (14.6% yield) as white solid. ¹*H*-NMR (CDCl₃) δ 1.22-1.18(t,3H), 2.55-2.49(m,2H), 3.39(s,3H), 3.55(s,3H), 4.55(s,2H), 5.39(s,2H), 7.24-7.21(d,2H), 7.28-7.27(m,1H), 7.44-7.42(d,2H). LCMS retention time 2.603 min; LCMS MH⁺ 492.

### Example 12k 7-(3-acetylbenzyl)-8-((4-ethyloxazol-2-yl)methylthio)-1,3-dimethyl-1H-purine-2,6(3H,7H)-dione (Compound 180)

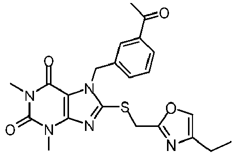

The title compound was prepared using the method of example 11 to give 10 mg (7.9% yield) as white solid. ¹*H*-NMR (CDCl₃) δ 1.24-1.20(t,3H), 2.57-2.51(q,2H), 2.61(s,3H), 3.42(s,3H), 3.58(s,3H), 4.58(s,2H), 5.53(s,2H), 7.13-7.31(m,1H), 7.46-7.42(t,1H), 7.56-7.54(d,1H), 7.91-7.89(d,1H), 7.99(s,1H). LCMS retention time 1.894 min; LCMS MH⁺ 454.

### Example 12l 7-(4-acetylbenzyl)-8-((4-ethyloxazol-2-yl)methylthio)-1,3-dimethyl-1H-purine-2,6(3H,7H)-dione (Compound 181)

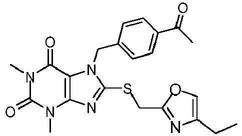

The title compound was prepared using the method of example 11 to give 10 mg (7.9% yield) as white solid. ¹*H*-NMR (CDCl₃) δ 1.24-1.20(t,3H), 2.57-2.51(q,2H), 2.59(s,3H), 3.41(s,3H), 3.59(s,3H), 4.58(s,2H), 5.53(s,2H), 7.31(s,1H), 7.42-7.39(d,2H), 7.93-7.91(d,2H). LCMS retention time 1.874 min; LCMS MH⁺ 454.

### Example 12m 4-((8-((4-ethyloxazol-2-yl)methylthio)-1,3-dimethyl-2,6-dioxo-2,3-dihydro-1H-purin-7(6H)-yl)methyl)benzonitrile (Compound 169)

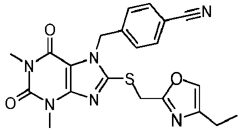

The title compound was prepared using the method of example 11 except the temperature was room temperature to give 10 mg (8.2% yield) as white solid. ¹*H*-NMR (DMSO-d₆) δ 1.09-1.05(t,3H), 2.39-2.37(m,2H), 3.18(s,3H), 3.39(s,3H), 4.58(s,2H), 5.52(s,2H), 7.35-7.33(d,2H), 7.70(s,1H), 7.79-7.78(d,2H). LCMS retention time 2.220 min; LCMS MH⁺ 437.

### Example 12n 3-((8-((4-ethyloxazol-2-yl)methylthio)-1,3-dimethyl-2,6-dioxo-2,3-dihydro-1H-purin-7(6H)-yl)methyl)benzonitrile (Compound 170)

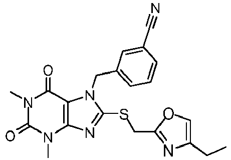

The title compound was prepared using the method of example 11 except the temperature was room temperature to give 10 mg (8.2% yield) as white solid. ¹*H*-NMR (DMSO-d₆) δ 1.09-1.05(t,3H), 2.48-2.37(m,2H), 3.18(s,3H), 3.39(s,3H), 4.58(s,2H), 5.49(s,2H), 7.54-7.52(,2H), 7.69-7.66(m,3H). LCMS retention time 2.402 min; LCMS MH⁺ 437.

### Example 12o 7-(3-bromobenzyl)-8-((4-ethyloxazol-2-yl)methylthio)-1,3-dimethyl-1H-purine-2,6(3H,7H)-dione (Compound 20)

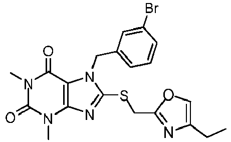

The title compound was prepared using the method of example 11 except the temperature was room temperature and the reaction was stirred overnight to give 81 mg (20.3% yield) as white solid. ¹*H*-NMR (DMSO-d₆) δ 1.09-1.05(t,3H), 2.39-2.37(q,2H), 3.19(s,3H), 3.39(s,3H), 4.59(s,2H), 5.42(s,2H), 7.16-7.14(d,1H), 7.29-7.25(t,1H), 7.49-7.45(m,2H), 7.69(s,1H). LCMS retention time 2.612 min; LCMS MH⁺ 492.

### Example 13 7-(3-bromobenzyl)-8-(2-(4-ethyloxazol-2-yl)propan-2-ylthio)-1,3-dimethyl-1H-purine-2,6(3H,7H)-dione (Compound 100)

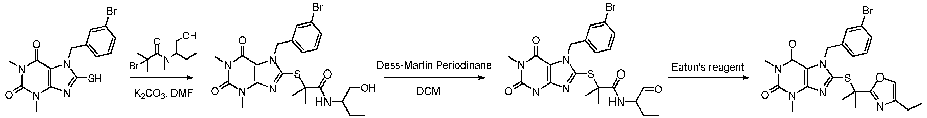

### Step 1 2-(7-(3-bromobenzyl)-1,3-dimethyl-2,6-dioxo-2,3,6,7-tetrahydro-1H-purin-8-ylthio)-N-(1-hydroxybutan-2-yl)-2-methylpropanamide

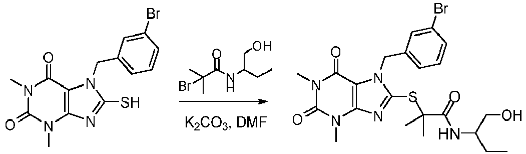

To a solution of 7-(3-bromobenzyl)-8-mercapto-1,3-dimethyl-1H-purine-2,6(3H,7H)-dione (1 g, 2.62 mmol, intermediate 1) in DMF (10 mL) was added 2-bromo-N-(1-hydroxybutan-2-yl)-2-methylpropanamide (0.69 g, 2.88 mmol, intermediate 9) followed by potassium carbonate (0.54 g, 3.93 mmol). The mixture was stirred at 80 °C overnight. The mixture was diluted with ethyl acetate and washed with saturated aqueous ammonium chloride, dried and concentrated to give 2-(7-(3-bromobenzyl)-1,3-dimethyl-2,6-dioxo-2,3,6,7-tetrahydro-1H-purin-8-ylthio)-N-(1-hydroxybutan-2-yl)-2-methylpropanamide (1.1 g, 77.9% yield) as yellow solid. LCMS retention time 1.571 min, LCMS MH⁺ 540.

### Step 2 2-(7-(3-bromobenzyl)-1,3-dimethyl-2,6-dioxo-2,3,6,7-tetrahydro-1H-purin-8-ylthio)-2-methyl-N-(1-oxobutan-2-yl)propanamide

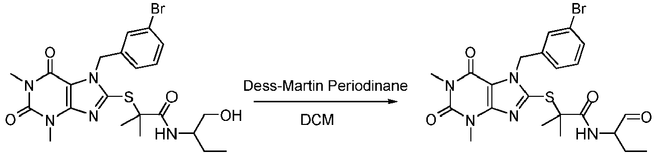

To a solution of 2-(7-(3-bromobenzyl)-1,3-dimethyl-2,6-dioxo-2,3,6,7-tetrahydro-1H-purin-8-ylthio)-N-(1-hydroxybutan-2-yl)-2-methylpropanamide (1 g, 1.86 mmol) in DCM (20 mL) was added Dess-Martin Periodinane (1.18 g, 27.8 mmol) at 0 °C. The resulting mixture was stirred at room temperature for 16 h. The mixture was quenched with saturated aqueous sodium bicarbonate and the layers were separated. The organic layer was washed with brine, dried and concentrated to give crude product, which was purified via silica gel chromatography eluting with DCM/ methanol = 60 : 1 to 40 :1 to give 2-(7-(3-bromobenzyl)-1,3-dimethyl-2,6-dioxo-2,3,6,7-tetrahydro-1H-purin-8-ylthio)-N-(1-hydroxybutan-2-yl)-2-methylpropanamide (540 mg, 54.1% yield) as yellow solid. LCMS retention time 1.896 min, LCMS MH⁺ 536.

### Step 3 7-(3-bromobenzyl)-8-(2-(4-ethyloxazol-2-yl)propan-2-ylthio)-1,3-dimethyl-1H-purine-2,6(3H,7H)-dione

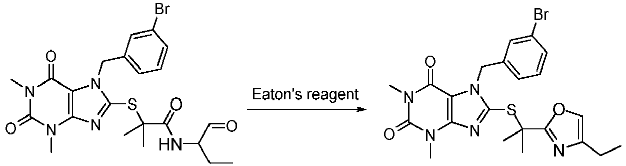

A mixture of 2-(7-(3-bromobenzyl)-1,3-dimethyl-2,6-dioxo-2,3,6,7-tetrahydro-1H-purin-8-ylthio)-2-methyl-N-(1-oxobutan-2-yl)propanamide (50 mg, 0.093 mmol) in Eaton's reagent (0.5 mL) in a sealed vial was reacted with microwave irradiation at 120 °C for 30 min. The mixture was poured into ice-water and extracted with ethyl acetate. The organic layer was washed with saturated aqueous sodium bicarbonate solution twice, dried and concentrated to give crude product, which was purified via preparative HPLC to give 7-(3-bromobenzyl)-8-(2-(4-ethyloxazol-2-yl)propan-2-ylthio)-1,3-dimethyl-1H-purine-2,6(3H,7H)-dione (11.2 mg, 22.9% yield) as light yellow solid. ¹*H*-NMR (CDCl₃) δ 7.38-7.39 (d,1H), 7.37 (s, 1H), 7.35 (s, 1H), 7.14-7.16 (dd, 1H), 5.24 (s, 2H), 3.51 (s, 3H), 3.36 (s, 3H), 2.45-2.51 (q, 2H), 1.88 (s, 6H), 1.14-1.17 (t, 3H). LCMS retention time 3.134 min; LCMS MH⁺ 518.

### Example 14 1,3-dimethyl-8-(propoxymethyl)-7-(3-(trifluoromethoxy)benzyl)-1H-purine-2,6(3H,7H)-dione (Compound 88)

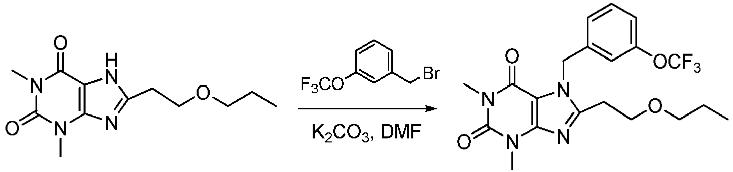

To a solution of 1,3-dimethyl-8-(propoxymethyl)-1H-purine-2,6(3H,7H)-dione (80 mg, 0.3 mmol, intermediate 3) in DMF (2 mL) was added 1-(bromomethyl)-3-(trifluoromethoxy)benzene (91 mg, 0.36 mmol) followed by potassium carbonate (62 mg, 0.45 mmol). The mixture was stirred at 50° C for 1.5 h. The mixture was partitioned between ethyl acetate and water. The organic layers were combined, dried over sodium sulfate, then filtered and concentrated to give a crude product, which was collected, washed with ethanol, dried in vacuo to give 1,3-dimethyl-8-(propoxymethyl)-7-(3-(trifluoromethoxy)benzyl)-1H-purine-2,6(3H,7H)-dione (50 mg, 38%) as a white solid. ¹*H*-NMR (DMSO-*d₆*) *δ* 0.78 (t, 3H), 1.41 (q, 2H), 2.98 (t, 2H),3.24 (s, 3H), 3.25 (t, 2H), 3.37 (s. 3H), 3.65 (t, 2H), 5.54 (s, 2H), 7.17 (d, 1H), 7.26 (s, 1H), 7.30 (d, 2H), 7.48 (t, 1H). LCMS retention time 2.971; LCMS MH⁺ 441.

### The following compounds were prepared using the method of example 14.

### Example 15a 7-(4-methoxybenzyl)-1,3-dimethyl-8-(propoxymethyl)-1H-purine-2,6(3H,7H)-dione (Compound 89)

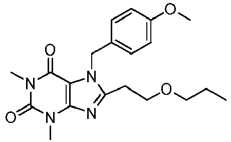

60 mg , 52% yield, white solid. ¹*H*-NMR (DMSO-*d₆*) *δ* 0.81(t, 3 H), 1.44(q, 2 H), 2.97(t, 2 H),3.22(s, 3 H), 3.28(t, 2 H), 3.42(s,3 H), 3.66(t, 2 H), 3.72(s, 3 H), 5.52(s, 2 H), 6.89(d, 2 H), 7.17(d, 2 H). LCMS retention time 2.646 min; LCMS MH⁺ 387.

### Example 15b 1,3-dimethyl-7-(3-methylbenzyl)-8-(propoxymethyl)-1H-purine-2,6(3H,7H)-dione (Compound 90)

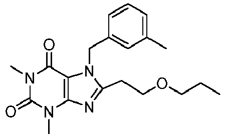

50 mg,45% yield, white solid. ¹*H*-NMR (DMSO-*d₆*) *δ* 0.80(t, 3H), 1.44(q, 2H),2.27 (s, 3H), 2.94(t, 2H), 3.22(s, 3H), 3.27(t, 2H), 3.44(s,3H), 3.64(t, 2H), 5.56(s, 2H), 6.90(d, 1H), 7.01(s, 1H), 7.11(d,1H),7.20(t, 1H). LCMS retention time 2.814 min; LCMS MH⁺ 371.

### Example 15c 7-(3-methoxybenzyl)-1,3-dimethyl-8-(propoxymethyl)-1H-purine-2,6(3H,7H)-dione (Compound 91)

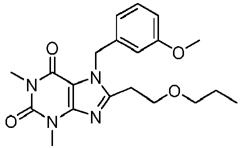

65 mg, 56% yield, white solid. ¹*H*-NMR (DMSO-d₆) δ 0.77(t, 3H), 1.41(q, 2H), 2.92(t, 2H), 3.19(s, 3H), 3.24(t, 2H), 3.41(s, 3H), 3.62(t, 2 H), 3.69(s, 3 H), 5.54(s, 2 H), 6.67(d, 1 H), 6.74(s, 1 H),6.84(d, 1 H),7.23(t, 1 H). LCMS retention time 2.661 min; LCMS MH⁺ 387.

### Example 15d 7-(3-fluorobenzyl)-1,3-dimethyl-8-(propoxymethyl)-1H-purine-2,6(3H,7H)-dione (Compound 92)

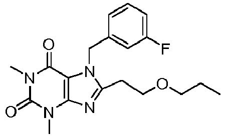

53mg, 47% yield, white solid. ¹*H*-NMR (DMSO-*d₆*) *δ* 0.79(t, 3 H), 1.41(q, 2 H), 2.97(t, 2 H), 3.21(s, 3 H), 3.27(t, 2 H), 3.44(s,3 H), 3.66(t, 2 H), 5.61(s, 2 H), 7.03(t, 2 H), 7.11-7.15(m, 1 H),7.36-7.42(m, 1 H). LCMS retention time 2.701 min; LCMS MH⁺ 375.

### Example 15e 7-(4-chlorobenzyl)-1,3-dimethyl-8-(propoxymethyl)-1H-purine-2,6(3H,7H)-dione (Compound 93)

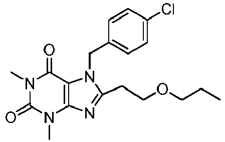

68 mg, 55% yield, white solid. ¹*H*-NMR (DMSO-*d₆*) *δ* 0.79 (t, 3H), 1.41(q, 2H), 2.96(t, 2H), 3.21(s, 3H), 3.27(t, 2H), 3.43(s, 3H), 3.66(t, 2H), 5.58(s, 2H), 7.21(d, 2H), 7.41(d, 2H). LCMS retention time 2.858 min; LCMS MH⁺ 391.

### Example 15f 7-(3-chlorobenzyl)-1,3-dimethyl-8-(propoxymethyl)-1H-purine-2,6(3H,7H)-dione (Compound 107)

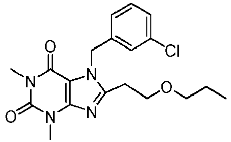

72 mg, 62% yield, white solid. ¹*H*-NMR (DMSO-*d₆*) *δ* 0.79(t, 3H), 1.42(q, 2H), 2.98(t, 2H), 3.18(s, 3H), 3.25(t, 2H), 3.44(s,3H), 3.66(t, 2H), 5.56(s, 2H), 7.13(d, 1H), 7.29(s, 1H),7.36-7.38(m, 2H). LCMS retention time 2.588 min; LCMS MH⁺ 391.

### Example 15g 7-(4-fluorobenzyl)-1,3-dimethyl-8-(propoxymethyl)-1H-purine-2,6(3H,7H)-dione (Compound 108)

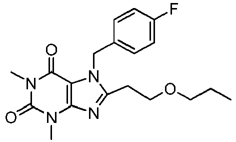

68 mg, 55% yield, white solid. ¹*H*-NMR (DMSO-*d₆*) *δ* 0.80(t, 3H), 1.43(q, 2H), 2.98(t, 2H), 3.22(s, 3H), 3.28(t, 2H), 3.43(s,3H), 3.66(t, 2H), 5.58(s, 2H), 7.17(t, 2H), 7.27(dd, 2H). LCMS retention time 2.429min; LCMS MH⁺ 375.

### Example 15h 1,3-dimethyl-8-(propoxymethyl)-7-(4-(trifluoromethoxy)benzyl)-1H-purine-2,6(3H,7H)-dione (Compound 109)

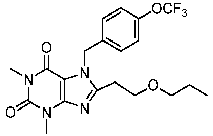

48mg, 31% yield, white solid. ¹*H*-NMR (DMSO-*d₆*) *δ* 0.78 (t, 3 H), 1.40(q, 2 H), 2.98(t, 2 H), 3.21(s, 3 H), 3.25(t, 2 H), 3.44(s,3 H), 3.65(t, 2 H), 5.63(s, 2 H), 7.33(s, 4H). LCMS retention time 2.761min; LCMS MH⁺ 441.

### Example 15i 4-((1,3-dimethyl-2,6-dioxo-8-(propoxymethyl)-2,3-dihydro-1H-purin-7(6H)-yl)methyl)benzonitrile (Compound 118)

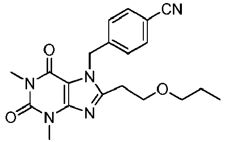

80mg, 70% yield, white solid. ¹*H*-NMR (DMSO-*d₆*) *δ* 0.77 (t, 3H), 1.39(q, 2H), 2.96(t, 2H), 3.19(s, 3H), 3.25(t, 2H), 3.44(s, 3H), 3.66(t, 2H), 5.68(s, 2H), 7.35(d, 2H), 7.82(d, 2H). LCMS retention time 2.165 min; LCMS MH⁺ 382.

### Example 15j 3-((1,3-dimethyl-2,6-dioxo-8-(propoxymethyl)-2,3-dihydro-1H-purin-7(6H)-yl)methyl)benzonitrile (Compound 119)

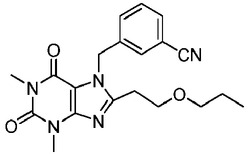

75mg, 65% yield, white solid. ¹*H*-NMR (DMSO-*d₆*) *δ* 0.77 (t, 3H), 1.41(q, 2H), 2.99(t, 2H), 3.20(s, 3H), 3.26(t, 2H), 3.44(s,3H), 3.67(t, 2H), 5.64(s, 2H), 7.54-7.58(m, 2H),7.66(s, 1H), 7.78(d, 1H). LCMS retention time 2.173 min; LCMS MH⁺ 382.

### Example 15k Methyl 4-((1,3-dimethyl-2,6-dioxo-8-(propoxymethyl)-2,3-dihydro-1H-purin-7(6H)-yl)methyl)benzoate (Compound 218)

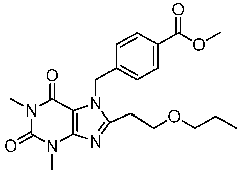

46mg, 38% yield, white solid. LCMS MH⁺ 415.

### Example 15l 7-(4-acetylbenzyl)-1,3-dimethyl-8-(propoxymethyl)-1H-purine-2,6(3H,7H)-dione (Compound 158)

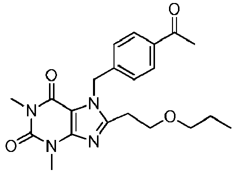

Title product was prepared using intermediate 19. 75mg, 50% yield, white solid. ¹*H*-NMR (DMSO-*d₆*) *δ* 0.78(t, 3 H), 1.41(q, 2 H), 2.55(s, 3 H), 2.96(t, 2 H), 3.20(s, 3 H), 3.26(t, 2 H), 3.45(s,3 H), 3.67(t, 2 H), 5.68(s, 2 H), 7.29(d, 2 H), 7.92(d, 2 H). LCMS retention time 2.382 min; LCMS MH⁺ 399.

### Example 15m 7-(3-acetylbenzyl)-1,3-dimethyl-8-(propoxymethyl)-1H-purine-2,6(3H,7H)-dione (Compound 159)

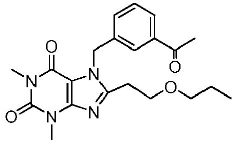

Title product was prepared using intermediate 32. 83mg, 55% yield, white solid. ¹*H*-NMR (DMSO-*d₆*) *δ* 0.78(t, 3 H), 1.41(q, 2 H), 2.58(s, 3 H), 2.98(t, 2 H), 3.21(s, 3 H), 3.25(t, 2 H), 3.44(s,3 H), 3.65(t, 2 H), 5.68(s, 2 H), 7.39(d, 1 H), 7.50(t, 1 H),7.82(s, 1 H), 7.90(d, 1 H). LCMS retention time 2.055 min; LCMS MH⁺ 399.

### Example 15n 1,3-dimethyl-8-(propoxymethyl)-7-(pyridin-2-ylmethyl)-1H-purine-2,6(3H,7H)-dione (Compound 219)

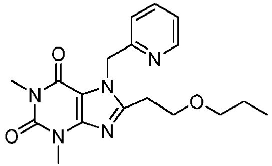

46mg, 34% yield, white solid. ¹*H*-NMR (DMSO-*d₆*) *δ* 0.81(t, 3H), 1.44(q, 2H), 3.03(t, 2H), 3.19(s, 3H), 3.29(t, 2H), 3.44(s,3H), 3.69(t, 2H), 5.67(s, 2H), 7.26-7.33(m, 2H), 7.79(t, 1H),8.49(d, 1H). LCMS retention time 1.710 min; LCMS MH⁺ 358.

### Example 15o 1,3-dimethyl-8-(propoxymethyl)-7-(pyridin-3-ylmethyl)-1H-purine-2,6(3H,7H)-dione (Compound 220)

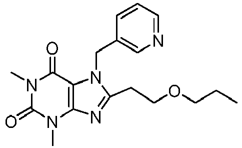

52mg, 38% yield, white solid. ¹*H*-NMR (DMSO-*d₆*) *δ* 0.78(t, 3 H), 1.41(q, 2 H), 3.03(t, 2 H), 3.21(s, 3 H), 3.26(t, 2 H), 3.43(s,3 H), 3.63(t, 2 H), 5.62(s, 2 H), 7.34-7.37(m, 1 H), 7.58(t, 1 H),8.49-8.51(m, 2 H). LCMS retention time 1.702 min; LCMS MH⁺ 358.

### Example 15p 1,3-dimethyl-8-(propoxymethyl)-7-(pyridin-4-ylmethyl)-1H-purine-2,6(3H,7H)-dione (Compound 221)

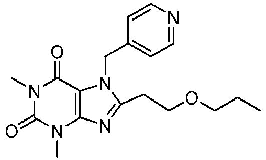

45mg, 34% yield, white solid. ¹*H*-NMR (DMSO-*d₆*) *δ* 0.78(t, 3H), 1.40(q, 2H), 3.96(t, 2H), 3.19(s, 3H), 3.26(t, 2H), 3.45(s, 3H), 3.67(t, 2H), 5.63(s, 2H), 7.12(d, 2H), 8.51(d, 2H). LCMS retention time 1.672 min; LCMS MH⁺ 358.

### Example 15q 1,3-dimethyl-8-(propoxymethyl)-7-(pyrimidin-4-ylmethyl)-1H-purine-2,6(3H,7H)-dione (Compound 222)

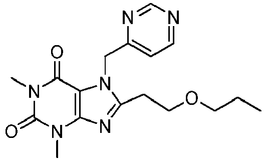

45mg, 34% yield, white solid. ¹*H*-NMR (DMSO-*d₆*) *δ* 0.75(t, 3 H), 1.37(q, 2 H), 2.99(t, 2 H), 3.13(s, 3 H), 3.24(t, 2 H), 3.42(s,3 H), 3.67(t, 2 H), 5.67(s, 2 H), 7.33(d, 1 H), 8.74(d, 1 H), 9.07(s, 1 H). LCMS retention time 1.713 min; LCMS MH⁺ 359.

### Example 15r 7-(biphenyl-3-ylmethyl)-1,3-dimethyl-8-(2-propoxyethyl)-1H-purine-2,6(3H,7H)-dione (Compound 128)

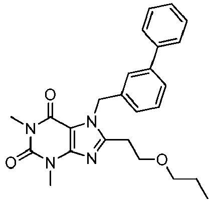

60 mg, 46% yield, white solid. ¹*H*-NMR (CDCl₃) *δ* 0.88(t, 3 H), 1.53(q, 2 H), 2.99(t, 2 H),3.34(t, 2 H), 3.40(s, 3 H), 3.59(s,3 H), 3.74(t, 2 H), 5.71(s, 2 H), 7.18(d, 1 H), 7.37-7.42(m, 5 H), 7.45-7.52(m, 3 H). LCMS retention time 2.683 min; LCMS MH⁺ 433.

### Example 15s 7-(biphenyl-4-ylmethyl)-1,3-dimethyl-8-(2-propoxyethyl)-1H-purine-2,6(3H,7H)-dione (Compound 129)

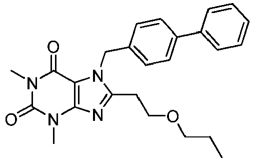

61 mg, 46% yield, white solid. ¹*H*-NMR (DMSO-*d₆*) *δ* 0.78(t, 3 H), 1.40(q, 2 H), 2.99(t, 2 H),3.23(s, 3 H), 3.27(t, 2 H), 3.44(s,3 H), 3.68(t, 2 H), 5.64(s, 2 H), 7.28(d, 1 H), 7.36(t, 1 H), 7.46(t, 2 H),7.63 (d, 4 H). LCMS retention time 2.684 min; LCMS MH⁺ 433.

### Example 15t 1,3-dimethyl-7-(4-methylbenzyl)-8-(2-propoxyethyl)-1H-purine-2,6(3H,7H)-dione (Compound 94)

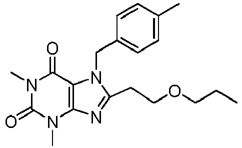

60 mg, 54% yield, white solid. ¹*H*-NMR (DMSO-*d₆*) *δ* 0.80(t, 3H), 1.44(q, 2H), 2.27 (s, 3H), 2.94(t, 2H),3.22(s, 3H), 3.27(t, 2H), 3.43(s, 3H), 3.65(t, 2H), 5.55(s, 2H), 7.08(d, 2H), 7.15 (d, 2H). LCMS retention time 2.838 min; LCMS MH⁺ 371.

### Example 15u 7-(4-bromobenzyl)-1,3-dimethyl-8-(2-propoxyethyl)-1H-purine-2,6(3H,7H)-dione (Compound 95)

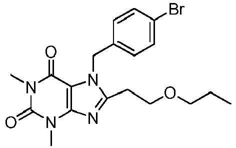

53 mg, 41 % yield, white solid. ¹*H*-NMR (DMSO-*d₆*) *δ* 0.79(t, 3 H), 1.42(q, 2 H), 2.96(t, 2 H),3.21(s, 3 H), 3.26(t, 2 H), 3.43(s,3 H), 3.66(t, 2 H), 5.57(s, 2 H), 7.15(d, 2 H), 7.54 (d, 2 H). LCMS retention time 2.909 min; LCMS MH⁺ 435.

### Example 16 3-((1,3-dimethyl-2,6-dioxo-8-(propoxymethyl)-2,3-dihydro-1H-purin-7(6H)-yl)methyl)benzamide (Compound 121)

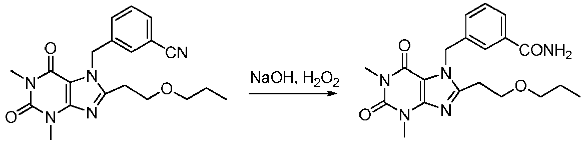

To a solution of 3-((1,3-dimethyl-2,6-dioxo-8-(propoxymethyl)-2,3-dihydro-1H-purin-7(6H)-yl)methyl)benzonitrile(100 mg, 0.26 mmol, example 15j) in ethanol (5 mL) was added 1 N sodium hydroxide (0.9 mL, 0.91 mmol), aqueous. hydrogen peroxide (0.035 mL, 0.26 mmol), the mixture was heated at 60°C overnight. Then it was cooled to room temperature and partitioned between DCM and water, the organic layers were combined, dried over sodium sulfate, filtered and concentrated to afford a crude product which was purified by pre-HPLC to give 3-((1,3-dimethyl-2,6-dioxo-8-(propoxymethyl)-2,3-dihydro-1H-purin-7(6H)-yl)methyl)benzamide(53 mg, 51%yield)as a white solid.Mass spec: 400(M+H). ¹*H*-NMR (400 Hz, DMSO-*d*₆). *δ* 0.79(t, 3H), 1.42(q, 2H), 2.96(t, 2H), 3.22(s, 3H), 3.26(t, 2H), 3.45(s,3H), 3.65(t, 2H), 5.65(s, 2H), 7.29(d, 1H),7.42(t, 2H), 7.68(s, 1H).7.79(d, 1H). LCMS retention time 1.483 min; LCMS MH⁺ 400.

### Example 17 4-((1,3-dimethyl-2,6-dioxo-8-(propoxymethyl)-2,3-dihydro-1H-purin-7(6H)-yl)methyl)benzamide (Compound 149)

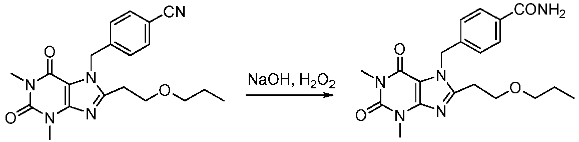

The title compound was prepared using the procedure of example 16 to give the white solid product (46 mg, 45% yield). ¹*H*-NMR (DMSO-*d₆*) *δ* 0.79(t, 3 H), 1.42(q, 2 H), 2.96(t, 2 H), 3.21(s, 3 H), 3.26(t, 2 H), 3.44(s,3 H), 3.66(t, 2 H), 5.64(s, 2 H), 7.23(d, 2 H),7.37(s, 1 H),7.82(d, 2 H). LCMS retention time 1.360 min; LCMS MH⁺ 400.

### Example 18 7-(4-(hydroxymethyl)benzyl)-1,3-dimethyl-8-(propoxymethyl)-1H-purine-2,6(3H,7H)-dione (Compound 151)

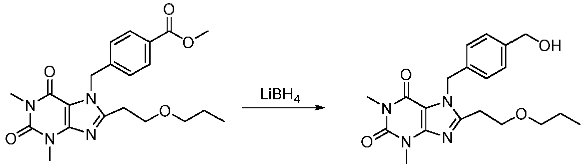

To a solution of methyl 4-((1,3-dimethyl-2,6-dioxo-8-(propoxymethyl)-2,3-dihydro-1H-purin-7(6H)-yl)methyl)benzoate (120 mg, 0.3 mmol, example 15k) in THF (5 mL) was added lithium borohydride (100 mg, 4.5 mmol). Then mixture was stirred at room temperature overnight. Aqueous ammonium chloride (5 mL) was added to the reaction mixture; then it was partitioned between ethyl acetate and water. The organic layers were combined, dried over sodium sulfate, filtered and concentrated to afford a crude product which was purified by flash chromatography eluting with DCM/methanol (80:1 to 40:1) to give 7-(4-(hydroxymethyl)benzyl)-1,3-dimethyl-8-(propoxymethyl)-1H-purine-2,6(3H,7H)-dione(70 mg, 60% yield) as a white solid. ¹*H*-NMR (DMSO-*d₆*) *δ* 0.81(t, 3 H), 1.44(q, 2 H), 2.95(t, 2 H), 3.22(s, 3 H), 3.29(t, 2 H), 3.43(s,3 H), 3.66(t, 2 H), 4.46(d, 2 H),5.18(t, 1 H),5.58(s, 2 H), 7.14(d, 2 H), 7.28(d, 2 H) LCMS retention time 1.906 min; LCMS MH⁺ 387.

### Example 19 7-(3-(hydroxymethyl)benzyl)-1,3-dimethyl-8-(propoxymethyl)-1H-purine-2,6(3H,7H)-dione (Compound 141)

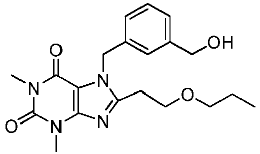

The title compound was prepared using the methods of examples 14 and 18 to give 20 (75mg, 65% yield) as a white solid. ¹*H*-NMR (DMSO-*d₆*) *δ* 0.78(t, 3 H), 1.41(q, 2 H), 2.91(t, 2 H), 3.19(s, 3 H), 3.25(t, 2 H), 3.41(s,3 H), 3.62(t, 2 H), 4.43(d, 2 H),5.18(t, 1 H),5.57(s, 2 H), 6.98(d, 1 H), 7.11(s, 1 H),7.20(d, 1 H), 7.25(d, 1 H). LCMS retention time 1.307 min; LCMS MH⁺ 387.

### Example 20 7-(3-bromobenzyl)-1,3-dimethyl-8-phenethyl-1H-purine-2,6(3H,7H)-dione (Compound 33)

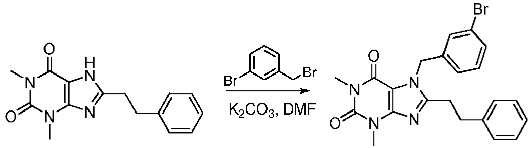

The title compound was prepared using the method of example 14 and using intermediate 10 to give 7-(3-bromobenzyl)-1,3-dimethyl-8-phenethyl-1H-purine-2,6(3H,7H)-dione (296 mg, 80.1% yield) as white solid. ¹*H*-NMR (DMSO-*d₆*) δ 7.49-7.51(d, 1H), 7.45(s, 1H), 7.25-7.31 (m, 3H), 7.09-7.19(m, 2H), 7.07-7.09 (d, 1H), 5.45(S, 2H), 3.43(s,3 H), 3.23(s, 3 H), 2.98-3.01 (t, 2H), 2.86-2.89(t, 2H). LCMS retention time 2.926 min; LCMS MH⁺ 453.

### Example 21 ethyl 3-(7-(3-bromobenzyl)-1,3-dimethyl-2,6-dioxo-2,3,6,7-tetrahydro-1H-purin-8-yl)propanoate (Compound 37)

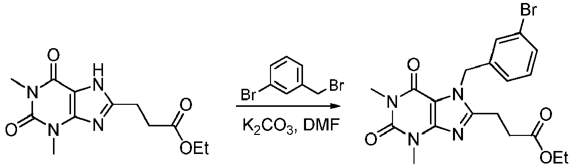

The title compound was prepared using the procedure of example 14 and using intermediate 11 to give ethyl 3-(1,3-dimethyl-2,6-dioxo-2,3,6,7-tetrahydro-1H-purin-8-yl)propanoate (35 mg, 34.6% yield) as white solid. ¹*H*-NMR (DMSO *d₆*) *δ* 7.48-7.50 (d, 1H), 7.45 (s, 1H), 7.28-7.32 (t, 1H), 7.13-7.15 (d, 1H), 5.56 (s, 2H), 4.00-4.05 (q, 2H), 3.37 (s, 3 H), 3.19 (s, 3H), 2.91-2.95 (t, 2H), 2.71-2.74 (t, 2H), 1.11-1.15 (t, 3H). LCMS retention time 2.489 min; LCMS MH⁺ 449.

### Example 22 ethyl 3-(7-(3-bromobenzyl)-1,3-dimethyl-2,6-dioxo-2,3,6,7-tetrahydro-1H-purin-8-yl) but-3-enoate (Compound 123)

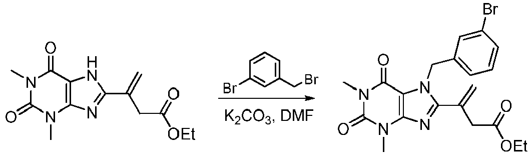

The title compound was prepared using the method of example 14 and using intermediate 12 to give 3-(7-(3-bromobenzyl)-1,3-dimethyl-2,6-dioxo-2,3,6,7-tetrahydro-1H-purin-8-yl)but-3-enoate (59 mg, 37.4% yield) as white solid. ¹*H*-NMR (DMSO *d*₆) δ 7.48-7.50 (d, 1H), 7.40 (s, 1H), 7.29-7.23 (dd, 1H), 7.07-7.09 (d, 1H), 5.70 (s, 1H), 5.67 (s, 2H), 5.36 (s, 1H), 3.98-4.03 (q, 2H), 3.61 (s, 2H), 3.43 (s, 3H), 1.09-1.13 (t, 3H). LCMS retention time 2.513 min; LCMS MH⁺ 461.

### Example 23 7-(3-bromobenzyl)-1,3-dimethyl-8-octyl-1H-purine-2,6(3H,7H)-dione (Compound 80)

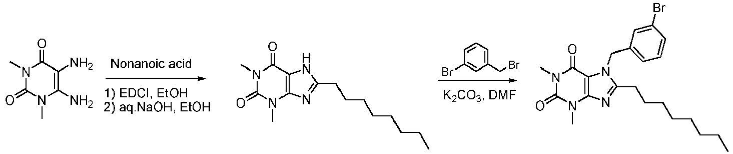

The title compound was prepared using the methods of intermediate 10, step 1 and example 14 to give the title compound (18 mg, 50.6% yield over three steps) as white solid. ¹*H*-NMR (DMSO *d*₆) 7.46-7.48 (d, 1H), 7.41 (s, 1H), 7.26-7.29 (dd, 1H), 7.10-7.12 (d, 1H), 5.55 (s, 2H), 3.40 (s, 3 H), 3.19 (s, 3H), 2.64-2.68 (t, 2H), 1.46-1.49 (m, 2H), 1.14-1.20 (m, 10H), 0.79-0.83 (t, 3H). LCMS retention time 3.467 min; LCMS MH⁺ 461, 463.

### Example 24 ethyl 3-(7-(3-bromobenzyl)-1,3-dimethyl-2,6-dioxo-2,3,6,7-tetrahydro-1H-purin-8-yl)propanoate (Compound 54)

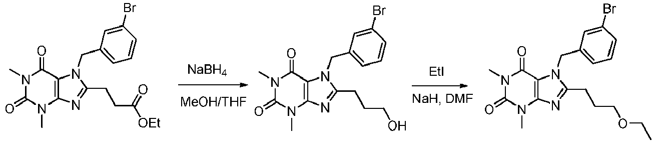

### Step 1 7-(3-bromobenzyl)-8-(3-hydroxypropyl)-1,3-dimethyl-1H-purine-2,6(3H,7H)-dione

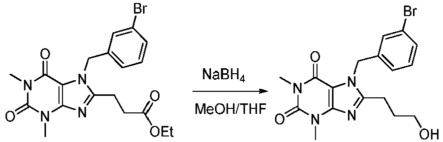

To a solution of ethyl 3-(7-(3-bromobenzyl)-1,3-dimethyl-2,6-dioxo-2,3,6,7-tetrahydro-1H-purin-8-yl)propanoate (250 mg, 0.556 mmol, product of example 22) in THF (5 mL) was added sodium borohydride (126 mg, 3.34 mmol) in portions. After addition, the mixture was stirred at reflux for 30 min. To the mixture, methanol (1 mL) was added dropwise carefully. The resulting mixture was stirred at this temperature for 30 min. The mixture was cooled and quenched with 2N HCl and diluted with ethyl acetate. The organic phase was washed with brine, dried and concentrated to give 7-(3-bromobenzyl)-8-(3-hydroxypropyl)-1,3-dimethyl-1H-purine-2,6(3H,7H)-dione (170 mg, 75.1% yield) as yellow solid. LCMS MH⁺ 407.

### Step 2 7-(3-bromobenzyl)-8-(3-ethoxypropyl)-1,3-dimethyl-1H-purine-2,6(3H,7H)-dione

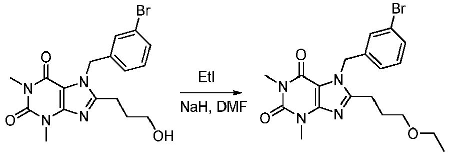

To a solution of 7-(3-bromobenzyl)-8-(3-hydroxypropyl)-1,3-dimethyl-1H-purine-2,6(3H,7H)-dione (100 mg, 0.245 mmol) in anhydrous DMF (2 mL) was added sodium hydride (14.7 mg, 0.37 mmol) in one portion at 0 °C. After addition, iodoethane (40 mg, 0.294 mmol) was added to the reaction and the resulting mixture was stirred at room temperature for 30 h. The mixture was quenched with dilute HCl (5 mL) and extracted with ethyl acetate. The organic phase was dried and concentrated to give crude product, which was purified via preparative HPLC to give 7-(3-bromobenzyl)-8-(3-ethoxypropyl)-1,3-dimethyl-1H-purine-2,6(3H,7H)-dione (31 mg, 29.1% yield) as white solid. ¹*H*-NMR (DMSO *d*₆) δ 7.49-7.51 (d, 1H), 7.44 (s, 1H), 7.29-7.33 (t, 1H), 7.11-7.13 (d, 1H), 5.56 (s, 2H), 3.43 (s, 3 H), 3.22 (s, 3H), 2.72-2.76 (t, 2H), 1.80-1.84 (m, 2H), 1.04-1.07 (t, 3H). LCMS retention time 2.597 min; LCMS MH⁺ 435.

### Example 25 7-(3-bromobenzyl)-1,3-dimethyl-8-(3-propoxypropyl)-1H-purine-2,6(3H,7H)-dione (Compound 82)

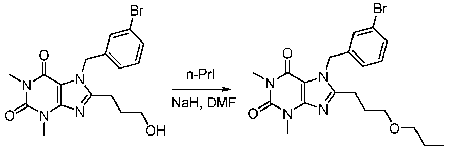

The title compound was prepared following the procedure of example 24 to give the title product (33 mg, 29.8%) as white solid. ¹*H*-NMR (DMSO *d*₆) δ 7.49-7.51 (d, 1H), 7.44 (s, 1H), 7.28-7.32 (dd, 1H), 7.11-7.13 (d, 1H), 5.56 (s, 2H), 3.43 (s, 3 H), 3.33-3.35 (t, 2H), 3.22-3.24 (t, 2H), 3.22 (s, 3H), 2.72-2.76 (t, 2H), 1.80-1.84 (t, 2H),1.42-1.46 (m, 2H), 0.80-0.83 (t, 3H). LCMS retention time 2.726 min; LCMS MH⁺ 449.

### Example 26 Ethyl 4-(7-(3-bromobenzyl)-1,3-dimethyl-2,6-dioxo-2,3,6,7-tetrahydro-1H-purin-8-yl)butanoate (Compound 50)

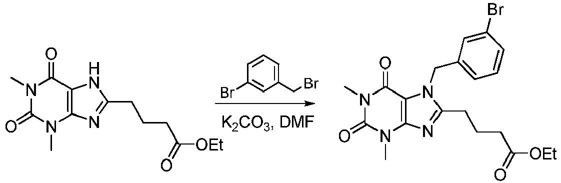

The title compound was prepared following the procedure of example 14 and using intermediate 13 to give 4-(1,3-dimethyl-2,6-dioxo-2,3,6,7-tetrahydro-1H-purin-8-yl)butanoate (0.69 g, 96%) as brown solid. ¹*H*-NMR (DMSO*d*₆) δ 7.48-7.50 (d, 1H), 7.44 (s, 1H), 7.27-7.30 (t, 1H), 7.10-7.12 (d, 1H), 5.55 (s, 2H), 3.98-4.04 (q, 2 H), 3.43 (s, 3H), 3.21 (s, 3H), 2.71-2.75 (t, 2H), 2.34-2.38 (t, 2H), 1.82-1.86 (m, 2H), 1.12-1.16 (t, 3H). LCMS retention time 2.571 min; LCMS MH⁺ 463.

### Example 27 7-(3-bromobenzyl)-8-(butoxymethyl)-1,3-dimethyl-1H-purine-2,6(3H,7H)-dione (Compound 55)

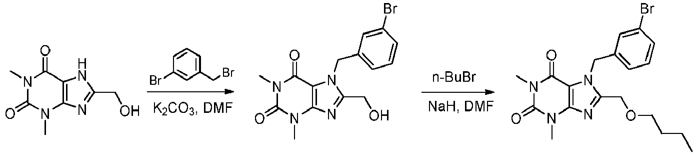

### Step 1 7-(3-bromobenzyl)-8-(hydroxymethyl)-1,3-dimethyl-1H-purine-2,6(3H,7H)-dione

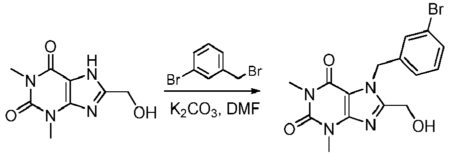

The title compound was prepared using the method of example 14 and using intermediate 14 to give 7-(3-bromobenzyl)-8-(hydroxymethyl)-1,3-dimethyl-1H-purine-2,6(3H,7H)-dione (1.26 g, 80.26% yield) as yellow solid. ¹*H*-NMR (DMSO *d*₆) δ 7.47-7.49 (d, 1H), 7.27-7.29 (t, 1H), 7.19-7.21 (d, 1H), 5.79-5.82 (t, 1H), 5.59 (s, 2H), 4.58-4.59 (d, 1 H), 3.43 (s, 3H), 3.20 (s, 3H). LCMS retention time 1.822 min; LCMS MH⁺ 379.

### Step 2 7-(3-bromobenzyl)-8-(butoxymethyl)-1,3-dimethyl-1H-purine-2,6(3H,7H)-dione

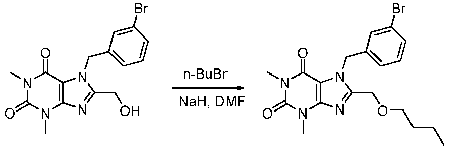

To a solution of 7-(3-bromobenzyl)-8-(hydroxymethyl)-1,3-dimethyl-1H-purine-2,6(3H,7H)-dione (100 mg, 0.265 mmol) in anhydrous DMF (2 mL) was added sodium hydride (21.2 mg, 0.53 mmol, 60% dispersion in mineral oil) in portions at 0 °C. The mixture was stirred at room temperature for 30 min; then 1-bromobutane (65 mg, 0.53 mmol) was added. The resulting mixture was stirred at room temperature for 3 h. The mixture was quenched with saturated aqueous ammonium chloride (5 mL). The mixture was extracted with ethyl acetate and the organic phase was concentrated to give crude product, which was purified via preparative HPLC to give 7-(3-bromobenzyl)-8-(butoxymethyl)-1,3-dimethyl-1H-purine-2,6(3H,7H)-dione (29 mg, 25.1% yield) as white solid. ¹*H*-NMR (DMSO *d*₆) δ 7.42 (d, 1H), 7.40 (s, 1H), 7.18-7.20 (dd, 1H), 5.63 (s, 2H), 4.53 (s, 2H), 3.59 (s, 3H), 3.45-3.48 (t, 2H), 3.39 (s, 3H), 1.50-1.54 (t, 3H), 1.29-1.33 (t, 3H), 0.87-0.89 (t, 3H). LCMS retention time 2.900 min; LCMS MH⁺ 435.

### Example 28 7-(3-bromobenzyl)-1,3-dimethyl-8-pentanoyl-1H-purine-2,6(3H,7H)-dione (Compound 105)

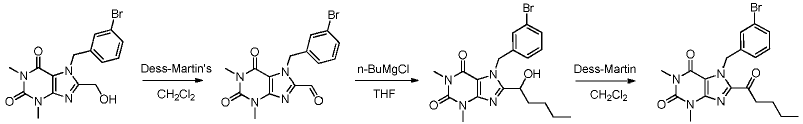

### Step 1 7-(3-bromobenzyl)-1,3-dimethyl-2,6-dioxo-2,3,6,7-tetrahydro-1H-purine-8-carbaldehyde

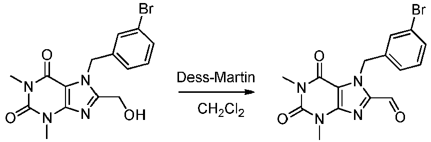

To a solution of 7-(3-bromobenzyl)-8-(hydroxymethyl)-1,3-dimethyl-1H-purine-2,6(3H,7H)-dione (100 mg, 0.265 mmol, product of example 28 step 2) in DCM (3 mL) was added Dess-Martin periodinane (224 mg, 0.53 mmol) at 0 °C. After addition, the mixture was stirred at room temperature for 16 h. The mixture was diluted with DCM and washed with saturated aqueous sodium bicarbonate. The organic phase was concentrated to give the crude product, which was purified via silica gel chromatography eluting with DCM/methanol (50: 1) to give 7-(3-bromobenzyl)-1,3-dimethyl-2,6-dioxo-2,3,6,7-tetrahydro-1H-purine-8-carbaldehyde (71 mg, 70.6% yield) as yellow solid. LCMS MH⁺ 377.

### Step 2 7-(3-bromobenzyl)-8-(1-hydroxypentyl)-1,3-dimethyl-1H-purine-2,6(3H,7H)-dione

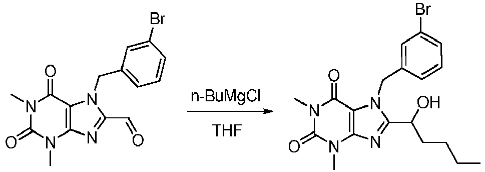

To a -78 °C solution of 7-(3-bromobenzyl)-1,3-dimethyl-2,6-dioxo-2,3,6,7-tetrahydro-1H-purine-8-carbaldehyde (71 mg, 0.188 mmol) was added n-butylmagnesium chloride (0.28 mL, 0.28 mmol, 1M solution in THF) dropwise. Then the mixture was stirred at this temperature for 2 h. The mixture was quenched with saturated aqueous ammonium chloride and extracted with ethyl acetate. The organic layer was concentrated to dryness to give crude product, which was purified via preparative HPLC to give 7-(3-bromobenzyl)-8-(1-hydroxypentyl)-1,3-dimethyl-1H-purine-2,6(3H,7H)-dione (11.2 mg, 13.7%) as white solid. ¹*H*-NMR (DMSO-*d₆*) δ 7.31-7.33 (d, 1H), 7.30 (s, 1H), 7.27-7.29 (dd, 1H), 7.14(d, 1H), 5.77-5.78 (d, 1H), 5.73 (s, 1H), 5.60(s, 1H), 5.56 (s,1H), 4.67-4.69 (m, 1H), 3.44 (s, 3H), 3.27 (s, 3H), 1.67-1.73 (m, 2H), 1.07-1.19 (m, 4H), 0.77-0.80 (t, 3H). LCMS retention time 2.599 min; LCMS MH⁺ 435.

### Step 3 7-(3-bromobenzyl)-1,3-dimethyl-8-pentanoyl-1H-purine-2,6(3H,7H)-dione

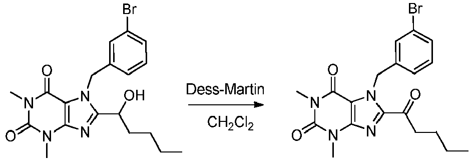

The title compound was prepared following the procedure of example 28 step 1 and purified via preparative HPLC to give 7-(3-bromobenzyl)-1,3-dimethyl-8-pentanoyl-1H-purine-2,6(3H,7H)-dione (3.1 mg, 42.6%) as white solid. **¹***H*-NMR (DMSO-*d₆*) δ 7.41-7.43 (d, 1H), 7.39 (s, 1H), 7.23-7.27 (t, 1H), 7.12-7.14 (d, 1H), 5.88 (s, 2H), 3.46 (s, 3H), 3.22 (s, 3H), 3.06-3.09 (t, 2H), 1.52-1.55 (m, 2H), 1.21-1.28 (m, 2H), 0.82-0.85 (t, 3H). LCMS retention time 3.161 min; LCMS MH⁺ 433.

### Example 29 ethyl 3-((7-(3-bromobenzyl)-1,3-dimethyl-2,6-dioxo-2,3,6,7-tetrahydro-1H-purin-8-yl)methyl)benzoate (Compound 102)

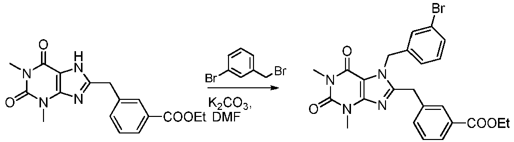

The title compound was prepared following the procedure of example 14 and using intermediate 18 to give 24 (130 mg, 79.2% yield) as white solid. ¹*H*-NMR (DMSO-*d₆*) δ 7.75 (d, 1H), 7.72 (s, 1H), 7.34-7.40(m, 3H), 7.13-7.17(m, 2H), 5.63 (s, 2H), 4.26-4.31 (m, 4H), 3.44(s, 3H), 3.21(s, 3H), 1.28-1.32 (t, 3H). LCMS retention time 2.825 min; LCMS MH⁺ 511.

### Example 30 7-(3-bromobenzyl)-8-(1-(3-(ethoxymethyl)phenyl)propyl)-1,3-dimethyl-1H-purine-2,6(3H,7H)-dione (Compound 125)

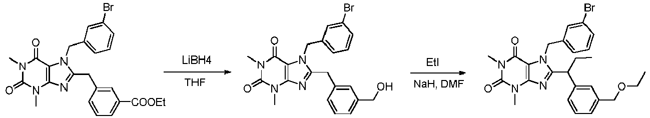

### Step 1 7-(3-bromobenzyl)-8-(3-(hydroxymethyl)benzyl)-1,3-dimethyl-1H-purine-2,6(3H,7H)-dione

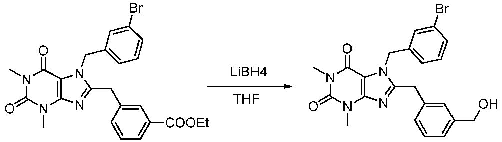

To a solution of ethyl 3-((7-(3-bromobenzyl)-1,3-dimethyl-2,6-dioxo-2,3,6,7-tetrahydro-1H-purin-8-yl)methyl)benzoate (200 mg, 0.392 mmol, product of example 29) in THF (6 mL) was added lithium borohydride (17 mg, 0.385 mmol). The resulting mixture was stirred at room temperature for 16 h. The mixture was quenched with dilute hydrochloride acid and extracted with ethyl acetate. The organic layer was concentrated to give the product, which was purified via silica gel eluted with DCM / methanol (40: 1) to give 7-(3-bromobenzyl)-8-(3-(hydroxymethyl)benzyl)-1,3-dimethyl-1H-purine-2,6(3H,7H)-dione (80 mg, 43.5% yield) as yellow solid. ¹*H*-NMR (400 Hz, DMSO-*d*₆) δ= 7.42-7.44 (d, 1H), 7.28 (s, 1H), 7.12-7.24 (m, 3H), 7.00-7.06 (m, 3H), 5.58 (s, 2H), 5.12-5.15 (t, 1H), 4.38-4.39 (d, 2H), 4.17 (s, 2H), 3.44(s, 3H), 3.21(s, 3H). LCMS retention time 2.055 min; LCMS MH⁺ 469.

### Step 2 7-(3-bromobenzyl)-8-(1-(3-(ethoxymethyl)phenyl)propyl)-1,3-dimethyl-1H-purine-2,6(3H,7H)-dione

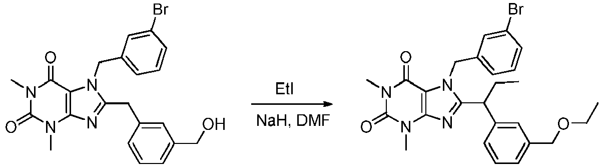

To a solution of 7-(3-bromobenzyl)-8-(3-(hydroxymethyl)benzyl)-1,3-dimethyl-1H-purine-2,6(3H,7H)-dione (50 mg, 0.107 mmol) in DMF (1 mL) was added sodium hydride (8.5 mg, 0.21 mmol, 60% dispersion in mineral oil) at 0 °C. After addition, the mixture was stirred at room temperature for 16 h. The mixture was quenched with saturated aqueous ammonium chloride and extracted with ethyl acetate. The organic layer was concentrated to give crude product, which was purified via preparative HPLC to give 7-(3-bromobenzyl)-8-(1-(3-(ethoxymethyl)phenyl)propyl)-1,3-dimethyl-1H-purine-2,6(3H,7H)-dione (21 mg, 37.3% yield) as white solid. **¹***H*-NMR (400 Hz, DMSO-*d*₆) δ= 7.38-7.40 (d, 1H), 7.08-7.19 (m, 6H), 6.95-6.97 (d, 1H), 5.51-5.67 (dd, 2H), 4.32 (s, 2H), 4.13-4.17 (t, 1H), 3.50 (s, 3H), 3.37-3.43 (m, 2H), 3.18 (s, 3H), 2.09-2.15 (m, 1H), 1.91-1.96 (m, 1H), 1.10-1.14 (t, 3H), 0.76-0.79 (t, 3H). LCMS retention time 2.882 min; LCMS MH⁺ 525.

### Example 31 7-(3-bromobenzyl)-8-(3-butyrylbenzyl)-1,3-dimethyl-1H-purine-2,6(3H,7H)-dione (Compound 161)

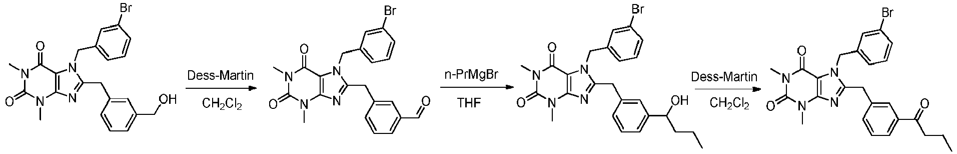

The title compound was prepared following the method of example 28 and purified via preparative HPLC to give 7-(3-bromobenzyl)-8-(3-butyrylbenzyl)-1,3-dimethyl-1H-purine-2,6(3H,7H)-dione (11.5 mg, 74.5% yield for the last step) as a white solid. ¹*H*-NMR (DMSO-d*₆*) δ 7.74-7.76 (d, 1H), 7.69 (s, 1H), 7.35-7.37 (m, 3H), 7.13-7.17 (m, 2H), 6.98-7.00 (d, 1H), 5.63 (s, 2H), 4.29 (s, 2H), 3.44 (s, 3H), 3.20 (s, 3H), 2.85-2.89 (t, 2H), 1.57-1.62 (m, 2H), 0.89-0.93 (t, 3H). LCMS retention time 2.693 min; LCMS MH⁺ 509.

### Example 32 1,3-dimethyl-8-(3-(4-methyloxazol-2-yl)benzyl)-1H-purine-2,6(3H,7H)-dione (LY-000266-122) (Compound 104)

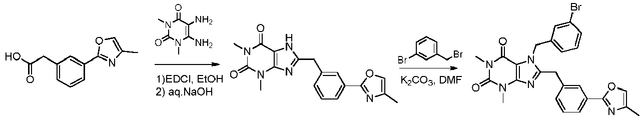

The title compound was prepared using the methods of intermediate 10, step 1 (with intermediate 20) and example 14 to give 25 (10.3 mg, 29.1% yield) as a white solid. ¹*H*-NMR (DMSO-d₆) δ 7.88 (s, 1H), 7.72-7.74 (d, 1H), 7.68 (s, 1H), 7.33-7.35 (m, 2H), 7.28 (dd, 1H), 7.22 (s, 1H), 7.12-7.14(t, 1H), 7.03 (d, 1H), 5.65 (s, 2H), 4.30 (s, 2H), 3.44(s, 3H), 3.21(s, 3H), 2.33(s, 3H). LCMS retention time 2.672 min; LCMS MH⁺ 520.

### Example 33 7-((6-chloropyridin-3-yl)methyl)-1,3-dimethyl-8-(3-(trifluoromethyl)phenoxy)-1H-purine-2,6(3H,7H)-dione (Compound 143)

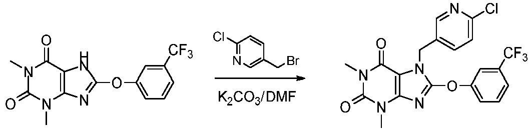

To a solution of 1,3-dimethyl-8-(3-(trifluoromethyl)phenoxy)-1H-purine-2,6(3H,7H)-dione (100 mg, 0.294 mmol, Intermediate 5) in DMF (3 mL) was added 5-(bromomethyl)-2-chloropyridine (137 mg, 0.668 mmol) followed by potassium carbonate (122 mg, 0.883 mmol). The mixture was stirred at 80 °C overnight. The mixture was diluted with ethyl acetate and water, and the phases were separated. The organic phase was washed with brine, dried over sodium sulfate, filtered and concentrated to give crude product. This material was purified by silica gel chromatography eluting with DCM/ methanol = 100: 1 and washed with ethanol and dried in vacuo to give 7-((6-chloropyridin-3-yl)methyl)-1,3-dimethyl-8-(3-(trifluoromethyl)phenoxy)-1H-purine-2,6(3H,7H)-dione (20 mg, 14.6%) as white solid. ¹*H*-NMR (DMSO-*d₆*) δ 8.52-8.51(d,1H), 7.92-7.86(m,2H), 7.76-7.71(m,3H), 7.55-7.53(d,1H, )3.24(s,3H), 3.29(s,3H). LCMS retention time 2.645 min; LCMS MH⁺ 466.

The following examples 34a to 34p were prepared using the general method of Example 33.

### Example 34a Tert-butyl 2-(7-(3-bromobenzyl)-1,3-dimethyl-2,6-dioxo-2,3,6,7-tetrahydro-1H-purin-8-ylthio)propanoate (Compound 175)

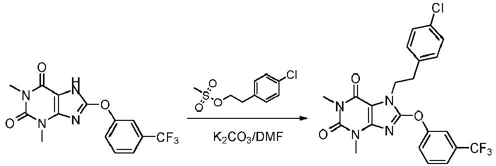

The title product was prepared using 4-chlorophenethyl methanesulfonate (intermediate 7 step 1) as the alkylating and the crude product was purified by preparative HPLC and lyophilized to give the white solid product: 20 mg (14.6%); ¹*H*-NMR (DMSO-*d₆*) δ 7.66-7.65(m, 2H), 7.38-7.36(m, 2H), 7.28-7.26(d, 2H), 7.18-7.16(d, 2H), 4.44-4.41(t, 2H), 3.274(s, 6H), 3.12-3.09(t, 2H). LCMS retention time 3.315 min; LCMS MH⁺ 479.

### Example 34b 1,3-dimethyl-7-(naphthalen-1-ylmethyl)-8-(3-(trifluoromethyl)phenoxy)-1H-purine-2,6(3H,7H)-dione (Compound 179)

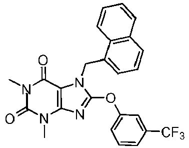

White solid, 20 mg, 23.6% yield: ¹*H*-NMR (DMSO-*d₆*) δ 8.26-8.23(d, 1H), 8.01-7.98(d, 1H), 7.90-7.88(d, 1H), 7.67-7.59(m, 6H), 7.47-7.43(t, 1H), 7.12-7.10(d, 1H), 6.02(s, 2H), 3.35(s, 3H), 3.22(s, 3H). LCMS retention time 3.308 min; LCMS MH⁺ 481.

### Example 34c 7-(1-(4-chlorophenyl)ethyl)-1,3-dimethyl-8-(3-(trifluoromethyl)phenoxy)-1H-purine-2,6(3H,7H)-dione (Compound 187)

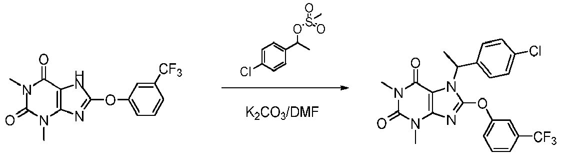

The title product was prepared using 1-(4-chlorophenyl)ethyl methanesulfonate (intermediate 21) as the alkylating agent and the crude product was purified by preparative HPLC to give the white solid product: 18 mg, 18.3% yield: ¹*H*-NMR (DMSO-*d₆*) δ 1.97-1.95(d,3H), 3.19(s,3H), 3.26(s,3H), 6.24-6.22(q,1H), 7.42-7.36(m,4H), 7.67-7.58(m,4H). LCMS retention time 3.456 min; LCMS MH⁺ 479.

### Example 34d 7-((2-chloropyridin-4-yl)methyl)-1,3-dimethyl-8-(3-(trifluoromethyl)phenoxy)-1H-purine-2,6(3H,7H)-dione (Compound 223)

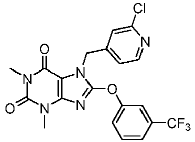

White solid purified by preparative TLC: 30 mg, 25.2% yield: ¹*H*-NMR (DMSO-*d₆*) δ 8.40-8.39(d, 1H), 7.85(s, 1H), 7.74-7.71(m, 3H), 7.52(s, 1H), 7.37-7.35(m, 1H), 5.46(s, 2H), 3.32(s, 3H), 3.22(s, 3H). LCMS retention time 2.908 min; LCMS MH⁺ 466.

### Example 34e 1,3-dimethyl-7-((5-methylpyridin-3-yl)methyl)-8-(3-(trifluoromethyl)phenoxy)-1H-purine-2,6(3H,7H)-dione (Compound 224)

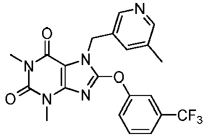

White solid: 25 mg, 24.3% yield: ¹*H*-NMR (DMSO-*d₆*) δ 8.45(s,1H), 8.36(s,1H), 7.80(s,1H), 7.73-7.69(m,3H), 7.61(s,1H), 5.48(s,2H), 3.35(s,3H), 3.25(s,3H), 2.27(s,3H). LCMS retention time 2.431 min; LCMS MH⁺ 446.

### Example 34f 7-((6-chloropyridin-2-yl)methyl)-1,3-dimethyl-8-(3-(trifluoromethyl)phenoxy)-1H-purine-2,6(3H,7H)-dione (Compound 225)

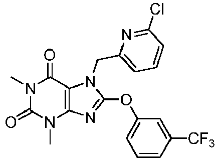

White solid: 25 mg, 25.8%yield: ¹*H*-NMR (DMSO*-d₆*) δ 7.91-7.87(t, 1H), 7.76-7.69(m, 4H), 7.46-7.44(m, 2H), 5.59(s, 2H), 3.35(s, 3H), 3.19(s, 3H). LCMS retention time 2.616 min; LCMS MH⁺ 466.

### Example 34g 1,3-dimethyl-7-((6-methylpyridin-2-yl)methyl)-8-(3-(trifluoromethyl)phenoxy)-1H-purine-2,6(3H,7H)-dione (Compound 226)

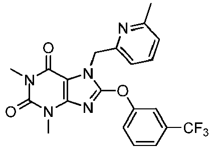

White solid: 30 mg, 32.2%yield: ¹*H*-NMR (DMSO-*d₆*) δ 7.73-7.68(m,5H), 7.17-7.14(m,2H), 5.53(s,2H), 3.35(s,3H), 3.19(s,3H), 2.35(s,3H). LCMS retention time 2.303 min; LCMS MH⁺ 446.

### Example 34h 1,3-dimethyl-7-((6-methylpyridin-3-yl)methyl)-8-(3-(trifluoromethyl)phenoxy)-1H-purine-2,6(3H,7H)-dione (Compound 227)

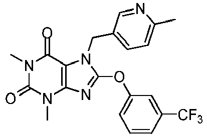

White solid: 30 mg, 17.6 %yield: ¹*H*-NMR (DMSO-*d₆*) δ 8.64(s,1H), 7.79-7.77(dd, 1H), 7.59-7.49(m, 4H), 7.18-7.16(d, 1H), 5.48(s, 2H), 3.46-3.44(d, 6H), 2.56(s, 3H). LCMS retention time 1.764 min; LCMS MH⁺ 446.

### Example 34i 1,3-dimethyl-8-(3-(trifluoromethyl)phenoxy)-7-((6-(trifluoromethyl)pyridin-3-yl)methyl)-1H-purine-2,6(3H,7H)-dione (Compound 228)

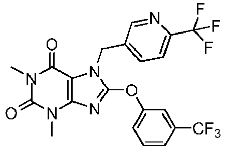

White solid: 30 mg, 20.4 %yield: ¹*H*-NMR (DMSO-*d₆*) δ 8.86-8.85(d, 1H), 8.12-8.09(dd, 1H), 7.93-7.91(d, 1H), 7.85(s, 1H), 7.79-7.68(m, 3H), 5.62(s, 2H), 3.30(s, 3H), 3.29(s, 3H). LCMS retention time 2.865 min; LCMS MH⁺ 500.

### Example 34j 1,3-dimethyl-7-((5-methylthiazol-2-yl)methyl)-8-(3-(trifluoromethyl)phenoxy)-1H-purine-2,6(3H,7H)-dione (Compound 229)

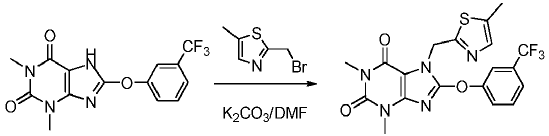

The title product was prepared using 2-(bromomethyl)-5-methylthiazole (intermediate 22) as the alkylating agent and the crude product was purified by silica gel chromatography eluting with DCM/methanol (80:1) to give the white solid product: 49 mg, 46.1% yield: ¹*H*-NMR (DMSO-*d₆*) δ 7.85(s, 1H), 7.76-7.75(m, 2H), 7.73-7.72(bs, 2H), 5.62(s, 2H), 3.28(s, 3H), 3.26(s, 3H), 2.60(s, 3H). LCMS retention time 2.547 min; LCMS MH⁺ 452.

### Example 34k 1,3-dimethyl-7-((5-methylpyridin-2-yl)methyl)-8-(3-(trifluoromethyl)phenoxy)-1H-purine-2,6(3H,7H)-dione (Compound 230)

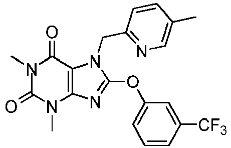

White solid: 46 mg, 35.1% yield: ¹*H*-NMR (DMSO-*d₆*) δ 8.29(s, 1H), 7.69-7.60(m, 4H), 7.32-7.29(d, 1H), 5.54(s, 2H), 3.31(s, 3H), 3.18(s, 3H), 2.25(s, 3H). LCMS retention time 2.597 min; LCMS MH⁺ 446.

### Example 34l 1,3-dimethyl-8-(3-(trifluoromethyl)phenoxy)-7-((5-(trifluoromethyl)pyridin-2-yl)methyl)-1H-purine-2,6(3H,7H)-dione (Compound 231)

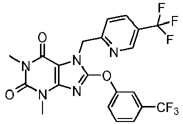

White solid: 61 mg, 41.7 %yield: ¹*H*-NMR (DMSO-*d₆*) δ 8.89(s,1H), 8.26-8.23(d,1H), 7.71-7.67(m, 5H), 5.72(s, 2H), 3.37(s, 3H), 3.18(s, 3H). LCMS retention time 2.917 min; LCMS MH⁺ 500.

### Example 34m 7-((2-chlorothiazol-5-yl)methyl)-1,3-dimethyl-8-(3-(trifluoromethyl)phenoxy)-1H-purine-2,6(3H,7H)-dione (Compound 232)

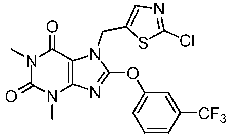

White solid recrystallized from ethanol: 10.5 mg, 15.1 %yield: ¹*H*-NMR (DMSO-*d₆*) δ 7.87(s, 1H), 7.78-7.69(m, 4H), 5.62(s, 2H), 3.26(s, 3H), 3.24(s, 3H). LCMS retention time 3.016min; LCMS MH⁺ 472.

### Example 34n 1,3-dimethyl-7-(naphthalen-2-ylmethyl)-8-(3-(trifluoromethyl)phenoxy)-1H-purine-2,6(3H,7H)-dione (Compound 185)

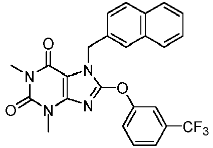

White solid recrystallized from ethanol: 20.5 mg, 29.6 % yield: ¹*H*-NMR (DMSO-*d₆*) δ 7.93-7.88(m, 4H), 7.77(s, 1H), 7.71-7.66(m, 3H), 7.57-7.50(m, 3H), 5.64(s, 2H), 3.31(s, 3H), 3.24(s, 3H). LCMS retention time 3.354min; LCMS MH⁺ 481.

### Example 34o 1,3-dimethyl-7-(thiazol-2-ylmethyl)-8-(3-(trifluoromethyl)phenoxy)-1H-purine-2,6(3H,7H)-dione (Compound 233)

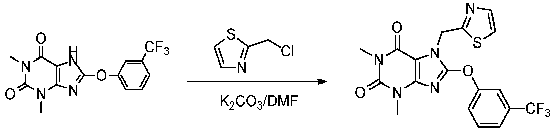

The title product was prepared using 2-chloromethylthiazole (intermediate 23) as the alkylating agent and the crude product was recrystallized from ethanol to give the pale white solid product: 5 mg, 7.6 %yield: **¹***H*-NMR (DMSO-*d₆*) δ 7.77-7.76(m,3H), 7.72-7.71(m,3H), 5.82(s,2H), 3.31(s,3H), 3.22(s,3H). LCMS retention time 2.517 min; LCMS MH⁺ 438.

### Example 34p 1,3-dimethyl-7-(pyrimidin-4-ylmethyl)-8-(3-(trifluoromethyl)phenoxy)-1H-purine-2,6(3H,7H)-dione (Compound 233)

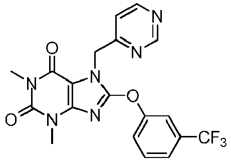

White solid purified by preparative HPLC:30 mg, 46.1% yield: ¹*H*-NMR (DMSO-d₆) δ 9.11-9.10(d,1H), 8.81-8.80(d,1H), 7.78(s,1H), 7.72-7.68(m,3H), 7.59-7.58(m,1H), 5.64(s,2H), 3.33(s,3H), 3.17(s,3H). LCMS retention time 2.517 min; LCMS MH⁺ 433.

### Example 35 1,3-dimethyl-7-(3-(trifluoromethoxy)benzyl)-8-(3-(trifluoromethoxy)phenoxy)-1H-purine-2,6(3H,7H)-dione (Compound 166)

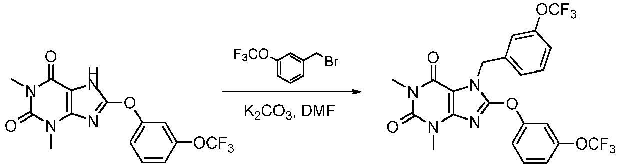

To a solution of 1,3-dimethyl-8-(3-(trifluoromethoxy)phenoxy)-1H-purine-2,6(3H,7H)-dione (50 mg, 0.14 mmol, Intermediate 5) in DMF (3 mL) was added 1-(bromomethyl)-3-(trifluoromethoxy)benzene (42 mg, 0.168 mmol) followed by potassium carbonate (30 mg, 0.21 mmol). The mixture was stirred at 50 °C for 2 h. The mixture was cooled and partitioned between ethyl acetate and water. The organic layers were combined, dried over sodium sulfate, filtered and concentrated to give a crude product which was re-crystallized from ethanol and dried in vacuo to give 1,3-dimethyl-7-(3-(trifluoromethoxy)benzyl)-8-(3-(trifluoromethoxy)phenoxy)-1H-purine-2,6(3H,7H)-dione (40 mg, 53.9%) as white solid. ¹*H-*NMR (DMSO-*d₆*) *δ* 7.61 (t, 1H),7.51 (t, 1 H), 7.40-7.45 (m, 4 H), 7.33 (d, 2 H), 5.51(s, 2 H), 3.31(s, 3 H), 3.24(s, 3 H). LCMS retention time 2.636 min; LCMS MH⁺ 531.

The following examples 36a to 36r were prepared following the general procedure of example 35.

### Example 36a 7-(3-methoxybenzyl)-1,3-dimethyl-8-(3-(trifluoromethoxy)phenoxy)-1H-purine-2,6(3H,7H)-dione (Compound 160)

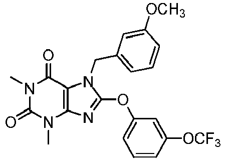

White solid: 31 mg, 46.5%: ¹*H*-NMR (DMSO-*d₆*) *δ* 7.61 (t, 1H),7.47 (s, 1H), 7.41 (dd, 1H),7.26-7.34 (m, 2H), 6.87-6.96 (m, 3H), 5.41(s, 2H), 3.71 (s, 3H), 3.30 (s, 3H), 3.24(s, 3H). LCMS retention time 2.341 min; LCMS MH⁺ 477.

### Example 36b 1,3-dimethyl-7-(3-methylbenzyl)-8-(3-(trifluoromethoxy)phenoxy)-1H-purine-2,6(3H,7H)-dione (Compound 167)

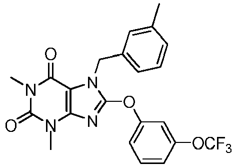

White solid: 29 mg, 45.4% yield: ¹*H*-NMR (DMSO-*d₆*) *δ*= 7.60 (t, 1H),7.45 (s, 1H), 7.40 (dd, 1H),7.31-7.34 (m, 1H),7.24 (t, 1H), 7.10-7.18 (m, 3H), 5.42(s, 2H), 3.30 (s, 3H), 3.24(s, 3H), 2.26 (s, 3H). LCMS retention time 2.524 min; LCMS MH⁺) 461.

### Example 36c 7-(3-chlorobenzyl)-1,3-dimethyl-8-(3-(trifluoromethoxy)phenoxy)-1H-purine-2,6(3H,7H)-dione (Compound 168)

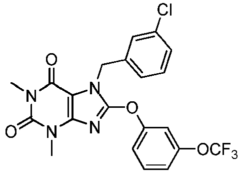

White solid: 29 mg, 44.6 % yield: ¹*H*-NMR (DMSO-*d₆*) *δ* 7.61 (t, 1H),7.48 (d, 2H), 7.40 (dd, 1H),7.34-7.44 (m, 3H),7.33 (d, 2H), 5.46 (s, 2H), 3.30 (s, 3H), 3.24(s, 3H). LCMS retention time 2.551 min; LCMS MH⁺ 481.

### Example 36d 1,3-dimethyl-7-(naphthalen-2-ylmethyl)-8-(3-(trifluoromethoxy)phenoxy)-1H-purine-2,6(3H,7H)-dione (Compound 191)

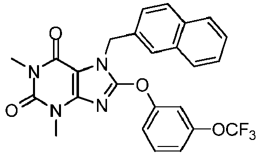

White solid: 25 mg, 38.5% yield: ¹*H*-NMR (DMSO-*d₆*) *δ* 7.86-7.93(m, 4H),7.50-7.61(m, 5H),7.43 (dd, 1H), 7.31(d, 1H) 5.63 (s, 2H), 3.31(s, 3H), 3.24(s, 3H). LCMS retention time 2.793 min; LCMS MH⁺ 468.

### Example 36e 7-(3,4-dichlorobenzyl)-1,3-dimethyl-8-(3-(trifluoromethoxy)phenoxy)-1H-purine-2,6(3H,7H)-dione (Compound 197)

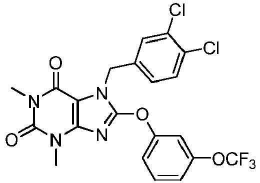

White solid: 26 mg, 40.6% yield: ¹*H*-NMR (DMSO-*d*₆) *δ* 7.59-7.68(m, 3H),7.43-7.50(m, 2H),7.32-7.37 (m, 2H), 5.45 (s, 2H), 3.31(s, 3H), 3.24(s, 3H). LCMS retention time 3.448 min; LCMS MH⁺ 515.

### Example 36f 7-(4-chloro-3-fluorobenzyl)-1,3-dimethyl-8-(3-(trifluoromethoxy)phenoxy)-1H-purine-2,6(3H,7H)-dione (Compound 198)

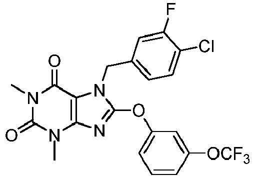

White solid: 28 mg, 40.6% yield: ¹*H*-NMR (DMSO-*d₆*) *δ* 7.60(q, 2 H),7.43-7.51(m, 3 H),7.33 (d, 1 H), 7.25 (d, 1 H),5.46 (s, 2 H), 3.31(s, 3 H), 3.24(s, 3 H). LCMS retention time 3.307 min; LCMS MH⁺ 499.

### Example 36g 7-(4-chloro-3-methoxybenzyl)-1,3-dimethyl-8-(3-(trifluoromethoxy)phenoxy)-1H-purine-2,6(3H,7H)-dione (Compound 205)

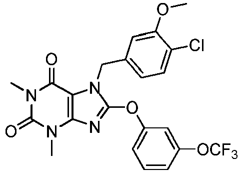

White solid: 28 mg, 40.6% yield: ¹*H*-NMR (DMSO-*d₆*) *δ* 7.61(t, 1 H),7.50 (s, 1H), 7.39-7.45(m, 2 H),7.33 (d, 1 H),7.23 (d, 1H), 6.91 (dd, 1 H),5.44 (s, 2 H), 3.80 (s, 3 H),3.30(s, 3 H), 3.24(s, 3 H). LCMS retention time 3.253 min; LCMS MH⁺ 511.

### Example 36h 7-(4-chloro-3-methylbenzyl)-1,3-dimethyl-8-(3-(trifluoromethoxy)phenoxy)-1H-purine-2,6(3H,7H)-dione (Compound 206)

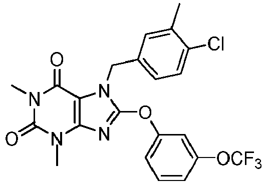

White solid: 39 mg, 56.5% yield: ¹*H*-NMR (DMSO-*d₆*) *δ* 7.61(t, 1H),7.46 (s, 1H), 7.38-7.43(m, 2H), 7.31-7.35(m, 2H),7.21 (dd, 1H),5.41 (s, 2H), 3.30(s, 3H), 3.24(s, 3H). 2.29 (s, 3H). LCMS retention time 3.458 min; LCMS MH⁺ 495.

### Example 36i 7-(4-chlorobenzyl)-1,3-dimethyl-8-(3-(trifluoromethoxy)phenoxy)-1H-purine-2,6(3H,7H)-dione (Compound 142)

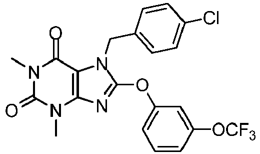

White solid: 21 mg, 31.3% yield: ¹*H*-NMR (DMSO-*d₆*) *δ* 7.61(t, 1H),7.50 (s, 1H), 7.43(s, 5H), 7.33 (d, 1H),5.45 (s, 2H), 3.31(s, 3H), 3.24(s, 3H). LCMS retention time 3.029 min; LCMS MH⁺ 481.

### Example 36j 1,3-dimethyl-8-(3-(trifluoromethoxy)phenoxy)-7-(4-(trifluoromethyl)benzyl)-1H-purine-2,6(3H,7H)-dione (Compound 234)

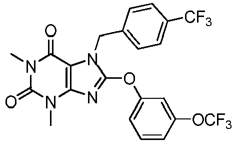

White solid: 40 mg, 55.5% yield: ¹*H*-NMR (DMSO-*d₆*) *δ* 7.74(d, 2H),7.56-7.63 (m, 3H), 7.43-7.47(m, 2H), 7.33 (d, 1H),5.56 (s, 2H), 3.31(s, 3H), 3.23(s, 3H). LCMS retention time 3.094 min; LCMS MH⁺ 515.

### Example 36k 1,3-dimethyl-7-(4-(trifluoromethoxy)benzyl)-8-(3-(trifluoromethoxy)phenoxy)-1H-purine-2,6(3H,7H)-dione (Compound 177)

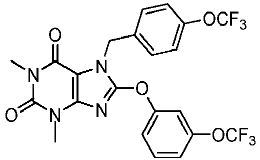

White solid: 23 mg, 31.1% yield: ¹*H*-NMR (DMSO-*d₆*) *δ* 7.61(t, 1H),7.53(d, 2H),7.43-7.49 (m, 2H), 7.32-7.38(m, 3H), 5.49 (s, 2H), 3.30(s, 3H), 3.24(s, 3H). LCMS retention time 3.183 min; LCMS MH⁺ 531.

### Example 36l 1,3-dimethyl-7-(4-methylbenzyl)-8-(3-(trifluoromethoxy)phenoxy)-1H-purine-2,6(3H,7H)-dione (Compound 178)

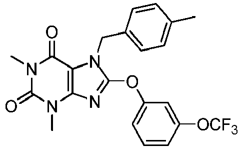

White solid: 33 mg, 51.5% yield: ¹*H*-NMR (DMSO-*d₆*) *δ* 7.61(t, 1H),7.41-7.46(m, 2H),7.27-7.34(m, 3H), 7.16(d, 2H), 5.40 (s, 2H), 3.29(s, 3H), 3.24(s, 3H). 2.27(s, 3H). LCMS retention time 3.047 min; LCMS MH⁺ 461.

### Example 36m 7-(3,5-dichlorobenzyl)-1,3-dimethyl-8-(3-(trifluoromethoxy)phenoxy)-1H-purine-2,6(3H,7H)-dione (Compound 204)

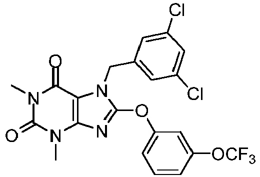

White solid: 30 mg, 41.6% yield: ¹*H*-NMR (DMSO-*d₆*) *δ* 7.57-7.63(m, 2H), 7.50(s, 1H),7.43-7.45(m, 3H), 7.31-7.34(m, 1H), 5.46 (s, 2H), 3.31(s, 3H), 3.24(s, 3H). LCMS retention time 3.484 min; LCMS MH⁺ 515.

### Example 36n 1,3-dimethyl-7-((4-methylthiazol-2-yl)methyl)-8-(3-(trifluoromethoxy)phenoxy)-1H-purine-2,6(3H,7H)-dione (Compound 235)

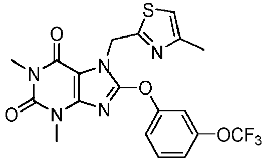

The title product was prepared using 2-(chloromethyl)-4-methylthiazole (intermediate 40). White solid: 45 mg, 34.6% yield: ¹*H*-NMR (DMSO-*d₆*) *δ* 7.63(t, 1H), 7.42-7.47(m, 2H), 7.35 (d, 1H), 7.26 (s, 1H) 5.74 (s, 2H), 3.31(s, 3H), 3.22(s, 3H), 2.30(s, 3H). LCMS retention time 2.793 min; LCMS MH⁺ 468.

### Example 36o 1,3-dimethyl-7-((2-methylpyrimidin-5-yl)methyl)-8-(3-(trifluoromethoxy)phenoxy)-1H-purine-2,6(3H,7H)-dione (Compound 236)

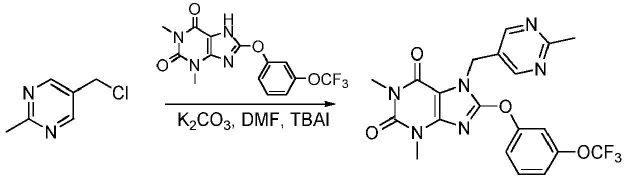

The title product was prepared using 5-(chloromethyl)-2-methylpyrimidine (intermediate 24) as the alkylating agent and with TBAI added to catalyze the reaction. The crude product was recrystallized from ethanol to give the white solid product: 21 mg, 16.2% yield: ¹*H*-NMR (DMSO-*d₆*) *δ* 8.76(s, 1H), 7.63 (t, 2H), 7.50 (dd, 1H), 7.35 (d, 1H) 5.46 (s, 2H), 3.29(s, 3H), 3.23(s, 3H), 2.60(s, 3H). LCMS retention time 1.413 min; LCMS MH⁺ 463.

### Example 36p 1,3-dimethyl-7-((5-methylpyridin-3-yl)methyl)-8-(3-(trifluoromethoxy)phenoxy)-1H-purine-2,6(3H,7H)-dione (Compound 237)

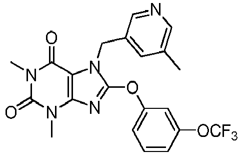

The title compound was prepared using the method of example 35 with TBAI added to catalyze the reaction. White solid: 17 mg, 26.1% yield: ¹*H*-NMR (DMSO-*d₆*) *δ* 8.44(s, 1H), 8.36(s, 1H),7.61 (t, 2H), 7.52 (s, 1H), 7.43-7.45(m, 1H),7.33(d, 1H) 5.46 (s, 2H), 3.30(s, 3H), 3.24(s, 3H), 2.27(s, 3H). LCMS retention time 2.314 min; LCMS MH⁺ 462.

### Example 36q 1,3-dimethyl-7-((5-methylpyrazin-2-yl)methyl)-8-(3-(trifluoromethoxy)phenoxy)-1H-purine-2,6(3H,7H)-dione (Compound 238)

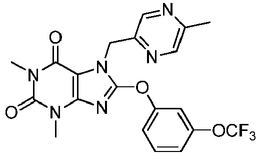

The title compound was prepared with 2-(chloromethyl)-5-methylpyrazine (intermediate 41) using the method of example 35 with TBAI added to catalyze the reaction. White solid: 15 mg, 23.1% yield: ¹*H*-NMR (DMSO-*d₆*) *δ* 8.63(s, 1H), 8.44(s, 1H),7.60 (t, 1H), 7.40-7.46(m, 2H),7.31-7.33(m, 1H) 5.60(s, 2H), 3.31(s, 3H), 3.17(s, 3H), 2.46(s, 3H). LCMS retention time 2.563 min; LCMS MH⁺ 463.

### Example 36r 7-((5-chloropyridin-3-yl)methyl)-1,3-dimethyl-8-(3-(trifluoromethoxy)phenoxy)-1H-purine-2,6(3H,7H)-dione (Compound 239)

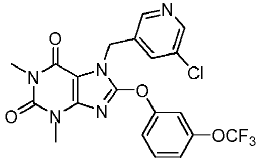

The title compound was prepared using the method of example 35 with TBAI added to catalyze the reaction. White solid: 65 mg, 48.5% yield: ¹*H*-NMR (DMSO-*d₆*) *δ* 8.52(d, 1H), 7.95(dd, 1H),7.60 (t, 1H), 7.51(d, 1H),7.41(q, 2H),7.31(d, 1H), 5.58(s, 2H), 3.32(s, 3H), 3.17(s, 3H). LCMS retention time 2.990 min; LCMS MH⁺ 482.

### Example 37 7-(4-((dimethylamino)methyl)benzyl)-1,3-dimethyl-8-(3-(trifluoromethoxy)phenoxy)-1H-purine-2,6(3H,7H)-dione (Compound 185)

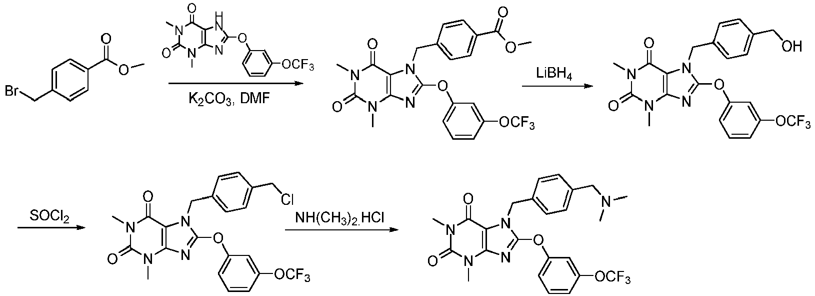

### Step 1 methyl 4-((1,3-dimethyl-2,6-dioxo-8-(3-(trifluoromethoxy)phenoxy)-2,3-dihydro-1H-purin-7(6H)-yl)methyl)benzoate

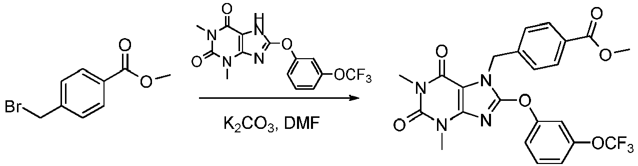

The title compound was prepared following the method of example 35. White solid: 250 mg, 100%. LCMS retention time 1.763 min; LCMS MH⁺ 505.

### Step 2 7-(4-(hydroxymethyl)benzyl)-1,3-dimethyl-8-(3-(trifluoromethoxy)phenoxy)-1H-purine-2,6(3H,7H)-dione

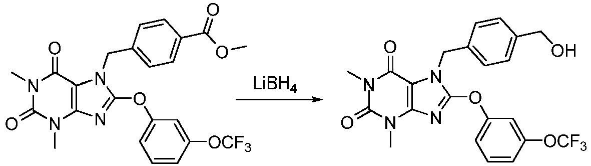

To a solution of methyl 4-((1,3-dimethyl-2,6-dioxo-8-(3-(trifluoromethoxy)phenoxy)-2,3-dihydro-1H-purin-7(6H)-yl)methyl)benzoate(200 mg, 0.4 mmol) in 1,2-dimethoxyethane(5 mL) was added lithium borohydride(44 mg, 2.0 mmol), the mixture was stirred at 50 °C overnight. The mixture was quenched with aqueous ammonium chloride, extracted with ethyl acetate, dried over sodium sulfate, filtered and concentrated to afford a crude product. This material was washed with methanol and dried in vacuo to give the white solid product 7-(4-(hydroxymethyl)benzyl)-1,3-dimethyl-8-(3-(trifluoromethoxy)phenoxy)-1H-purine-2,6(3H,7H)-dione. LCMS retention time 1.521 min; LCMS MH⁺ 477.

### Step 3 7-(4-(chloromethyl)benzyl)-1,3-dimethyl-8-(3-(trifluoromethoxy)phenoxy)-1H-purine-2,6(3H,7H)-dione

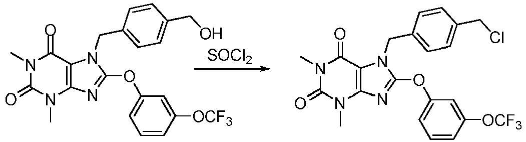

A solution of 7-(4-(hydroxymethyl)benzyl)-1,3-dimethyl-8-(3-(trifluoromethoxy)phenoxy)-1H-purine-2,6(3H,7H)-dione (40 mg, 0.84 mmol) in thionyl chloride(1 mL) was stirred at room temperature for 2 h. The mixture was concentrated to dryness and the crude product was used without purification. LCMS retention time 1.872 min; LCMS MH⁺ 495.

### Step 4 7-(4-((dimethylamino)methyl)benzyl)-1,3-dimethyl-8-(3-(trifluoromethoxy)phenoxy)-1H-purine-2,6(3H,7H)-dione

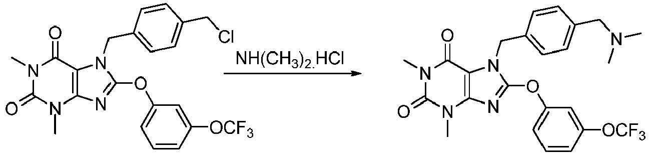

To a solution of 7-(4-(chloromethyl)benzyl)-1,3-dimethyl-8-(3-(trifluoromethoxy)phenoxy)-1H-purine-2,6(3H,7H)-dione (40 mg, 0.081 mmol) in DMF (2 mL) was added dimethylamine hydrochloride (33 mg, 0.4 mmol), potassium carbonate(23 mg, 0.17 mmol). The reaction was stirred at 30 °C for 16 h. The mixture was partitioned between ethyl acetate and water. The organic layers were combined, dried over sodium sulfate, filtered and concentrated to afford a crude product. This material was purified by preparative HPLC to give 7-(4-((dimethylamino)methyl)benzyl)-1,3-dimethyl-8-(3-(trifluoromethoxy)phenoxy)-1H-purine-2,6(3H,7H)-dione (16 mg, 39.1% yield) as white solid. ¹*H*-NMR (DMSO-*d₆*) *δ* 7.43(d, 3H), 7.31(d, 2H),7.23 (d, 2H), 7.15(d, 1H), 5.47(s, 2H), 3.47(s, 3H), 3.44(s, 5H),2.25(s, 6H). LCMS retention time 1.944 min; LCMS MH⁺ 504.

### Example 38 Ethyl 3-(7-(3-bromobenzyl)-1,3-dimethyl-2,6-dioxo-2,3,6,7-tetrahydro-1H-purin-8-yloxy)benzoate (Compound 57)

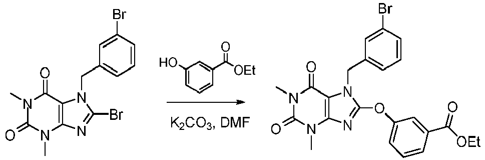

The title compound was prepared using the method of Intermediate 5 step 1 to give ethyl 3-(7-(3-bromobenzyl)-1,3-dimethyl-2,6-dioxo-2,3,6,7-tetrahydro-1H-purin-8-yloxy)benzoate (110 mg, 74.3 yield) as white solid. ¹*H*-NMR (DMSO-*d₆*) δ 7.87-7.88 (d,1H), 7.84 (s, 1H), 7.61-7.64 (m, 3H), 7.50-7.52 (d, 1H), 7.31-7.36 (m, 2H), 5.46 (s, 2H), 4.29-4.34 (m, 2H), 3.27 (s, 3H), 3.23 (s, 3H), 1.30-1.33 (t, 3H). LCMS retention time 3.007 min; LCMS MH⁺ 513.

### Example 39 7-(3-bromobenzyl)-1,3-dimethyl-8-(3-(4-methyloxazol-2-yl)phenoxy)-1H-purine-2,6(3H,7H)-dione (Compound 103)

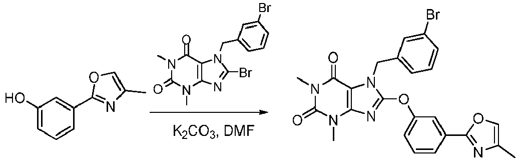

The title compound was prepared using the method of Intermediate 5 step 1 and using intermediate 25 and purified via preparative HPLC to give7-(3-bromobenzyl)-1,3-dimethyl-8-(3-(4-methyloxazol-2-yl)phenoxy)-1H-purine-2,6(3H,7H)-dione (15 mg, 33.6% yield) as white solid. ¹*H*-NMR (DMSO-*d₆*) δ 7.95 (s, 1H), 7.86-7.88 (d, 1H), 7.82 (s, 1H), 7.60-7.64 (m, 2H), 7.48-7.53 (m, 2H), 7.34-7.37 (m, 2H), 5.48 (s, 2H), 3.31(s, 3H), 3.24 (s, 3H), 2.17 (s, 3H). LCMS retention time 3.131 min; LCMS MH⁺ 522.

### Example 40 7-(3-bromobenzyl)-1,3-dimethyl-8-(3-(4-methyloxazol-2-yl)phenoxy)-1H-purine-2,6(3H,7H)-dione (Compound 126)

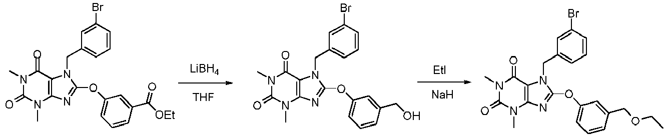

### Step 1 7-(3-bromobenzyl)-8-(3-(hydroxymethyl)phenoxy)-1,3-dimethyl-1H-purine-2,6(3H,7H)-dione

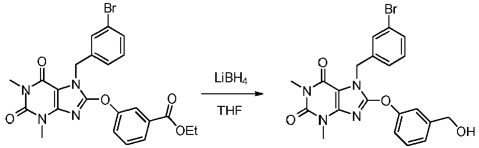

The title compound was prepared from the product of example 38 using the method of example 31 step 1 to give 7-(3-bromobenzyl)-8-(3-(hydroxymethyl)phenoxy)-1,3-dimethyl-1H-purine-2,6(3H,7H)-dione (170 mg, 74% yield) as yellow syrup. LCMS MH⁺ 471.

### Step 2 7-(3-bromobenzyl)-8-(3-(ethoxymethyl)phenoxy)-1,3-dimethyl-1H-purine-2,6(3H,7H)-dione

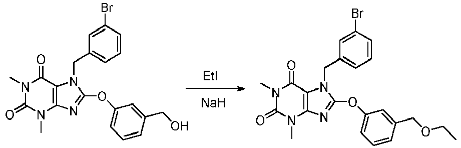

The title compound was prepared using the method of example 30 step 2 to give 7-(3-bromobenzyl)-8-(3-(ethoxymethyl)phenoxy)-1,3-dimethyl-1H-purine-2,6(3H,7H)-dione (19 mg, 79.6% yield) as white solid. ¹*H*-NMR (DMSO-*d₆*) δ 7.49-7.51 (d, 1H), 7.48 (s, 1H), 7.24-7.31 (m, 3H), 6.97-6.99 (d, 2H), 6.90-6.92 (d, 1H), 5.50 (s,2H), 5.27 (s, 2H), 3.96-4.01 (q, 2H), 3.41(s, 3H), 3.21 (s, 3H), 1.30-1.34 (t, 3H). LCMS retention time 3.077 min; LCMS MH⁺ 499.

### Example 41 7-(3-bromobenzyl)-8-(3-(1-hydroxybutyl)phenoxy)-1,3-dimethyl-1H-purine-2,6(3H,7H)-dione (Compound 173)

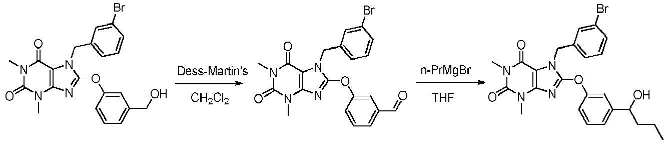

### Step 1 Synthesis of 3-(7-(3-bromobenzyl)-1,3-dimethyl-2,6-dioxo-2,3,6,7-tetrahydro-1H-purin-8-yloxy)benzaldehyde

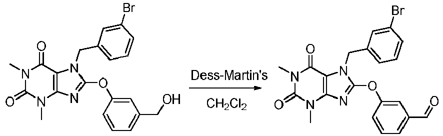

The title compound was prepared from the product of example 40, step 1 using the method of example 28, step 1 to give 3-(7-(3-bromobenzyl)-1,3-dimethyl-2,6-dioxo-2,3,6,7-tetrahydro-1H-purin-8-yloxy)benzaldehyde (370 mg, 82.6%) as yellow solid. LCMS MH⁺ 470.

### Step 2 Synthesis of 7-(3-bromobenzyl)-8-(3-(1-hydroxybutyl)phenoxy)-1,3-dimethyl-1H-purine-2,6(3H,7H)-dione

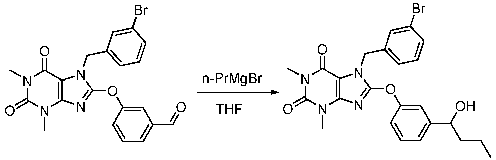

The title compound was prepared using the method of example 28, step 2 using to give -(3-bromobenzyl)-8-(3-(1-hydroxybutyl)phenoxy)-1,3-dimethyl-1H-purine-2,6(3H,7H)-dione (240 mg, 59.1% yield) as white solid. ¹*H*-NMR (DMSO-*d₆*) δ 7.57 (s, 1H), 7.46-7.47 (d, 1H), 7.31-7.36 (m, 3H), 7.15-7.22 (m, 3H), 5.42 (s,2H), 5.21-5.22 (d, 1H), 3.21(s, 3H), 3.19 (s, 3H), 1.52 (m, 2H), 1.46 (m, 2H), 081-0.85 (t, 3H). LCMS retention time 2.927 min; LCMS MH⁺ 513.

### Example 42 7-(3-bromobenzyl)-8-(3-butyrylphenoxy)-1,3-dimethyl-1H-purine-2,6(3H,7H)-dione (Compound 155)

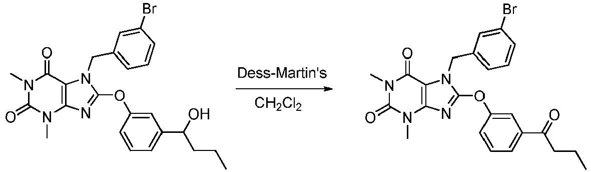

The title compound was prepared from the product of example 41 using the method of example 28 step 3 to give 7-(3-bromobenzyl)-8-(3-butyrylphenoxy)-1,3-dimethyl-1H-purine-2,6(3H,7H)-dione (19 mg, 38.3% yield) as white solid. ¹*H*-NMR (CDCl₃) δ 7.85-7.89 (m, 2H), 7.61 (s, 1H), 7.28-7.57 (m, 3H), 7.23-7.25 (t, 1H), 5.47 (s,2H), 3.44 (s, 3H), 3.45 (s, 3H), 2.95-2.98 (t, 2H), 1.77-1.83 (m, 2H), 1.01-1.05 (t, 3H). LCMS retention time 2.790 min; LCMS MH⁺ 511.

### Example 43 7-(3-bromobenzyl)-8-(3-butylphenoxy)-1,3-dimethyl-1H-purine-2,6(3H,7H)-dione (Compound 174)

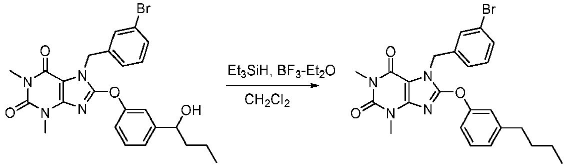

To a solution of 7-(3-bromobenzyl)-8-(3-(1-hydroxybutyl)phenoxy)-1,3-dimethyl-1H-purine-2,6(3H,7H)-dione (40 mg, 0.078 mmol, example 41 product) in DCM (2 mL) was added triethylsilane (18.1 mg, 0.156 mmol) followed by boron trifluoride etherate (0.1 mL) at 0 °C. The mixture was stirred at room temperature for 16 h. The mixture was diluted with DCM and washed with brine, dried and concentrated to give crude product. This crude material was purified via preparative HPLC to give 7-(3-bromobenzyl)-8-(3-butylphenoxy)-1,3-dimethyl-1H-purine-2,6(3H,7H)-dione (13 mg, 33.5% yield) as white solid. ¹*H*-NMR (CDCl₃) δ 7.62 (s, 1H), 7.39-7.44 (m, 2H), 7.28-7.32 (t, 3H), 7.19-7.23 (t, 1H), 7.02-7.09 (m, 3H), 5.41 (s,2H), 3.41 (s, 3H), 3.43 (s, 3H), 2.61-2.65 (t, 2H), 1.58-1.64 (m, 2H), 1.33-1.39 (m, 2H), 0.91-0.94 (t, 3H). LCMS retention time 3.622 min; LCMS MH⁺ 497.

### Example 44 7-(3-bromobenzyl)-8-(3-chlorophenoxy)-1,3-dimethyl-1H-purine-2,6(3H,7H)-dione (Compound 110)

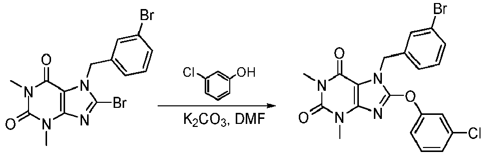

To a solution of 8-bromo-7-(3-bromobenzyl)-1,3-dimethyl-1H-purine-2, 6(3H,7H)-dione (50 mg, 0.12 mmol, intermediate 1 step 2) in DMF (3mL) was added 3-chlorophenol (23 mg, 0.17 mmol) and potassium carbonate (75 g, 0.24 mmol). The mixture was stirred at 80°C for 12 h. The mixture was partitioned between ethyl acetate and water. The organic phase was washed with brine, dried over sodium sulfate, filtered and concentrated to give crude product. This crude product was collected, washed with ethanol and dried under vacuum to give 7-(3-bromobenzyl)-8-(3-chlorophenoxy)-1,3-dimethyl-1H-purine-2,6 (3H,7H)-dione (15 mg, 26.7% yield) as white solid.¹*H*-NMR (DMSO-*d₆*) *δ* 7.60(s, 1H), 7.48-7.50(m, 3H), 7.32-7.40(m, 4H),5.44(s, 2H), 3.31(s, 3H), 3.24(s, 3H). LCMS retention time 3.151 min; LCMS MH⁺ 477.

The following examples 45a to 45n were prepared using the method of example 44.

### Example 45a 7-(3-bromobenzyl)-8-(3-fluorophenoxy)-1,3-dimethyl-1H-purine-2,6(3H,7H)-dione (Compound 111)

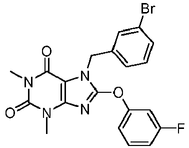

White solid, 12 mg, 22.6% yield: ¹*H*-NMR (DMSO-*d₆*) *δ* 7.60(s, 1H), 7.50-7.53(m, 2H), 7.30-7.35(m, 3H),7.17-7.21(m, 2H),5.44(s, 2H)3.31(s, 3H), 3.24(s, 3H). LCMS retention time 2.979 min; LCMS MH⁺ 459.

### Example 45b 3-(7-(3-bromobenzyl)-1,3-dimethyl-2,6-dioxo-2,3,6,7-tetrahydro-1H-purin-8-yloxy)benzonitrile (Compound 112)

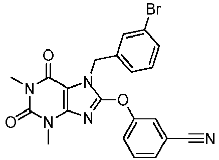

White solid, 16 mg, 29.7% yield: ¹*H*-NMR (DMSO-*d₆*) *δ* 7.95(s, 1H), 7.67-7.82(m, 3H), 7.61(s, 1H), 7.51-7.54(m, 1H),7.32-7.39(m, 2H),5.46(s, 2H), 3.31(s, 3H), 3.25(s, 3H). LCMS retention time 2.784 min; LCMS MH⁺ 466.

### Example 45c 7-(3-bromobenzyl)-1,3-dimethyl-8-(3-propylphenoxy)-1H-purine-2,6(3H,7H)-dione (Compound 113)

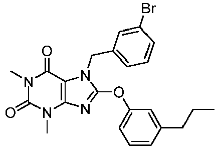

White solid, 15 mg, 26.7% yield: ¹*H*-NMR (DMSO-*d₆*) *δ* 7.59(s, 1 H), 7.52(t, 1 H), 7.34-7.38(m,3 H), 7.11-7.13(m, 2H), 7.08(d, 1H), 5.44(s, 2H), 3.29(s, 3H), 3.24(s, 3H), 2.57(d, 2H), 1.58 (q, 2H), 0.89(t, 3H). LCMS retention time 3.413 min; LCMS MH⁺ 485.

### Example 45d 8-(3-acetylphenoxy)-7-(3-bromobenzyl)-1,3-dimethyl-1H-purine-2,6(3H,7H)-dione (Compound 114)

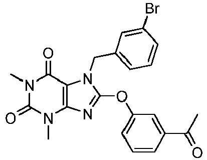

White solid, 21 mg, 37.5% yield: ¹*H*-NMR (DMSO-*d₆*) *δ* 7.90(t, 1H), 7.84(s, 1H), 7.62-7.65(m, 3H), 7.52-7.54(m, 1H), 7.35-7.37(m, 2H), 5.48(s, 2H), 3.29(s, 3H), 3.25(s, 3H), 2.61(s, 3H). LCMS retention time 2.764 min; LCMS MH⁺ 485.

### Example 45e 7-(3-bromobenzyl)-1,3-dimethyl-8-(3-(trifluoromethoxy)phenoxy)-1H-purine-2,6(3H,7H)-dione (Compound 115)

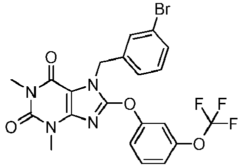

White solid, 23 mg, 37.6% yield: ¹*H*-NMR (DMSO-*d₆*) *δ* 7.62(t, 2H), 7.50-7.54(m, 2H), 7.43(d, 1H), 7.33-7.36(m, 3H), 5.46(s, 2H), 3.31(s, 3H), 3.25(s, 3H). LCMS retention time 3.180 min; LCMS MH⁺ 527.

### Example 45f 7-(4-chlorobenzyl)-1,3-dimethyl-8-(3-(trifluoromethyl)phenoxy)-1H-purine-2,6(3H,7H)-dione (Compound 139)

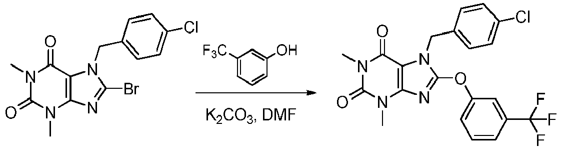

The title compound was prepared from intermediate 6. White solid, 21 mg, 19.8% yield: ¹*H-*NMR (DMSO-*d₆*) *δ* 7.86(d, 2H), 7.59(d, 2H), 7.43(s, 4H), 5.46(s, 2H), 3.31(s, 3H), 3.25(s, 3H). LCMS retention time 2.996 min; LCMS MH⁺ 465.

### Example 45g 7-(4-chlorobenzyl)-1,3-dimethyl-8-(5-(trifluoromethyl)pyridin-3-yloxy)-1H-purine-2,6(3H,7H)-dione (Compound 184)

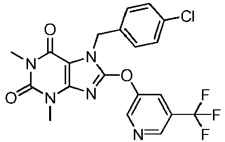

The title compound was prepared from intermediate 6. White solid, 18.6 mg, 22.7% yield: ¹*H-*NMR (DMSO-*d₆*) *δ* 9.05(d, 1H), 8.96(s, 1H), 8.44(t, 1H), 7.43-7.49(m, 4H), 5.47(s, 2H), 3.30(s, 3H), 3.24(s, 3H). LCMS retention time 2.880 min; LCMS MH⁺ 466.

### Example 45h 8-(4-chloro-3-(trifluoromethyl)phenoxy)-7-(4-chlorobenzyl)-1,3-dimethyl-1H-purine-2,6(3H,7H)-dione (Compound 200)

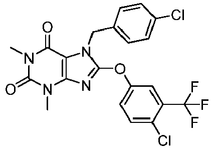

The title compound was prepared from intermediate 6. White solid, 27.1 mg, 29.5% yield: ¹*H-*NMR (DMSO-*d₆*) *δ* 7.95(d, 1H), 7.77-7.87(m, 2H), 7.43(s, 4H), 5.45(s, 2H), 3.30(s, 3H), 3.23(s, 3H). LCMS retention time 3.422 min; LCMS MH⁺ 499.

### Example 45i 7-(4-chlorobenzyl)-8-(4-fluoro-3-(trifluoromethyl)phenoxy)-1,3-dimethyl-1H-purine-2,6(3H,7H)-dione (Compound 201)

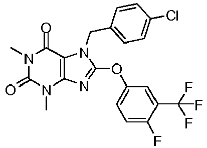

The title compound was prepared from intermediate 6. White solid, 22.8 mg, 35.4% yield: ¹*H-*NMR (DMSO-*d₆*) *δ* 7.81-7.90(m, 2H), 7.66(t, 1H), 7.44(s, 4H), 5.45(s, 2H), 3.29(s, 3H), 3.23(s, 3H). LCMS retention time 3.271 min; LCMS MH⁺ 483.

### Example 45j 8-(3-chloro-5-(trifluoromethoxy)phenoxy)-7-(4-chlorobenzyl)-1,3-dimethyl-1H-purine-2,6(3H,7H)-dione (Compound 202)

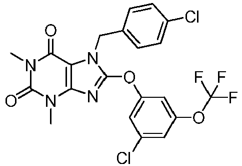

The title compound was prepared from intermediate 6. White solid, 11.8 mg, 26.6% yield: ¹*H-*NMR (DMSO-*d₆*) *δ* 7.65(d, 1H), 7.58(s, 1H), 7.55(s, 1H), 7.43(s, 4H), 5.43(s, 2H), 3.31(s, 3H), 3.24(s, 3H). LCMS retention time 3.562 min; LCMS MH⁺ 515.

### Example 45k 8-(3,5-bis(trifluoromethyl)phenoxy)-7-(4-chlorobenzyl)-1,3-dimethyl-1H-purine-2,6(3H,7H)-dione (Compound 203)

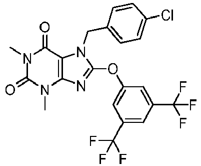

The title compound was prepared from intermediate 6. White solid, 33.7mg, 47.8% yield: ¹*H-*NMR (DMSO-*d₆*) *δ* 8.25(s, 2H), 8.10(s, 1H), 7.41-7.48(m, 4H), 5.46(s, 2H), 3.32(s, 3H), 3.24(s, 3H). LCMS retention time 3.501 min; LCMS MH⁺ 533.

### Example 45l 7-(4-chlorobenzyl)-1,3-dimethyl-8-(2-methyl-3-(trifluoromethyl)phenoxy)-1H-purine-2,6(3H,7H)-dione (Compound 207)

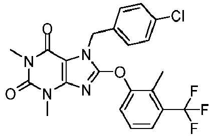

The title compound was prepared from intermediate 6. White solid, 70.9 mg, 56.5% yield: ¹*H-*NMR (DMSO-*d₆*) *δ* 7.61-7.68(m, 2H), 7.42-7.53(m, 5H), 5.49(s, 2H), 3.32(s, 3H), 3.24(s, 3H), 2.19(s, 3H). LCMS retention time 3.308 min; LCMS MH⁺ 479.

### Example 45m 7-(4-chlorobenzyl)-1,3-dimethyl-8-(3-(trifluoromethyl)benzyloxy)-1H-purine-2,6(3H,7H)-dione (Compound 145)

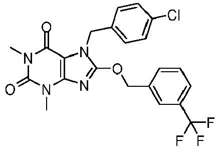

The title compound was prepared from intermediate 6 using the method of example 45 with the reaction being facilitated with microwave irradiation at 120 °C for 20 min: white solid, 20 mg, 16.5% yield: ¹*H*-NMR (DMSO-*d₆*) *δ* 7.74-7.80(m, 3H), 7.62-7.66(m, 1H), 7.37-7.39(m, 2H), 7.28(d, 2H), 5.63(s, 2H), 5.27(d, 2H), 3.48(s, 3H), 3.18(s, 3H). LCMS retention time 2.992 min; LCMS MH⁺ 479.

### Example 45n 8-(4-bromophenoxy)-7-(3-methoxybenzyl)-1,3-dimethyl-1H-purine-2,6(3H,7H)-dione (Compound 194)

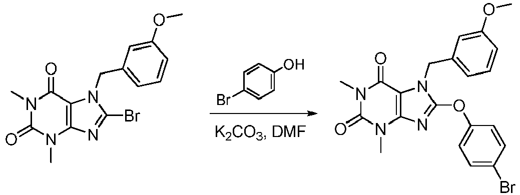

The title product was prepared from intermediate 26: white solid, 161 mg, 86.4% yield: ¹*H*-NMR (DMSO-*d₆*) *δ* 7.65-7.67 (d, 2H), 7.30-7.32 (d, 2H), 7.26-7.28 (d, 1H), 6.87-6.94 (m, 3H), 5.40(s, 2H), 3.71 (s, 3H), 3.28 (s, 3H), 3.23 (s, 3H). LCMS retention time 3.173 min; LCMS MH⁺ 471.

### Example 46 7-(3-methoxybenzyl)-1,3-dimethyl-8-(4-(pyridin-2-yl)phenoxy)-1H-purine-2,6(3H,7H)-dione (Compound 189)

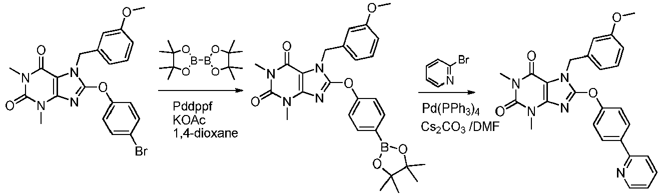

### Step 1 7-(3-methoxybenzyl)-1,3-dimethyl-8-(4-(4,4,5,5-tetramethyl-1,3,2-dioxaborolan-2-yl)phenoxy)-1H-purine-2,6(3H,7H)-dione

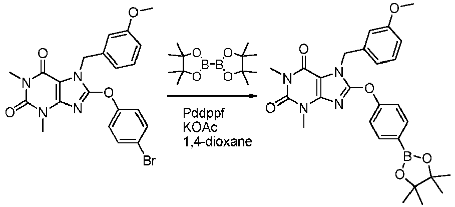

To a solution of 8-(4-bromophenoxy)-7-(3-methoxybenzyl)-1,3-dimethyl-1H-purine-2,6(3H,7H)-dione (100 mg, 0.212 mmol, product of example 45n) in 1,4-dioxane (6 mL) was added 4,4,4',4',5,5,5',5'-octamethyl-2,2'-bi(1,3,2-dioxaborolane) (108 mg, 0.425 mmol) followed by potassium acetate (42 mg, 0.427 mmol). Then the mixture was degassed under nitrogen atmosphere three times. Pd-dppf (8.66 mg, 0.01 mmol) was added under nitrogen atmosphere. The resulting mixture was stirred at 100 °C under nitrogen atmosphere for 2 h. The mixture was cooled and filtered. The filtrate was concentrated to dryness to give crude product. This crude material was purified via silica gel chromatography eluting with petroleum ether/acetate (3:1) to give 7-(3-methoxybenzyl)-1,3-dimethyl-8-(4-(4,4,5,5-tetramethyl-1,3,2-dioxaborolan-2-yl)phenoxy)-1H-purine-2,6(3H,7H)-dione (93 mg, 84.6% yield) as yellow solid. LCMS retention time 1.948 min, LCMS MH⁺ 519.

### Step 2 7-(3-methoxybenzyl)-1,3-dimethyl-8-(4-(pyridin-2-yl)phenoxy)-1H-purine-2,6(3H,7H)-dione

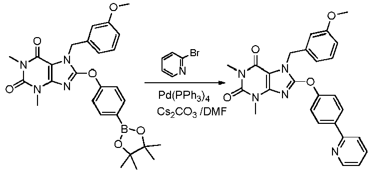

To a solution of 7-(3-methoxybenzyl)-1,3-dimethyl-8-(4-(4,4,5,5-tetramethyl-1,3,2-dioxaborolan-2-yl)phenoxy)-1H-purine-2,6 (3H,7H)-dione (70 mg, 0.135 mmol) in DMF (2 mL) was added 2-bromopyridine (30 mg, 0.19 mmol) followed by cesium carbonate (88 mg, 0.27 mmol) and
tetrakis(triphenylphosphine)palladium (15.6 mg, 0.013 mmol) under nitrogen atmosphere. After addition, the mixture was subjected to microwave irradiation at 110 °C for 30 min. The mixture was diluted with ethyl acetate and washed with brine, dried and concentrated to give crude product. This crude material was purified via preparative HPLC to give 7-(3-methoxybenzyl)-1,3-dimethyl-8-(4-(pyridin-2-yl)phenoxy)-1H-purine-2,6(3H,7H)-dione (13.9 mg, 21.9% yield) as white solid. ¹*H*-NMR (DMSO-*d₆*) *δ* 8.71 (s, 1H), 8.17-8.19 (d, 2H), 7.99-8.01 (m, 1H), 7.88-7.92 (m, 1H), 7.28-7.45 (m, 4H), 6.88-6.98 (m, 3H), 5.44(s, 2H), 3.71 (s, 3H), 3.72 (s, 3H), 3.30 (s, 3H), 3.25 (s, 3H). LCMS retention time 2.454 min; LCMS MH⁺ 470.

### Example 47 7-(4-chlorobenzyl)-1,3-dimethyl-8-(3-(trifluoromethyl)phenylthio)-1H-purine-2,6(3H,7H)-dione (Compound 192)

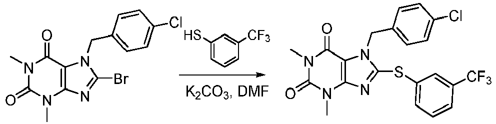

To a solution of 8-bromo-7-(4-chlorobenzyl)-1,3-dimethyl-1H-purine-2,6(3H,7H)-dione (100 mg, 0.261 mmol, intermediate 6) in DMF (3 mL) was added 3-(trifluoromethyl)benzenethiol (55.7 mg, 0.313 mmol) followed by potassium carbonate (54 mg, 0.39 mmol). The resulting mixture was stirred at 80 °C for 3 h. The mixture was diluted with ethyl acetate and washed with brine, dried and concentrated to give crude product. This crude material was purified via silica gel chromatography eluting with DCM/methanol (60: 1) to give 7-(4-chlorobenzyl)-1,3-dimethyl-8-(3-(trifluoromethyl)phenylthio)-1H-purine-2,6(3H,7H)-dione (89 mg, 70.9% yield) as white solid. ¹*H*-NMR (DMSO-*d₆*) *δ* 7.65-7.67 (d, 1H), 7.62 (s, 1H), 7.53-7.58 (m, 2H), 7.29-7.31 (d, 2H), 7.20-7.22 (d, 2H), 5.64 (s, 2H), 3.42 (s, 3H), 3.24 (s, 3H). LCMS retention time 3.194 min; LCMS MH⁺ 481.

### Example 48 7-(4-chlorobenzyl)-1,3-dimethyl-8-(3-(trifluoromethyl)phenylsulfonyl)-1H-purine-2,6(3H,7H)-dione (Compound 193)

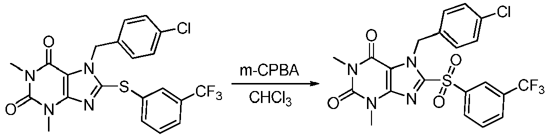

To a solution of 7-(4-chlorobenzyl)-1,3-dimethyl-8-(3-(trifluoromethyl)phenylthio)-1H-purine-2,6(3H,7H)-dione (89 mg, 0.179 mmol, product of example 47) in chloroform (2 mL) was added MCPBA (46.4 mg, 0.269 mmol). Then the mixture was stirred at 35 °C for 16 h. The mixture was diluted with DCM and washed with saturated aqueous sodium bisulfite solution and brine. The organic phase was dried and concentrated to give crude product which was purified via silica gel chromatography eluting with DCM/ methanol (45: 1) to give 7-(4-chlorobenzyl)-1,3-dimethyl-8-(3-(trifluoromethyl)phenylsulfonyl)-1H-purine-2,6(3H,7H)-dione (46 mg, 50.1% yield) as white solid. ¹*H*-NMR (DMSO-*d₆*) *δ* 8.13-8.18 (m, 2H), 7.83-7.86 (m, 2H), 7.30-7.32 (d, 2H), 7.09-7.11 (d, 2H), 5.99 (s, 2H), 3.40 (s, 3H), 3.20 (s, 3H). LCMS retention time 3.030 min; LCMS MH⁺ 513.

### Example 49 7-(3-bromobenzyl)-1,3-dimethyl-8-(methyl(3-(trifluoromethyl)phenyl)amino)-1H-purine-2,6(3H,7H)-dione (Compound 182)

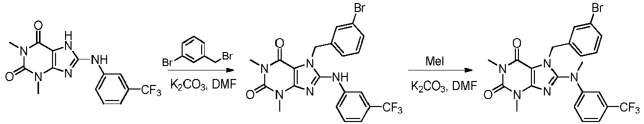

### Step 1 7-(3-bromobenzyl)-1,3-dimethyl-8-(3-(trifluoromethyl)phenylamino)-1H-purine-2,6(3H,7H)-dione

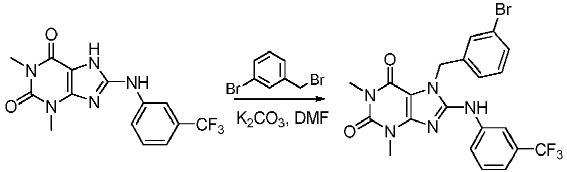

The title compound was prepared from intermediate 31 using the method of intermediate 1 step 2 to give 7-(3-bromobenzyl)-1,3-dimethyl-8-(3-(trifluoromethyl)phenylamino)-1H-purine-2,6(3H,7H)-dione (29 mg, 41.9% yield) as white solid. ¹*H*-NMR (DMSO-*d₆*) δ 9.64 (s,1 H), 8.13 (s, 1H), 7.96-7.98 (d, 1H), 7.52-7.56 (t, 1H), 7.46 (s, 1H), 7.44 (d, 1H), 7.25-7.30 (m, 4H), 7.10-7.12 (d, 1H), 5.57 (s, 2H), 3.42 (s, 3H), 3.17 (s, 3H). LCMS retention time 3.100 min; LCMS MH⁺ 508.

### Step 2 7-(3-bromobenzyl)-1,3-dimethyl-8-(methyl(3-(trifluoromethyl)phenyl)amino)-1H-purine-2,6(3H,7H)-dione

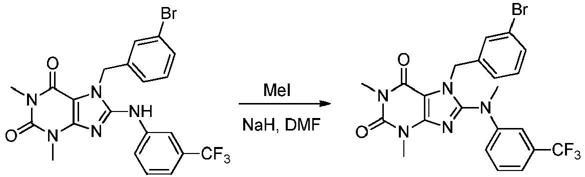

To a solution of 7-(3-bromobenzyl)-1,3-dimethyl-8-(3-(trifluoromethyl)phenylamino)-1H-purine-2,6(3H,7H)-dione (15 mg, 0.029 mmol) in anhydrous DMF (0.5 mL) was added sodium hydride (4.6 mg, 0.11 mmol, 60% dispersion in mineral oil) at 0 °C. The mixture was stirred at 0 °C for 30 min and iodomethane (8.3 mg, 0.059 mmol) was added. The resulting mixture was stirred at room temperature for 16 h. The mixture was quenched with saturated aqueous ammonium chloride solution and extracted with ethyl acetate. The organic layer was dried and concentrated to give crude product, which was purified via preparative HPLC to give 7-(3-bromobenzyl)-1,3-dimethyl-8-(methyl(3-(trifluoromethyl)phenyl)amino)-1H-purine-2,6(3H,7H)-dione (11.6 mg, 80.8% yield) as white solid. ¹*H*-NMR (DMSO-*d₆*) δ 7.39-7.41 (d, 2H), 7.24-7.26 (d, 1H), 7.16-7.19 (m, 2H), 7.00-7.02 (m, 2H), 6.95 (s, 1H), 5.20 (s, 2H), 3.42 (s, 3H), 3.26 (s, 3H), 3.25 (s, 3H). LCMS retention time 3.068 min; LCMS MH⁺ 522.

### Example 50 7-(4-chlorophenethyl)-1,3-dimethyl-8-(3-(morpholinomethyl)phenoxy)-1H-purine-2,6(3H,7H)-dione (Compound 209)

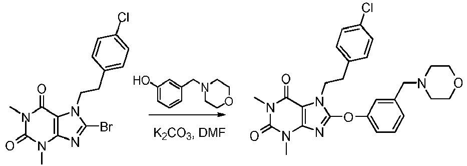

To a solution of 8-bromo-7-(4-chlorophenethyl)-1,3-dimethyl-1H-purine-2,6(3H,7H)-dione (100 mg, 0.25 mmol, intermediate 7) in DMF (3 mL) was added 3-(morpholinomethyl)phenol (58.3 mg, 0.30 mmol, intermediate 27) followed by potassium carbonate (104 mg, 0.75 mmol). Then the mixture was heated to 80 °C for 4 h. The mixture was diluted with ethyl acetate. This organic phase was washed with brine and saturated aqueous ammonium chloride, then it was dried and concentrated to give crude product. This crude product was purified by preparative HPLC to give 7-(4-chlorophenethyl)-1,3-dimethyl-8-(3-(morpholinomethyl)phenoxy)-1H-purine-2,6(3H,7H)-dione (45 mg, 35.3% yield) as white solid. ¹*H*-NMR (DMSO-*d₆*) δ 7.53-7.55 (d, 1H), 7.45-7.49 (m, 2H), 7.28-7.30 (d, 2H), 7.18-7.20 (d, 2H), 7.01-7.03(dd, 1H), 4.38-4.41 (t, 2H), 4.34 (s, 2H), 3.82-3.94 (m, 4H), 3.26 (s, 6H), 3.16-3.21 (m, 2H), 3.04-3.11 (m, 4H). LCMS retention time 1.782 min; LCMS MH⁺ 510.

The following examples 51a to 51e were prepared using the method of example 50.

### Example 51a 8-(4-chloro-3-(trifluoromethyl)phenoxy)-7-(4-chlorophenethyl)-1,3-dimethyl-1H-purine-2,6(3H,7H)-dione (Compound 208)

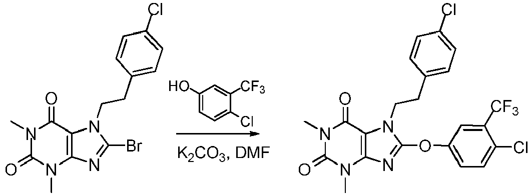

White solid, 41 mg, 31.3% yield: ¹*H*-NMR (DMSO-*d₆*) δ 7.76-7.78 (d, 1H), 7.50 (s, 1H), 7.44-7.47 (d, 1H), 7.22-7.24 (d, 2H), 7.13-7.15(d, 2H), 4.40-4.43 (t, 2H), 3.28 (s, 3H), 3.27 (s, 3H), 3.06-3.09 (t, 2H). LCMS retention time 3.266 min; LCMS MH⁺ 513.

### Example 51b 8-(3,5-bis(trifluoromethyl)phenoxy)-7-(4-chlorophenethyl)-1,3-dimethyl-1H-purine-2,6(3H,7H)-dione (Compound 210)

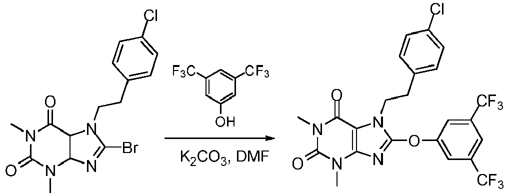

White solid, 43 mg, 31.1% yield: ¹*H*-NMR (DMSO-*d₆*) δ 8.05 (s, 1H), 7.85 (s, 2H), 7.15-7.21 (t, 4H), 4.43-4.46 (t, 2H), 3.28 (s, 3H), 3.27 (s, 3H), 3.07-3.10 (t, 2H). LCMS retention time 3.351 min; LCMS MH⁺ 547.

### Example 51c 8-(4-tert-butylphenoxy)-7-(4-chlorophenethyl)-1,3-dimethyl-1H-purine-2,6(3H,7H)-dione (Compound 211)

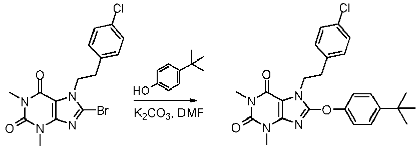

White solid, 51 mg, 53.6% yield: ¹*H*-NMR (DMSO-*d₆*) δ 7.37-7.39 (d, 2H), 7.27-7.30 (d, 2H), 7.13-7.15 (d, 2H), 6.84-6.86 (d, 2H), 4.35-4.38 (t, 2H), 3.26 (s, 3H),3.25 (s, 3H), 3.07-3.10 (t, 2H), 1.28 (s, 9H). LCMS retention time 3.432 min; LCMS MH⁺ 467.

### Example 51d 8-(4-chloro-3-(morpholinomethyl)phenoxy)-7-(4-chlorobenzyl)-1,3-dimethyl-1H-purine-2,6(3H,7H)-dione (Compound 213)

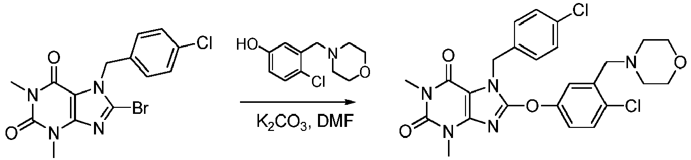

The title compound was prepare using the method of example 50 from intermediates 6 and 29. White solid, 91 mg, 64.9% yield: ¹*H*-NMR (DMSO-*d₆*) δ 7.52-7.54 (d, 1H), 7.39-7.45 (m, 5H), 7.27-7.30(dd, 1H),5.44 (s, 2H), 3.56-3.57(d, 6H),3.27 (s, 3H), 3.23 (s, 3H), 2.42 (m, 4H). LCMS retention time 1.995 min; LCMS MH⁺ 530.

### Example 51e 8-(4-chloro-3-(morpholinomethyl)phenoxy)-7-(4-chlorobenzyl)-1,3-dimethyl-1H-purine-2,6(3H,7H)-dione (Compound 212)

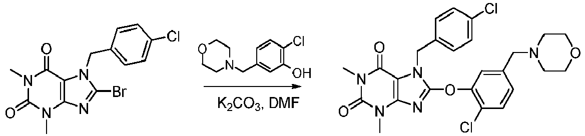

The title compound was prepare using the method of example 50 from intermediates 6 and 30. White solid, 94 mg, 67.9% yield: ¹*H*-NMR (DMSO-*d₆*) δ 7.45-7.46 (m, 7H), 5.47 (s, 2H), 3.57-3.60(m, 6H), 3.23 (s, 6H), 2.42 (m, 4H). LCMS retention time 1.842 min; LCMS MH⁺ 530.

### Example 52 7-(4-chlorobenzyl)-1,3-dimethyl-8-(2-(3-(trifluoromethoxy)phenoxy)ethoxy)-1H-purine-2,6(3H,7H)-dione (Compound 214)

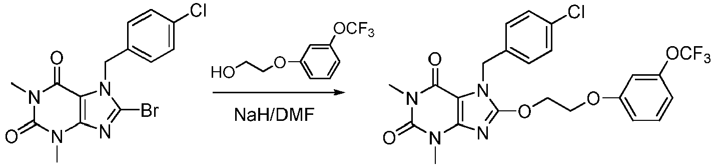

To a solution of 2-(3-(trifluoromethoxy)phenoxy)ethanol (61 mg, 0.275 mmol, intermediate 17) in anhydrous THF (10 mL) was added sodium hydride (66 mg, 2.75 mmol)at 0 °C under nitrogen atmosphere, and the mixture was stirred at 0 °C for 20 miniutes. A solution of 8-bromo-7-(4-chlorobenzyl)-1,3-dimethyl-1H-purine-2,6(3H,7H)-dione (90 mg, 0.235 mmol, Intermediate 6) in THF (3 mL) was added dropwise , and the resulting mixture was stirred at 0 °C to room temperature for 16 h under nitrogen. The reaction was quenched with ice-water and partitioned between ethyl acetate and brine. The organic layer was dried over sodium sulfate, and concentrated to give a crude product, which was purified by preparative HPLC to give 7-(4-chlorobenzyl)-1,3-dimethyl-8-(2-(3-(trifluoromethoxy)phenoxy)ethoxy)-1H-purine-2,6(3H,7H)-dione (70 mg, 26.7% yield) as white solid. ¹*H*-NMR (DMSO-*d₆*) δ 7.45-7.41(m, 1H), 7.32-7.26(m, 4H), 7.02-6.95(m, 3H), 5.19(s, 2H), 4.82-4.80(m, 2H), 4.42-4.39(m, 2H), 3.37(s, 3H), 3.20(s, 3H). LCMS retention time 3.251min; LCMS MH⁺ 525.

### Example 53 2-(7-(3-bromobenzyl)-1,3-dimethyl-2,6-dioxo-2,3,6,7-tetrahydro-1H-purin-8-yl)acetic acid (Compound 25)

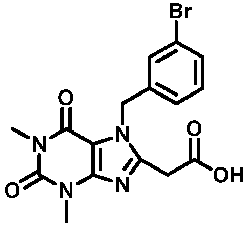

Diethyl 2-(7-(3-bromobenzyl)-1,3-dimethyl-2,6-dioxo-2,3,6,7-tetrahydro-1H-purin-8-yl)malonate (12.57 g, 24.78 mmol, intermediate 33) was slurried in 18% aqueous HCl (300 mL) and THF (300 mL). The reaction was heated at 100°C for 5 h. The clear solution was cooled in an ice bath and enough 2 N sodium hydroxide was added until PH 12 to 14 then extracted with ethyl acetate (2 x 200 mL). The aqueous layer was cooled in an ice bath and enough concentrated HCl was added until PH = 1 and a white solid formed. Solid was filtered, washed with water (100 mL), diethyl ether (2 x 75 mL) and high vacuum dried for 15 h to give 2-(7-(3-bromobenzyl)-1,3-dimethyl-2,6-dioxo-2,3,6,7-tetrahydro-1*H*-purin-8-yl)acetic acid (5.58 g, 55% yield) as a white solid. LCMS retention time = 2.562 min and 100% purity, LCMS MH⁺ 407. ¹*H*-NMR (DMSO-*d₆*) δ: 12.89 (brd s, 1H), 7.43-7.48 (m, 2H), 7.26 (t, 1H, J = 8 Hz), 7.15 (d, 1H, J = 4 Hz), 5.55 (s, 2H), 3.91 (s, 2H), 3.41 (s, 3H), 3.19 (s, 3H).

### Example 54 7-(3-bromobenzyl)-1,3,8-trimethyl-1H-purine-2,6(3H,7H)-dione (Compound 24)

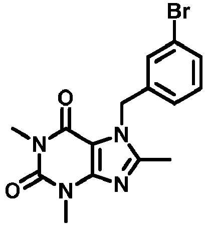

A portion of the ethyl acetate layer, from example 53, was dried with magnesium sulfate, filtered and evaporated under reduced pressure to give 7-(3-bromobenzyl)-1,3,8-trimethyl-1*H*-purine-2,6(3H,7H)-dione (0.31 g) as a white solid. LCMS retention time = 2.877 min and 99% purity, LCMS MH⁺ 363. ¹*H*-NMR (DMSO-*d₆*) δ: 12.89 (brd s, 1H), 7.43-7.49 (m, 2H), 7.29 (t, 1H, J = 8 Hz), 7.14 (d, 1H, J = 8 Hz), 5.52 (s, 2H), 3.39 (s, 3H), 3.19 (s, 3H), 2.38 (s, 3H).

### Example 55 Propyl-2-(7-(3-bromobenzyl)-1,3-dimethyl-2,6-dioxo-2,3,6,7-tetrahydro-1H-purin-8-yl)-acetate (Compound 26)

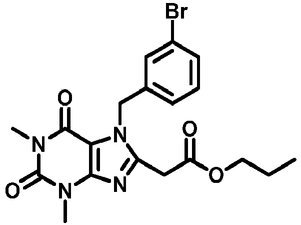

2-(7-(3-bromobenzyl)-1,3-dimethyl-2,6-dioxo-2,3,6,7-tetrahydro-1*H*-purin-8-yl)acetic acid (0.061 g, 0.15 mmol, example 53) was slurried in 1-propanol (8 mL) and sulfuric acid (1 drop). The reaction was heated at reflux for 1 hour, evaporated under reduced pressure to a clear oil. Oil was purified using a 2000 micron preparative TLC plate eluted with 100% ethyl acetate. Target band was scraped off plate, eluted off silica gel with ethyl acetate, evaporated under reduced pressure to give propyl-2-(7-(3-bromobenzyl)-1,3-dimethyl-2,6-dioxo-2,3,6,7-tetrahydro-1*H-*purin-8-yl)acetate (0.042 g, 63% yield) as a white solid. LCMS retention time = 3.368 min and 99% purity, LCMS MH⁺ 449. ¹*H*-NMR (DMSO-*d₆*) δ: 7.47 (d, 1H, J = 8 Hz), 7.43-7.45 (m, 1H), 7.27 (t, 1H, J = 8 Hz), 7.16 (d, 1H, J = 8 Hz), 5.57 (s, 2H), 4.04 (s, 2H), 3.86 (t, 2H, J = 8 Hz), 3.41 (s, 3H), 3.20 (s, 3H), 1.42-1.52 (m, 2H), 0.80 (t, 3H, J = 8 Hz).

### Example 56 Ethyl 2-(7-(3-bromobenzyl)-1,3-dimethyl-2,6-dioxo-2,3,6,7-tetrahydro-1H-purin-8yl)acetate (Compound 34)

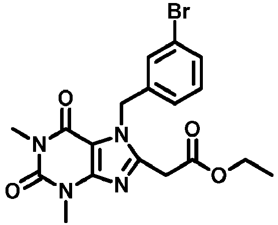

2-(7-(3-Bromobenzyl)-1,3-dimethyl-2,6-dioxo-2,3,6,7-tetrahydro-1*H*-purin-8yl)acetic acid (3.57 g, 8.77 mmol, example 53) was slurried in ethanol (80 mL) and sulfuric acid (2 drops) was added and the reaction was heated at reflux for 2 h. The clear solution was cooled to room temperature and a white solid crystallized. Filtered solid, washed with ethanol(10 mL) and high vacuum dried for 3 h to give ethyl 2-(7-(3-bromobenzyl)-1,3-dimethyl-2,6-dioxo-2,3,6,7-tetrahydro-1H-purin-8yl)acetate (2.01 g, 52.6% yield) as a white solid. LCMS retention time = 3.139 min and 97% purity, LCMS MH⁺ 435. ¹*H*-NMR (CDCl₃) δ: 7.41 (d, 1H, J = 8 Hz), 7.25-7.28 (m, 1H), 7.21 (t, 1H, J = 8 Hz), 7.09 (d, 1H, J = 8 Hz), 5.59 (s, 2H), 4.13 (q, 2H, J = 8 Hz and 16 Hz), 3.59 (s, 3H), 3.41 (s, 3H), 1.24 (t, 3H, J = 8 Hz).

### Example 57 2-(7-(3-Bromobenzyl)-1,3-dimethyl-2,6-dioxo-2,3,6,7-tetrahydro-1H-purin-8-yl)-N-(2-hydroxyethyl)acetamide (Compound 38)

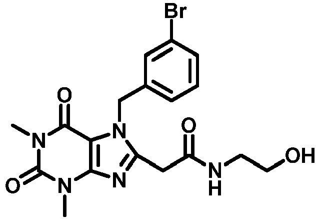

2-(7-(3-Bromobenzyl)-1,3-dimethyl-2,6-dioxo-2,3,6,7-tetrahydro-1*H*-purin-8-yl)acetic acid (0.25 g, 0.61 mmol, example 53) was slurried in DMF (4 mL) and carbonyldiimidazole (0.10 g, 0.61 mmol) was added. The reaction was stirred for 10 min then 2-aminoethanol (0.11 mL, 1.83 mmol) was added. After stirring reaction for 15 h a white solid formed. Filtered solid, washed with water (2 x 10 mL), ethanol (10 mL), diethyl ether (10 mL), high vacuum dried to give 2-(7-(3-bromobenzyl)-1,3-dimethyl-2,6-dioxo-2,3,6,7-tetrahydro-1*H*-purin-8-yl)-*N*-(2-hydroxyethyl)acetamide (0.21 g, 75% yield) as a white solid. LCMS retention time = 2.288 min and 95% purity, LCMS MH⁺ 450. ¹*H*-NMR (DMSO-*d₆*) δ: 8.18-8.28 (m, 1H), 7.41-7.49 (m, 2H), 7.27 (t, 1H, J = 8 Hz), 7.13 (d, 1H, J = 8 Hz), 5.56 (s, 2H), 4.68 (d, 1H, J = 8 Hz), 3.74 (s, 2H), 3.41 (s, 3H), 3.38 (q, 2H, J = 8 Hz and 12 Hz), 3.19 (s, 3H), 3.08 (q, 2H, J = 8 Hz and 12 Hz).

### Example 58 2-(7-(3-Bromobenzyl)-1,3-dimethyl-2,6-dioxo-2,3,6,7-tetrahydro-1H-purin-8-yl)-N-propylacetamide (Compound 27)

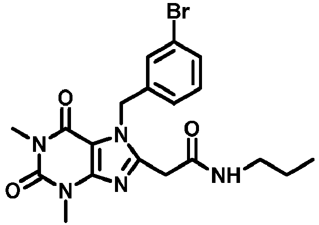

The title compound was prepared using the method of example 57. Off-white solid; LCMS retention time = 2.815 min and 100% purity, LCMS MH⁺ 448. ¹*H*-NMR (DMSO-*d₆*) δ: 8.15 (t, 1H, J = 4 Hz), 7.46 (d, 1H, J = 8 Hz), 7.41-7.44 (m, 1H), 7.26 (t, 1H, J = 8 Hz), 7.12 (d, 1H, J = 8 Hz), 5.56 (s, 2H), 3.71 (s, 2H), 3.41 (s, 3H), 3.19 (s, 3H), 2.93-2.99 (m, 2H), 1.32-1.41 (m, 2H), 0.82 (t, 3H, J = 8 Hz).

### Example 59 7-(3-Bromobenzyl)-1,3-dimethyl-8-(2-(4-methylpiperazin-1-yl)-2-oxoethyl)-1H-purine-2,6(3H,7H)-dione (Compound 31)

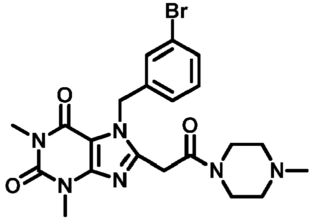

The title compound was prepared using using the method of example 57. White solid; LCMS retention time = 3.291 min and 95% purity, LCMS MH⁺ 411. ¹*H*-NMR (DMSO-*d₆*) δ: 7.38-7.51 (m, 2H), 7.22-7.32 (m, 1H), 7.09-7.18 (m, 1H), 5.50 (s, 2H), 4.00 (s, 2H), 3.42-3.52 (m, 7H), 3.20 (s, 3H), 2.10-2.34 (m, 7H).

### Example 60 7-(3-Bromobenzyl)-8-(2-hydroxyethyl)-1,3-dimethyl-1H-purine-2,6(3H,7H)-dione (Compound 39)

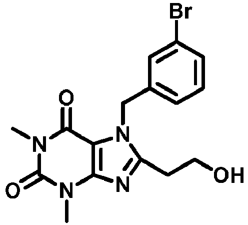

Ethyl 2-(7-(3-bromobenzyl)-1,3-dimethyl-2,6-dioxo-2,3,6,7-tetrahydro-1*H*-purin-8yl)acetate (0.25 g, 0.57 mmol, example 56) was dissolved in THF (4 mL) and finely powdered sodium borohydride (0.13 g, 3.45 mmol) was added. The reaction was heated at reflux and methanol (2 mL) was slowly added drop wise (violent gas evolution). After heating for 15 min longer the reaction was cooled and 1N HCl (2 mL) was added. The reaction was diluted with water (75 mL) and extracted with ethyl acetate (3 x 50 mL). The combined extracts were washed with saturated sodium bicarbonate (25 mL), dried with magnesium sulfate and evaporated under reduced pressure to give 7-(3-bromobenzyl)-8-(2-hydroxyethyl)-1,3-dimethyl-1*H*-purine-2,6(3*H*,7*H*)-dione (0.21 g, 92% yield) as a white solid. LCMS retention time = 2.534 min and 94% purity, LCMS MH⁺ 393. ¹*H*-NMR (CDCl₃) δ: 7.43 (d, 1H, J = 8 Hz), 7.29 (s, 1H), 7.20 (t, 1H, J = 8 Hz), 7.09 (d, 1H, J = 8 Hz), 5.54 (s, 2H), 3.95-4.06 (m, 2H), 3.58 (s, 3H), 3.45 (t, 1H, J = 4 Hz), 3.40 (s, 3H), 2.85 (t, 2 H, J = 8 Hz).

### Example 61 7-(3-Bromobenzyl)-8-(2-chloroethyl)-1,3-dimethyl-1H-purine-2,6(3H,7H)-dione (Compound 40)

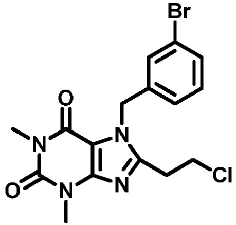

7-(3-Bromobenzyl)-8-(2-hydroxyethyl)-1,3-dimethyl-1*H*-purine-2,6(3*H*,7*H*)-dione (1.7 g, 4.32 mmol, example 60) and thionyl chloride (20 mL) were combined and stirred at room temperature for 72 h. The reaction was evaporated under reduced pressure, diluted with water (200 mL) and extracted with ethyl acetate (3 x 50 mL). The combined extracts were washed with saturated sodium bicarbonate (100 mL), dried with magnesium sulfate and evaporated under reduced pressure to a light golden solid. The solid was purified using a 40 g silica gel flash column eluted with 2% methanol / dichloromethane to give 7-(3-bromobenzyl)-8-(2-chloroethyl)-1,3-dimethyl-1*H*-purine-2,6(3*H*,7*H*)-dione (1.01 g, 56% yield) as a light tan solid. LCMS retention time = 3.291 min and 95% purity, LCMS MH⁺ 411. ¹*H*-NMR (CDCl₃) δ: 7.43 (d, 1H, J = 8 Hz), 7.28 (s, 1H), 7.21 (t, 1H, J = 8 Hz), 7.09 (d, 1H, J = 8 Hz), 5.59 (s, 2H), 3.88 (t, 2H, J = 8 Hz), 3.59 (s, 3H), 3.40 (s, 3H), 3.11 (t, 2H, J = 8 Hz).

### Example 62 7-(3-Bromobenzyl)-1,3-dimethyl-8-vinyl-1H-purine-2,6(3H,7H)-dione (Compound 41)

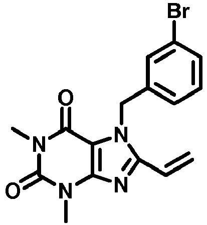

and

### 7-(3-Bromobenzyl)-1,3-dimethyl-8-(2-propoxyethyl)-1H-purine-2,6(3H,7H)-dione (Compound 42)

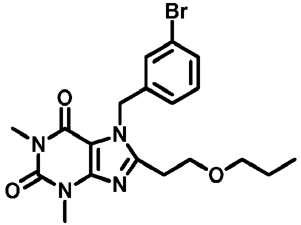

Sodium (0.029 g, 1.21 mmol) was dissolved in 1-propanol (5 mL) and 7-(3-bromobenzyl)-8-(2-chloroethyl)-1,3-dimethyl-1*H*-purine-2,6(3*H*,7*H*)-dione (0.25 g, 0.61 mmol, example 61) was added followed by THF (2 mL) and MC (2 mL) to aid solubility. The reaction was stirred at room temperature for 10 min, diluted with water (100 ml) and extracted with ethyl acetate (3 x 75 mL). The combined extracts were dried with magnesium sulfate and evaporated under reduced pressure to a light golden oil. This oil was purified using two 2000 micron silica gel preparative TLC plates eluted with 50% ethyl acetate / hexanes. The upper (less polar) band was scraped off plate, eluted off the silica gel with ethyl acetate and evaporated under reduced pressure to give 7-(3-bromobenzyl)-1,3-dimethyl-8-vinyl-1*H*-purine-2,6(3*H*,7*H*)-dione (0.066 g) as a white solid. LCMS retention time = 3.250 min and 96% purity, LCMS MH⁺ 375. ¹*H*-NMR (CDCl₃) δ: 7.42 (d, 1H, J = 8 Hz), 7.31 (s, 1H), 7.20 (t, 1H, J = 8 Hz), 7.12 (d, 1H, J = 8 Hz), 6.46-6.63 (m, 2H), 5.73 (dd, 1H, J = 4 Hz and 8 Hz), 3.62 (s, 3H), 3.40 (s, 3H).

The lower (more polar) band was scraped off plate, eluted off silica gel with ethyl acetate and evaporated to give 7-(3-bromobenzyl)-1,3-dimethyl-8-(2-propoxyethyl)-1*H*-purine-2,6(3*H*,7*H*)-dione (0.099 g, 38% yield) as a white solid. LCMS retention time = 3.536 min and 96% purity, LCMS MH⁺ 435. ¹*H*-NMR (CDCl₃) δ: 7.41 (d, 1H, J = 8 Hz), 7.28 (s, 1H), 7.19 (t, 1H, J = 8 Hz), 7.10 (d, 1H, J = 8 Hz), 5.62 (s, 3H), 3.74 (t, 2H, J = 8 Hz), 3.59 (s, 3H), 3.39 (s, 3H), 3.35 (t, 2H, J = 8 Hz), 2.93 (t, 2H, J = 8 Hz), 1.48-1.58 (m, 2H), 0.87 (t, 3H, J = 8 Hz).

### Example 63 7-(3-Bromobenzyl)-1,3-dimethyl-8-(2-(propylamino)ethyl-1H-purine-2,6(3H,7H)-dione (Compound 46)

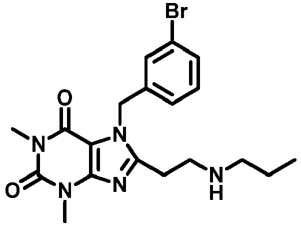

7-(3-Bromobenzyl)-8-(2-chloroethyl)-1,3-dimethyl-1*H*-purine-2,6(3*H*,7*H*)-dione (0.22 g, 0.53 mmol, example 61) was dissolved in propan-1-amine (4 mL) and stirred at room temperature for 15 h. The reaction was evaporated under reduced pressure and high vacuum dried to give 7-(3-bromobenzyl)-1,3-dimethyl-8-(2-(propylamino)ethyl-1*H*-purine-2,6(3*H*,7*H*)-dione (0.23 g, 100% yield) as an off-white solid. LCMS retention time = 2.009 min and 98% purity, LCMS MH⁺ 434.

The hydrochloride was prepared by dissolving 0.05 g of this solid product in diethyl ether (2 mL) and excess 1N HCl in diethyl ether was added. The mixture was evaporated under reduced pressure, azeotroped with ethyl acetate (2 x 50 mL), diethyl ether (2 x 5 mL) and high vacuum dried to a give 7-(3-bromobenzyl)-1,3-dimethyl-8-(2-(propylamino)ethyl-1*H*-purine-2,6(3*H*,7*H*)-dione hydrochloride (0.029 g) as a white solid. LCMS retention time = 2.120 min and 96% purity, LCMS MH⁺ 434. ¹*H*-NMR DMSO-*d₆* δ: 9.00 (brd s, HCl and NH), 7.43-7.53 (m, 2H), 7.31 (t, 1H), J = 8 Hz), 7.18 (d, 1H, J = 8 Hz), 5.59 (s, 2H), 3.36 (s, 3H), 3.20-3.31 (m, 5H), 3.12-3.18 (m, 2H), 2.83-2.91 (m, 2H), 1.57-1.67 (m, 2H), 0.91 (t, 3H, J = 8 Hz).

### Example 64 8-(2-(Benzylamino)ethyl)-7-(3-bromobenzyl)-1,3-dimethyl-1H-purine-2,6(3H,7H)-dione (Compound 47)

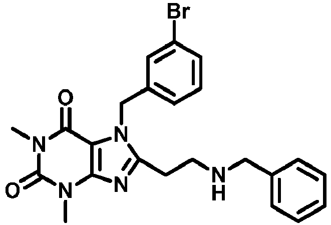

The title compound was prepared using the method of example 63 and isolated as the free base. Golden oil; LCMS retention time = 2.181 min and 99% purity, LCMS MH⁺ 482. ¹*H*-NMR (CDCl₃) δ: 7.41 (d, 1H, J = 8 Hz), 7.23-7.34 (m, 6H), 7.18 (t, 1H, J = 8 Hz), 7.07 (d, 1H, J = 8 Hz), 5.52 (s, 2H), 3.78 (s, 2H), 3.58 (s, 3H), 3.40 (s, 3H), 3.01 (t, 2H, J = 8 Hz), 2.83 (t, 2H, J = 8 Hz).

### Example 65 7-(3-Bromobenzyl)-1,3-dimethyl-8-(2-(methyl(propyl)amino)ethyl)-1H-purine-2,6(3H,7H)-dione hydrochloride (Compound 48)

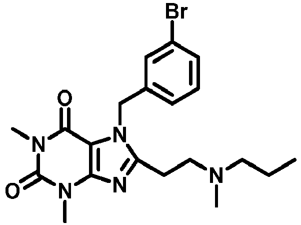

7-(3-Bromobenzyl)-1,3-dimethyl-8-(2-(propylamino)ethyl-1*H*-purine-2,6(3*H*,7*H*)-dione (0.10 g, 0.23 mmol, example 63), formic acid (1 mL) and formaldehyde (37% by weight in water, 1 mL) were dissolved in ethanol (5 mL) in a sealed tube and heated at 85°C for 15 h. The reaction was cooled to room temperature, diluted with water (50 mL) and treated with 1N sodium hydroxide until pH = 12 to 14. The reaction was then extracted with ethyl acetate (3 x 50 mL). The combined organic extracts were dried with magnesium sulfate, filtered and the solvent was removed under reduced pressure to a light golden oil. This oil was purified using a 2000 micron silica gel preparative TLC plate eluted with 5% methanol / MC. The target band was scraped off plate, eluted off silica gel with ethyl acetate and evaporated under reduced pressure to a clear oil. The oil was dissolved in diethyl ether (3 mL) and excess 1N HCl in diethyl ether was added. Mix was evaporated under reduced pressure, high vacuum dried for 2 h to give 7-(3-bromobenzyl)-1,3-dimethyl-8-(2-(methyl(propyl)amino)ethyl)-1*H*-purine-2,6(3*H*,7*H*)-dione hydrochloride (0.076 g, 68% yield) as a white solid. LCMS retention time = 2.044 min and 99% purity, LCMS MH⁺ 448. ¹*H*-NMR (DMSO-*d₆*) δ: 10.24 (brd s, 1H), 7.48-7.53 (m, 2H), 7.29 (t, 1H, J = 8 Hz), 7.19 (d, 1H, J = 8 Hz), 5.60 (s, 2H), 3.32-3.54 (m, 5H), 3.26 (t, 2H, J = 8 Hz), 3.22 (s, 3H), 2.90-3.03 (m, 1H), 3.08-3.17 (m, 1H), 2.76 (d, 3H, J = 4 Hz), 1.61-1.72 (m, 2H), 0.89 (t, 3H, J = 8 Hz).

### Example 66 N-(2-7-(3-bromobenzyl)-1,3-dimethyl-2,6-dioxo-2,3,6,7-tetrahydro-1H-purin-8-yl)ethyl)-N-propylacetamide (Compound 49)

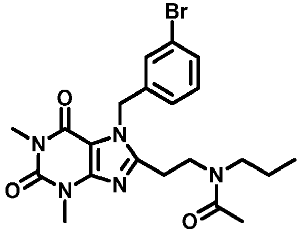

7-(3-Bromobenzyl)-1,3-dimethyl-8-(2-(propylamino)ethyl-1*H*-purine-2,6(3*H*,7*H*)-dione (0.098 g, 0.226 mmol, example 63) and triethylamine (0.063 mL, 0.45 mmol) were dissolved in THF (5 mL) and acetyl chloride (0.032 mL, 0.45 mmol) was added slowly drop wise. The reaction was stirred at room temperature for 15 h then diluted with water (50 mL) and extracted with ethyl acetate (3 x 25 mL). The combined extracts were dried with magnesium sulfate, filtered and the solvent was removed under reduced pressure to a light golden oil. This oil was crystallized by trituration with diethyl ether (1 mL). The solid was filtered and high vacuum dried to give N-(2-7-(3-bromobenzyl)-1,3-dimethyl-2,6-dioxo-2,3,6,7-tetrahydro-1*H*-purin-8-yl)ethyl)-*N-*propylacetamide (0.053 g, 50% yield) as a white solid. LCMS retention time = 2.991 min and 98% purity, LCMS MH⁺ 476. ¹*H*-NMR (CDCl₃) δ: 7.40 (d, 1H, J = 8 Hz), 7.30 (s, 1H), 7.15-7.22 (m, 2H), 5.61 (s, 2H), 3.55-3.65 (m, 5H), 3.40 (s, 3H), 3.19 (t, 2H, J = 8 Hz), 2.98 (t, 2H, J = 8 Hz), 2.07 (s, 3H), 1.53-1.61 (m, 2H), 0.91 (t, 3H, J = 8 Hz).

### Example 67 Ethyl 3-(7-(3-bromobenzyl)-1,3-dimethyl-2,6-dioxo-2,3,6,7-tetrahydro-1H-purin-8-yl)butanoate (Compound 240)

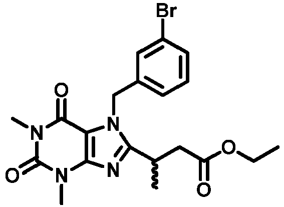

and

### Ethyl 3-(7-(3-bromobenzyl)-1,3-dimethyl-2,6-dioxo-2,3,6,7-tetrahydro-1H-purin-8-yl)-2-methylpropanoate (Compound 241)

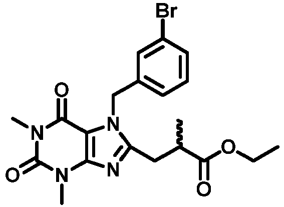

A mixture of ethyl 3-(1,3-dimethyl-2,6-dioxo-2,3,6,7-tetrahydro-1*H*-purin-8-yl)2-methylpropanoate and ethyl 3-(1,3-dimethyl-2,6-dioxo-2,3,6,7-tetrahydro-1*H*-purin-8-yl)butanoate (2.07 g, 7.0 mmol, intermediates 34), potassium carbonate (3.8 g, 28.0 mmol) and 1-bromo-3-(bromomethyl)benzene (3.8 g, 15.4 mmol) in DMF (15 mL) was stirred at room temperature for 15 h. The reaction was diluted with water (100 mL) and extracted with ethyl acetate (3 x 75 mL). The combined extracts were washed with 1N LiCl (2 x 100 mL), dried with magnesium sulfate, filtered and evaporated under reduced pressure to leave a golden oil. The oil was purified using an 80 g silica gel flash column eluted with 20% ethyl acetate / hexanes to give ethyl 3-(7-(3-bromobenzyl)-1,3-dimethyl-2,6-dioxo-2,3,6,7-tetrahydro-1*H*-purin-8-yl)butanoate (0.53 g , 16% yield) as a white foam, LCMS retention time = 3.165 min and 96% purity, LCMS MH⁺ 463. Continued elution gave ethyl 3-(7-(3-bromobenzyl)-1,3-dimethyl-2,6-dioxo-2,3,6,7-tetrahydro-1*H*-purin-8-yl)-2-methylpropanoate (0.85 g, 26% yield) as a white foam. LCMS retention time = 3.163 min and 93% purity, LCMS MH⁺ 463..

### Example 68 Ethyl 4-(7-(3-bromobenzyl)-1,3-dimethyl-2,6-dioxo-2,3,6,7-tetrahydro-1H-purin-8-yl) -2,2-dimethylbutanoate (Compound 242)

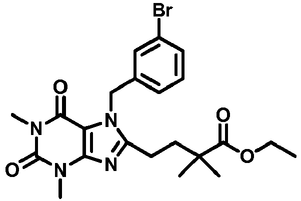

and

### Ethyl 4-(7-(3-bromobenzyl)-1,3-dimethyl-2,6-dioxo-2,3,6,7-tetrahydro-1H-purin-8-yl) -4-methylpentanoate (Compound 243)

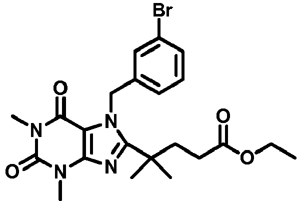

Ethyl 4-(7-(3-bromobenzyl)-1,3-dimethyl-2,6-dioxo-2,3,6,7-tetrahydro-1*H*-purin-8-yl) -2,2-dimethylbutanoate and Ethyl 4-(7-(3-bromobenzyl)-1,3-dimethyl-2,6-dioxo-2,3,6,7-tetrahydro-1*H*-purin-8-yl)-4-methylpentanoate were prepared using the methods of intermediate 34 and example 67.

Ethyl 4-(7-(3-bromobenzyl)-1,3-dimethyl-2,6-dioxo-2,3,6,7-tetrahydro-1*H*-purin-8-yl) -2,2-dimethylbutanoate: White solid, 0.054 g, 25% yield: LCMS retention time = 3.458 min and 98% purity, LCMS MH⁺ 491. ¹*H*-NMR (DMSO-*d₆*) δ: 7.49 (d, 1H, J = 8 Hz), 7.41-7.43 (m, 1H), 7.29 (t, 1H, J = 8 Hz), 7.10 (d, 1H, J = 8 Hz), 5.56 (s, 2H), 3.98 (q, 2H, J = 4 Hz and 12 Hz), 3.42 (s, 3H), 3.21 (s, 3H), 2.55-2.63 (m, 2H), 1.73-1.79 (m, 2H), 1.05-1.12 (m, 9H).

Ethyl 4-(7-(3-bromobenzyl)-1,3-dimethyl-2,6-dioxo-2,3,6,7-tetrahydro-1*H*-purin-8-yl) -4-methylpentanoate: White solid, 0.036 g, 17% yield: LCMS retention time = 3.486 min and 98% purity, LCMS MH⁺ 491. ¹*H*-NMR (DMSO-*d₆*) δ: 7.43 (d, 1H, J = 8 Hz), 7.30-7.33 (m, 1H), 7.24 (t, 1H, J = 8 Hz), 6.90 (d, 1H, J = 8 Hz), 5.75 (s, 2H), 3.90 (q, 2H, J = 8 Hz and 16 Hz), 3.44 (s, 3H), 3.16 (s, 3H), 2.06-2.12 (m, 2H), 1.88-1.96 (m, 2H), 1.28 (s, 6H), 1.08 (t, 3H, J = 8 Hz).

### Example 69 Ethyl 3-(7-(3-bromobenzyl)-1,3-dimethyl-2,6-dioxo-2,3,6,7-tetrahydro-1H-purin-8-yl) -3-methylbutanoate (Compound 96)

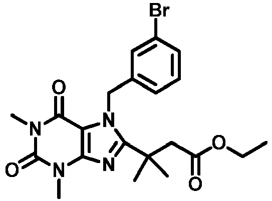

and

### Ethyl 3-(7-(3-bromobenzyl)-1,3-dimethyl-2,6-dioxo-2,3,6,7-tetrahydro-1H-purin-8-yl) -2-2-dimethylpropanoate (Compound 97)

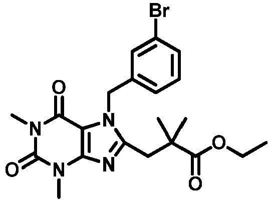

Ethyl 3-(7-(3-bromobenzyl)-1,3-dimethyl-2,6-dioxo-2,3,6,7-tetrahydro-1*H*-purin-8-yl)-3-methylbutanoate and ethyl 3-(7-(3-bromobenzyl)-1,3-dimethyl-2,6-dioxo-2,3,6,7-tetrahydro-1*H-*purin-8-yl)-2,2-dimethylpropanoate were prepared using the methods of intermediate 34 and example 67.

Ethyl 3-(7-(3-bromobenzyl)-1,3-dimethyl-2,6-dioxo-2,3,6,7-tetrahydro-1*H*-purin-8-yl)-3-methylbutanoate: White solid, 0.12 g, 41% yield: LCMS retention time = 3.322 min and 99% purity, LCMS MH⁺ 477. ¹*H*-NMR (DMSO-*d₆*) δ: 7.44 (d, 1H, J = 8 Hz), 7.29-7.33 (m, 1H), 7.26 (t, 1H, J = 8 Hz), 6.93 (d, 1H, J = 8 Hz), 5.76 (s, 2H), 3.88 (q, 2H, J = 4 Hz and 12 Hz), 3.42 (s, 3H), 3.15 (s, 3H), 2.77 (s, 2H), 1.36 (s, 6H), 1.04 (t, 3H, J = 8 Hz).

Ethyl 3-(7-(3-bromobenzyl)-1,3-dimethyl-2,6-dioxo-2,3,6,7-tetrahydro-1*H*-purin-8-yl) -2,2-dimethylpropanoate: Golden oil, 0.17 g, 65% yield: LCMS retention time = 3.406 min and 99% purity, LCMS MH⁺ 477. ¹*H*-NMR (DMSO-*d₆*) δ: 7.48 (d, 1H, J = 8 Hz), 7.38-7.43 (m, 1H), 7.29 (t, 1H, J = 8 Hz), 7.10 (d, 1H, J = 12 Hz), 5.57 (s, 2H), 4.00-4.08 (m, 2H), 3.37 (s, 3H), 3.19 (s, 3H), 2.95 (s, 2H), 1.18 (s, 6H), 1.12 (t, 3H, J = 8 Hz).

### Example 70 3-(7-(3-Bromobenzyl)-1,3-dimethyl-2,6-dioxo-2,3,6,7-tetrahydro-1H-purin-8-yl)-2-methylpropanoic acid (Compound 117)

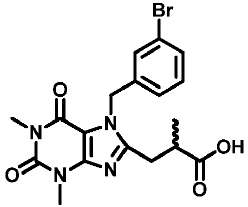

Ethyl 3-(7-(3-bromobenzyl)-1,3-dimethyl-2,6-dioxo-2,3,6,7-tetrahydro-1*H*-purin-8-yl)-2-methylpropanoate (0.85 g, 1.8 mmol, example 67) and potassium hydroxide (1.03 g, 18.3 mmol) were combined in ethanol (20 mL) and water (5 mL); then heated at reflux for 2 h. The reaction was concentrated and the residue was cooled in an ice bath. 2N HCl was added until the mixture reached pH = 2. The mixture was extracted with ethyl acetate (3 x 75 mL). The combined extracts were washed with water (1 x 50 mL), dried with magnesium sulfate, filtered and evaporated under reduced pressure to give 3-(7-(3-bromobenzyl)-1,3-dimethyl-2,6-dioxo-2,3,6,7-tetrahydro-1*H*-purin-8-yl)-2-methylpropanoic acid (0.69 g, 87% yield) as an off white foam. LCMS retention time = 2.595 min and 96% purity, LCMS MH⁺ 435. ¹*H*-NMR (DMSO-*d₆*) δ: 12.31 (brd s, 1H), 7.43-7.50 (m, 2H), 7.29 (t, 1H, J = 8 Hz), 5.57 (dd, 2H, J = 20 Hz and 28 Hz), 3.40 (s, 3H), 3.19 (s, 3H), 2.86-3.05 (m, 2H), 2.68-2.76 (m, 1H), 1.10 (d, 3H, J = 4 Hz).

### Example 71 Ethyl 4-(7-(3-bromobenzyl)-1,3-dimethyl-2,6-dioxo-2,3,6,7-tetrahydro-1H-purin -8-yl)pentanoate (Compound 135)

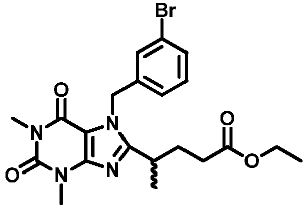

and

### Ethyl 4-(7-(3-bromobenzyl)-1,3-dimethyl-2,6-dioxo-2,3,6,7-tetrahydro-1H-purin-8-yl)-2-methylbutanoate (Compound 136)

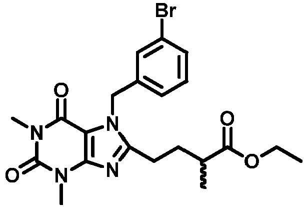

Ethyl 4-(7-(3-bromobenzyl)-1,3-dimethyl-2,6-dioxo-2,3,6,7-tetrahydro-1*H*-purin -8-yl)pentanoate and Ethyl 4-(7-(3-bromobenzyl)-1,3-dimethyl-2,6-dioxo-2,3,6,7-tetrahydro-1*H-*purin-8-yl)-2-methylbutanoate were prepared using the methods of intermediate 34 and example 67.

Ethyl 4-(7-(3-bromobenzyl)-1,3-dimethyl-2,6-dioxo-2,3,6,7-tetrahydro-1*H*-purin-8-yl)pentanoate: Clear oil, 0.17 g, 75% yield: LCMS retention time = 3.791 min and 99% purity. LCMS MH⁺ 477. ¹*H*-NMR (DMSO-*d₆*) δ: 7.48 (d, 1H, J = 8 Hz), 7.43 (s, 1H), 7.29 (t, 1H, J = 8 Hz), 7.10 (d, 1H, J = 12 Hz), 5.58 (q, 2H, J = 20 Hz and 16 Hz), 3.90-4.00 (m, 2H), 3.42 (s, 3H), 3.20 (s, 3H), 3.00-3.08 (m, 1H), 2.08-2.21 (m, 2H), 1.72-1.92 (m, 2H), 1.05-1.13 (m, 6H).

Ethyl 4-(7-(3-bromobenzyl)-1,3-dimethyl-2,6-dioxo-2,3,6,7-tetrahydro-1*H*-purin-8-yl)-2-methylbutanoate: Light golden solid, 0.078 g, 33% yield: LCMS retention time = 3.791 min and 99% purity. LCMS MH⁺ 477. ¹*H*-NMR (DMSO-*d₆*) δ: 7.48 (d, 1H, J = 8 Hz), 7.43 (s, 1H), 7.29 (t, 1H, J = 8 Hz), 7.10 (d, 1H, J = 12 Hz), 5.55 (s, 2H), 4.00 (q, 2H, J = 8 Hz and 12 Hz), 3.41 (s, 3H), 3.20 (s, 3H), 2.64-2.73 (m, 2H), 2.43-2.73 (m, 1H), 1.81-1.92 (m, 1H), 1.56-1.68 (m, 1H), 1.11 (t, 3H, J = 8 Hz), 1.04 (d, 3H, J = 8 Hz).

### Example 72 7-(3-Bromobenzyl)-1,3-dimethyl-8-(3-(trifluoromethyl)phenoxy)-1H-purine-2,6(3H,7H)-dione (Compound 138)

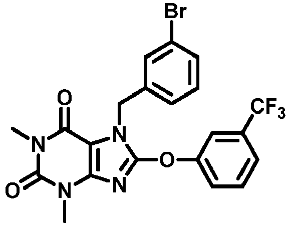

7-(3-Bromobenzyl)-8-chloro-1,3-dimethyl-1*H*-purine-2,6(3*H*,7*H*)-dione (0.50 g, 1.30 mmol, intermediate 39), potassium carbonate (0.54 g, 3.90 mmol) and 3-(trifluoromethyl)phenol (0.17 mL, 1.37 mmol) were combined in DMF (5 mL) and heated at 90°C for 15 h. The reaction was cooled, diluted with water (100 mL) and extracted with ethyl acetate (3 x 50 mL). The combined extracts were washed with 1 N LiCl (2 x 75 mL), dried with magnesium sulfate, filtered and evaporated under reduced pressure to a golden oil. The oil was purified using a 24 g silica gel flash column eluted with 20 % ethyl acetate / hexanes to give 7-(3-bromobenzyl)-1,3-dimethyl-8-(3-(trifluoromethyl)phenoxy)-1*H*-purine-2,6(3*H*,7*H*)-dione (0.26 g, 39% yield) as a white solid. LCMS retention time = 4.377 min and 99% purity, LCMS MH⁺ 509. ¹*H*-NMR (DMSO-*d₆*) δ: 7.59-7.76 (m, 5H), 7.49 (d, 1H, J = 8 Hz), 5.45 (s, 2H), 3.27 (s, 3H), 3.22 (s, 3H).

### Example 73 7-(3-Chlorobenzyl)-1,3-dimethyl-8-(3-(trifluoromethyl)phenoxy)-1H-purine-2,6(3H,7H)-dione (Compound 137)

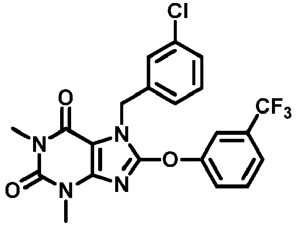

The title compound was prepared using the methods of intermediate 1, step 2 and example 72. White solid: LCMS retention time = 4.341 min and 99% purity, LCMS MH⁺ 465. ¹*H*-NMR (DMSO-*d₆*) δ: 7.73-7.78 (m, 1H), 7.63-7.72 (m, 3H), 7.44-7.47 (m, 1H), 7.33-7.39 (m, 3H), 5.45 (s, 2H), 3.27 (s, 3H), 3.22 (s, 3H).

### Example 74 7-(4-Chlorobenzyl)-1,3-dimethyl-8-(3-(trifluoromethyl)phenoxy)-1H-purine-2,6(3H,7H)-dione (Compound 139)

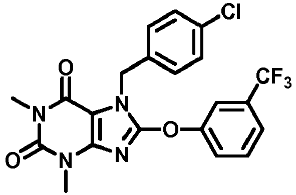

The title compound was prepared using the methods of intermediate 1, step 2 and example 72. White solid: LCMS retention time = 4.336 min and 100% purity, LCMS MH⁺ 465. ¹*H*-NMR (DMSO-*d₆*) δ: 7.75-7.78 (m, 1H), 7.64-7.72 (m, 3H), 7.41 (s, 4H), 5.44 (s, 2H), 3.27 (s, 3H), 3.22 (s, 3H).

### Example 75 7-(3-Chlorobenzyl)-1,3-dimethyl-8-(3-((methylamino)phenoxy)-1H-purine-2,6(3H,7H)-dione hydrochloride (Compound 152)

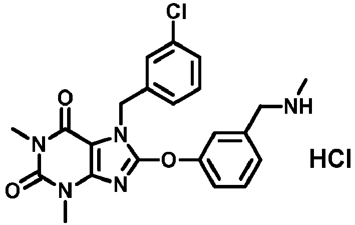

7-(3-Chlorobenzyl)-8-(3-chloromethyl)phenoxy)-1,3-dimethyl-1*H*-purine-2,6(3*H*,7*H*)-dione (0.17 g, 0.39 mmol, intermediate 38) and 2.0 M methylamine in THF (1.95 mL) were dissolved in THF (1 mL), then stirred at room temperature for 15 h. The reaction was evaporated to dryness under reduced pressure and purified using a 2000 micron preparative TLC plate eluted with 5% methanol / DCM to give a clear oil (0.052 g). The oil was dissolved in DCM (1 mL) and excess 2 N HCl in diethyl ether was added. The mixture was evaporated under reduced pressure, then azeotroped with 3 times with diethyl ether (5 mL) to give 7-(3-Chlorobenzyl)-1,3-dimethyl-8-(3-((methylamino)phenoxy)-1*H*-purine-2,6(3*H*,7*H*)-dione hydrochloride (0.52 g, 28% yield) as a white solid. LCMS retention time = 2.296 min and 98% purity, LCMS MH⁺ 439. ¹*H*-NMR (DMSO-*d₆*) δ: 9.27 (brd s, HCl and NH), 7.27-7.54 (m, 8H), 5.44 (s, 2H), 4.10-4.15 (m, 2H), 3.29 (s, 3H), 3.23 (s, 3H), 2.50-2.55 (m, 3H).

### Example 76 7-(3-Chlorobenzyl)-8-(3-((dimethylamino)methyl)phenoxy)-1,3-dimethyl-1H-purine-2,6(3H,7H)-dione hydrochloride (Compound 153)

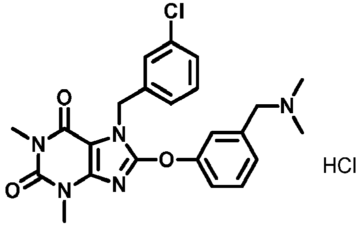

The title compound was prepared using the method of example 75. White foam, 0.10 g, 52% yield: LCMS retention time = 2.423 min and 97% purity, LCMS MH⁺ 454. ¹*H*-NMR (DMSO-*d₆*) δ: 10.93 (brd s, 1H), 7.27-7.62 (m, 8H), 5.45 (s, 2H), 4.10-4.28 (m, 2H), 3.28 (s, 3H), 3.22 (s, 3H), 2.67 (d, 6H, J = 4 Hz).

### Example 77 7-(3-Chlorobenzyl)-8-ethoxy-1,3-dimethyl-1H-purine-2,6(3H,7H)-dione (Compound 154)

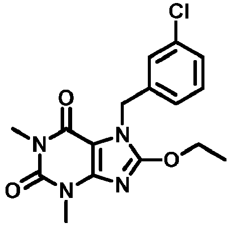

Sodium (0.090 g 3.90 mmol) was added to ethanol (2 mL) to form sodium ethoxide; then 7-(3-chlorobenzyl)-8-(3-(chloromethyl)phenoxy)-1,3-dimethyl-1*H*-purine-2,6(3*H*,7*H*)-dione (0.17 g, 0.39 mmol) was added. The reaction was stirred for 24 h; then the solvent was evaporated under reduced pressure. The reaction was diluted with water (50 mL) and extracted with ethyl acetate (2 x 50 mL). The combined extracts were dried with magnesium sulfate and evaporated under reduced pressure to leave a golden oil. This oil was purified using a 12 g silica gel flash column eluted with 2 % methanol / MC to give 7-(3-chlorobenzyl)-8-ethoxy-1,3-dimethyl-1*H*-purine-2,6(3H,7H)-dione (0.13 g, 96% yield) as a tan solid. LCMS retention time = 3.690 min and 95% purity, LCMS MH⁺ 349. ¹*H*-NMR (DMSO-*d₆*) δ: 7.32-7.39 (m, 3H), 7.19-7.24 (m, 1H), 5.21 (s, 2H), 4.48 (q, 2H, J = 4 Hz, and 28 Hz), 3.34 (s, 3H), 3.19 (s, 3H), 1.34 (t, 3H, J = 4 Hz).

### Example 78 7-(4-Chlorobenzyl)-1,3-dimethyl-8-(3-((methylaminol)methyl)phenoxy)-1H-purine-2,6(3H,7H)-dione hydrochloride (Compound 163)

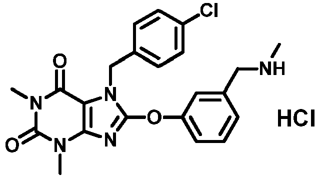

The title compound was prepared using the methods of intermediate 37 and example 75. Off-white solid, 0.034 g, 13% yield: LCMS retention time = 2.491 min and 97% purity, LCMS MH⁺ 440 and 97% pure. ¹*H*-NMR (DMSO-*d₆*) δ: 9.19 (brd s, 1H), 7.36-7.54 (m, 8H), 5.43 (s, 2H), 4.10-4.18 (m, 2H), 3.28 (s, 3H), 3.22 (s, 3H), 2.52 (t, 3H, J = 8 Hz).

### Example 79 7-(4-Chlorobenzyl)-8-(3-((dimethylaminol)methyl)phenoxy)-1,3-dimethyl-1H-purine-2,6(3H,7H)-dione hydrochloride (Compound 164)

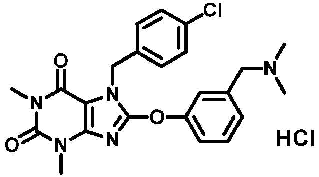

The title compound was prepared using the methods of intermediate 37 and example 75. Off-white solid, 0.078 g, 30% yield: LCMS retention time = 2.524 min and 98% purity, LCMS MH⁺ 454. ¹*H*-NMR (DMSO-*d₆*) δ: 10.85 (brd s, 1H), 7.38-7.62 (m, 8H), 5.43 (s, 2H), 4.29 (d, 2H, J = 8 Hz), 3.27 (s, 3H), 3.22 (s, 3H), 2.57 (d, 6H, J = 4 Hz).

### Example 80 7-(4-Chlorobenzyl)-1,3-dimethyl-8-(3-(morpholininomethyl)phenoxy)-1H-purine-2,6(3H,7H)-dione (Compound 176)

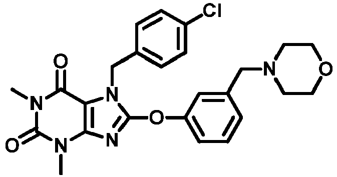

7-(4-Chlorobenzyl)-1,3-dimethyl-8-(3-chloromethyl)phenoxy)1,3-dimethyl-1*H*-purine-2,6(3*H*,7*H*)-dione (0.25 g, 0.55 mmol, intermediate 37) and morpholine (0.48 mL, 5.50 mmol) were combined in THF (5 mL) and stirred at room temperature for 15 h. The reaction was diluted with water (100 mL) and extracted with ethyl acetate (3 x 75 mL). The combined extracts were dried with magnesium sulfate, filtered and evaporated under reduced pressure to leave a golden oil. This oil was purified using a 12 g silica gel flash column eluted with 1.5% methanol / MC to give 7-(4-chlorobenzyl)-1,3-dimethyl-8-(3-(morpholininomethyl)phenoxy)-1*H*-purine-2,6(3*H*,7*H*)-dione (0.13 g, 47% yield) as a white solid. LCMS retention time = 2.595 min and 98% purity, LCMS MH⁺ 496. ¹*H*-NMR (DMSO-*d₆*) δ: 7.35-7.44 (m, 6H), 7.15-7.24 (m, 3H), 5.42 (s, 2H), 3.45-3.60 (m, 4H), 3.47 (s, 2H), 3.26 (s, 3H), 3.22 (s, 3H), 2.28-2.40 (m, 4H).

### Example 81 7-(4-Chlorobenzyl)-1,3-dimethyl-8-((3-(trifluoromethoxy)benzyl)oxy)-1H-purine-2,6(3H,7H)-dione (Compound 188)

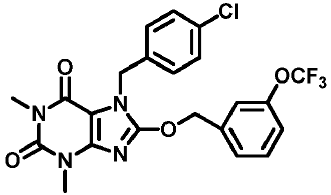

(3-(Trifluoromethoxy)phenyl)methanol (0.31 g, 1.62 mmol) was dissolved in THF (8 mL) and sodium hydride (60% in oil, 0.071 g, 1.76 mmol) was added. The reaction was stirred for 20 min then 8-chloro-7-(4-chlorobenzyl)-1.3-dimethyl-1H-purine-2,6(3H,7H)-dione (0.50 g, 1.47 mmol, intermediate 35) was added. The reaction was stirred at room temperature for 15 h; then it was diluted with water (100 mL) and extracted with ethyl acetate (2 x 100 mL). The combined extracts were dried with magnesium sulfate, filtered and evaporated under reduced pressure to give an off-white solid. Solid was purified using a 40 g silica gel flash column eluted with 20% ethyl acetate / hexanes to give 7-(4-chlorobenzyl)-1,3-dimethyl-8-((3-(trifluoromethoxy)benzyl)oxy)-1H-purine-2,6(3H,7H)-dione (0.42 g, 58% yield) as a white solid. LCMS retention time = 4.302 min and 99% purity, LCMS 495.¹*H*-NMR DMSO-*d₆* δ: 7.36-7.53 (m, 3H), 7.24-7.27 (m, 5H), 5.56 (s, 2H), 5.26 (s, 2H), 3.37 (s, 3H), 3.19 (s, 3H).

The following examples 82a through 82d were prepared using the method of example 81.

### Example 82a 7-(4-Chlorobenzyl)-8-(hexyloxy)-1,3-dimethyl-1H-purine-2,6(3H,7H)-dione (Compound 215)

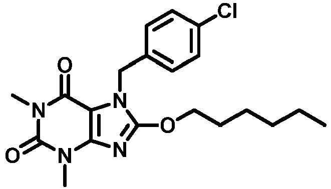

White solid: LCMS retention time = 4.567 min and 98% purity, LCMS MH⁺ 405. ¹*H*-NMR (DMSO-*d₆*) δ: 7.32 (dd, 4H, J = 36 Hz and 44 Hz), 5.21 (2, 2H), 4.42 (t, 2H, J = 8 Hz), 3.35 (s, 3H), 3.17 (s, 3H), 1.63-1.72 (m, 2H), 1.16-1.30 (m, 6H), 0.82 (t, 3H, J = 8 Hz).

### Example 82b 7-(4-Chlorobenzyl)-8-((5-hydroxypentyl)oxy)-1,3-dimethyl-1H-purine-2,6(3H,7H)-dione (Compound 217)

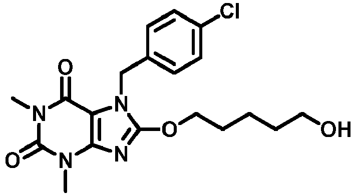

White solid, 0.15 g, 25 % yield: LCMS retention time = 2.934 min and 98% purity, LCMS MH⁺ 407. ¹*H*-NMR (DMSO-*d₆*) δ: 7.34 (dd, 4H, J = 8 Hz and 40 Hz), 5.20 (s, 2H), 4.43 (t, 2H, J = 8 Hz), 4.36 (t, 1H, J = 4 Hz), 3.33-3.41 (m, 5H), 1.66-1.76 (m, 2H), 1.37-1.45 (m, 2H), 1.28-1.36 (m, 2H).

### Example 82c 7-(4-Chlorobenzyl)-8-(cyclopentylmethoxy)-1,3-dimethyl-1H-purine-2,6(3H,7H)-dione (Compound 216)

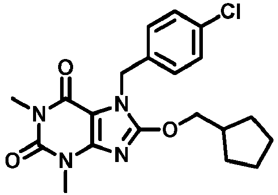

White solid: LCMS retention time = 4.377 min and 100% purity, LCMS MH⁺ 403. ¹*H*-NMR DMSO-*d₆* δ: 7.34 (dd, 4H, J = 8 Hz and 44 Hz), 5.21 (s, 2H), 4.32 (t, 2H, J = 8 Hz), 3.35 (s, 3H), 2.24-2.35 (m, 1H), 1.40-1.72 (m, 4H), 1.13-1.28 (m, 4H).

### Example 82d 7-(4-Chlorobenzyl)-8-(hexyloxy)-1,3-dimethyl-1H-purine-2,6(3H,7H)-dione (Compound 215)

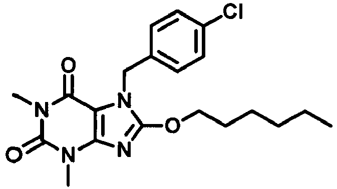

White solid: LCMS retention time = 4.574 min and 99% purity, LCMS MH⁺ 405. ¹*H*-NMR DMSO-*d₆* δ: 7.32 (dd, 4H, J = 8 Hz and 44 Hz), 5.21 (s, 2H), 4.42 (t, 2H, J = 8 Hz), 3.35 (s, 3H), 1.63-1.72 (m, 2H), 1.15-1.28 (m, 6H), 0.78-0.87 (t, 3H, J = 8 Hz).

## Claims

1. A compound of Formula I: 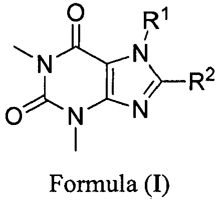 or a pharmaceutically acceptable salt thereof, wherein:
R¹ is 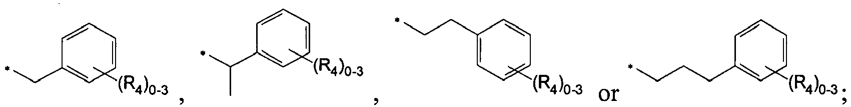
R² is C₁-C₆ alkyl, C₂-C₆ alkenyl, C₂-C₆ alkynyl, C₁-C₆ haloalkyl, hydroxyl, C₁-C₆ alkoxy, C₁-C₆ heteroalkyl, C₆-C₁₀ aryloxy, C₇-C₁₆ arylalkoxy, C₁-C₆ alkylamino, C₂-C₁₂ dialkylamino, - C(O)-, -S(O)-, -O-, heterocycloalkyl, heteroaryl, sulfonamidyl, amido, urea, sulfonylurea, acyl, nitro, cyano, wherein each of C₁-C₆ alkyl, C₂-C₆ alkenyl, C₂-C₆ alkynyl, C₁-C₆ haloalkyl, C₁-C₆ alkoxy, C₁-C₆ heteroalkyl, C₆-C₁₀ aryloxy, C₇-C₁₆ arylalkoxy, C₁-C₆ alkylamino, C₂-C₁₂ dialkylamino, -S(O)-, heterocycloalkyl, heteroaryl, sulfonamidyl, amido, urea, sulfonylurea, acyl, is optionally substituted with 1-3 R³;
each R³ is independently C₁-C₆ alkyl, C₁-C₆ heteroalkyl, halo, C₁-C₆ haloalkoxy, hydroxyl, C₁-C₆ alkoxy, amino, C₁-C₆ alkylamino, C₂-C₁₂ dialkylamino, cyano, nitro, amido, -S-, -S(O)₂-, -C(O)-, -C(O)O-C₁-C₆ alkyl, -C(O)-C₁-C₆ alkyl, C₃-C₇ cycloalkyl, heterocycloalkyl, phenyl, or naphthyl, wherein each of C₁-C₆ alkyl, C₁-C₆ heteroalkyl, C₁-C₆ haloalkoxy, C₁-C₆ alkoxy, amino, C₁-C₆ alkylamino, C₂-C₁₂ dialkylamino, amido, -S-, -S(O)₂-, -C(O)-, -C(O)O-C₁-C₆ alkyl, -C(O)-C₁-C₆ alkyl, C₃-C₇ cycloalkyl, heterocycloalkyl, phenyl, or naphthyl is optionally substituted with 1-3 R⁴; and
each R⁴ is independently C₁-C₆ alkyl, C₁-C₆ heteroalkyl, C₂-C₆ alkenyl, C₂-C₆ alkynyl, C₃-C₇ cycloalkyl, C₁-C₆ haloalkoxy, C₁-C₆ haloalkyl, heterocycloalkyl, C₆-C₁₀ aryl, heteroaryl, C₄-C₁₀ cycloalkylalkyl, heterocycloalkylalkyl, C₇-C₁₆ arylalkyl, heteroaryl-C₁-C₆ alkyl, halo, hydroxyl, C₁-C₆ alkoxy, C₁-C₆ hydroxyalkyl, C₆-C₁₀ aryloxy, C₇-C₁₆ arylalkoxy, C₂-C₈ alkoxyalkoxyl, amino, C₁-C₆ alkylamino, C₂-C₁₂ dialkylamino, -S-, -S-C₁-C₆ alkyl, -S(O)₂-C₁-C₆ alkyl, sulfonamidyl, amido, urea, sulfonylurea, acyl, -C(O)-C₆-C₁₀ aryl, -NHC(O)-C₆-C₁₀ aryl, - C(O)NH-C₆-C₁₀ aryl, -C(O)OH, -C(O)O-C₁-C₆ alkyl, -C(O)-C₁-C₆ alkyl, acyl, nitro, or cyano.

2. The compound according to claim 1, wherein R² is C₁-C₆ alkyl, C₂-C₆ alkenyl, C₁-C₆ alkoxy, C₁-C₆ heteroalkyl, C₆-C₁₀ aryloxy, C₇-C₁₆ arylalkoxy, C₁-C₆ alkylamino, C₂-C₁₂ dialkylamino, -C(O)-, -S(O)-, -O-, or heterocycloalkyl, wherein each of C₁-C₆ alkyl, C₂-C₆ alkenyl, C₁-C₆ alkoxy, C₁-C₆ heteroalkyl, C₆-C₁₀ aryloxy, C₇-C₁₆ arylalkoxy, C₁-C₆ alkylamino, C₂-C₁₂ dialkylamino, -S(O)-, or heterocycloalkyl is optionally substituted with 1-3 R³.

3. The compound according to claim 2, wherein each R³ is independently C₁-C₆ alkyl, C₁-C₆ heteroalkyl, halo, C₁-C₆ haloalkoxy, hydroxyl, C₁-C₆ alkoxy, amino, C₁-C₆ alkylamino, C₂-C₁₂ dialkylamino, cyano, amido, -C(O)-, -C(O)O-C₁-C₆ alkyl, -C(O)-C₁-C₆ alkyl, heterocycloalkyl, phenyl, or napthyl, wherein each of C₁-C₆ alkyl, C₁-C₆ heteroalkyl, C₁-C₆ haloalkoxy, hydroxyl, C₁-C₆ alkoxy, amino, C₁-C₆ alkylamino, C₂-C₁₂ dialkylamino, amido, - C(O)-, -C(O)O-C₁-C₆ alkyl, -C(O)-C₁-C₆ alkyl, heterocycloalkyl, phenyl, or napthyl is optionally substituted with 1-3 R⁴.

4. The compound according to claim 3, wherein each R⁴ is independently C₁-C₆ alkyl, C₁-C₆ heteroalkyl, C₃-C₇ cycloalkyl, C₁-C₆ haloalkoxy, C₁-C₆ haloalkyl, heterocycloalkyl, C₆-C₁₀ aryl, heteroaryl, C₄-C₁₀ cycloalkylalkyl, heterocycloalkylalkyl, heteroaryl-C₁-C₆ alkyl, halo, hydroxyl, C₁-C₆ alkoxy, C₁-C₆ hydroxyalkyl, amino, C₁-C₆ alkylamino, C₂-C₁₂ dialkylamino, amido, -C(O)O-C₁-C₆ alkyl, -C(O)-C₁-C₆ alkyl, or cyano.

5. The compound according to claim 1, wherein R² is selected from the group consisting of:

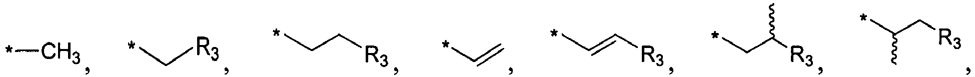

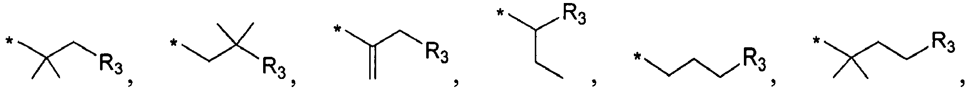

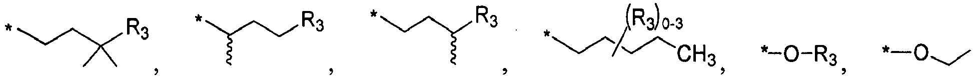

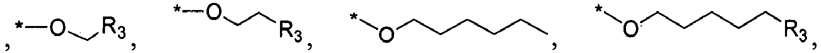

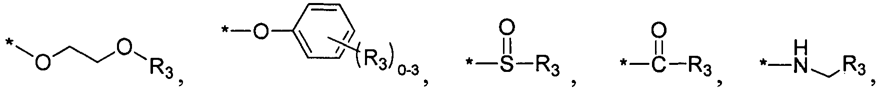

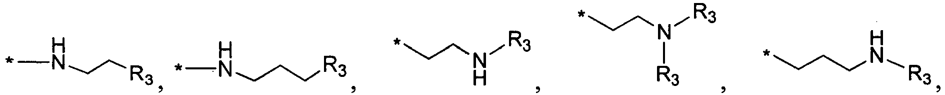

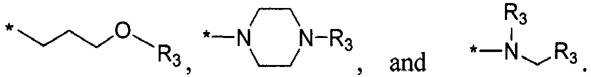

6. The compound according to claim 1, wherein each R³ is independently selected from the group consisting of: 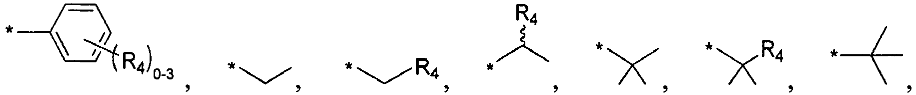 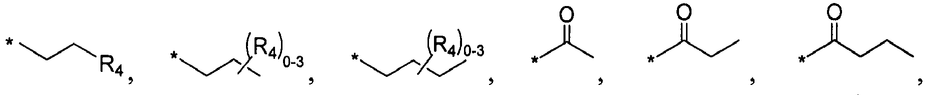 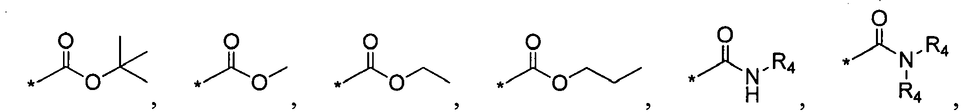 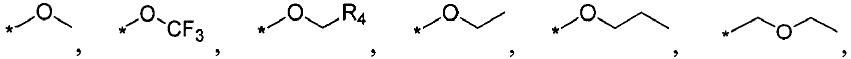 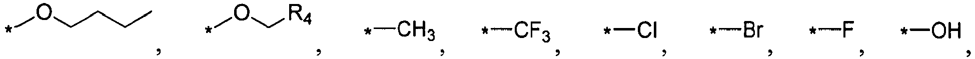 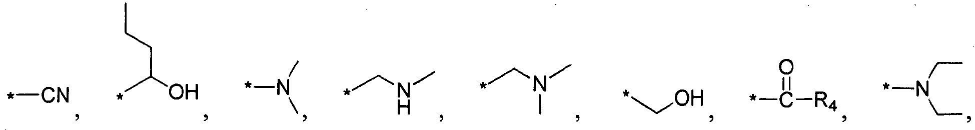 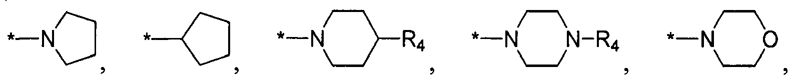 and

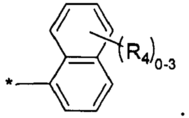

7. The compound according to claim 1, wherein each R⁴ is independently selected from the group consisting of:

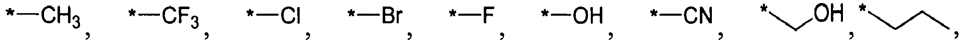

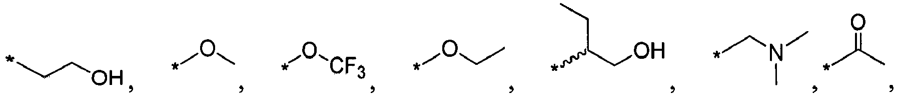

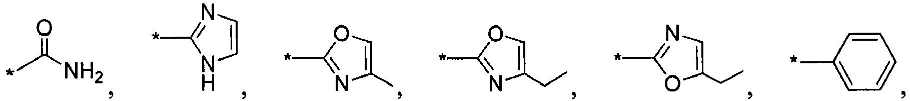

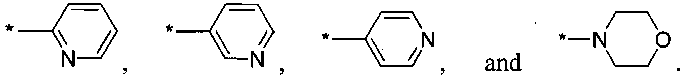

8. The compound according to claim 1, wherein:
R² is 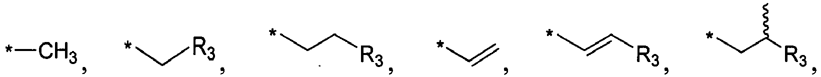 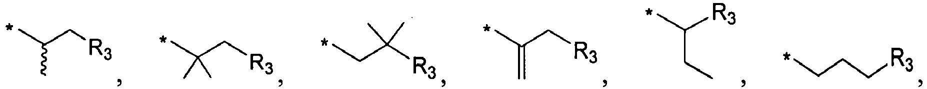 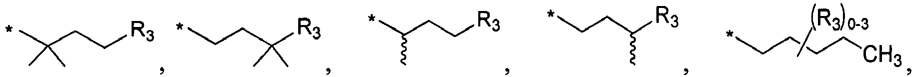 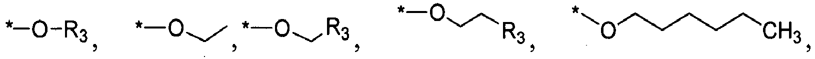 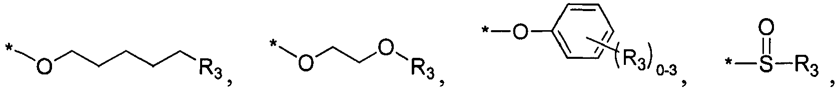 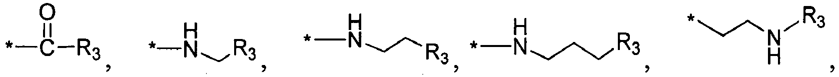 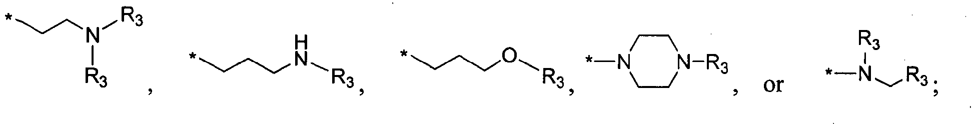
each R³ is independently 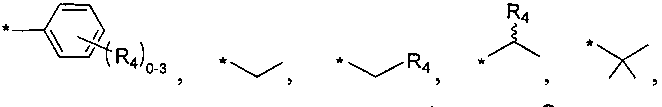 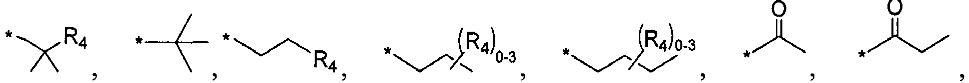 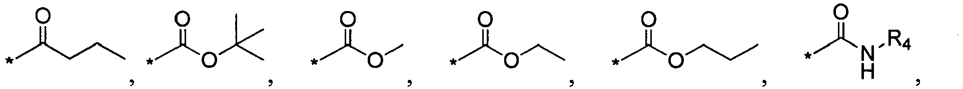 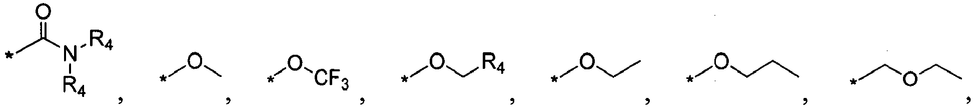 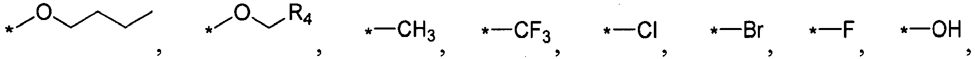 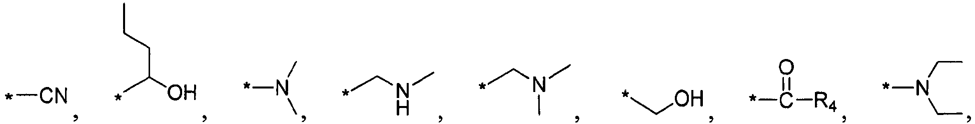 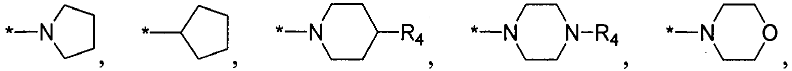 or 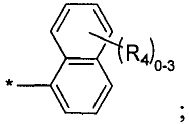 and
each R⁴ is independently

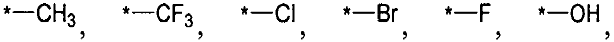

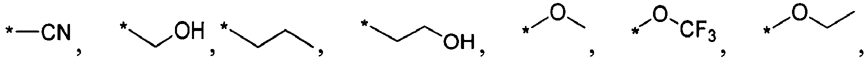

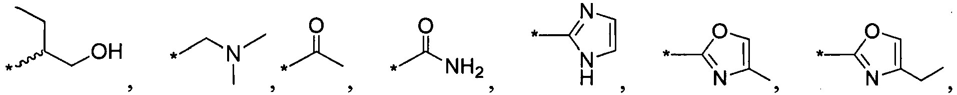

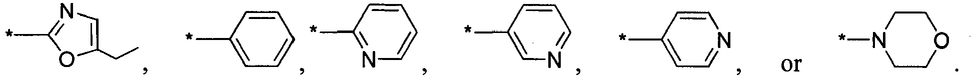

9. A compound selected from the group consisting of:

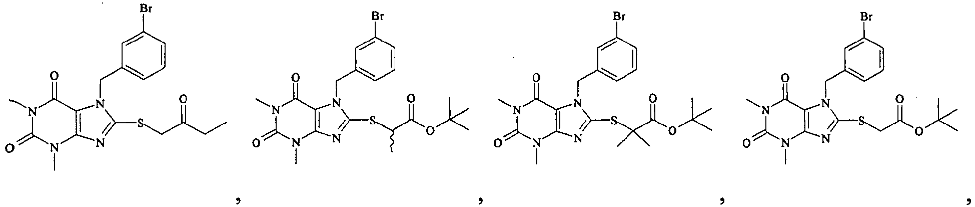

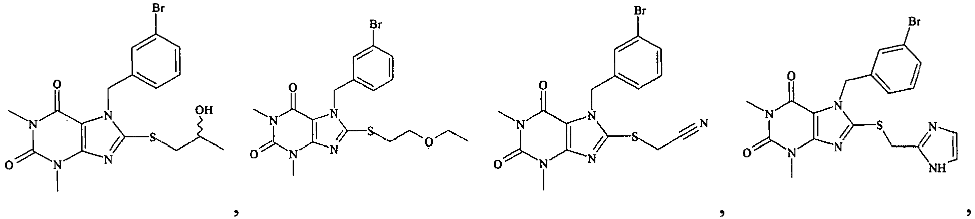

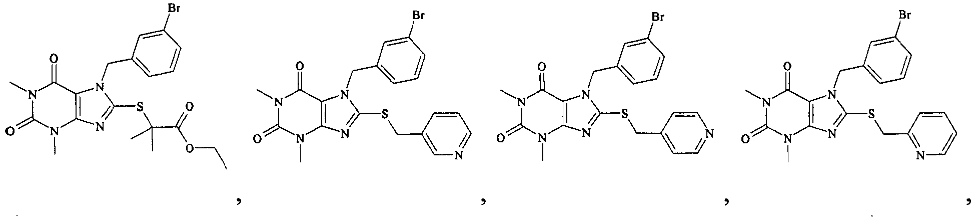

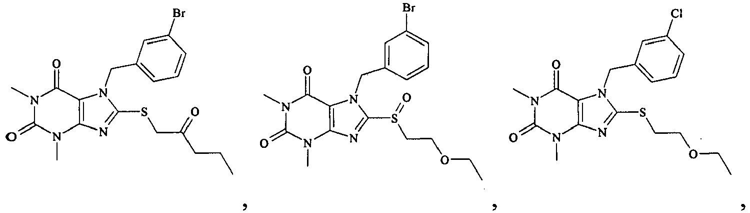

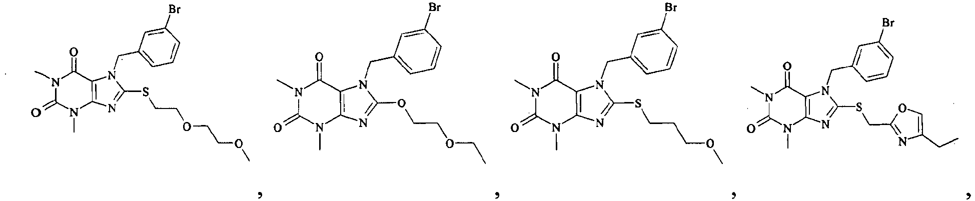

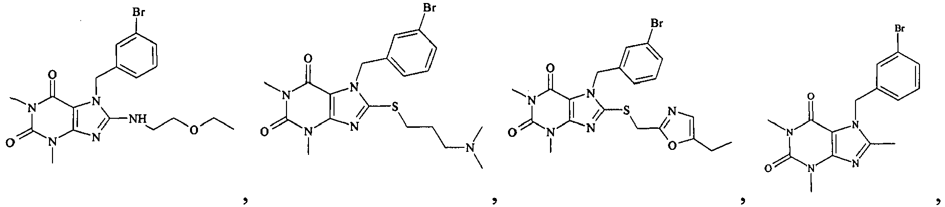

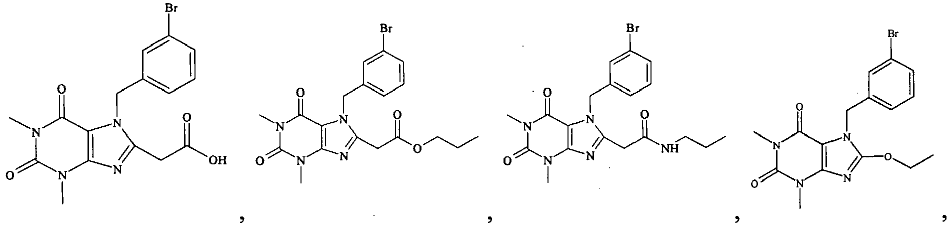

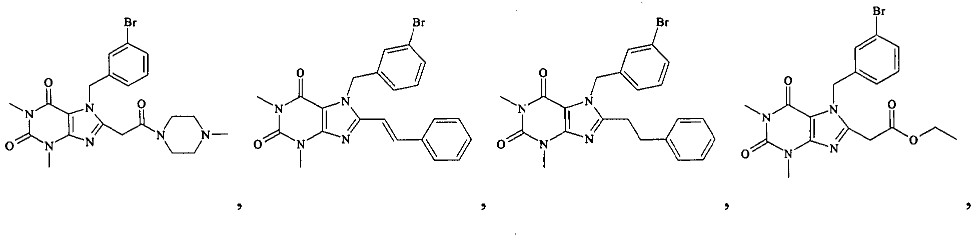

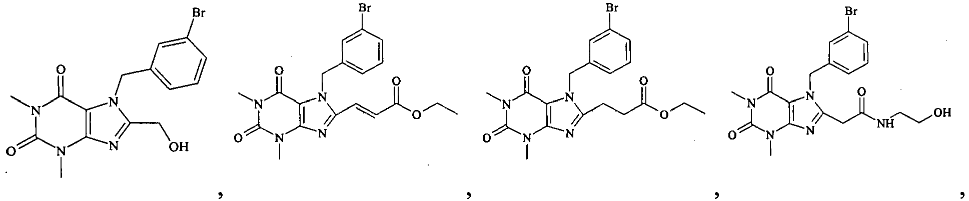

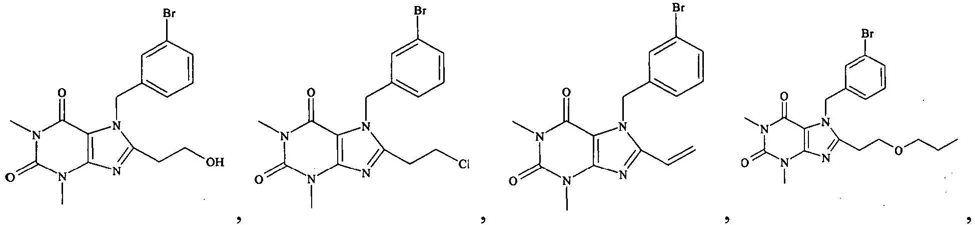

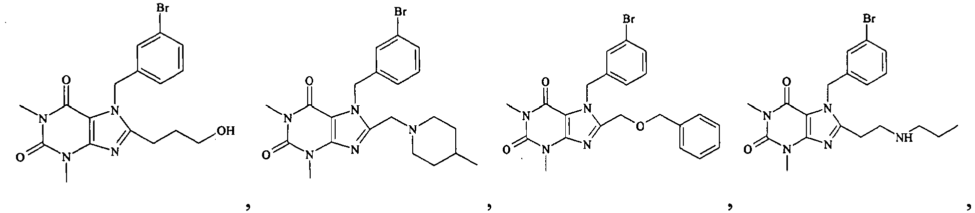

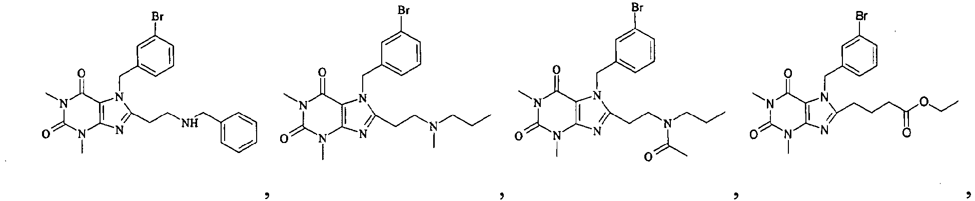

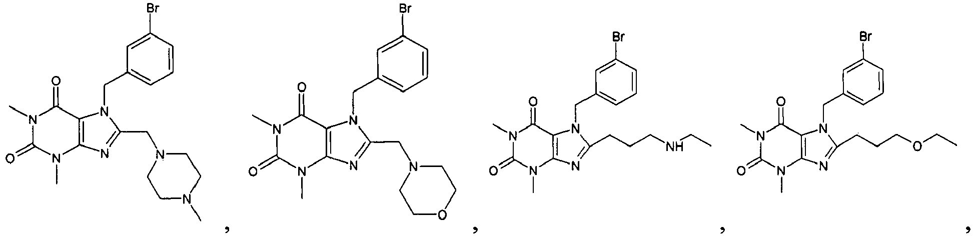

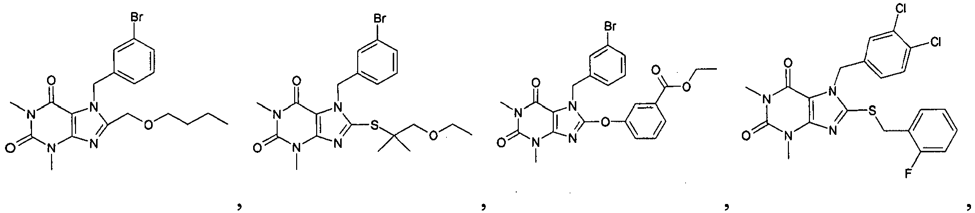

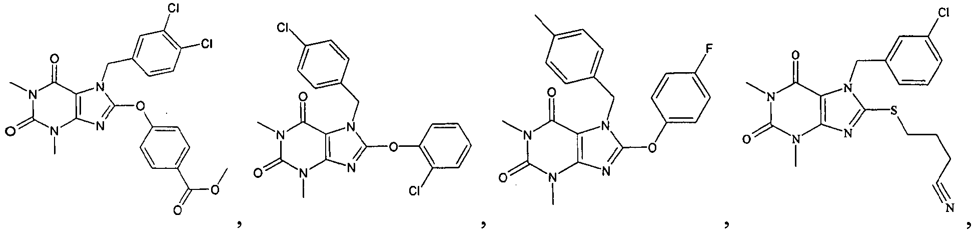

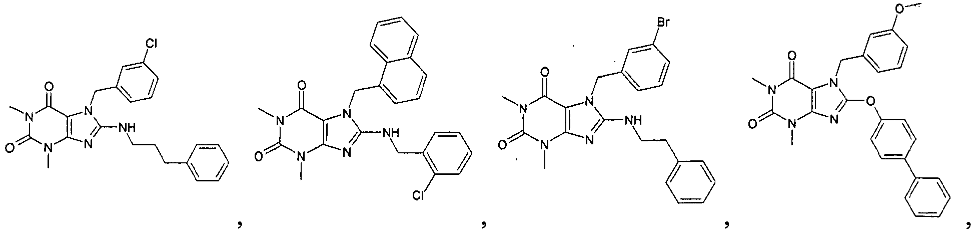

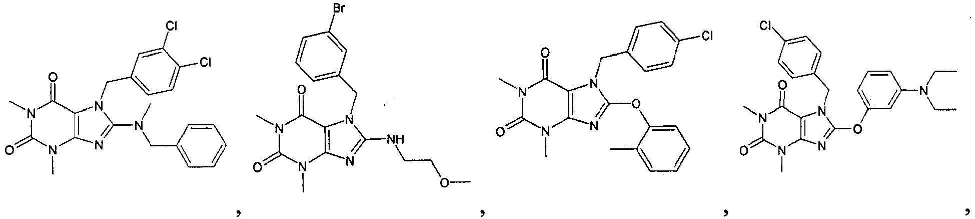

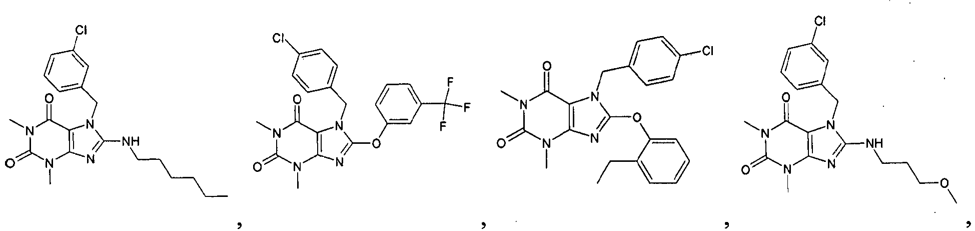

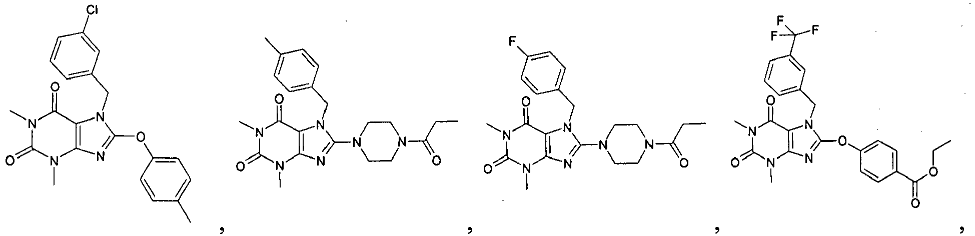

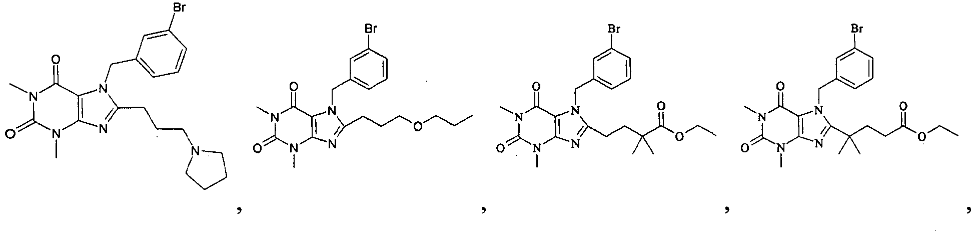

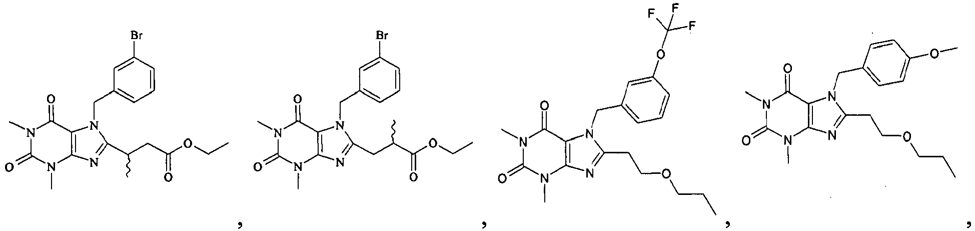

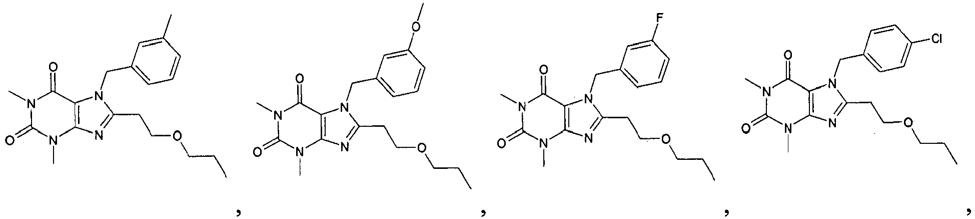

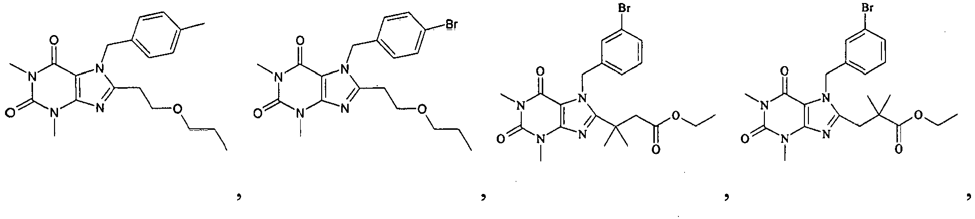

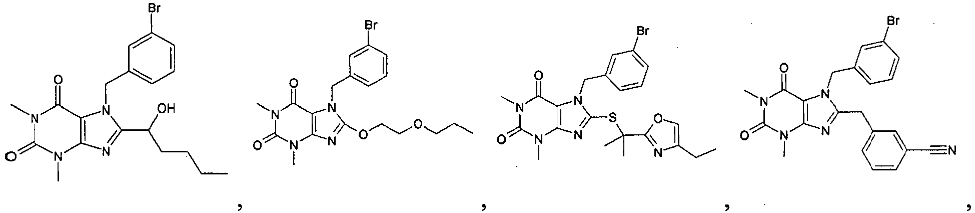

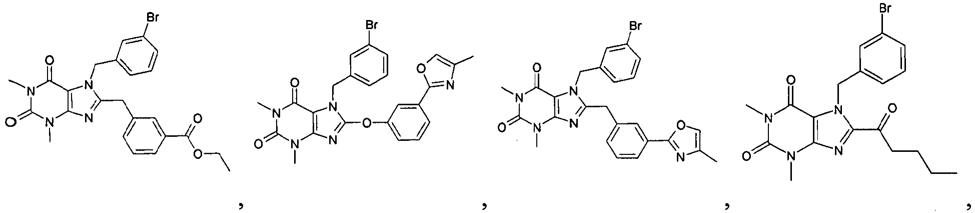

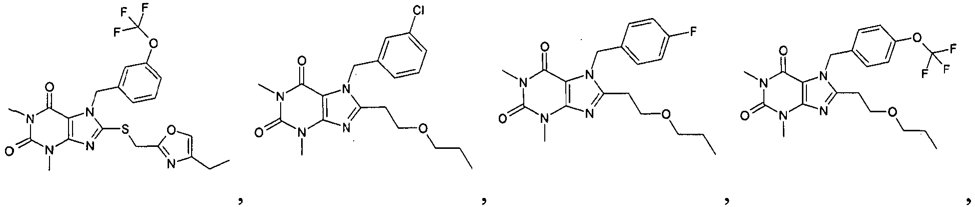

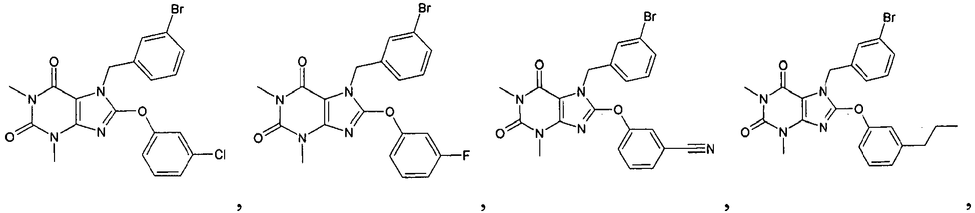

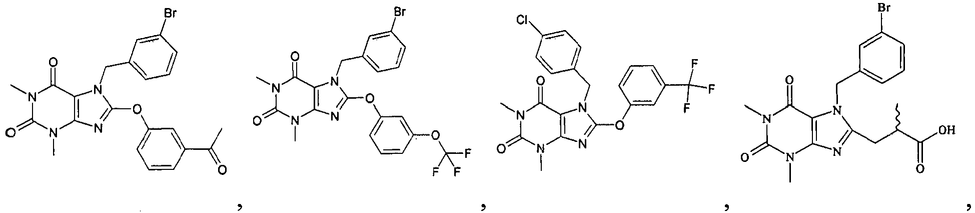

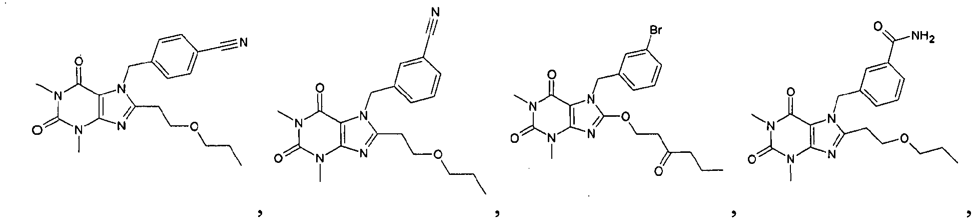

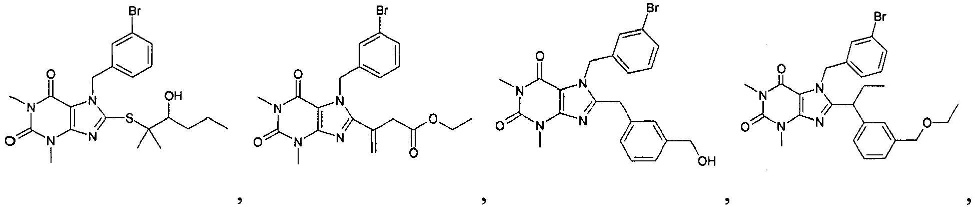

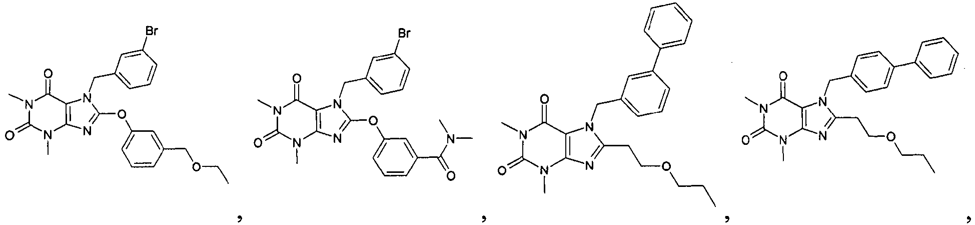

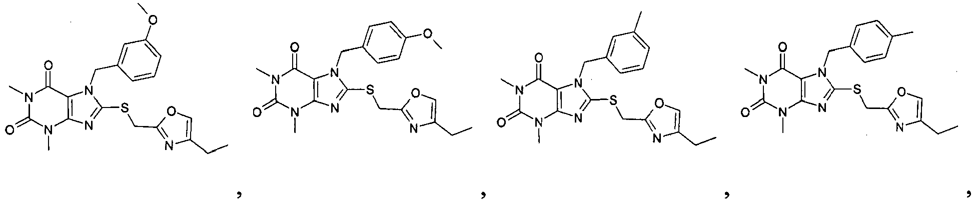

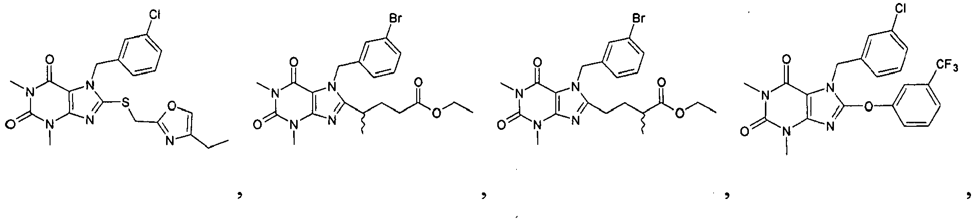

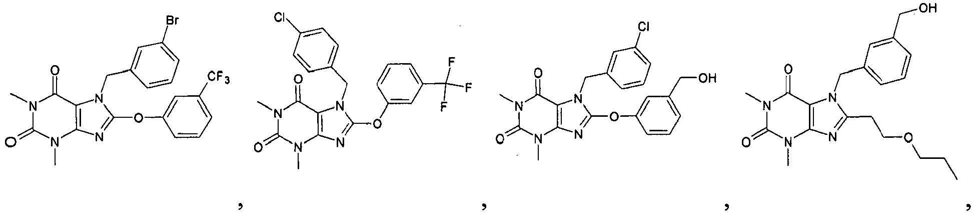

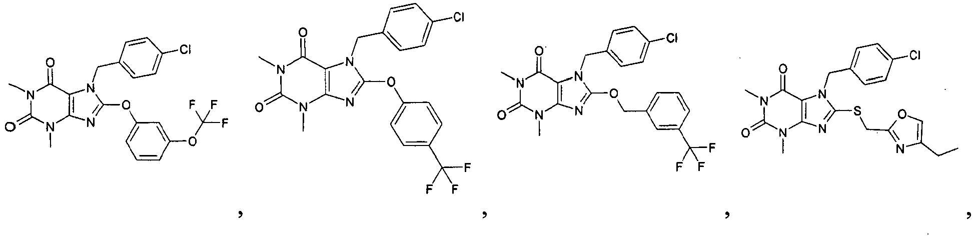

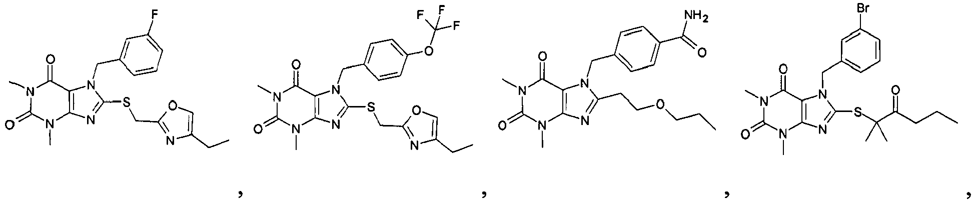

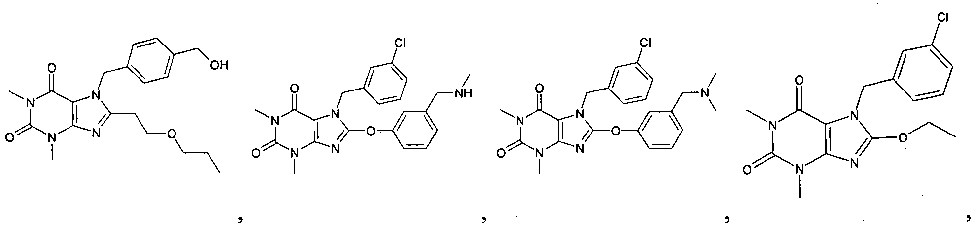

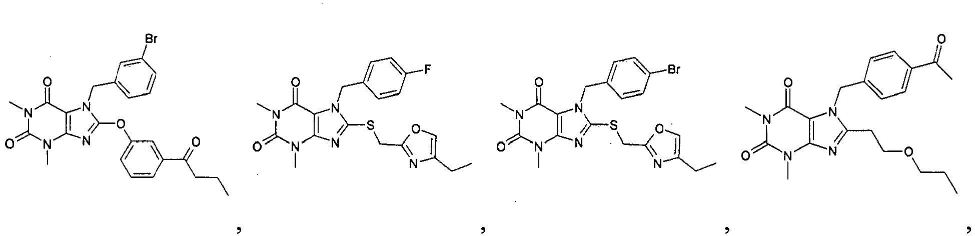

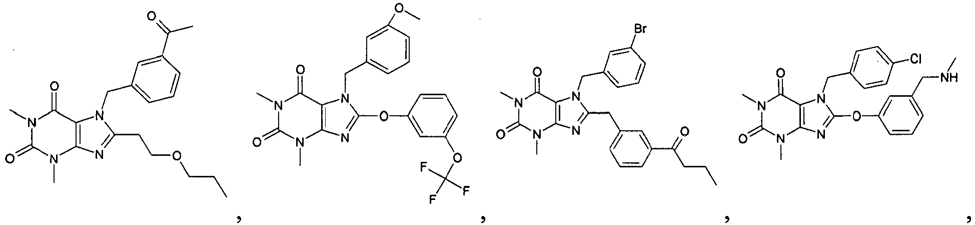

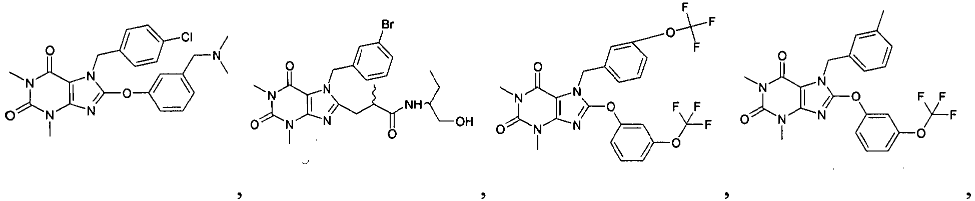

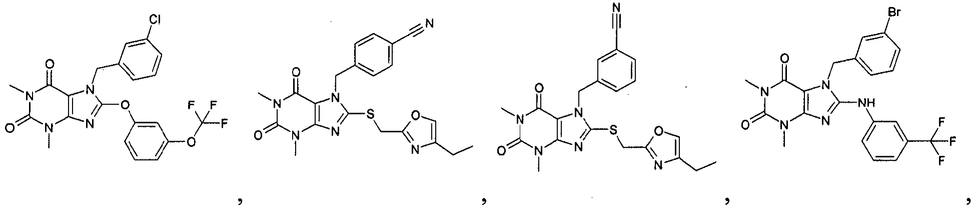

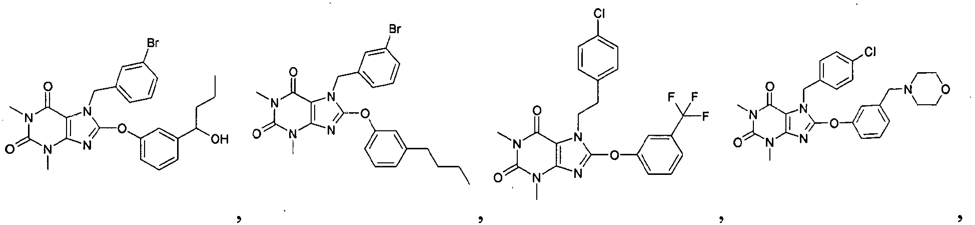

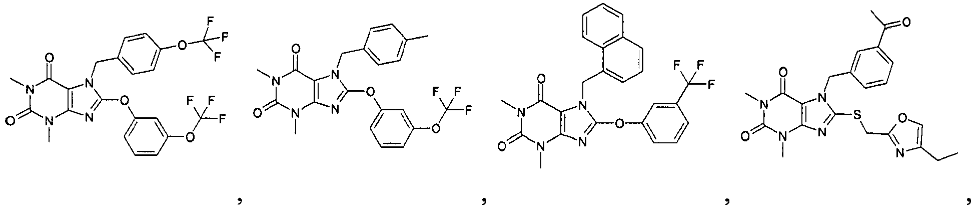

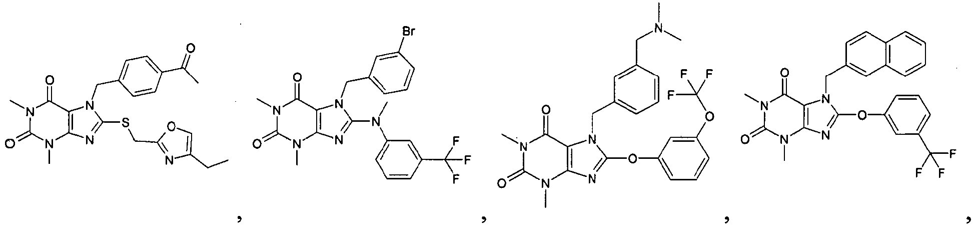

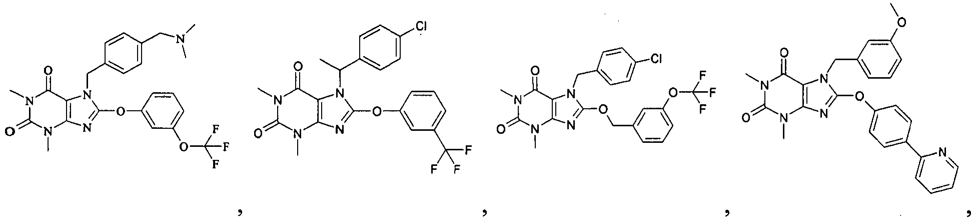

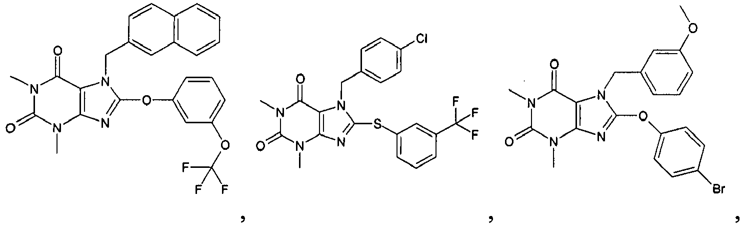

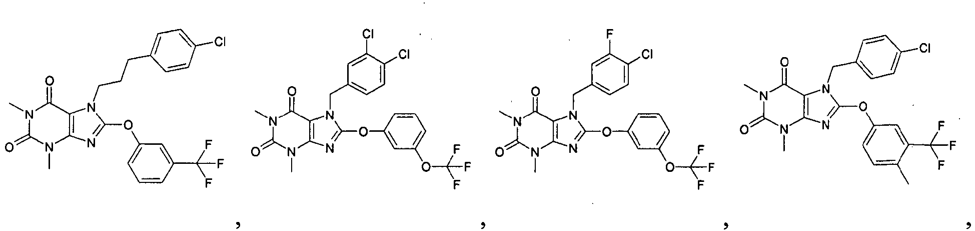

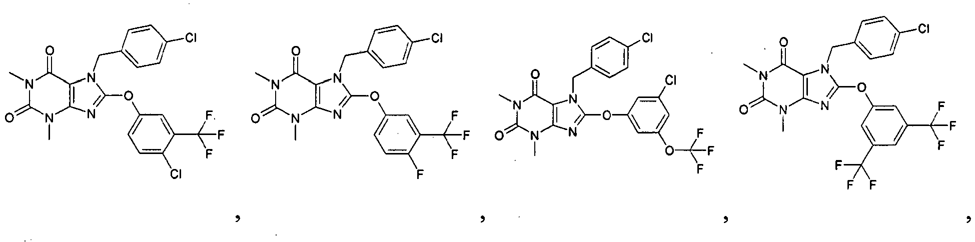

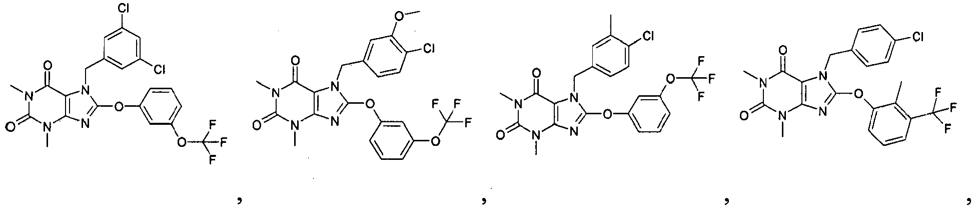

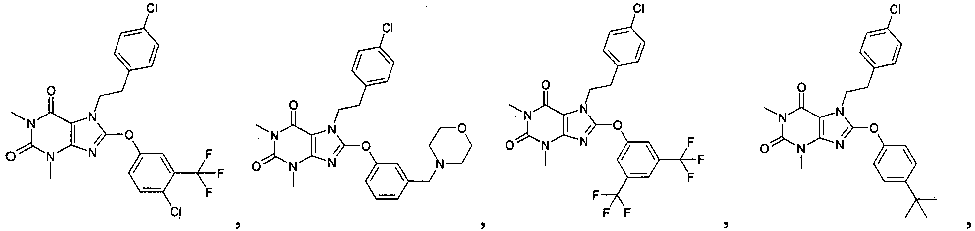

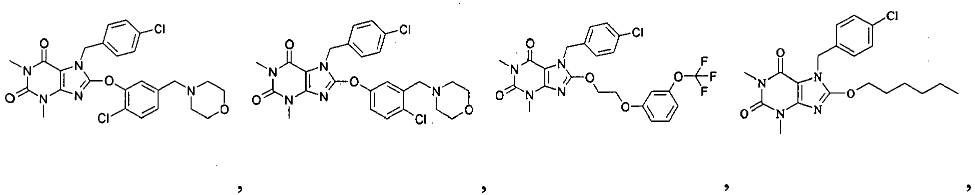

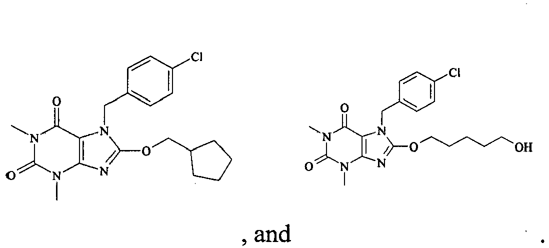

10. A compound of Formula II: 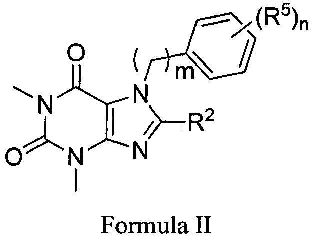 or a pharmaceutically acceptable salt thereof, wherein:
R² is C₁-C₆ alkoxy, C₁-C₆ heteroalkyl, C₆-C₁₀ aryloxy, C₇-C₁₆ arylalkoxy, or -C(O)-C₁-C₆ alkyl, each independently substituted with 1-3 R³;
each R³ is independently halo, C₁-C₆ alkyl, C₁-C₆ haloalkyl, C₁-C₆ haloalkoxy, C₁-C₆ heteroalkyl, hydroxyl, -C(O)O-C₁-C₆ alkyl, -C(O)-C₁-C₆ alkyl, phenyl, or heteroaryl, wherein each of C₁-C₆ alkyl, C₁-C₆ haloalkyl, C₁-C₆ haloalkoxy, C₁-C₆ heteroalkyl, -C(O)O-C₁-C₆ alkyl, -C(O)-C₁-C₆ alkyl, phenyl, or heteroaryl is optionally substituted with 1-3 R⁴;
R⁴ and R⁵ are each independently hydroxyl, C₁-C₆ alkyl, C₁-C₆ alkoxy, C₁-C₆ heteroalkyl, C₁-C₆ haloalkyl, C₁-C₆ haloalkoxy or halo;
n is 1 or 2; and
m is 1, 2, or 3.

11. A pharmaceutical composition comprising a compound according to any of the preceding claims, or a pharmaceutically acceptable salt thereof.

12. A compound or a pharmaceutical composition according to any of the preceding claims for use in a method of treating a TRPC5 mediated disorder, wherein the TRPC5 mediated disorder is selected from the group consisting of: neuropsychiatric disorder, neurodegenerative disorder, nephropathy, and seizure disorder.

## Patentansprüche

1. Verbindung der Formel (I): 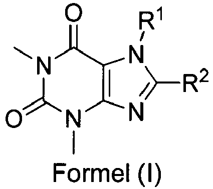 oder ein pharmazeutisch annehmbares Salz davon, worin:
R¹ 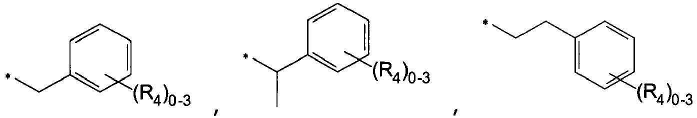 oder 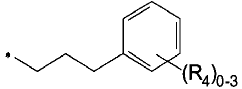 ist;
R² C₁₋₆-Alkyl, C₂₋₆-Alkenyl, C₂₋₆-Alkinyl, C₁₋₆-Halogenalkyl, Hydroxyl, C₁₋₆-Alkoxy, C₁₋₆-Heteroalkyl, C₆₋₁₀-Aryloxy, C₇₋₁₆-Arylalkoxy, C₁₋₆-Alkylamino, C₂₋₁₂-Dialkylamino, -C(O)-, -S(O)-, -O-, Heterocycloalkyl, Heteroaryl, Sulfonamidyl, Amido, Harnstoff, Sulfonylharnstoff, Acyl, Nitro oder Cyano ist, wobei jedes/jeder C₁₋₆-Alkyl, C₂₋₆-Alkenyl, C₂₋₆-Alkinyl, C₁₋₆-Halogenalkyl, C₁₋₆-Alkoxy, C₁₋₆-Heteroalkyl, C₆₋₁₀-Aryloxy, C₇₋₁₆-Arylalkoxy, C₁₋₆-Alkylamino, C₂₋₁₂-Dialkylamino, -S(O)-, Heterocycloalkyl, Heteroaryl, Sulfonamidyl, Amido, Harnstoff, Sulfonylharnstoff, Acyl gegebenenfalls mit 1 bis 3 R³ substituiert ist;
jedes R³ unabhängig C₁₋₆-Alkyl, C₁₋₆-Heteroalkyl, Halogen, C₁₋₆-Halogenalkoxy, Hydroxyl, C₁₋₆-Alkoxy, Amino, C₁₋₆-Alkylamino, C₂₋₁₂-Dialkylamino, Cyano, Nitro, Amido, -S-, -S(O)₂-, -C(O)-, -C(O)O-C₁₋₆-Alkyl, -C(O)-C₁₋₆-Alkyl, C₃₋₇-Cycloalkyl, Heterocycloalkyl, Phenyl oder Naphthyl ist, wobei jedes C₁₋₆-Alkyl, C₁₋₆-Heteroalkyl, C₁₋₆-Halogenalkoxy, C₁₋₆-Alkoxy, Amino, C₁₋₆-Alkylamino, C₂₋₁₂-Dialkylamino, Amido, -S-, -S(O)₂-, -C(O)-, -C(O)O-C₁₋₆-Alkyl, -C(O)-C₁₋₆-Alkyl, C₃₋₇-Cycloalkyl, Heterocycloalkyl, Phenyl oder Naphthyl gegebenenfalls mit 1 bis 3 R⁴ substituiert ist; und
jedes R⁴ unabhängig C₁₋₆-Alkyl, C₁₋₆-Heteroalkyl, C₂₋₆-Alkenyl, C₂₋₆-Alkinyl, C₃₋₇-Cycloalkyl, C₁₋₆-Halogenalkoxy, C₁₋₆-Halogenalkyl, Heterocycloalkyl, C₆₋₁₀-Aryl, Heteroaryl, C₄₋₁₀-Cycloalkylalkyl, Heterocycloalkylalkyl, C₇₋₁₆-Arylalkyl, Heteroaryl-C₁₋₆-alkyl, Halogen, Hydroxyl, C₁₋₆-Alkoxy, C₁₋₆-Hydroxyalkyl, C₆₋₁₀-Aryloxy, C₇₋₁₆-Arylalkoxy, C₂₋₈-Alkoxyalkoxyl, Amino, C₁₋₆-Alkylamino, C₂₋₁₂-Dialkylamino, -S-, -S-C₁₋₆-Alkyl, -S(O)₂-C₁₋₆-Alkyl, Sulfonamidyl, Amido, Harnstoff, Sulfonylharnstoff, Acyl, -C(O)-C₆₋₁₀-Aryl, -NHC(O)-C₆₋₁₀-Aryl, -C(O)NH-C₆₋₁₀-Aryl, -C(O)OH, -C(O)O-C₁₋₆-Alkyl, -C(O)-C₁₋₆-Alkyl, Acyl, Nitro oder Cyano ist.

2. Verbindung gemäss Anspruch 1, worin R² C₁₋₆-Alkyl, C₂₋₆-Alkenyl, C₁₋₆-Alkoxy, C₁₋₆-Heteroalkyl, C₆₋₁₀-Aryloxy, C₇₋₁₆-Arylalkoxy, C₁₋₆-Alkylamino, C₂₋₁₂-Dialkylamino, -C(O)-, -S(O)-, -O- oder Heterocycloalkyl ist, wobei jedes C₁₋₆-Alkyl, C₂₋₆-Alkenyl, C₁₋₆-Alkoxy, C₁₋₆-Heteroalkyl, C₆₋₁₀-Aryloxy, C₇₋₁₆-Arylalkoxy, C₁₋₆-Alkylamino, C₂₋₁₂-Dialkylamino, -S(0)- oder Heterocycloalkyl gegebenenfalls mit 1 bis 3 R³ substituiert ist.

3. Verbindung gemäss Anspruch 2, worin jedes R³ unabhängig C₁₋₆-Alkyl, C₁₋₆-Heteroalkyl, Halogen, C₁₋₆-Halogenalkoxy, Hydroxyl, C₁₋₆-Alkoxy, Amino, C₁₋₆-Alkylamino, C₂₋₁₂-Dialkylamino, Cyano, Amido, -C(O)-, -C(O)O-C₁₋₆-Alkyl, -C(O)-C₁₋₆-Alkyl, Heterocycloalkyl, Phenyl oder Napthyl ist, wobei jedes C₁₋₆-Alkyl, C₁₋₆-Heteroalkyl, C₁₋₆-Halogenalkoxy, Hydroxyl, C₁₋₆-Alkoxy, Amino, C₁₋₆-Alkylamino, C₂₋₁₂-Dialkylamino, Amido, -C(O)-, -C(O)O-C₁₋₆-Alkyl, -C(O)-C₁₋₆-Alkyl, Heterocycloalkyl, Phenyl oder Napthyl gegebenenfalls mit 1 bis 3 R⁴ substituiert ist.

4. Verbindung gemäss Anspruch 3, worin jedes R⁴ unabhängig C₁₋₆-Alkyl, C₁₋₆-Heteroalkyl, C₃₋₇-Cycloalkyl, C₁₋₆-Halogenalkoxy, C₁₋₆-Halogenalkyl, Heterocycloalkyl, C₆₋₁₀-Aryl, Heteroaryl, C₄₋₁₀-Cycloalkylalkyl, Heterocycloalkylalkyl, Heteroaryl-C₁₋₆-alkyl, Halogen, Hydroxyl, C₁₋₆-Alkoxy, C₁₋₆-Hydroxyalkyl, Amino, C₁₋₆-Alkylamino, C₂₋₁₂-Dialkylamino, Amido, -C(O)O-C₁₋₆-Alkyl, -C(O)-C₁₋₆-Alkyl oder Cyano ist.

5. Verbindung gemäss Anspruch 1, worin R² ausgewählt ist aus der Gruppe bestehend aus: 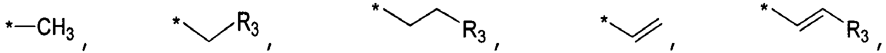 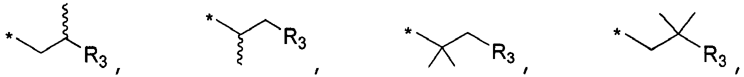 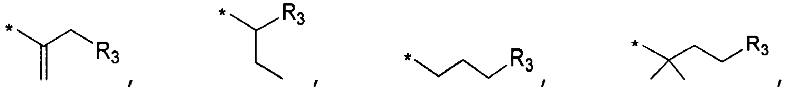 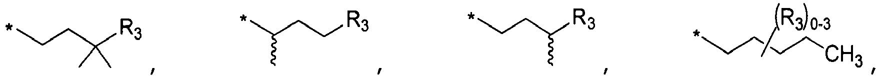 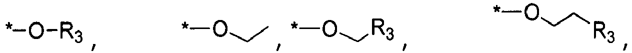 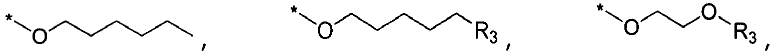 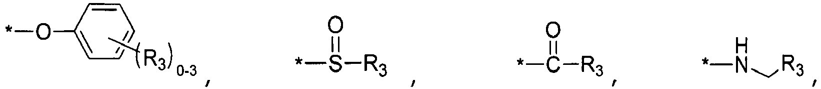 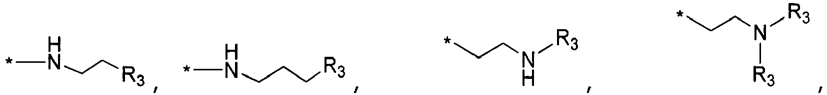 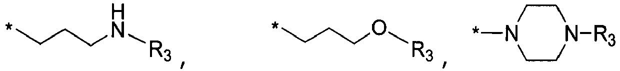 und

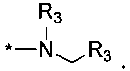

6. Verbindung gemäss Anspruch 1, worin R³ unabhängig ausgewählt ist aus der Gruppe bestehend aus:

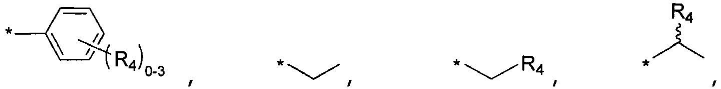

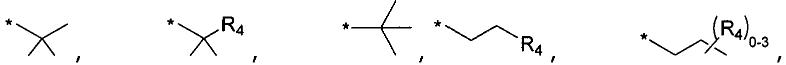

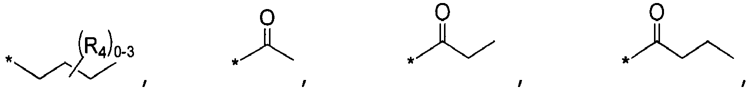

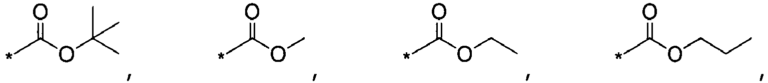

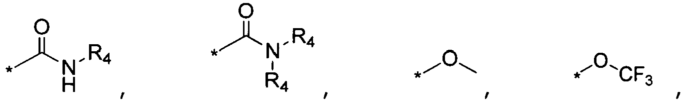

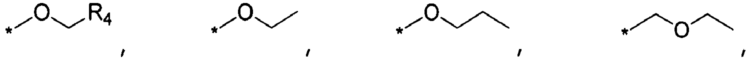

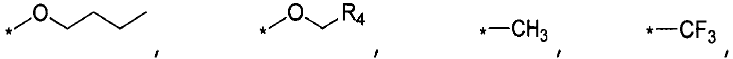

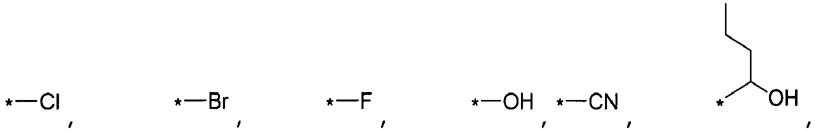

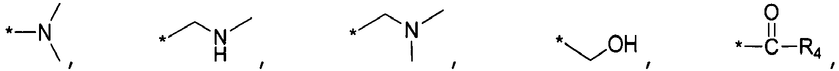

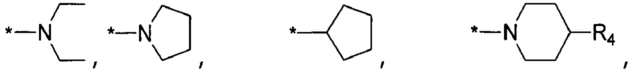

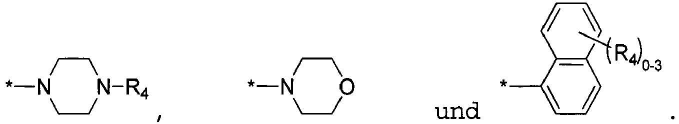

7. Verbindung gemäss Anspruch 1, worin R⁴ unabhängig ausgewählt ist aus der Gruppe bestehend aus:

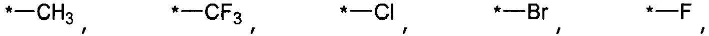

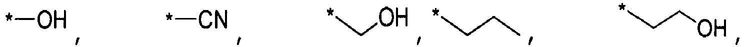

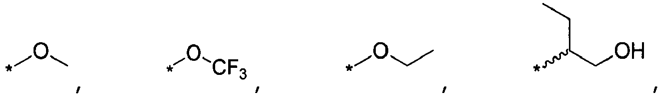

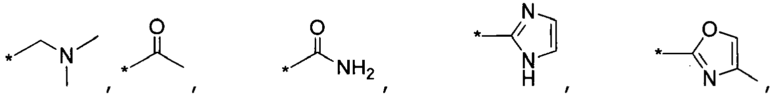

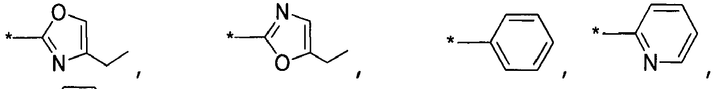

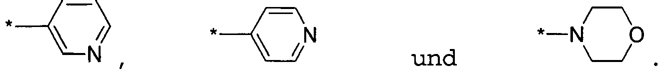

8. Verbindung gemäss Anspruch 1, worin
R² 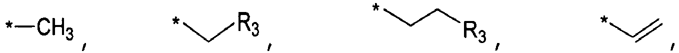 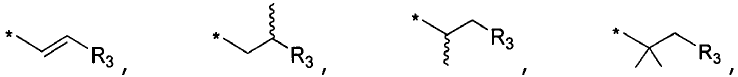 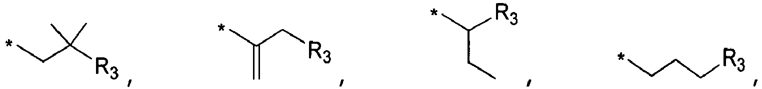 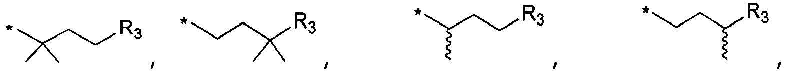 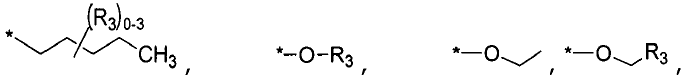 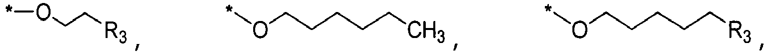 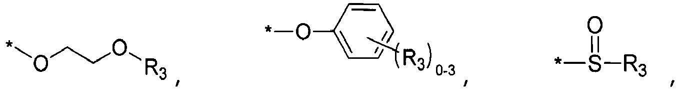 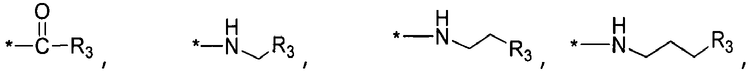 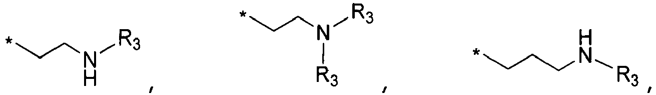 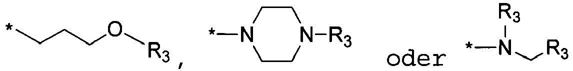 ist;
jedes R³ unabhängig 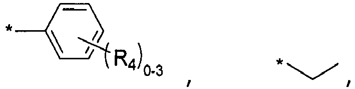 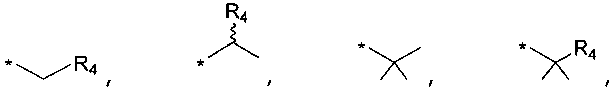 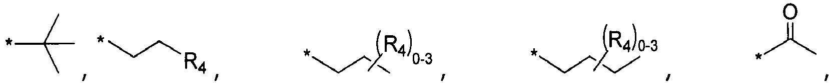 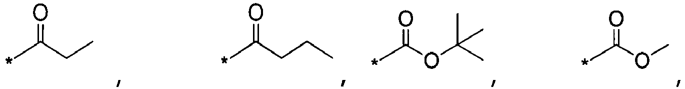 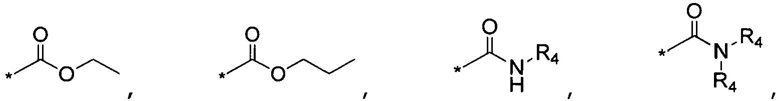 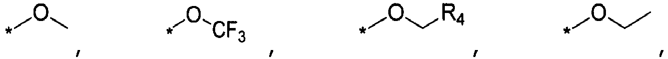 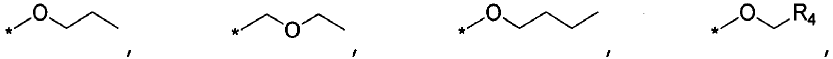 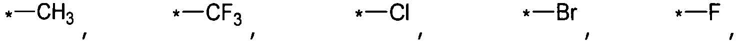 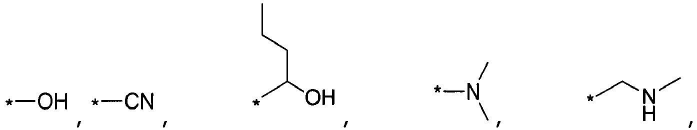 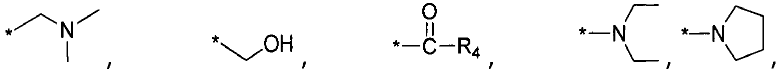 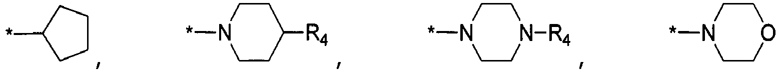 oder 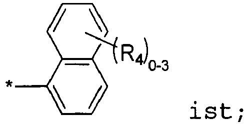 und
jedes R⁴ unabhängig 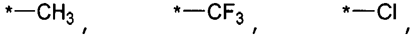 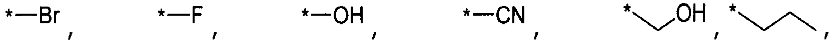 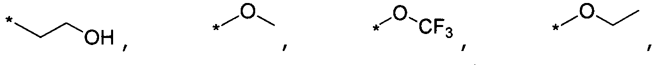 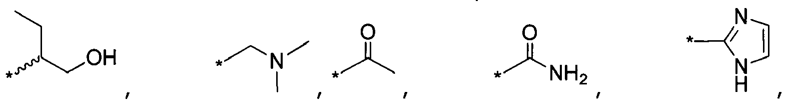 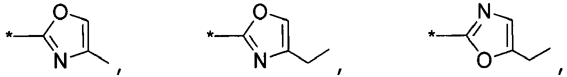 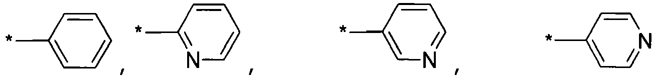 oder 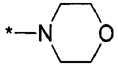 ist.

9. Verbindung, ausgewählt aus der Gruppe bestehend aus: 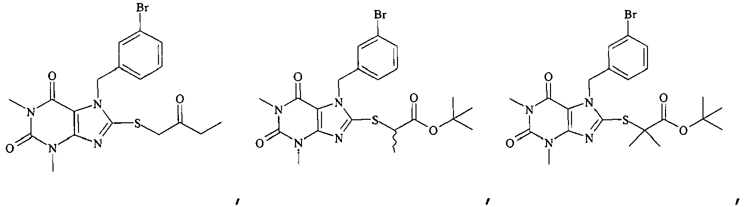 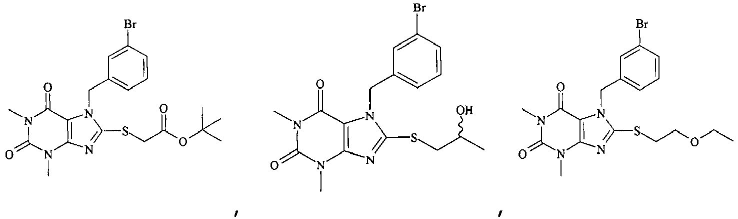 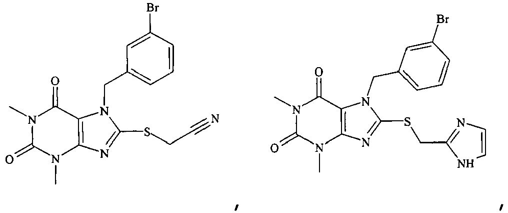 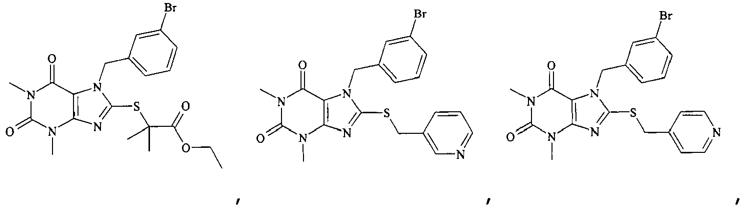 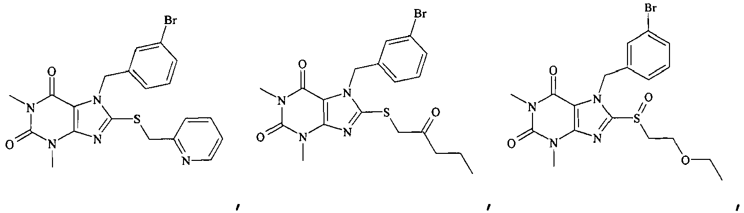 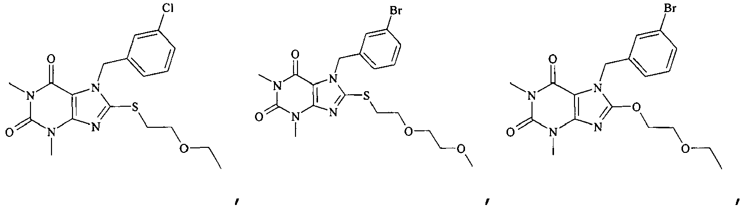 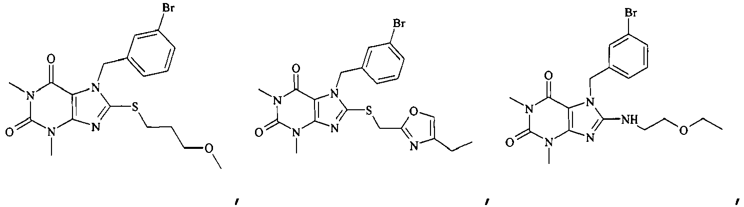 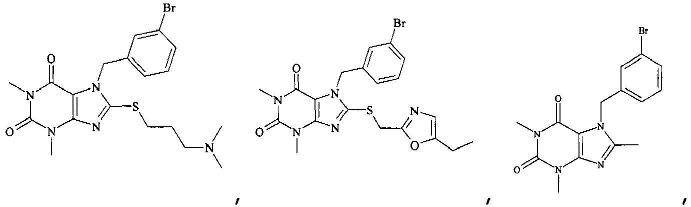 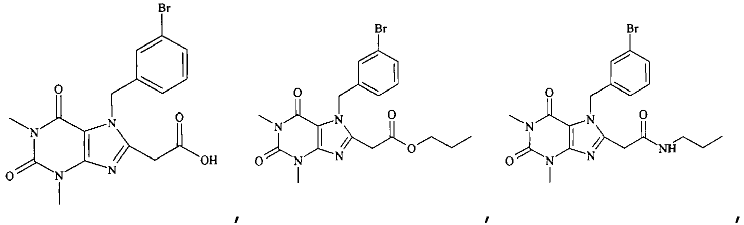 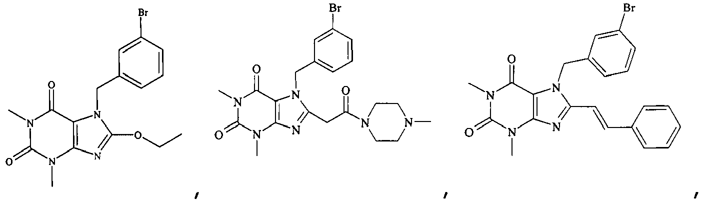 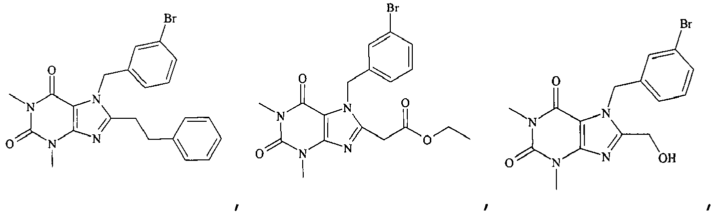 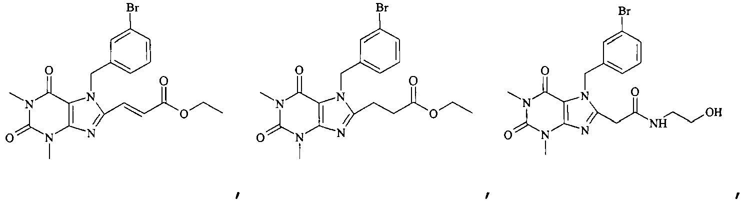 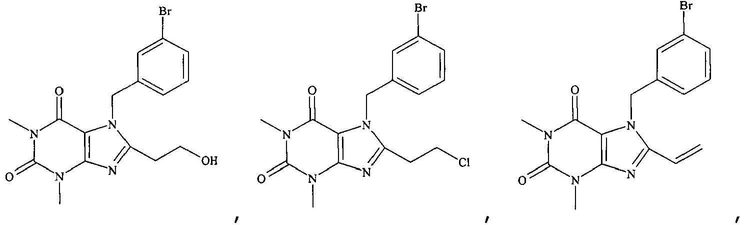 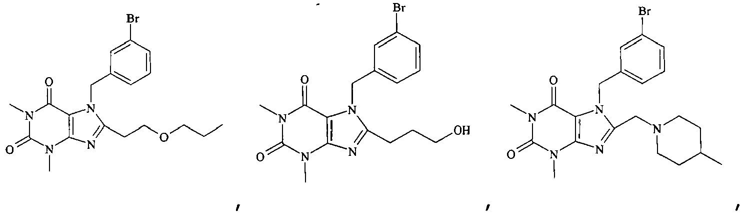 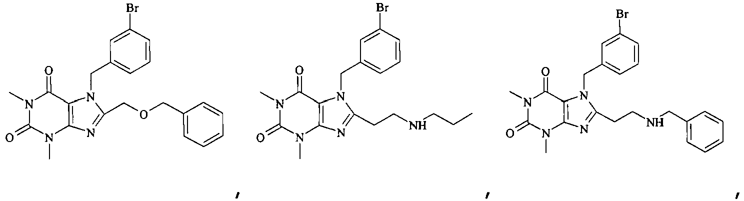 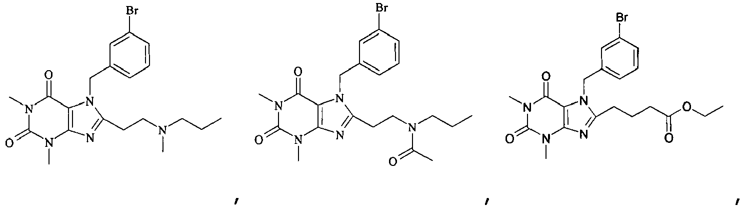 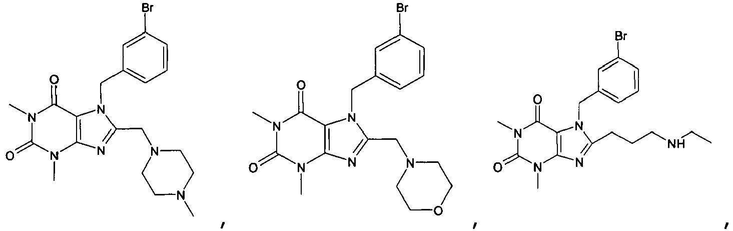 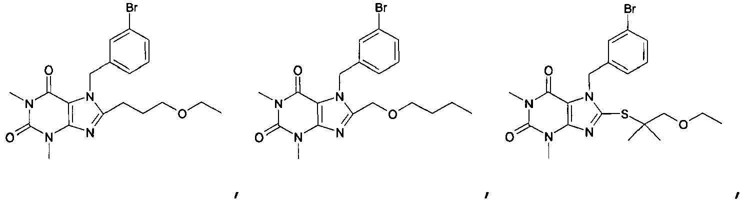 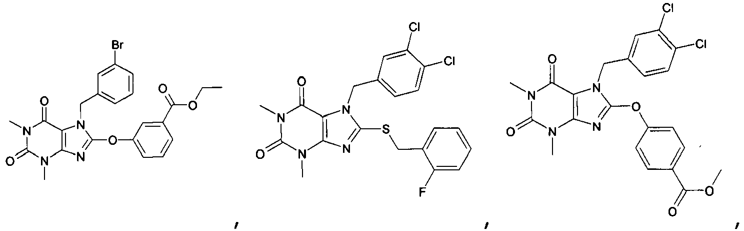 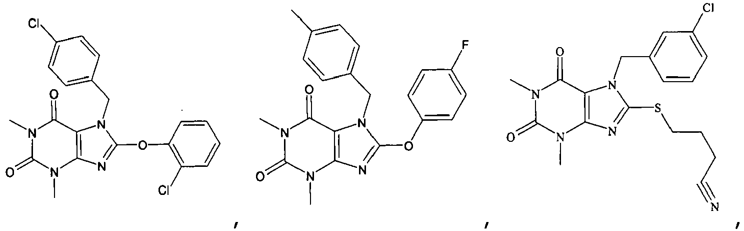 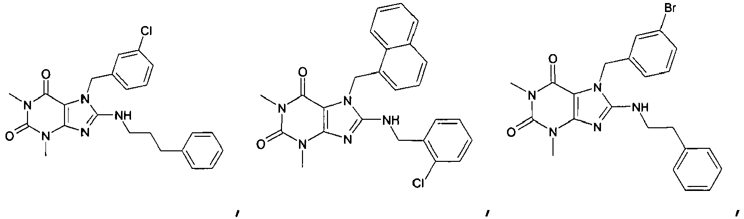 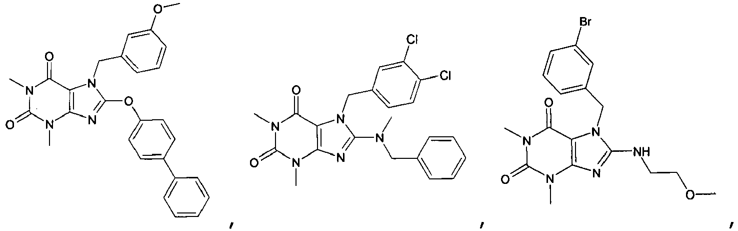 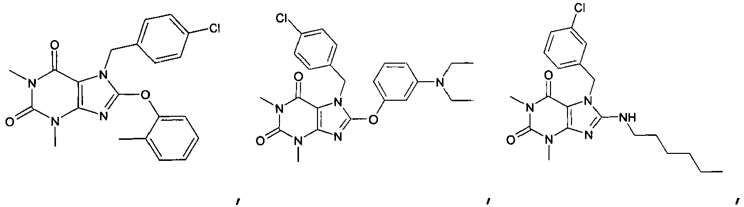 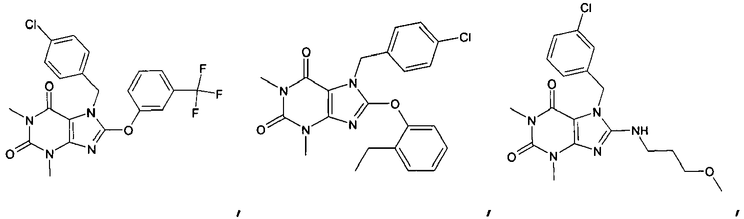 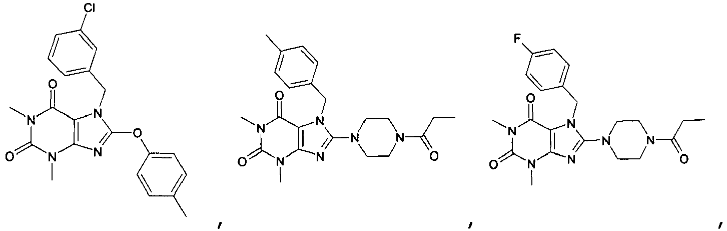 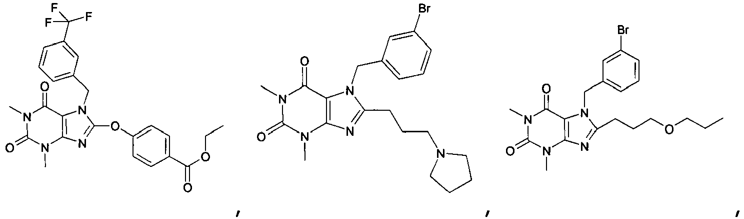 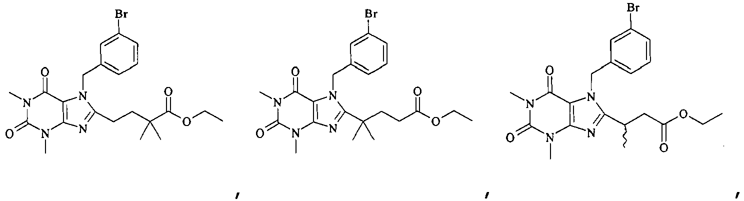 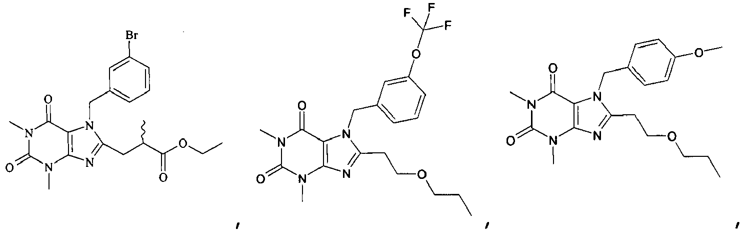 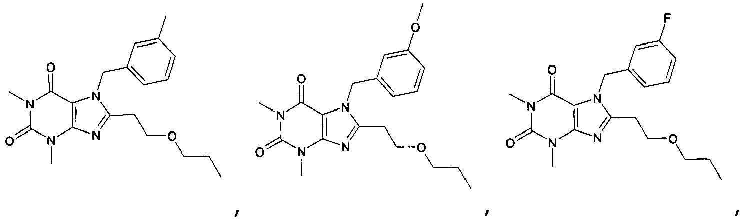 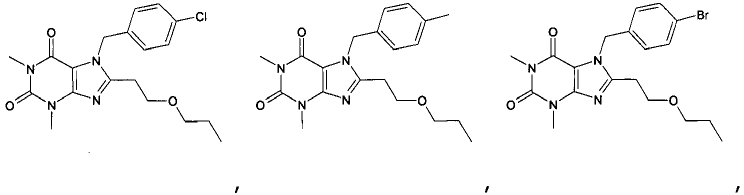 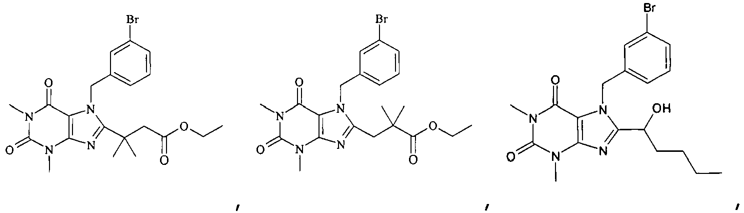 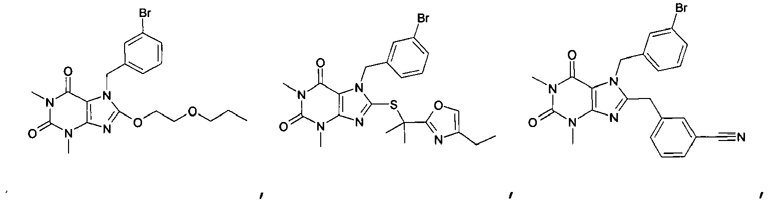 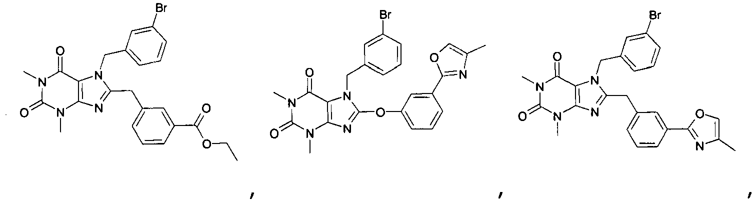 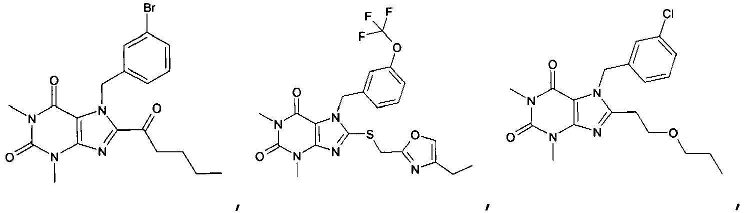 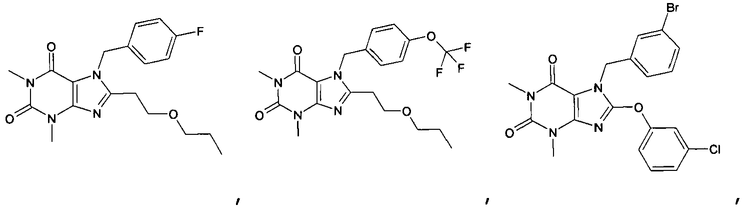 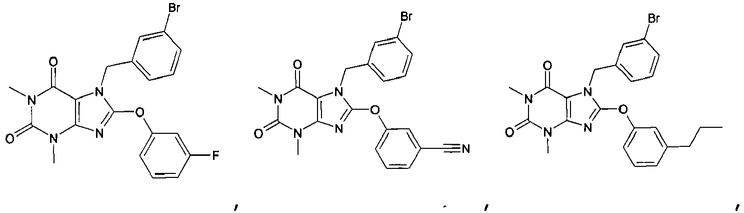 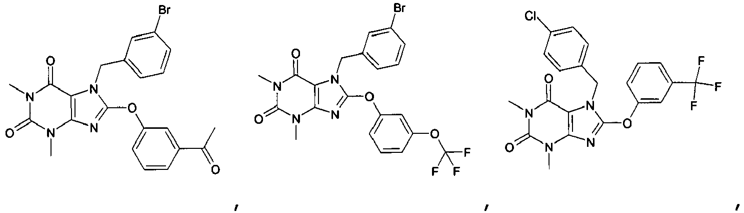 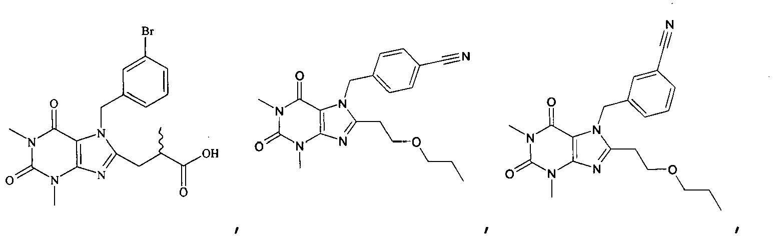 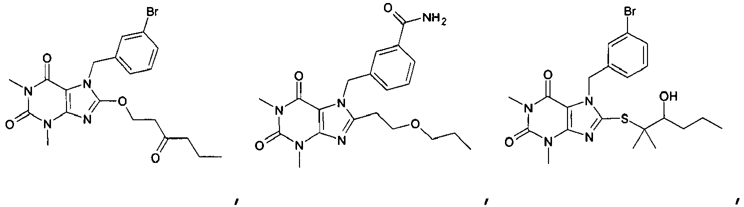 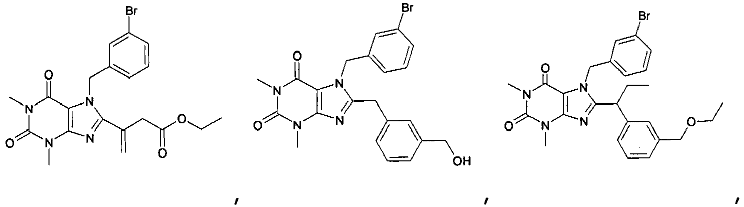 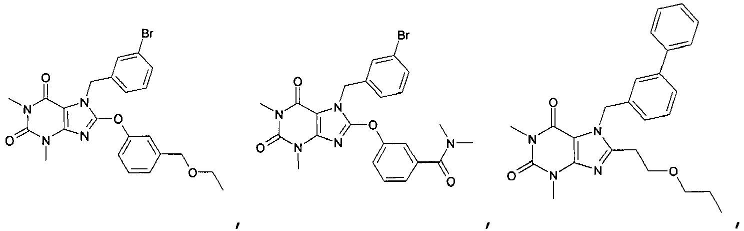 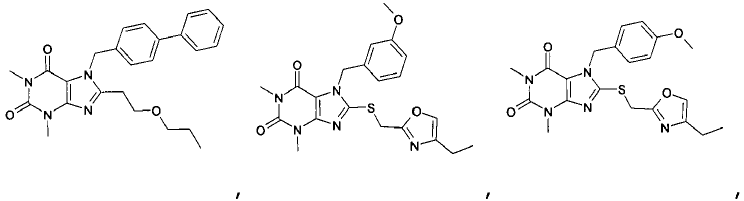 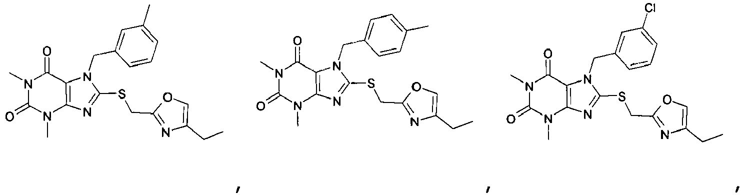 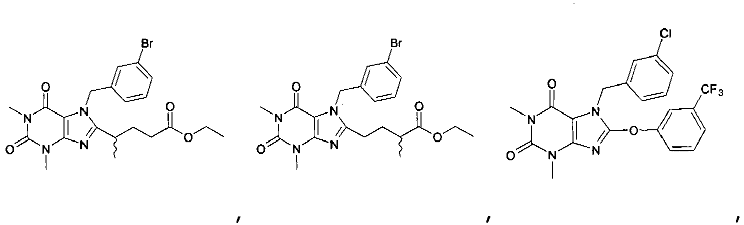 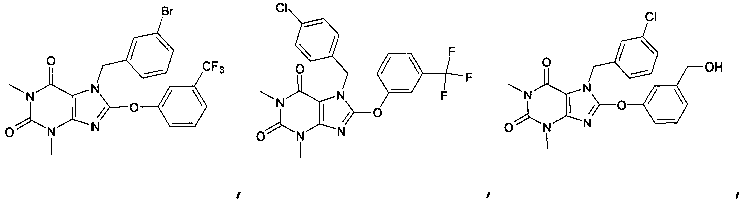 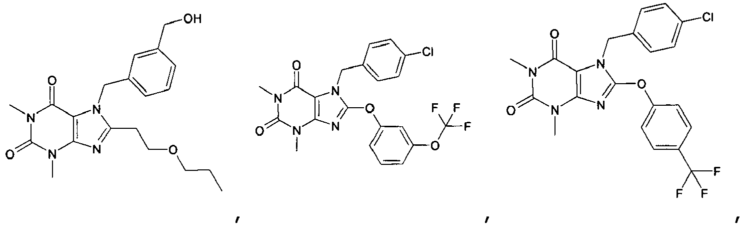 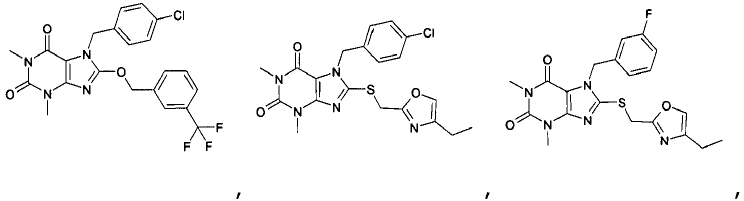 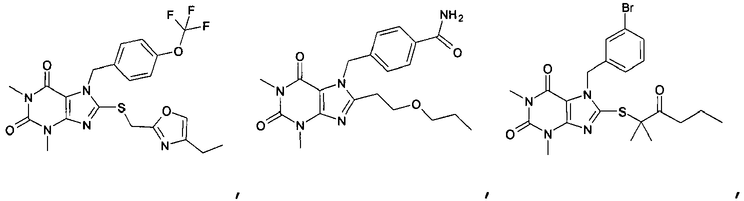 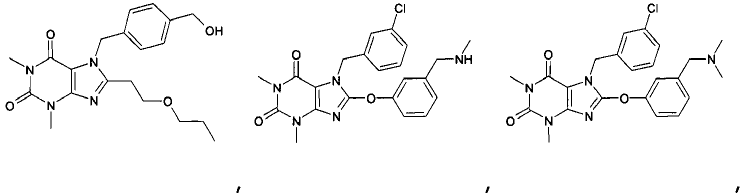 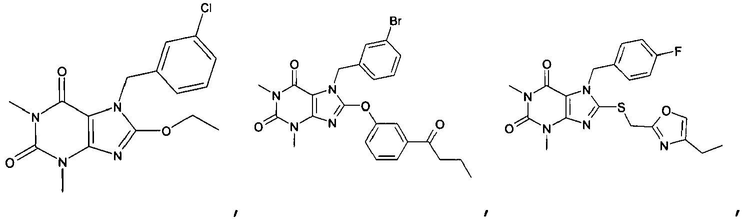 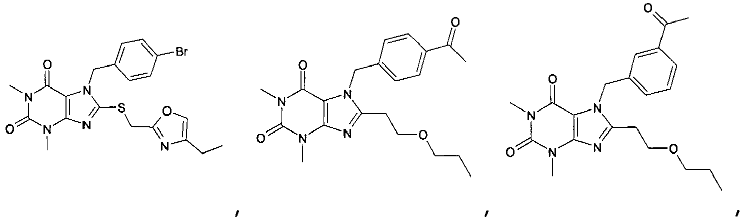 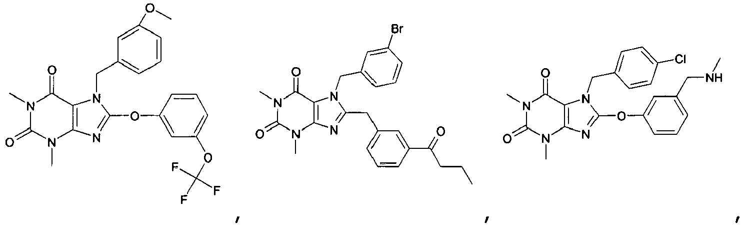 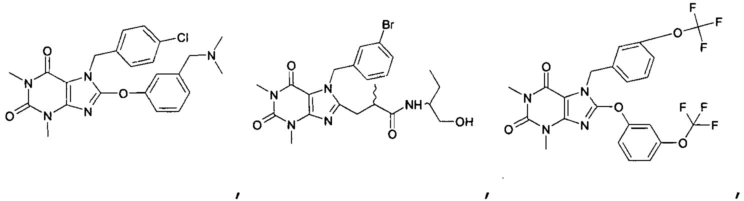 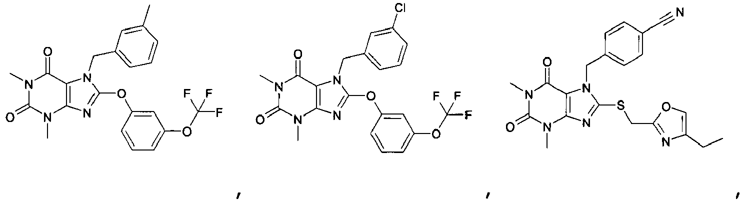 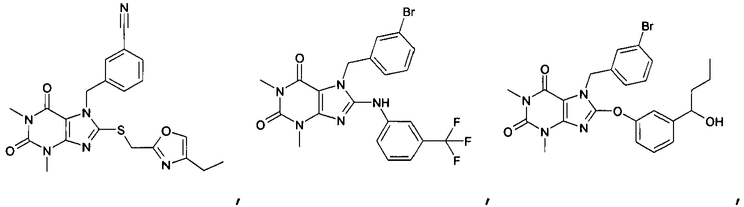 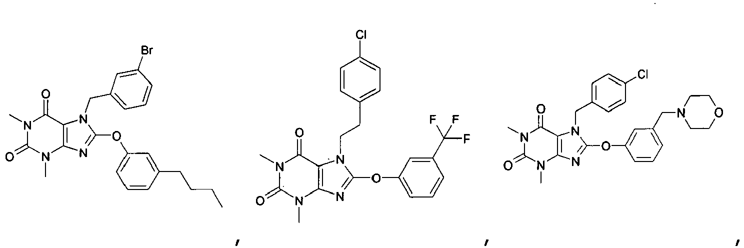 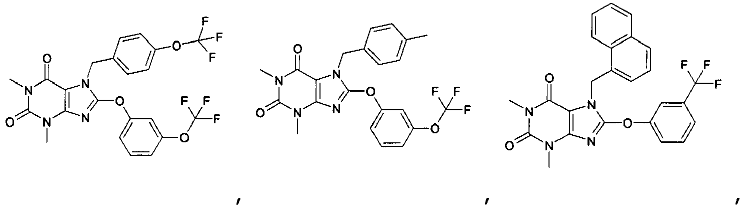 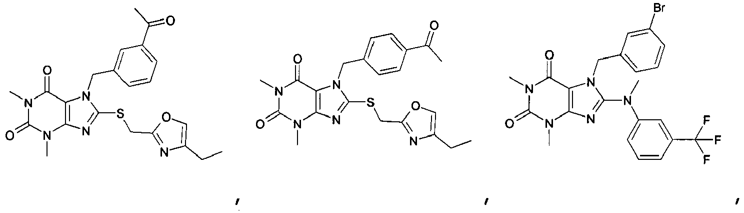 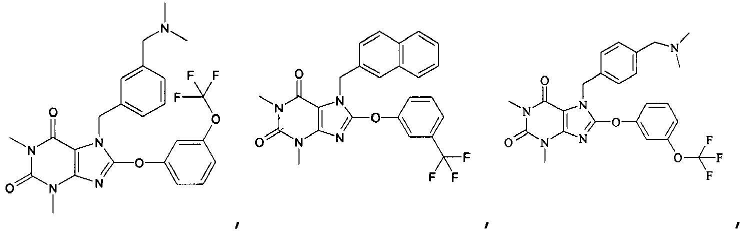 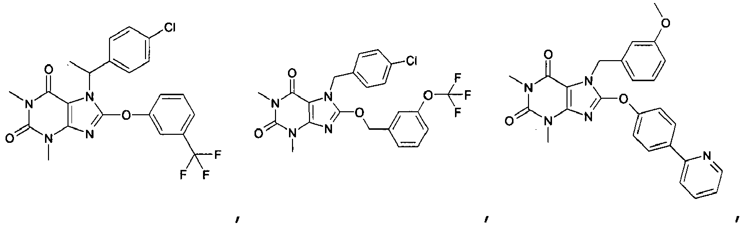 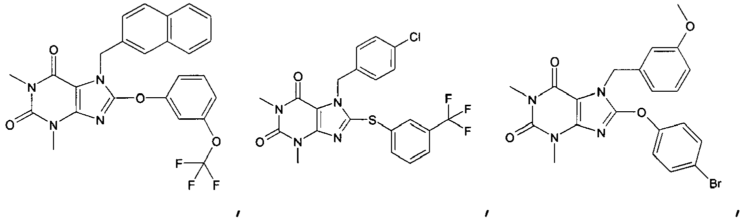 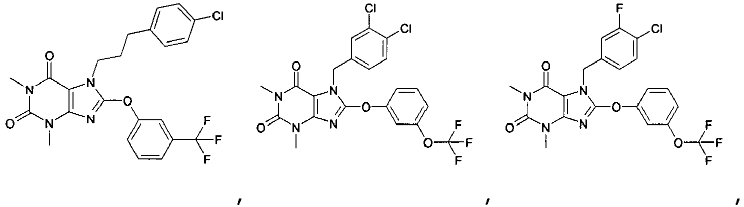 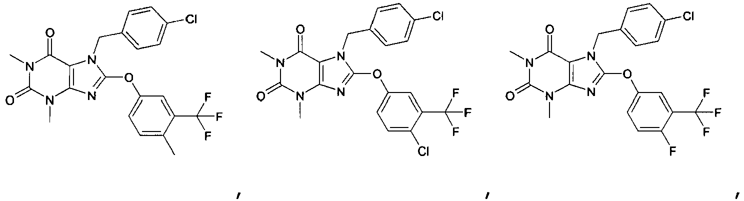 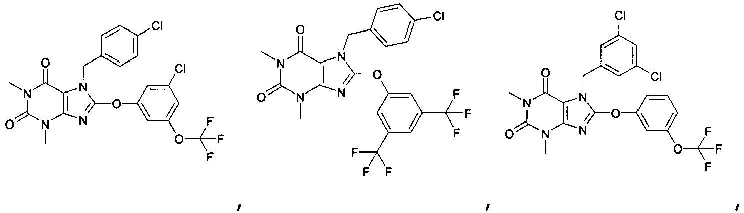 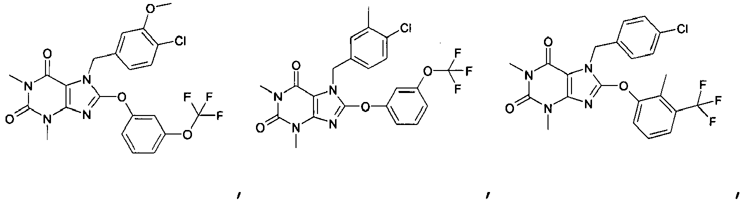 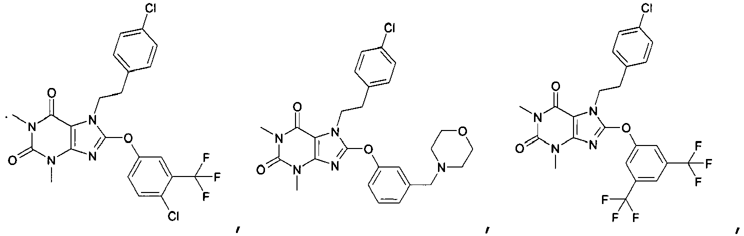 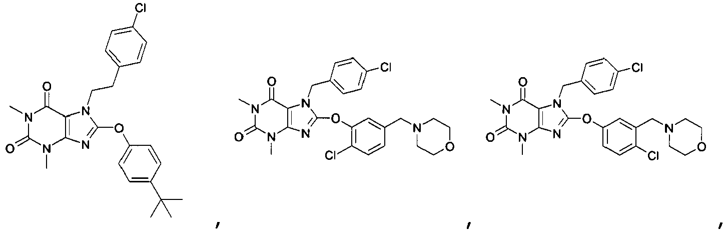 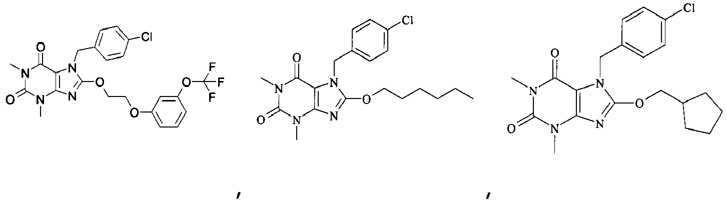 und

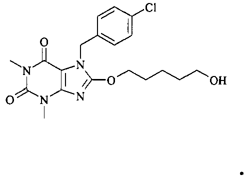

10. Verbindung der Formel (II): 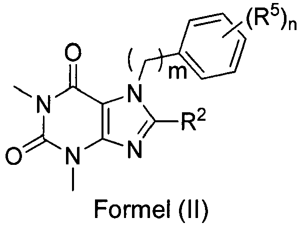 oder ein pharmazeutisch annehmbares Salz davon, worin:
R² C₁₋₆-Alkoxy, C₁₋₆-Heteroalkyl, C₆₋₁₀-Aryloxy, C₇₋₁₆-Arylalkoxy oder -C(O)-C₁₋₆-Alkyl ist, von denen jedes unabhängig mit 1 bis 3 R³ substituiert ist;
jedes R³ unabhängig Halogen, C₁₋₆-Alkyl, C₁₋₆-Halogenalkyl, C₁₋₆-Halogenalkoxy, C₁₋₆-Heteroalkyl, Hydroxyl, -C(O)O-C₁₋₆-Alkyl, -C(O)-C₁₋₆-Alkyl, Phenyl oder Heteroaryl ist, wobei jedes C₁₋₆-Alkyl, C₁₋₆-Halogenalkyl, C₁₋₆-Halogenalkoxy, C₁₋₆-Heteroalkyl, -C(O)O-C₁₋₆-Alkyl, -C(O)-C₁₋₆-Alkyl, Phenyl oder Heteroaryl gegebenenfalls mit 1 bis 3 R⁴ substituiert ist;
R⁴ und R⁵ jeweils unabhängig Hydroxyl, C₁₋₆-Alkyl, C₁₋₆-Alkoxy, C₁₋₆-Heteroalkyl, C₁₋₆-Halogenalkyl, C₁₋₆-Halogenalkoxy oder Halogen sind;
n 1 oder 2 ist; und
m 1, 2 oder 3 ist.

11. Pharmazeutische Zusammensetzung, umfassend eine Verbindung gemäss einem der vorhergehenden Ansprüche, oder ein pharmazeutisch annehmbares Salz davon.

12. Verbindung oder eine pharmazeutische Zusammensetzung gemäss einem der vorhergehenden Ansprüche zur Verwendung in einem Verfahren zur Behandlung einer durch TRPC5 vermittelten Störung, wobei die durch TRPC5 vermittelte Störung aus der Gruppe bestehend aus einer neuropsychiatrischen Störung, neurodegenerativen Störung, Nephropathie und einem Anfallsleiden ausgewählt ist.

## Revendications

1. Composé de Formule I : 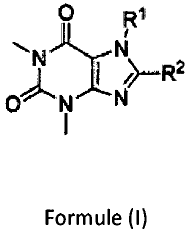 ou un sel pharmaceutiquement acceptable de celui-ci, dans lequel :
R¹ est 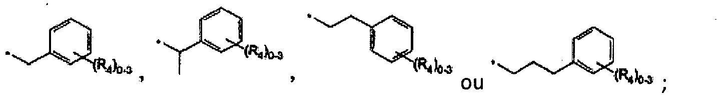
R² est un alkyle en C₁ à C₆, un alcényle en C₂ à C₆, un alcynyle en C₂ à C₆, un halogénoalkyle en C₁ à C₆, un hydroxyle, un alcoxy en C₁ à C₆, un hétéroalkyle en C₁ à C₆, un aryloxy en C₆ à C₁₀, un arylalcoxy en C₂ à C₁₆, un alkylamino en C₁ à C₆, un dialkylamino en C₂ à C₁₂, un -C(O)-, un -S(O)-, un -O-, un hétérocycloalkyle, un hétéroaryle, un sulfonamidyle, un amido, une urée, une sulfonylurée, un acyle, un nitro, un cyano, dans lequel chacun de l'alkyle en C₁ à C₆, de l'alcényle en C₂ à C₆, de l'alcynyle en C₂ à C₆, de l'halogénoalkyle en C₁ à C₆, de l'alcoxy en C₁ à C₆, de l'hétéroalkyle en C₁ à C₆, de l'aryloxy en C₆ à C₁₀, de l'arylalcoxy en C₇ à C₁₆, de l'alkylamino en C₁ à C₆, du dialkylamino en C₂ à C₁₂, du -S(O)-, de l'hétérocycloalkyle, de l'hétéroaryle, du sulfonamidyle, de l'amido, de l'urée, de la sulfonylurée, de l'acyle, est facultativement substitué par 1 à 3 R³ ;
chaque R³ est indépendamment un alkyle en C₁ à C₆, un hétéroalkyle en C₁ à C₆, un halogéno, un halogénoalcoxy en C₁ à C₆, un hydroxyle, un alcoxy en C₁ à C₆, un amino, un alkylamino en C₁ à C₆, un dialkylamino en C₂ à C₁₂, un cyano, un nitro, un amido, un -S-, un -S(O)₂-, un -C(O)-, un -C(O)O-alkyle en C₁ à C₆, un -C(O)-alkyle en C₁ à C₆, un cycloalkyle en C₃ à C₇, un hétérocycloalkyle, un phényle ou un naphtyle, dans lequel chacun parmi l'alkyle en C₁ à C₆, l'hétéroalkyle en C₁ à C₆, l'halogénoalcoxy en C₁ à C₆, l'alcoxy en C₁ à C₆, l'amino, l'alkylamino en C₂ à C₆,le dialkylamino en C₂ à C₁₂, l'amido, le -S-, le -S(O)₂-, le -C(O)-, le -C(O)O-alkyle en C₁ à C₆, le -C(O)-alkyle en C₁ à C₆, le cycloalkyle en C₃ à C₇, l'hétérocycloalkyle, le phényle ou le naphtyle est facultativement substitué par 1 à 3 R⁴ ; et
chaque R⁴ est indépendamment un alkyle en C₁ à C₆, un hétéroalkyle en C₁ à C₆, un alcényle en C₂ à C₆, un alcynyle en C₂ à C₆, un cycloalkyle en C₃ à C₇, un halogénoalcoxy en C₁ à C₆, un halogénoalkyle en C₁ à C₆, un hétérocycloalkyle, un aryle en C₆ à C₁₀, un hétéroaryle, un cycloalkylalkyle en C₂ à C₁₀, un hétérocycloalkylalkyle, un arylalkyle en C₇ à C₁₆, un hétéroaryl-alkyle en C₁ à C₆, un halogéno, un hydroxyle, un alcoxy en C₁ à C₆, un hydroxyalkyle en C₁ à C₆, un aryloxy en C₆ à C₁₀, un arylalcoxy en C₇ à C₁₆, un alcoxyalcoxyle en C₂ à C₈, un amino, un alkylamino en C₁ à C₆, un dialkylamino en C₂ à C₁₂, un -S-, un -S-alkyle en C₁ à C₆, un -S(O)₂-alkyle en C₁ à C₆, un sulfonamidyle, un amido, une urée, une sulfonylurée, un acyle, un -C(O)-aryle en C₆ à C₁₀, un -NHC(O)-aryle en C₆ à C₁₀, un -C(O)NH-aryle en C₆ à C₁₀, un -C(O)OH, un -C(O)O-alkyle en C₁ à C₆, un -C(O)-alkyle en C₁ à C₆, un acyle, un nitro ou un cyano.

2. Composé selon la revendication 1, dans lequel R² est un alkyle en C₁ à C₆, un alcényle en C₂ à C₆, un alcoxy en C₁ à C₆, un hétéroalkyle en C₂ à C₆, un aryloxy en C₆ à C₁₀, un arylalcoxy en C₇ à C₁₆, un alkylamino en C₁ à C₆, un dialkylamino en C₂ à C₁₂, un -C(O)-, un -S(O)-, un -O- ou un hétérocycloalkyle, dans lequel chacun parmi l'alkyle en C₂ à C₆, l'alcényle en C₂ à C₆, l'alcoxy en C₁ à C₆, l'hétéroalkyle en C₁ à C₆, l'aryloxy en C₆ à C₁₀, l'arylalcoxy en C₇ à C₁₆, l'alkylamino en C₁ à C₆, le dialkylamino en C₂ à C₁₂, le -S(O)-ou l'hétérocycloalkyle est facultativement substitué par 1 à 3 R³.

3. Composé selon la revendication 2, dans lequel chaque R³ est indépendamment un alkyle en C₁ à C₆, un hétéroalkyle en C₁ à C₆, un halogéno, un halogénoalcoxy en C₁ à C₆, un hydroxyle, un alcoxy en C₁ à C₆, un amino, un alkylamino en C₁ à C₆, un dialkylamino en C₂ à C₁₂, un cyano, un amido, un -C(O)-, un -C(O)O-alkyle en C₁ à C₆, un -C(O)-alkyle en C₁ à C₆, un hétérocycloalkyle, un phényle ou un napthyle, dans lequel chacun parmi l'alkyle en C₁ à C₆, l'hétéroalkyle en C₁ à C₆, l'halogénoalcoxy en C₁ à C₆, l'hydroxyle, l'alcoxy en C₁ à C₆, l'amino, l'alkylamino en C₁ à C₆, le dialkylamino en C₂ à C₁₂, l'amido, le -C(O)-, le -C(O)O-alkyle en C₁ à C₆, le -C(O)-alkyle en C₁ à C₆, l'hétérocycloalkyle, le phényle ou le napthyle est facultativement substitué par 1 à 3 R⁴.

4. Composé selon la revendication 3, dans lequel chaque R⁴ est indépendamment un alkyle en C₂ à C₆, un hétéroalkyle en C₁ à C₆, un cycloalkyle en C₃ à C₇, un halogénoalcoxy en C₂ à C₆, un halogénoalkyle en C₁ à C₆, un hétérocycloalkyle, un aryle en C₆ à C₁₀, un hétéroaryle, un cycloalkylalkyle en C₄ à C₁₀, un hétérocycloalkylalkyle, un hétéroaryl-alkyle en C₂ à C₆, un halogéno, un hydroxyle, un alcoxy en C₁ à C₆, un hydroxyalkyle en C₁ à C₆, un amino, un alkylamino en C₁ à C₆, un dialkylamino en C₂ à C₁₂, un amido, un -C(O)O-alkyle en C₁ à C₆, un -C(O)-alkyle en C₁ à C₆ ou un cyano.

5. Composé selon la revendication 1, dans lequel R² est choisi dans le groupe constitué de :

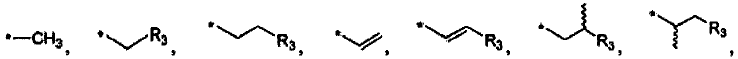

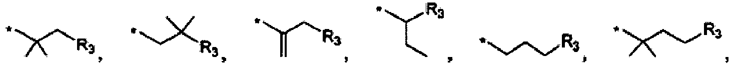

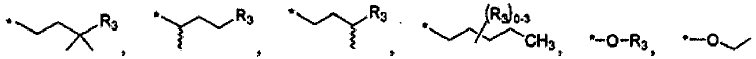

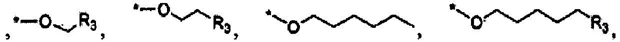

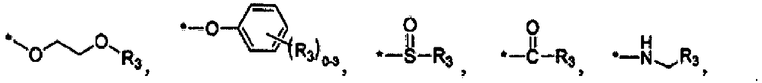

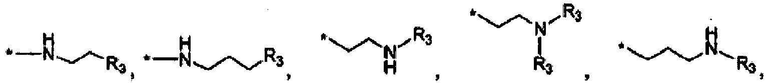

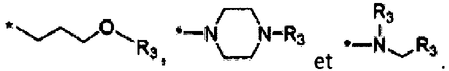

6. Composé selon la revendication 1, dans lequel chaque R³ est indépendamment choisi dans le groupe constitué de : 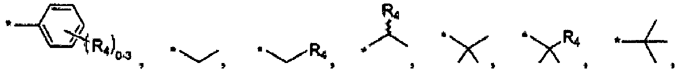 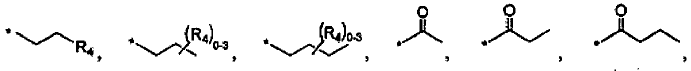 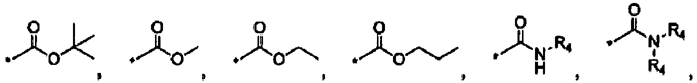 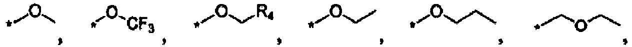 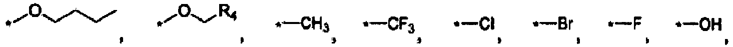 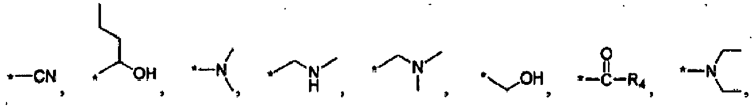 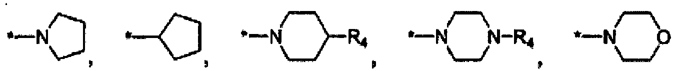 et

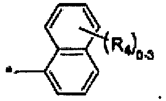

7. Composé selon la revendication 1, dans lequel chaque R⁴ est indépendamment choisi dans le groupe constitué de :

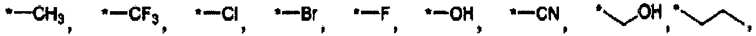

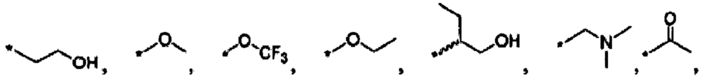

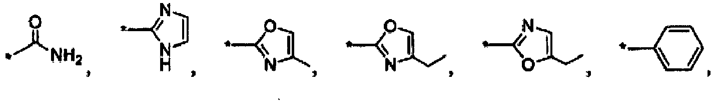

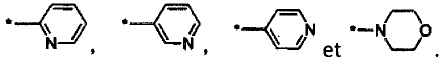

8. Composé selon la revendication 1, dans lequel :
R² est 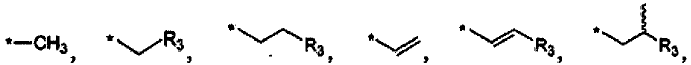 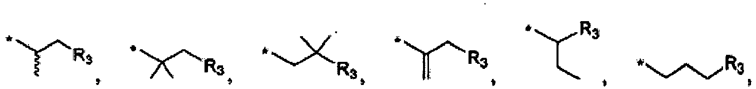 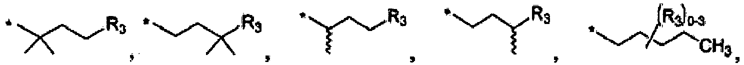 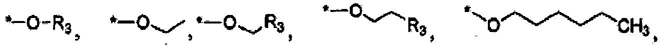 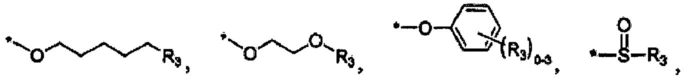 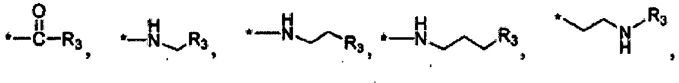 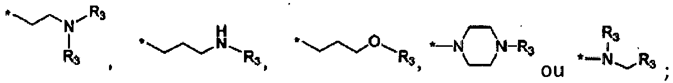 chaque R³ est indépendamment 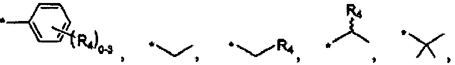 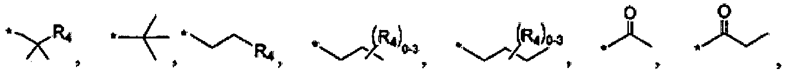 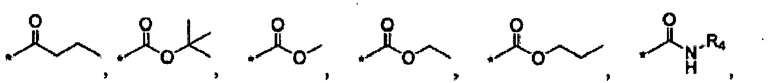 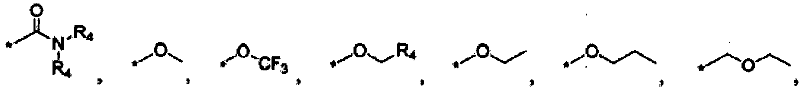 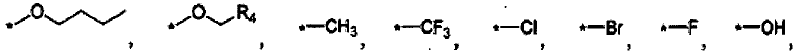 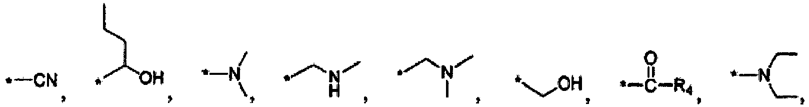 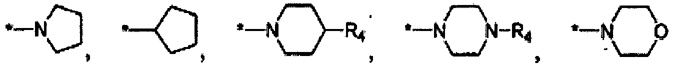 ou 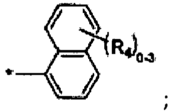 et
chaque R⁴ est indépendamment

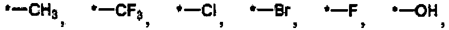

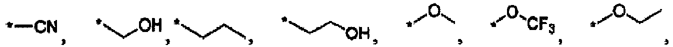

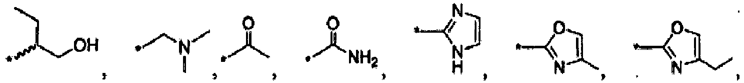

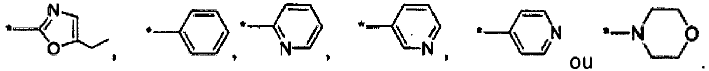

9. Composé choisi dans le groupe constitué de :

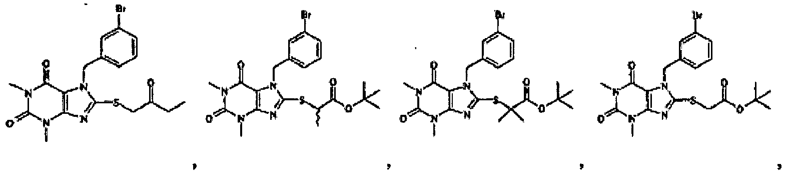

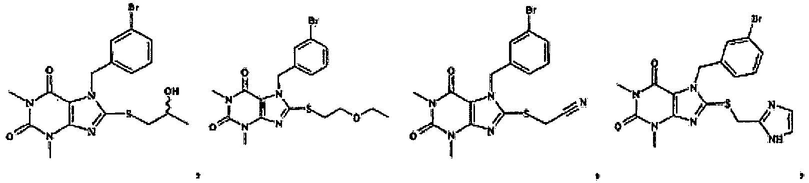

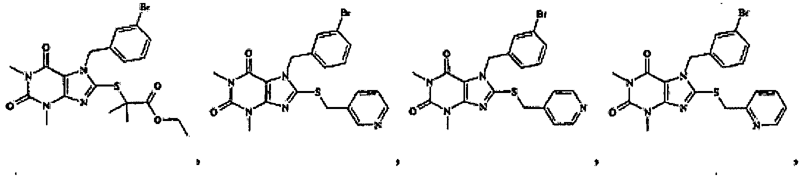

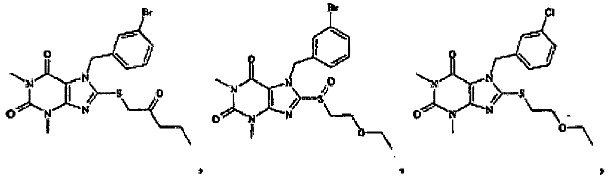

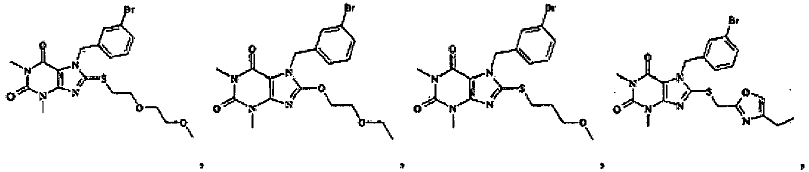

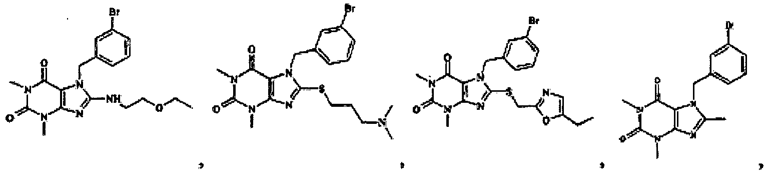

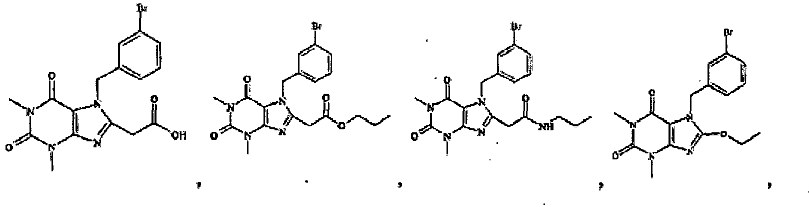

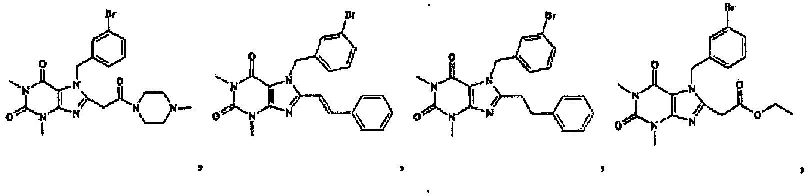

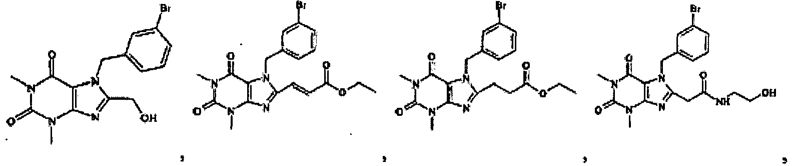

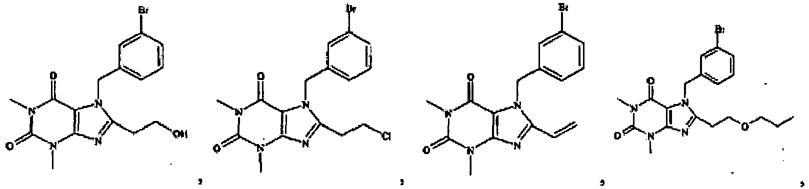

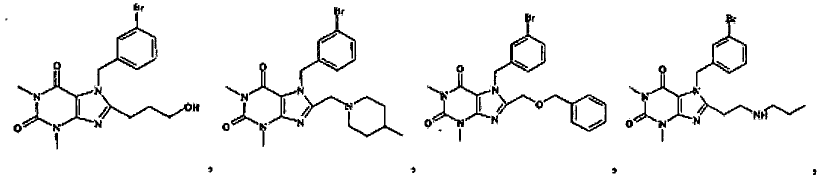

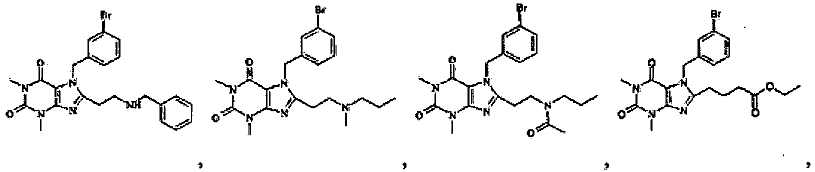

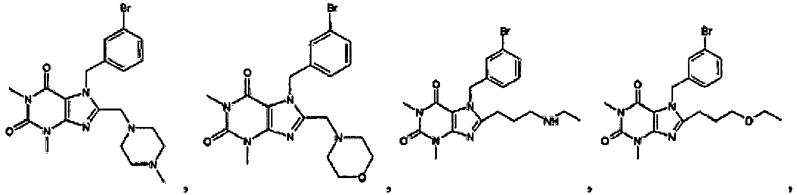

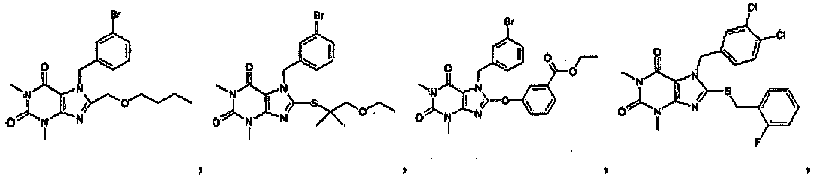

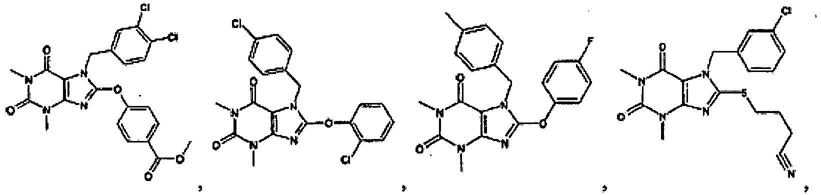

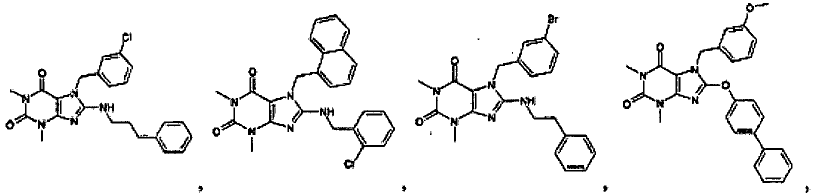

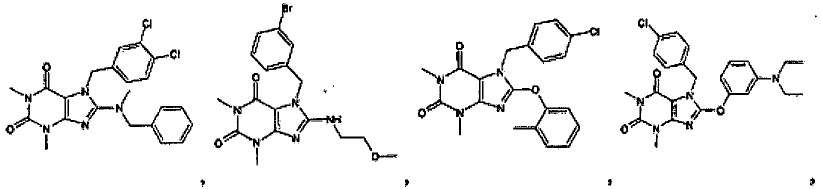

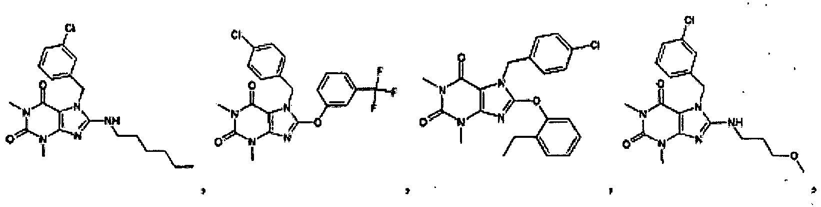

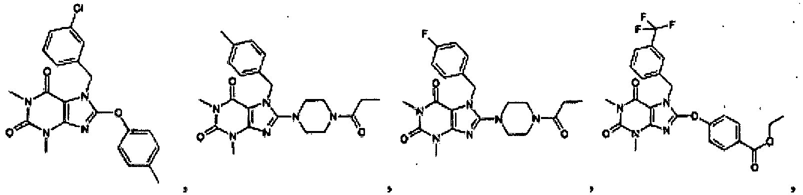

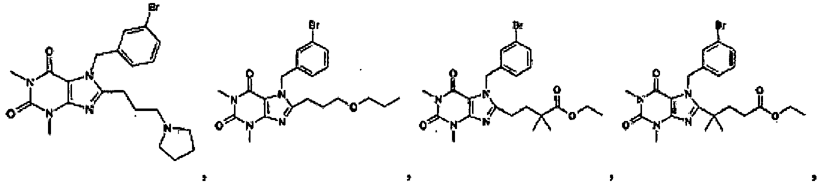

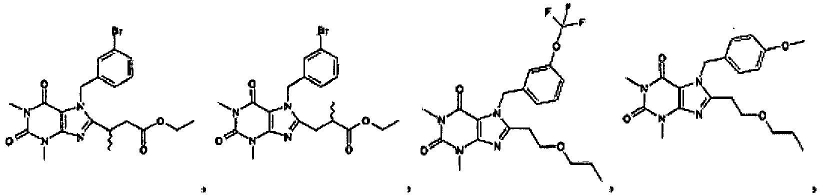

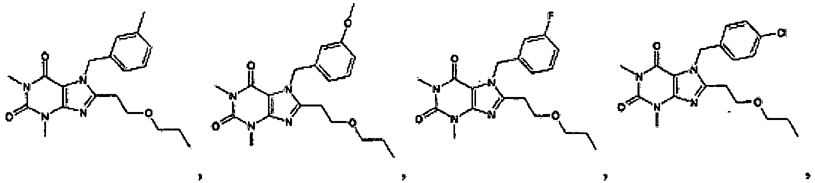

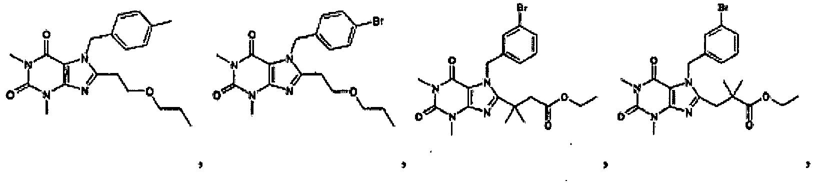

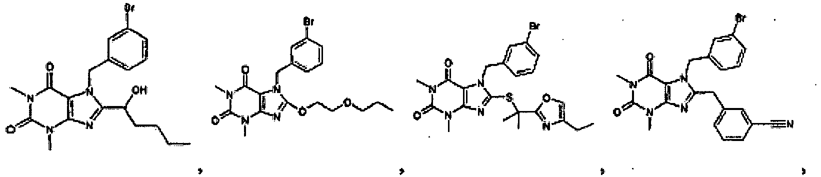

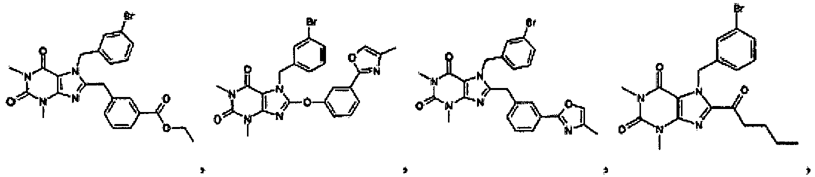

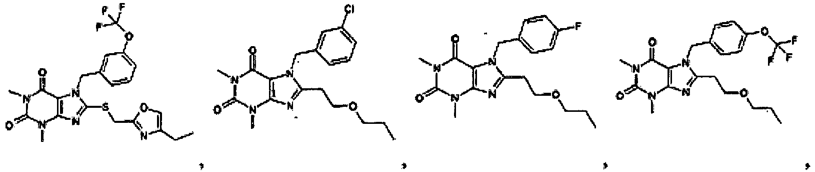

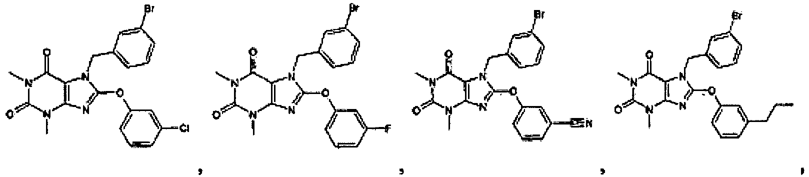

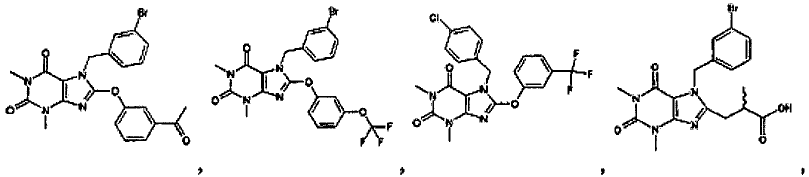

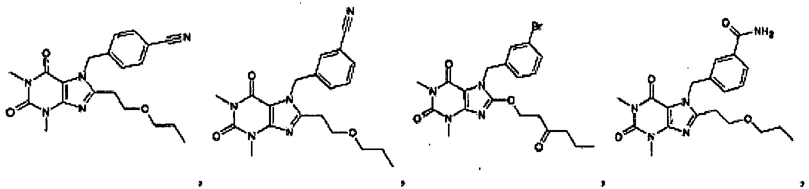

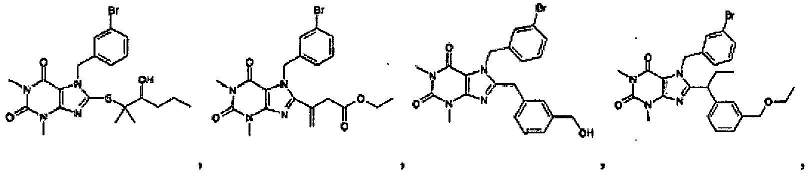

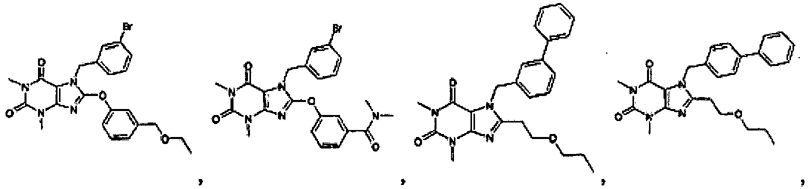

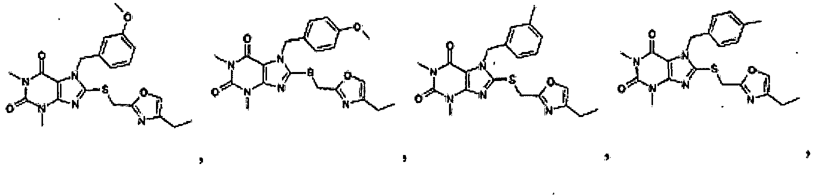

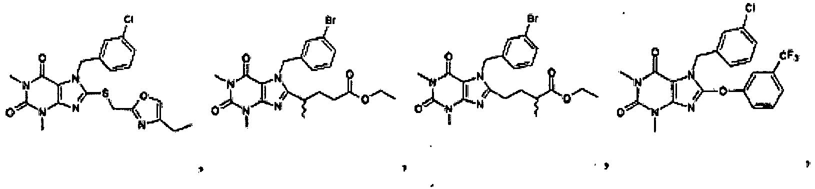

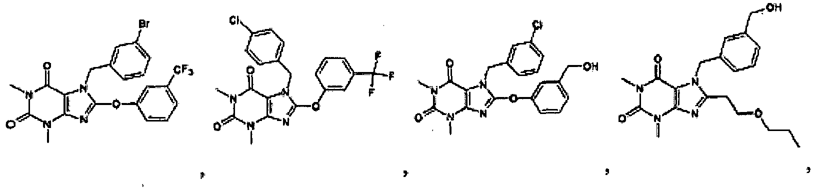

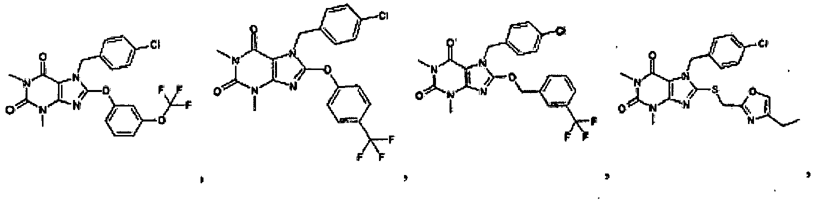

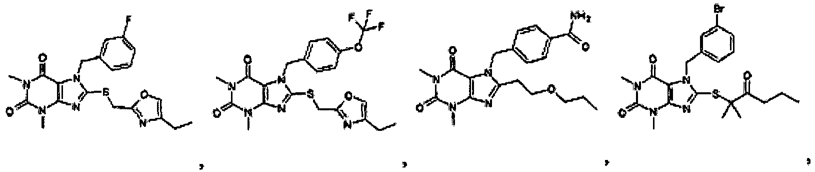

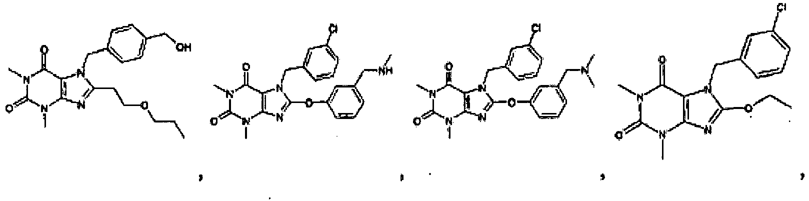

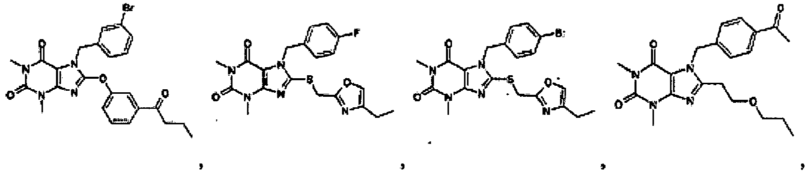

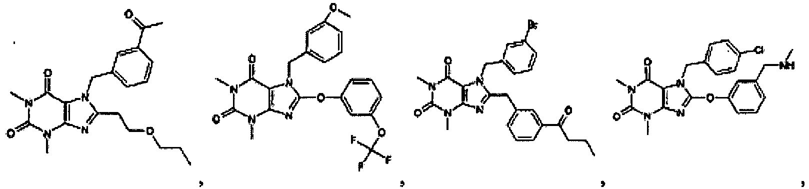

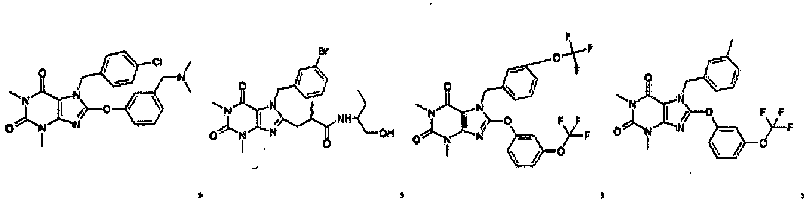

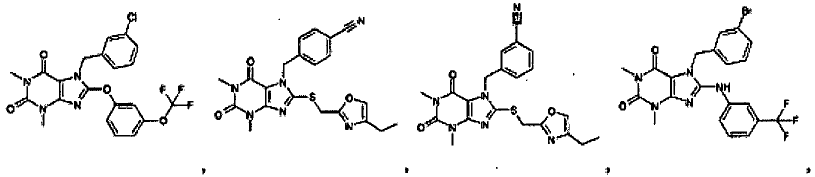

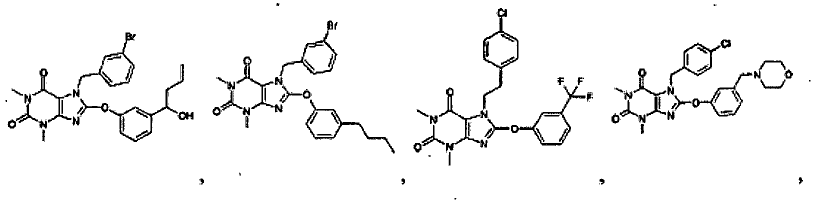

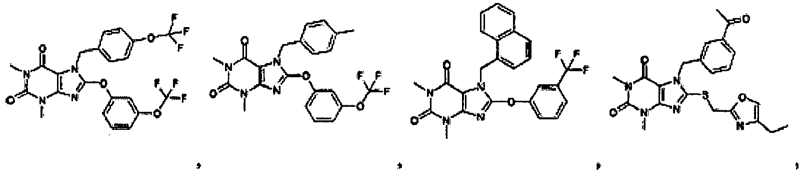

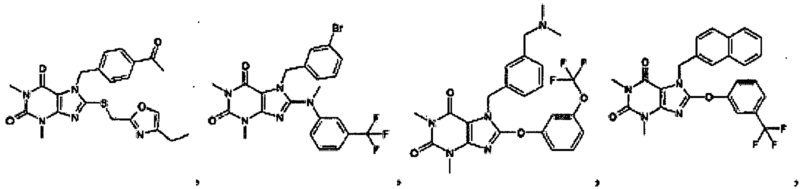

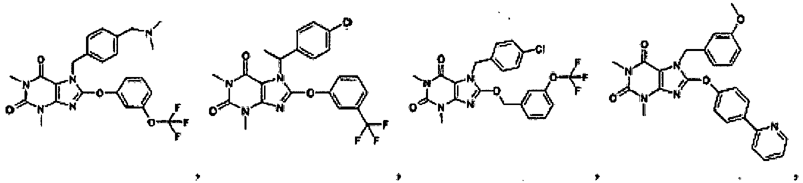

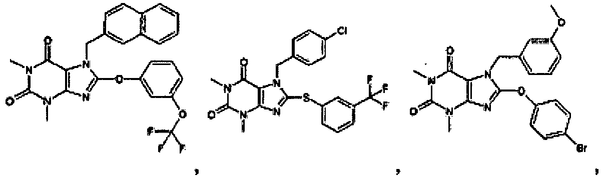

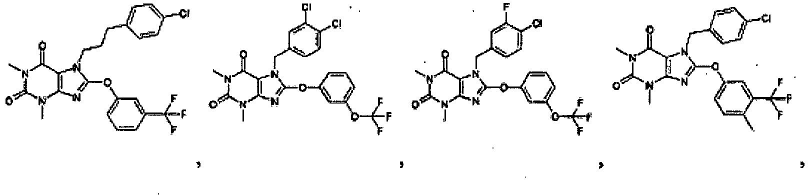

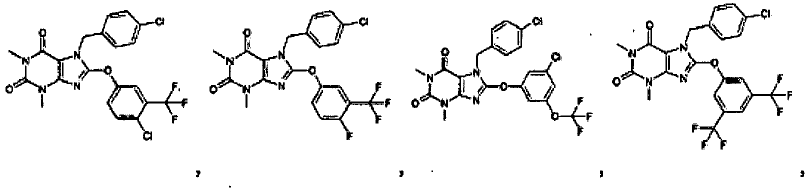

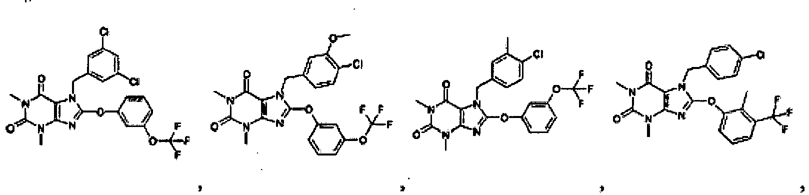

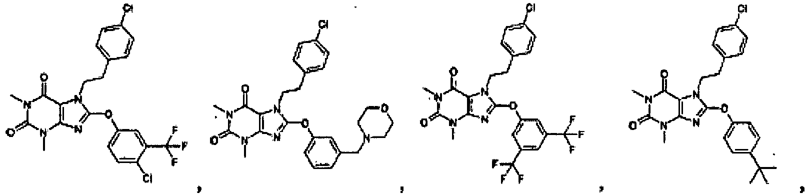

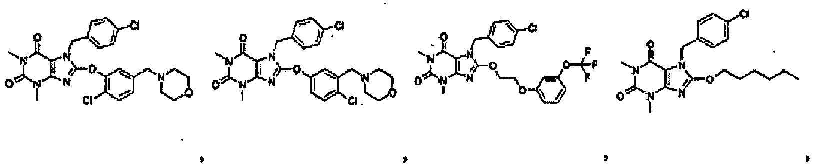

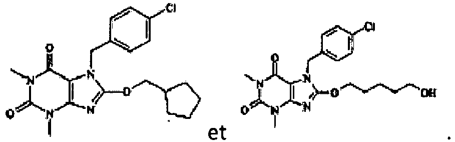

10. Composé de Formule II : 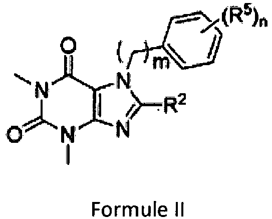 ou un sel pharmaceutiquement acceptable de celui-ci, dans lequel :
R² est un alcoxy en C₁ à C₆, un hétéroalkyle en C₁ à C₆, un aryloxy en C₆ à C₁₀, un arylalcoxy en C₇ à C₁₆ ou un -C(O)-alkyle en C₁ à C₆, chacun indépendamment substitué par 1 à 3 R³ ;
chaque R³ est indépendamment un halogéno, un alkyle en C₁ à C₆, un halogénoalkyle en C₁ à C₆, un halogénoalcoxy en C₁ à C₆, un hétéroalkyle en C₁ à C₆, un hydroxyle, un -C(O)O-alkyle en C₁ à C₆, un -C(O)-alkyle en C₁ à C₆, un phényle ou un hétéroaryle, dans lequel chacun parmi l'alkyle en C₁ à C₆, l'halogénoalkyle en C₁ à C₆, l'halogénoalcoxy en C₁ à C₆, l'hétéroalkyle en C₁ à C₆, le -C(O)O-alkyle en C₁ à C₆, le -C(O)-alkyle en C₁ à C₆, le phényle ou l'hétéroaryle est facultativement substitué par 1 à 3 R⁴ ;
R⁴ et R⁵ sont chacun indépendamment un hydroxyle, un alkyle en C₁ à C₆, un alcoxy en C₁ à C₆, un hétéroalkyle en C₁ à C₆, un halogénoalkyle en C₁ à C₆, un halogénoalcoxy en C₁ à C₆ ou un halogéno ;
n vaut 1 ou 2 ; et
m vaut 1, 2 ou 3.

11. Composition pharmaceutique comprenant un composé selon l'une quelconque des revendications précédentes, ou un sel pharmaceutiquement acceptable de celui-ci.

12. Composé ou composition pharmaceutique selon l'une quelconque des revendications précédentes pour utilisation dans un procédé de traitement d'un trouble induit par TRPC5, dans lequel le trouble induit par TRPC5 est choisi dans le groupe constitué de : un trouble neuropsychiatrique, un trouble neurodégénératif, une néphropathie et un trouble convulsif.
